(19) Europäisches Patentamt
European Patent Office
Office européen des brevets

(11) EP 4 055 641 B1

(12) **EUROPÄISCHE PATENTSCHRIFT**

(45) Veröffentlichungstag und Bekanntmachung des Hinweises auf die Patenterteilung:
**19.07.2023 Patentblatt 2023/29**

(21) Anmeldenummer: **20797135.9**

(22) Anmeldetag: **02.11.2020**

(51) Internationale Patentklassifikation (IPC):
***C07C 13/00*** $^{(2006.01)}$ ***C07D 209/82*** $^{(2006.01)}$
***H10K 85/60*** $^{(2023.01)}$ ***H10K 85/30*** $^{(2023.01)}$
***H10K 71/12*** $^{(2023.01)}$ *H10K 101/10* $^{(2023.01)}$

(52) Gemeinsame Patentklassifikation (CPC):
**C09K 11/06; C07D 487/06; H10K 85/342; H10K 85/615; H10K 85/654; H10K 85/6572; H10K 85/6574; H10K 85/6576;** H10K 50/11; H10K 2101/10; H10K 2101/90

(86) Internationale Anmeldenummer:
**PCT/EP2020/080617**

(87) Internationale Veröffentlichungsnummer:
**WO 2021/089447 (14.05.2021 Gazette 2021/19)**

# (54) ORGANISCHE ELEKTROLUMINESZIERENDE VORRICHTUNG

ORGANIC ELECTROLUMINESCENT APPARATUS

APPAREIL ÉLECTROLUMINESCENT ORGANIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.11.2019 EP 19206899**
**13.11.2019 EP 19208907**

(43) Veröffentlichungstag der Anmeldung:
**14.09.2022 Patentblatt 2022/37**

(73) Patentinhaber: **Merck Patent GmbH**
**64293 Darmstadt (DE)**

(72) Erfinder:
- **PARHAM, Amir Hossain**
  **64293 DARMSTADT (DE)**
- **KROEBER, Jonas Valentin**
  **64293 DARMSTADT (DE)**
- **ENGELHART, Jens**
  **64293 DARMSTADT (DE)**
- **EHRENREICH, Christian**
  **64293 DARMSTADT (DE)**
- **EICKHOFF, Christian**
  **64293 DARMSTADT (DE)**

(56) Entgegenhaltungen:
**KR-A- 20190 097 715** **US-A1- 2015 333 273**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Die vorliegende Erfindung betrifft eine organische elektrolumineszierende Vorrichtung enthaltend eine Mischung, die ein elektronentransportierendes Hostmaterial und ein lochtransportierendes Hostmaterial umfasst, sowie eine Formulierung enthaltend eine Mischung der Hostmaterialien und eine Mischung enthaltend die Hostmaterialien. Das elektronentransportierende Hostmaterial entspricht einer Verbindung der Formel (1) aus der Klasse von Verbindungen enthaltend eine Bispirofluorenyl-Einheit.

Der Aufbau organischer Elektrolumineszenzvorrichtungen (z.B. OLEDs - organic light emitting diodes oder OLECs - organic light emitting electrochemical cells), in denen organische Halbleiter als funktionelle Materialien eingesetzt werden, ist seit Langem bekannt. Als emittierende Materialien werden hierbei neben fluoreszierenden Emittern zunehmend metallorganische Komplexe eingesetzt, die Phosphoreszenz statt Fluoreszenz zeigen. Aus quantenmechanischen Gründen ist unter Verwendung metallorganischer Verbindungen als Phosphoreszenzemitter eine bis zu vierfach gesteigerte Energie- und Leistungseffizienz möglich. Generell gibt es bei OLEDs, insbesondere auch bei OLEDs die Triplettemission (Phosphoreszenz) zeigen, jedoch immer noch Verbesserungsbedarf, beispielsweise im Hinblick auf Effizienz, Betriebsspannung und Lebensdauer.

Die Eigenschaften organischer elektrolumineszierender Vorrichtungen werden nicht nur durch die eingesetzten Emitter bestimmt. Hier sind insbesondere auch die anderen verwendeten Materialien, wie Host- und Matrixmaterialien, Lochblockiermaterialien, Elektronentransportmaterialien, Lochtransportmaterialien und Elektronen- bzw. Exzitonenblockiermaterialien von besonderer Bedeutung, und davon insbesondere die Host bzw. Matrixmaterialien. Verbesserungen dieser Materialien können zu deutlichen Verbesserungen elektrolumineszierender Vorrichtungen führen.

Hostmaterialien zur Verwendung in organischen elektronischen Vorrichtungen sind dem Fachmann gut bekannt. Im Stand der Technik wird häufig auch der Begriff Matrixmaterial verwendet, wenn ein Hostmaterial für phosphoreszierende Emitter gemeint ist. Diese Verwendung des Begriffs gilt auch für die vorliegende Erfindung. Mittlerweile wurde eine Vielzahl von Hostmaterialien sowohl für fluoreszierende als auch für phosphoreszierende elektronische Vorrichtungen entwickelt.

Eine weitere Möglichkeit, die Leistungsdaten elektronischer Vorrichtungen, insbesondere von organischen Elektrolumineszenzvorrichtungen, zu verbessern, besteht darin, Kombinationen aus zwei oder mehr Materialien, insbesondere Hostmaterialien bzw. Matrixmaterialien, zu verwenden.

In US 6,392,250 B1 wird die Verwendung einer Mischung bestehend aus einem Elektronentransportmaterial, einem Lochtransportmaterial und einem fluoreszierenden Emitter in der Emissionsschicht einer OLED offenbart. Mit Hilfe dieser Mischung konnte die Lebensdauer der OLED gegenüber dem Stand der Technik verbessert werden.

In US 6,803,720 B1 wird die Verwendung einer Mischung enthaltend einen phosphoreszierenden Emitter sowie ein Loch- und ein Elektronentransportmaterial in der Emissionsschicht einer OLED offenbart. Dabei sind sowohl das Loch- und das Elektrontransportmaterial kleine organische Moleküle.

In WO2011088877 werden spezielle heterocyclische Verbindungen beschrieben, die in einer organischen lichtemittierenden Vorrichtung als lichtemittierende Verbindung, als Hostmaterial oder lochtransportierendem Material eingesetzt werden können.

Gemäß WO2015169412 können beispielsweise Triazin-Dibenzofuran-Arylderivate und Triazin-Dibenzothiophen-Arylderivate in einer lichtemittierenden Schicht als Hostmaterial verwendet werden.

In KR20170113318 werden spezielle heterocyclische Verbindungen beschrieben, die als Hostmaterial in einer lichtemittierenden Schicht einer organischen lichtemittierenden Vorrichtung eingesetzt werden können.

Gemäß US20180337348 können beispielsweise Triazin-Dibenzofuran-Arylderivate und Triazin-Dibenzothiophen-Arylderivate in einer Mischung mit einem speziellen Biscarbazol verwendet werden. Die Verbindung der folgenden Struktur

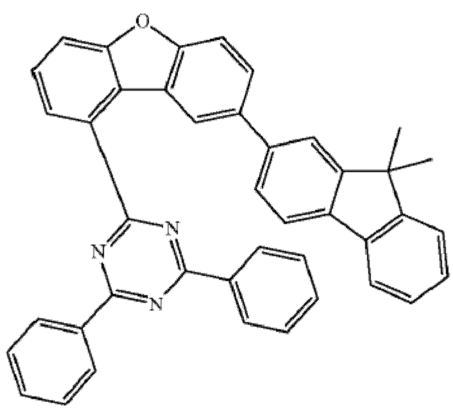

wird zu Vergleichszwecken in einem Einzelhostsystem herangezogen.

In US2019013490 werden spezielle Dibenzofuranverbindungen oder Dibenzothiophenverbindungen beschrieben und deren Verwendung als Hostmaterial in Kombination mit weiteren Hostmaterialien.

In US2019047991 werden doppelt substituierte Triazin-Dibenzofuranderivate beschrieben und deren Verwendung als organisches Material in einer organischen lichtemittierenden Vorrichtung.

In WO19031679 werden organische lichtemittierende Vorrichtungen beschrieben, enthaltend in der emittierenden Schicht ein doppelt substituiertes Triazin-Dibenzofuranderivat als Hostmaterial und ein zweites Hostmaterial.

US2015333273 (A) offenbart eine organische elektrolumineszierende Vorrichtung umfassend eine lichtemittierende Schicht, wobei die lichtemittierende Schicht zwei Hostmaterialen enthält.

Allerdings besteht bei Verwendung dieser Materialien oder bei der Verwendung von Mischungen der Materialien noch Verbesserungsbedarf, insbesondere in Bezug auf Effizienz, Betriebsspannung und/oder Lebensdauer der organischen elektrolumineszierenden Vorrichtung. Aufgabe der vorliegenden Erfindung ist daher die Bereitstellung einer Kombination von Hostmaterialien, welche sich für den Einsatz in einer organischen elektrolumineszierenden Vorrichtung, insbesondere in einer fluoreszierenden oder phosphoreszierenden OLED eignen und zu guten Device-Eigenschaften insbesondere im Hinblick auf eine verbesserte Lebensdauer, führen, sowie die Bereitstellung der entsprechenden elektrolumineszierenden Vorrichtung.

Es wurde nun gefunden, dass die Kombination mindestens einer Verbindung der Formel (1) als erstes Hostmaterial und mindestens einer lochtransportierenden Verbindung der Formel (2) als zweites Hostmaterial in einer Licht emittierenden Schicht einer organischen elektrolumineszierenden Vorrichtung, diese Aufgabe lösen und die Nachteile aus dem Stand der Technik beseitigen. Die Verwendung einer derartigen Materialkombination zur Herstellung der lichtemittierenden Schicht in einer organischen elektrolumineszierenden Vorrichtung führt zu sehr guten Eigenschaften dieser Vorrichtungen, insbesondere hinsichtlich der Lebensdauer, insbesondere bei gleicher oder verbesserter Effizienz und/oder Betriebsspannung. Die Vorteile zeigen sich insbesondere auch bei Anwesenheit einer lichtemittierenden Komponente in der Emissionsschicht, insbesondere bei Kombination mit Emittern der Formel (5), bei Konzentrationen zwischen 2 und 15 Gew.-%.

Ein erster Gegenstand der vorliegenden Erfindung ist daher eine organische elektrolumineszierende Vorrichtung umfassend eine Anode, eine Kathode und mindestens eine organische Schicht, enthaltend mindestens eine lichtemittierende Schicht, wobei die mindestens eine lichtemittierende Schicht mindestens eine Verbindung der Formel (1) als Hostmaterial 1 und mindestens eine Verbindung der Formel (2) als Hostmaterial 2 enthält,

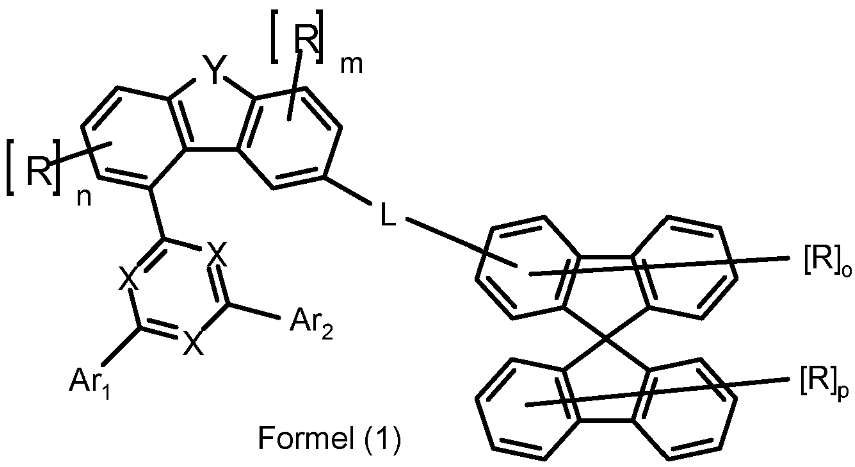

Formel (1)

,

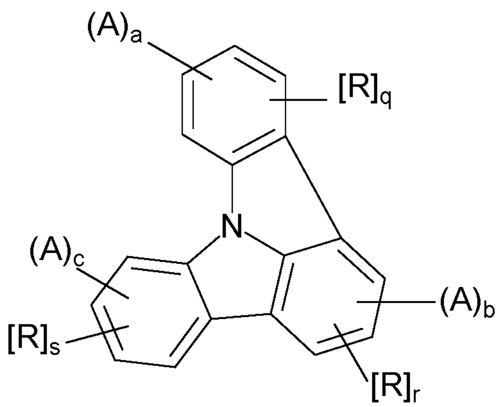

Formel (2) ,

wobei für die verwendeten Symbole und Indizes gilt:

X ist bei jedem Auftreten gleich oder verschieden $CR^0$ oder N, mit der Maßgabe, dass mindestens zwei Gruppen X für N stehen;

Y ist ausgewählt aus O oder S;

L ist bei jedem Auftreten gleich oder verschieden eine Einfachbindung oder ein Linker L-1 bis L-13,

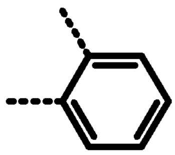

L-1

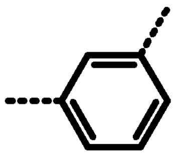

L-2

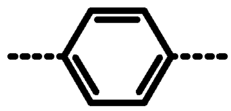

L-3

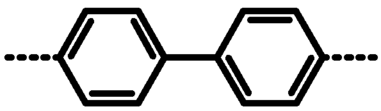

L-4

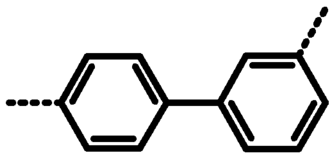

L-5

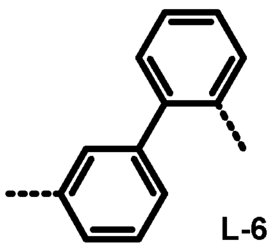

L-6

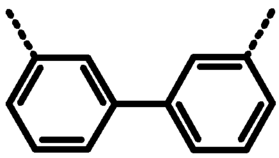

L-7

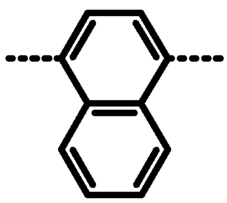

L-8

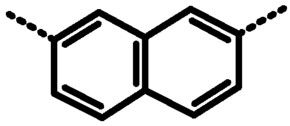

**L-9**

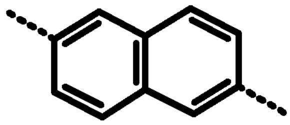

**L-10**

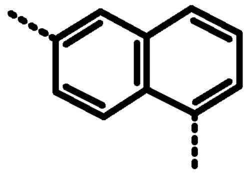

**L-11**

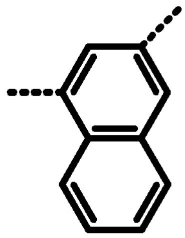

**L-12**

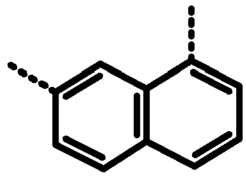

**L-13** ,

wobei die Linker L-1 bis L-13 noch mit einem oder mehreren Substituenten R substituiert sein können und die gestrichelte Linie die jeweilige Bindung an den Rest der Formel (1) kennzeichnet;

R ist bei jedem Auftreten gleich oder verschieden ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen;

$Ar_1$, $Ar_2$ sind jeweils unabhängig voneinander bei jedem Auftreten eine Aryl- oder Heteroarylgruppe mit 5 bis 40 aromatischen Ringatomen, die mit einem oder mehreren Resten R substituiert sein können;

A ist bei jedem Auftreten unabhängig voneinander eine Gruppe der Formel (3) oder (4),

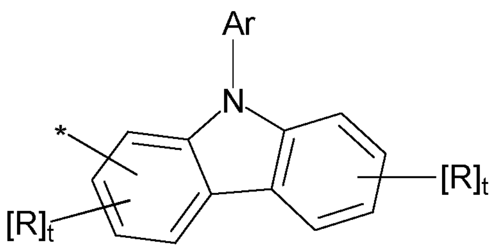

Formel (3) ,

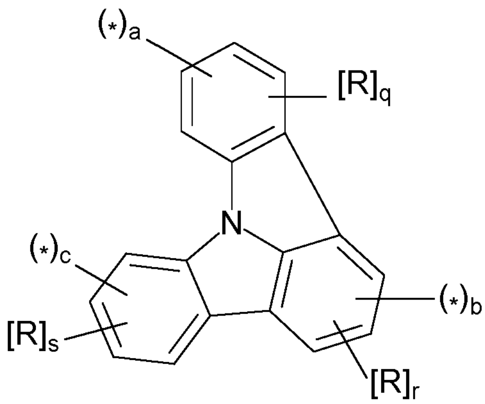

Formel (4) ;

Ar ist jeweils unabhängig voneinander bei jedem Auftreten eine Arylgruppe mit 6 bis 40 aromatischen Ringatomen, die mit einem oder mehreren Resten R substituiert sein kann, oder eine Heterarylgruppe mit 5 bis 40 aromatischen Ringatomen, enthaltend O als Heteroatom, die mit einem oder mehreren Resten R substituiert sein kann;

* kennzeichnet die Bindungsstelle an die Formel (2);

a, b, c bedeuten jeweils unabhängig voneinander bei jedem Auftreten 0 oder 1, wobei die Summe der Indizes bei jedem Auftreten a+b+c 1 bedeutet;

n und m bedeuten unabhängig voneinander bei jedem Auftreten 0, 1, 2 oder 3;

o bedeutet unabhängig bei jedem Auftreten 0, 1, 2, 3, 4, 5, 6 oder 7;

p bedeutet unabhängig bei jedem Auftreten 0, 1, 2, 3, 4, 5, 6, 7 oder 8;

q, r, s, t bedeuten jeweils unabhängig bei jedem Auftreten 0 oder 1;

$R^0$ ist bei jedem Auftreten unabhängig voneinander H oder ein unsubstituiertes oder teilweise oder vollständig deuteriertes aromatisches Ringsystem mit 6 bis 18 C-Atomen.

Weitere Gegenstände der Erfindung umfassen ein Verfahren zur Herstellung der organischen elektrolumineszierenden Vorrichtungen sowie Mischungen enthaltend mindestens eine Verbindung der Formel (1) und mindestens eine Verbindung der Formel (2), spezielle Materialkombinationen und Formulierungen, die derartige Mischungen bzw. Materialkombinationen enthalten. Die entsprechenden bevorzugten Ausführungsformen, wie nachfolgend beschrieben, sind ebenfalls Gegenstand der vorliegenden Erfindung. Die überraschenden und vorteilhaften Effekte werden durch spezifische Selektion der Verbindungen der Formel (1) und der Verbindungen der Formel (2) erreicht.

Die erfindungsgemäße organische elektrolumineszierende Vorrichtung ist beispielsweise ein organischer lichtemittierender Transistor (OLET), ein organisches Feld-Quench-Device (OFQD), eine organische lichtemittierende elektrochemische Zelle (OLEC, LEC, LEEC), eine organische Laserdiode (O-Laser) oder eine organische lichtemittierende Diode (OLED). Die erfindungsgemäße organische elektrolumineszierende Vorrichtung ist insbesondere eine organische lichtemittierende Diode oder eine organische lichtemittierende elektrochemische Zelle. Besonders bevorzugt ist die erfindungsgemäße Vorrichtung eine OLED.

Die organische Schicht der erfindungsgemäßen Vorrichtung, die die lichtemittierende Schicht enthaltend die Materialkombination aus mindestens einer Verbindung der Formel (1) und mindestens einer Verbindung der Formel (2) enthält, wie zuvor beschrieben oder nachfolgend beschrieben, enthält bevorzugt neben dieser lichtemittierenden Schicht (EML), eine Lochinjektionsschicht (HIL), eine Lochtransportschicht (HTL), eine Elektronentransportschicht (ETL), eine Elektroneninjektionsschicht (EIL) und/oder eine Lochblockierschicht (HBL). Es können in der erfindungsgemäßen Vorrichtung auch mehrere Schichten dieser Gruppe ausgewählt aus EML, HIL, HTL, ETL, EIL und HBL enthalten sein.

Die Vorrichtung kann aber auch anorganische Materialien enthalten oder auch Schichten, welche vollständig aus anorganischen Materialien aufgebaut sind.

Es ist bevorzugt, dass die lichtemittierende Schicht enthaltend mindestens eine Verbindung der Formel (1) und mindestens eine Verbindung der Formel (2) eine phosphoreszierende Schicht ist, die dadurch gekennzeichnet ist, dass sie zusätzlich zu der Hostmaterialienkombination der Verbindungen der Formel (1) und Formel (2), wie zuvor beschrieben, mindestens einen phosphoreszierenden Emitter enthält. Eine geeignete Auswahl an Emittern und bevorzugte Emitter werden nachfolgend beschrieben.

Eine Arylgruppe im Sinne dieser Erfindung enthält 6 bis 40 aromatische Ringatome, bevorzugt C-Atome. Eine Heteroarylgruppe im Sinne dieser Erfindung enthält 5 bis 40 aromatische Ringatome, wobei die Ringatome C-Atome und mindestens ein Heteroatom umfassen, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Phenyl, abgeleitet von Benzol, bzw. ein einfacher heteroaromatischer Cyclus, beispielsweise abgeleitet von Pyridin, Pyrimidin oder Thiophen, oder eine kondensierte Aryl- oder Heteroarylgruppe, beispielsweise abgeleitet von Naphthalin, Anthracen, Phenanthren, Chinolin oder Isochinolin, verstanden. Eine Arylgruppe mit 6 bis 18 C-Atomen ist daher vorzugsweise Phenyl, Naphthyl, Phenanthryl oder Triphenylenyl, wobei die Anbindung der Arylgruppe als Substituent dabei nicht eingeschränkt ist. Die Aryl- oder Heteroarylgruppe im Sinne dieser Erfindung kann einen oder mehrere Reste R tragen, wobei der Substituent R nachfolgend beschrieben wird.

Ein aromatisches Ringsystem im Sinne dieser Erfindung enthält 6 bis 40 C-Atome im Ringsystem. Das aromatisches Ringsystem umfasst auch Arylgruppen, wie zuvor beschrieben.

Ein aromatisches Ringsystem mit 6 bis 18 C-Atomen wird vorzugsweise aus Phenyl, Biphenyl, Naphthyl, Phenanthryl und Triphenylenyl ausgewählt.

Ein heteroaromatisches Ringsystem im Sinne dieser Erfindung enthält 5 bis 40 Ringatome und mindestens ein Heteroatom. Ein bevorzugtes heteroaromatisches Ringsystem hat 10 bis 40 Ringatome und mindestens ein Heteroatom. Das heteroaromatische Ringsystem umfasst auch Heteroarylgruppen, wie zuvor beschrieben. Die Heteroatome im heteroaromatischen Ringsystem sind bevorzugt ausgewählt aus N, O und/oder S.

Unter einem aromatischen oder heteroaromatischen Ringsystem im Sinne dieser Erfindung wird ein System verstanden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können.

So sollen beispielsweise auch Systeme wie 9,9'-Spirobifluoren, 9,9-Diarylfluoren, Triarylamin, Diarylether, Stilben, etc. als aromatische bzw. heteroaromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sind Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls

von der Definition des aromatischen bzw. heteroaromatischen Ringsystems umfasst.

Unter einem aromatischen oder heteroaromatischen Ringsystem mit 5 - 40 aromatischen Ringatomen, welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Die Abkürzungen $Ar_1$ und $Ar_2$ sind bei jedem Auftreten jeweils unabhängig voneinander eine Aryl- oder Heteroarylgruppe mit 5 bis 40 aromatischen Ringatomen, die mit einem oder mehreren Resten R substituiert sein können, wobei der Rest R eine Bedeutung hat, wie zuvor oder nachfolgend beschrieben. Die genannten Ausführungen für die Aryl- und Heteroarylgruppen mit 5 bis 40 aromatischen Ringatomen gelten hier entsprechend.

Die Abkürzung Ar ist jeweils unabhängig voneinander bei jedem Auftreten eine Arylgruppe mit 6 bis 40 aromatischen Ringatomen, die mit einem oder mehreren Resten R substituiert sein kann, oder eine Heterarylgruppe mit 5 bis 40 aromatischen Ringatomen, enthaltend O als Heteroatom, die mit einem oder mehreren Resten R substituiert sein kann, wobei die Ausführungen für die Arylgruppe bzw. Heteroarylgruppe entsprechend gelten, wie zuvor beschrieben. Der Rest R oder die Reste R haben eine Bedeutung, wie zuvor beschrieben oder nachfolgend beschrieben.

Unter einer cyclischen Alkyl-, Alkoxy- oder Thioalkylgruppe im Sinne dieser Erfindung wird eine monocyclische, eine bicyclische oder eine polycyclische Gruppe verstanden.

Im Rahmen der vorliegenden Erfindung werden unter einer geradkettigen, verzweigten oder cyclischen $C_1$- bis $C_{20}$-Alkylgruppe beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden.

Unter einer geradkettigen oder verzweigten $C_1$- bis $C_{20}$-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einer geradkettigen $C_1$- bis $C_{20}$-Thioalkylgruppe werden beispielsweise S-Alkylgruppen verstanden, beispielsweise Thiomethyl, 1-Thioethyl, 1-Thio-i-propyl, 1-Thio-n-propoyl, 1-Thio-i-butyl, 1-Thio-n-butyl oder 1-Thio-t-butyl.

Eine Aryloxy- oder Heteroaryloxygruppe mit 5 bis 40 aromatischen Ringatomen bedeutet O-Aryl oder O-Heteroaryl und bedeutet, dass die Aryl- bzw. Heteroarylgruppe über ein Sauerstoffatom gebunden wird, wobei die Aryl- bzw. Heteroarylgruppe eine Bedeutung hat, wie zuvor beschrieben.

Eine Aralkyl- oder Heteroaralkylgruppe mit 5 bis 40 aromatischen Ringatomen bedeutet, dass eine Alkylgruppe, wie zuvor beschrieben, mit einer Arylgruppe bzw. Heteroarylgruppe substituiert ist, wobei die Aryl- bzw. Heteroarylgruppe eine Bedeutung hat, wie zuvor beschrieben.

Ein phosphoreszierender Emitter im Sinne der vorliegenden Erfindung ist eine Verbindung, die Lumineszenz aus einem angeregten Zustand mit höherer Spinmultiplizität zeigt, also einem Spinzustand > 1, insbesondere aus einem angeregten Triplettzustand. Im Sinne dieser Anmeldung sollen alle lumineszierenden Komplexe mit Übergangsmetallen oder Lanthaniden als phosphoreszierende Emitter angesehen werden. Eine genauere Definition erfolgt nachfolgend.

Wenn die Hostmaterialien der lichtemittierenden Schicht umfassend mindestens eine Verbindung der Formel (1), wie zuvor beschrieben oder nachfolgend bevorzugt beschrieben, und mindestens eine Verbindung der Formel (2), wie zuvor beschrieben oder nachfolgend beschrieben, für einen phosphoreszierenden Emitter eingesetzt wird, ist es bevorzugt, wenn deren Triplettenergie nicht wesentlich kleiner als die Triplettenergie des phosphoreszierenden Emitters

ist. Dabei gilt bevorzugt für das Triplettniveau $T_1$(Emitter) - $T_1$(Matrix) $\leq$ 0.2 eV, besonders bevorzugt $\leq$ 0.15 eV, ganz besonders bevorzugt $\leq$ 0.1 eV. Dabei ist $T_1$(Matrix) das Triplettniveau des Matrixmaterials in der Emissionsschicht, wobei diese Bedingung für jedes der beiden Matrixmaterialien gilt, und $T_1$(Emitter) ist das Triplettniveau des phosphoreszierenden Emitters. Enthält die Emissionsschicht mehr als zwei Matrixmaterialien, so gilt die oben genannte Beziehung bevorzugt auch für jedes weitere Matrixmaterial.

Im Folgenden wird das Hostmaterial 1 und dessen bevorzugte Ausführungsformen beschrieben, welches/welche in der erfindungsgemäßen Vorrichtung enthalten ist/sind. Die bevorzugten Ausführungsformen des Hostmaterials 1 der Formel (1) gelten auch für die erfindungsgemäße Mischung und/oder Formulierung.

In Verbindungen der Formel (1) wird Y aus O oder S ausgewählt.

In einer bevorzugten Ausführungsform des Hostmaterials der Formel (1) steht Y für O.

Demzufolge ist ein weiterer Gegenstand der Erfindung die organische elektrolumineszierende Vorrichtung, wie zuvor beschrieben, wobei im Hostmaterial 1 Y für O steht.

In Verbindungen der Formel (1) oder der bevorzugten Ausführungsform des Hostmaterials der Formel (1) steht das Symbol X zwei Mal für N und ein Mal für CR° oder drei Mal für N.

Der Substituent

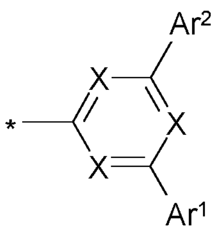

hat daher die folgenden Bedeutungen, wobei * die Verknüpfungsstelle mit dem Dibenzofuran bzw. Dibenzothiophen kennzeichnet und $R^0$, $Ar^1$ und $Ar^2$ eine zuvor angegebene Bedeutung oder eine bevorzugt angegebene Bedeutung haben:

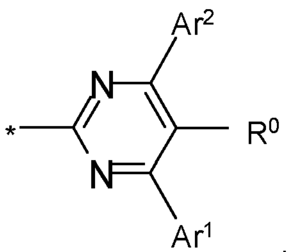 , 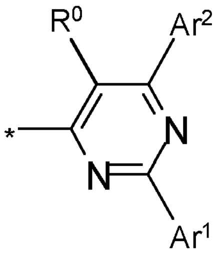 , 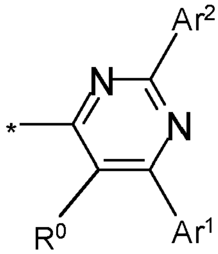 , 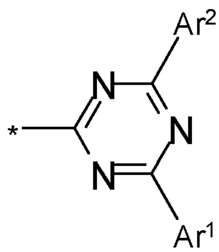 .

$R^0$ ist bei jedem Auftreten gleich oder verschieden bevorzugt ausgewählt aus der Gruppe H oder ein unsubstituiertes oder teilweise oder vollständig deuteriertes aromatisches Ringsystem mit 6 bis 18 C-Atomen. $R^0$ ist bei jedem Auftreten bevorzugt H oder ein unsubstituiertes aromatisches Ringsystem mit 6 bis 18 C-Atomen. $R^0$ ist bei jedem Auftreten besonders bevorzugt H.

Verbindungen der Formel (1), in denen X bei jedem Auftreten gleich N bedeutet werden durch die Formel (1a) dargestellt,

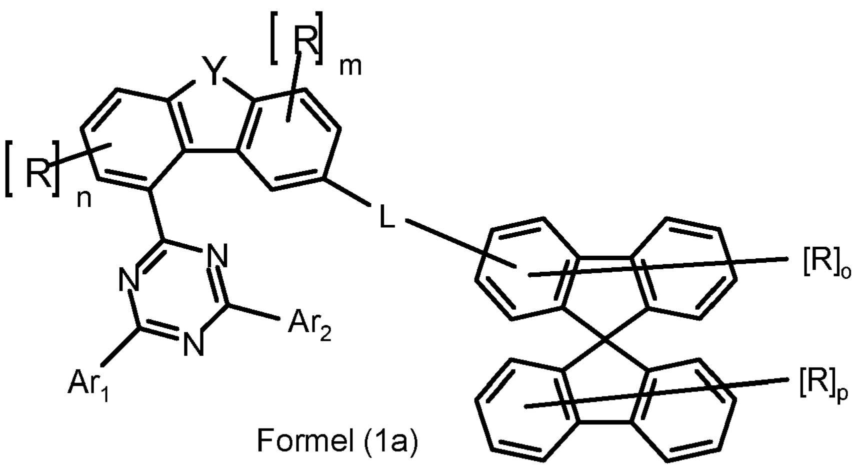

Formel (1a)

,

wobei Y, L, $Ar_1$, $Ar_2$, R, n, m, o und p eine zuvor angegebene Bedeutung haben oder eine nachfolgend angegebene Bedeutung haben.

Verbindungen der Formel (1a) sind bevorzugte Ausführungsformen der Verbindungen der Formel (1). In Verbindungen der Formel (1a) steht Y bevorzugt für O.

In Verbindungen der Formeln (1) oder (1a) oder bevorzugt beschriebenen Verbindungen der Formeln (1) oder (1a), stehen $Ar_1$ und $Ar_2$ jeweils unabhängig voneinander bevorzugt für eine Arylgruppe mit 6 bis 40 C-Atomen, wie zuvor beschrieben oder bevorzugt beschrieben, die mit einem oder mehreren Resten R substituiert sein kann oder für eine Dibenzofuranyl- oder Dibenzothiophenylgruppe, die mit einem oder mehreren Resten R substituiert sein können.

Die Verknüpfung der Arylgruppe oder der Dibenzofuranyl- oder Dibenzothiophenylgruppe ist dabei nicht eingeschränkt.

$Ar_1$ und $Ar_2$ können daher bevorzugt aus folgenden Gruppen Ar-1 bis Ar-12 ausgewählt werden, wobei R eine zuvor angegebene oder bevorzugt angegebene Bedeutung hat:

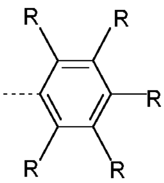

Ar-1

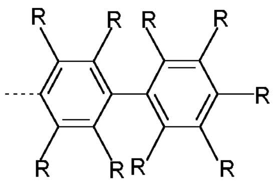

Ar-2

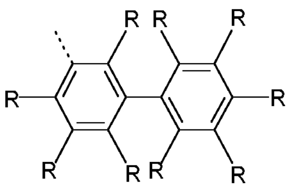

Ar-3

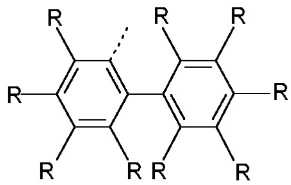

Ar-4

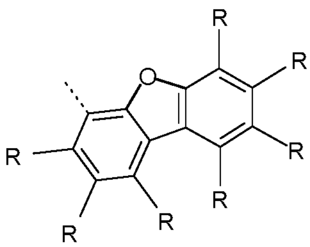

Ar-5

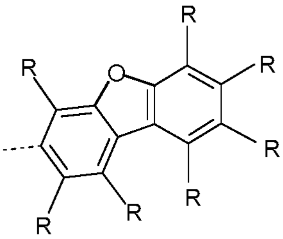

Ar-6

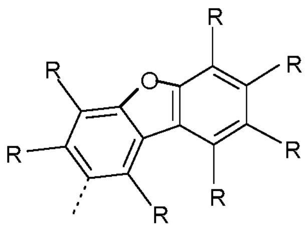

Ar-7

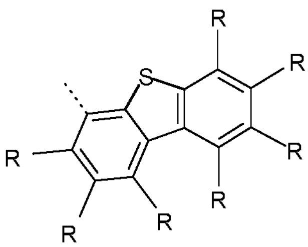

Ar-8

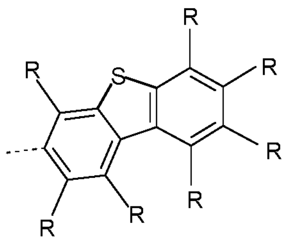

Ar-9

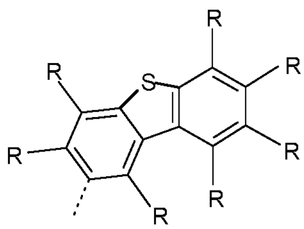

Ar-10

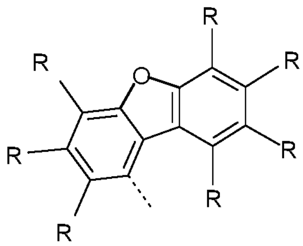

Ar-11

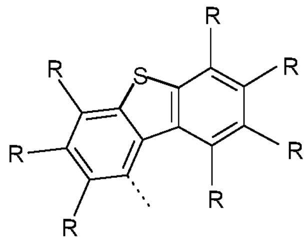

Ar-12 .

Besonders bevorzugt steht mindestens ein $Ar_1$ oder $Ar_2$ für Phenyl und der andere aromatische Substituent steht für eine Arylgruppe mit 6 bis 40 C-Atomen, die mit einem oder mehreren Resten R substituiert sein kann oder für eine Dibenzofuranyl oder eine Dibenzothiophenylgruppe; vorzugsweise steht der andere aromatische Substituent für eine Gruppe ausgewählt aus Ar-1 bis Ar-12. Besonders bevorzugt steht mindestens ein Substituent $Ar_1$ oder $Ar_2$ für Phenyl und der andere aromatische Substituent steht für eine Phenylgruppe, die mit einem oder mehreren Resten R substituiert sein kann oder für Dibenzofuranyl. Ganz besonders bevorzugt sind beide Gruppen $Ar_1$ und $Ar_2$ gleich. Ganz besonders bevorzugt stehen beide Gruppen $Ar_1$ und $Ar_2$ für Phenyl oder beide Gruppen $Ar_1$ und $Ar_2$ stehen für Dibenzofuranyl, bevorzugt ausgewählt aus Ar-5, Ar-6, Ar-7 oder Ar-11. Ganz besonders bevorzugt ist die Arylgruppe oder Heteroarylgruppe in $Ar_1$ und $Ar_2$ unsubstituiert.

R in Verbindungen der Formel (1) und der Formel (1a) oder bevorzugten Verbindungen der Formeln (1) und (1a) , wie zuvor beschrieben, ist bei jedem Auftreten gleich oder verschieden ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen. Der Substituent R steht bei jedem Auftreten unabhängig voneinander bevorzugt für CN oder eine Arylgruppe mit 6 bis 40 C-Atomen, wie zuvor beschrieben. R ist bei jedem Auftreten unabhängig voneinander besonders bevorzugt Phenyl.

In Verbindungen der Formeln (1) oder (1a) oder in bevorzugt beschriebenen Verbindungen der Formeln (1) oder (1a) ist n bevorzugt 0 oder 1, wobei R eine zuvor angegebene Bedeutung hat. Besonders bevorzugt ist n 0.

In Verbindungen der Formeln (1) oder (1a) oder in bevorzugt beschriebenen Verbindungen der Formeln (1) oder (1a) ist m bevorzugt 0 oder 1, wobei R eine zuvor angegebene Bedeutung hat. Besonders bevorzugt ist m 0.

In Verbindungen der Formeln (1) oder (1a) oder in bevorzugt beschriebenen Verbindungen der Formeln (1) oder (1a) ist o bevorzugt 0, 1 oder 2, wobei R eine zuvor angegebene Bedeutung hat. Besonders bevorzugt ist o 0.

In Verbindungen der Formeln (1) oder (1a) oder in bevorzugt beschriebenen Verbindungen der Formeln (1) oder (1a) ist p bevorzugt 0, 1 oder 2, wobei R eine zuvor angegebene Bedeutung hat. Besonders bevorzugt ist p 0.

In Verbindungen der Formeln (1) oder (1a) oder in bevorzugt beschriebenen Verbindungen der Formeln (1) oder (1a) steht L für eine Einfachbindung oder L wird aus der Gruppe der Linker L-1 bis L-13 ausgewählt, wobei die Linker L-1 bis L-13 noch mit einem oder mehreren Substituenten R substituiert sein können. Bevorzugt sind die Linker L-1 bis L-13 unsubstituiert oder tragen einen Substituenten R, wie zuvor beschrieben oder bevorzugt beschrieben. Besonders bevorzugt sind die Linker L-1 bis L-13 unsubstituiert.

In Verbindungen der Formeln (1) oder (1a), wie zuvor beschrieben oder bevorzugt beschrieben, wird L bevorzugt aus einer Einfachbindung oder den Linkern L-1, L-2 und L-3,

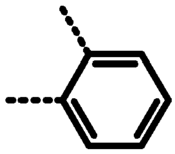 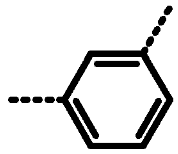 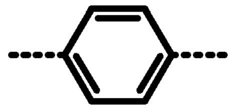

L-1 L-2 L-3 ,

ausgewählt.

Ein weiterer Gegenstand der Erfindung ist demzufolge eine organische elektrolumineszierende Vorrichtung, wie zuvor beschrieben oder bevorzugt beschrieben, wobei der Linker L im Hostmaterial 1 eine Einfachbindung bedeutet oder aus den Linkern L-1, L-2 oder L-3 ausgewählt wird.

Bevorzugte Ausführungsformen der Verbindungen der Formeln (1) oder (1a) sind Verbindungen der Formel (1b), in denen L eine Einfachbindung bedeutet, n und m 0 bedeuten und Y, $Ar_1$, $Ar_2$, R, o und p eine zuvor genannte oder eine bevorzugt genannte Bedeutung haben,

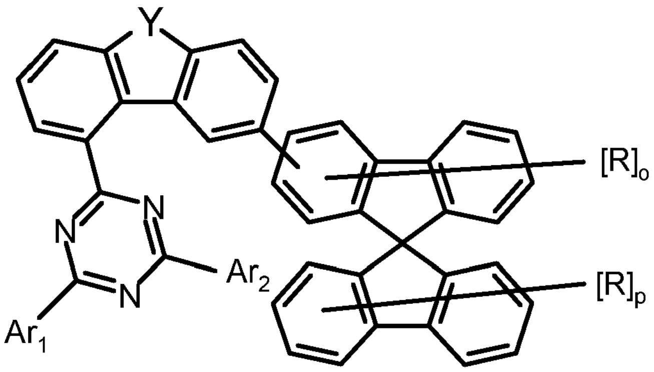

Formel (1b) .

Bevorzugte Ausführungsformen der Verbindungen der Formeln (1) oder (1a) sind Verbindungen der Formel (1c), in denen n und m 0 bedeuten und Y, L, $Ar_1$, $Ar_2$, R, o und p eine zuvor genannte oder eine bevorzugt genannte Bedeutung haben,

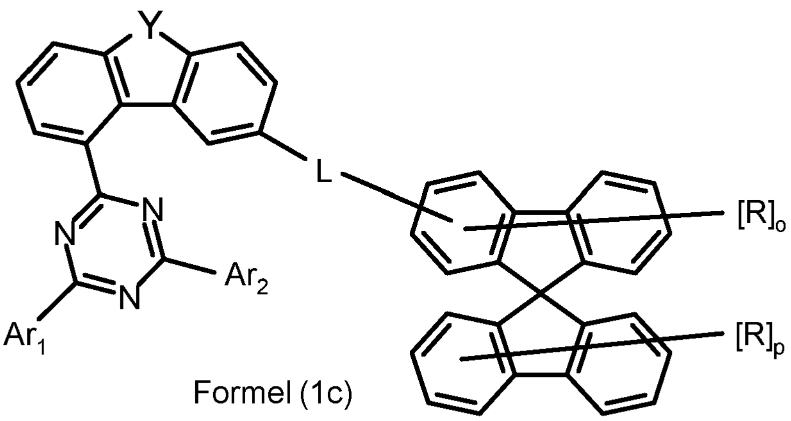

Formel (1c) .

In Verbindungen der Formeln (1), (1a), (1b) und (1c) oder in bevorzugt beschriebenen Verbindungen der Formeln (1), (1a), (1b) und (1c) kann L in beliebiger Position an das Bispirofluorenyl gebunden sein.

L, wie zuvor beschrieben oder als bevorzugt beschrieben, ist bevorzugt in Position 2, 3 oder 4 des Bispirofluorenylrestes verknüpft, oder ganz besonders bevorzugt in Position 2 des Bispirofluorenylrestes verknüpft.

Beispiele für geeignete Hostmaterialien der Formel (1), die erfindungsgemäß ausgewählt werden, und bevorzugt in Kombination mit mindestens einer Verbindung der Formel (2) in der erfindungsgemäßen elektrolumineszierenden Vorrichtung verwendet werden, sind die nachstehend genannten Strukturen der Tabelle 1.

Tabelle 1:

| | |
|---|---|
| 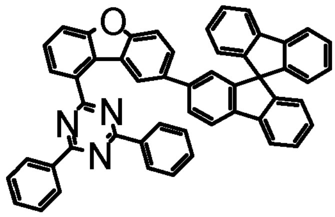 **1** | 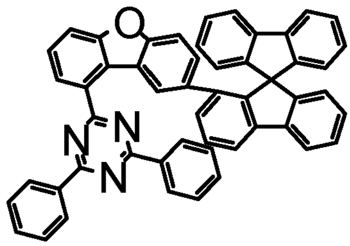 |
| 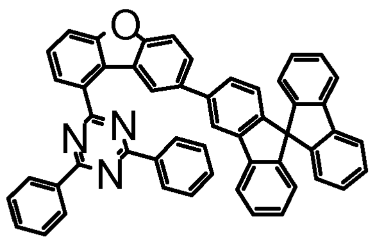 | 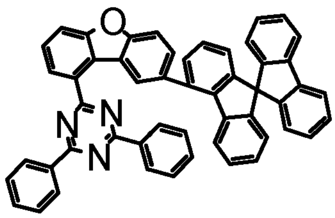 |
| 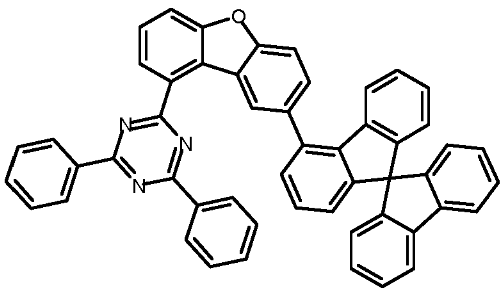 **2** | 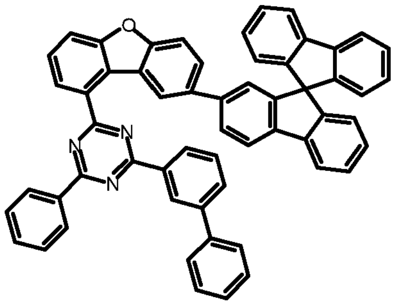 **3** |

| | |
|---|---|
| 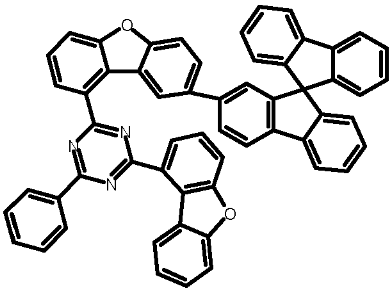 **4** | 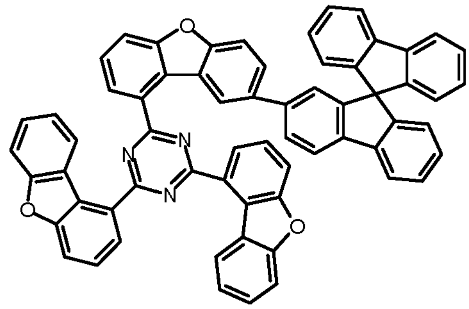 **5** |
| 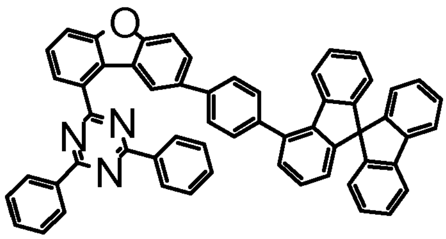 | 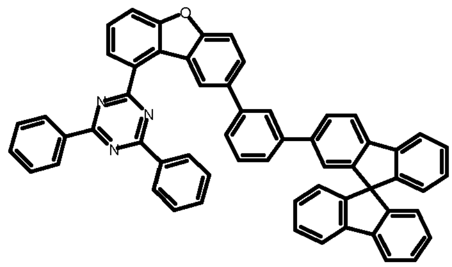 **6** |
| 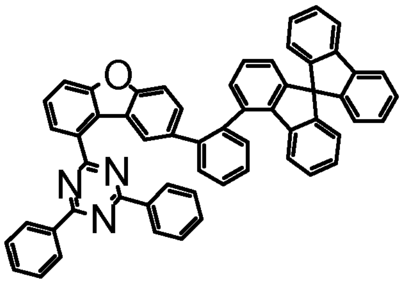 | 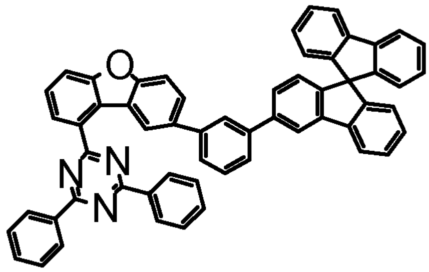 |
| 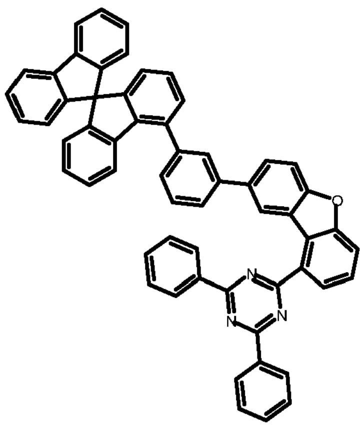 **7** | 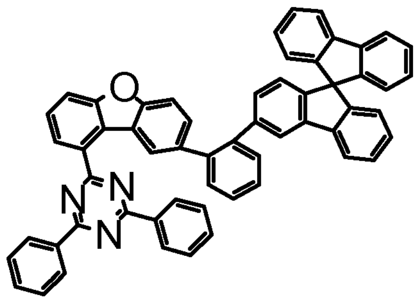 |

| | |
|---|---|
| 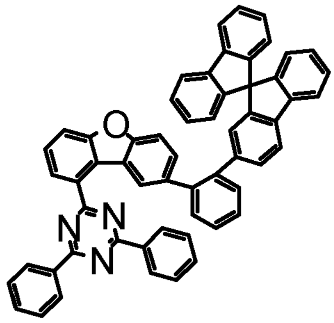 | 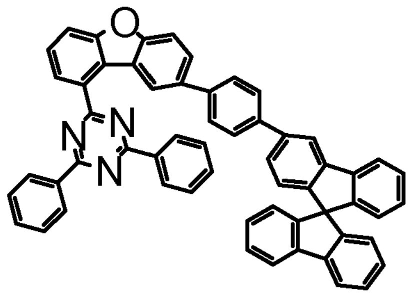 |
| 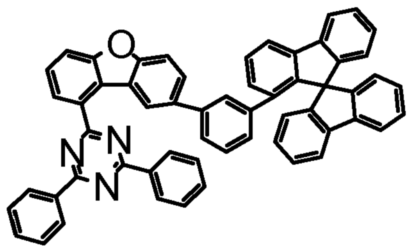 | 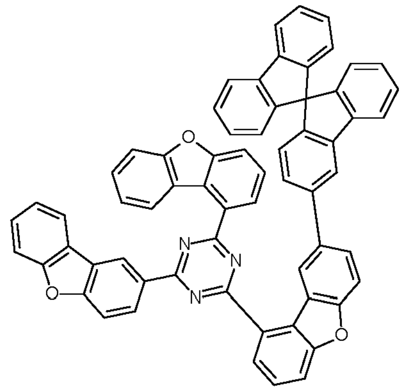 8 |
| 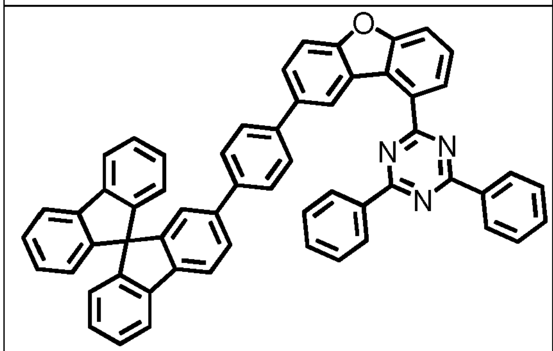 9 | 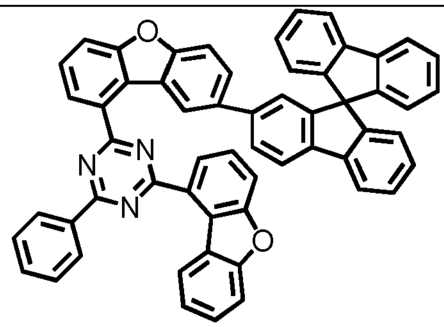 10 |
| 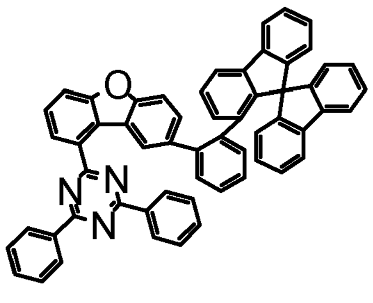 | 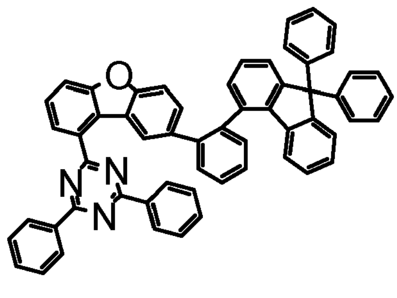 |

| | |
|---|---|
| 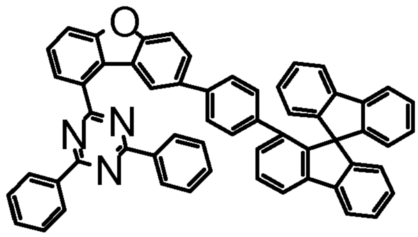 | 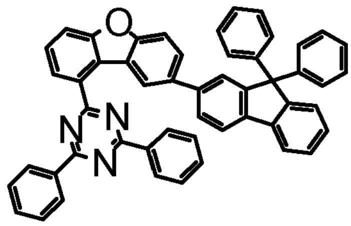 |
| 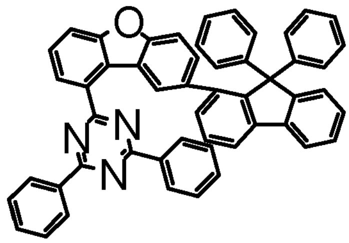 | 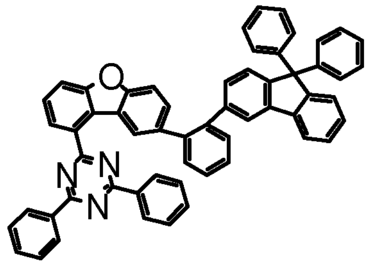 |
| 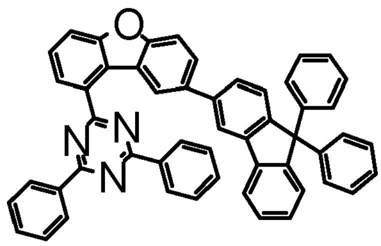 | 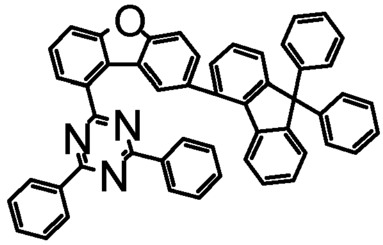 |
| 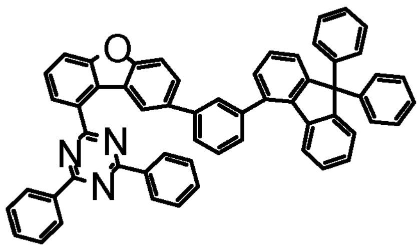 | 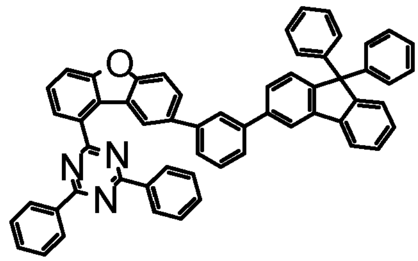 |
| 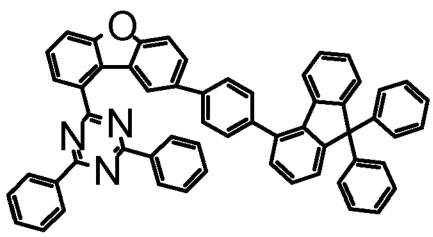 | 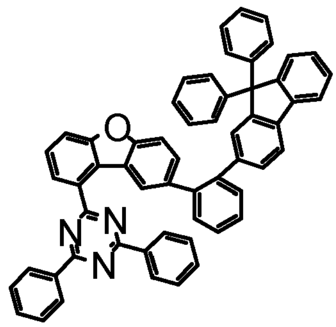 |

| | |
|---|---|
| 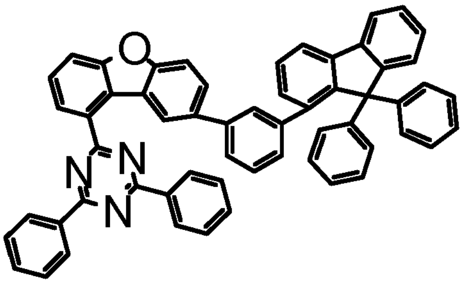 | 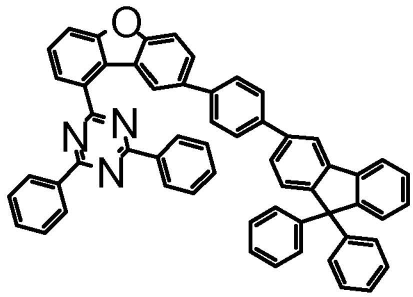 |
| 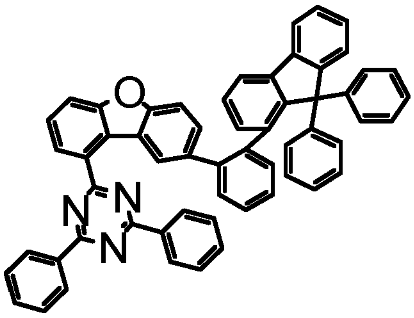 | 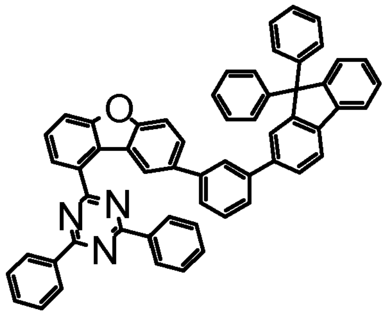 |
| 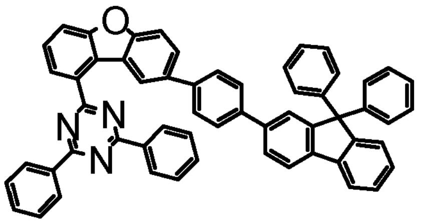 | 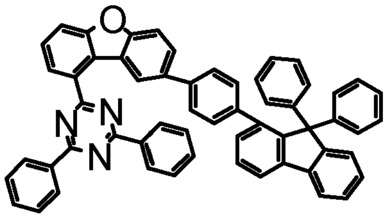 |
| 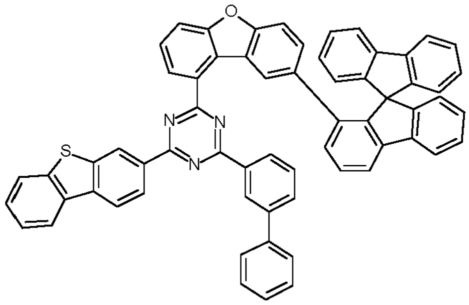 | 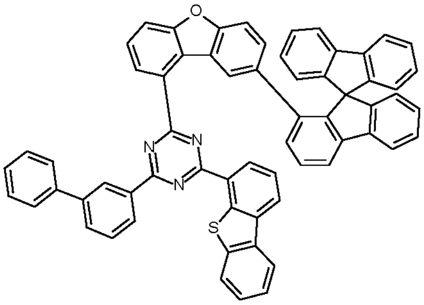 |
| 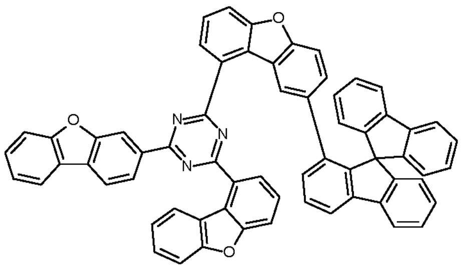 | 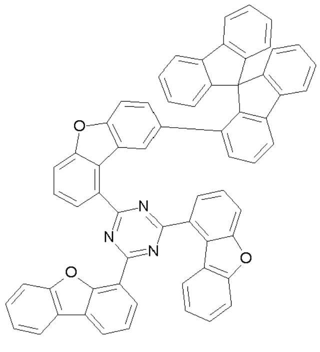 |

| | |
|---|---|
| 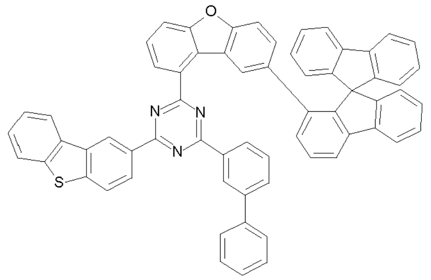 | 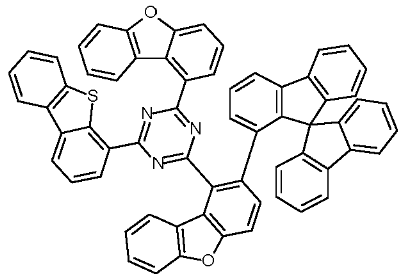 |
| 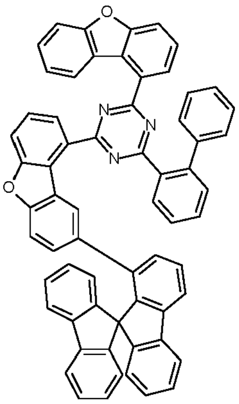 | 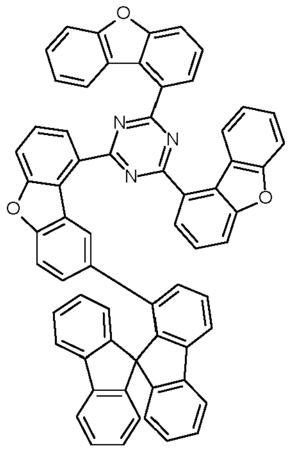 |
| 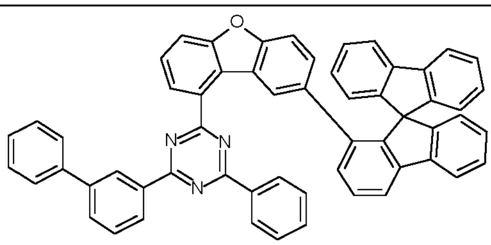 | 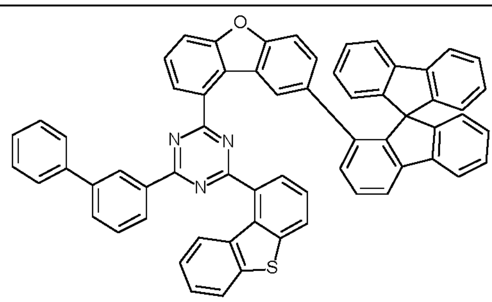 |
| 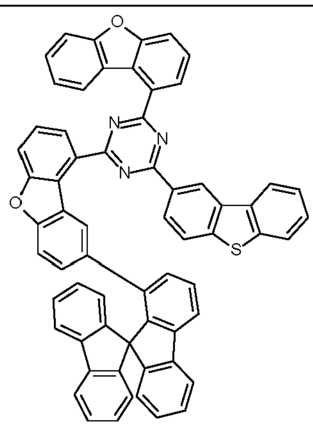 | 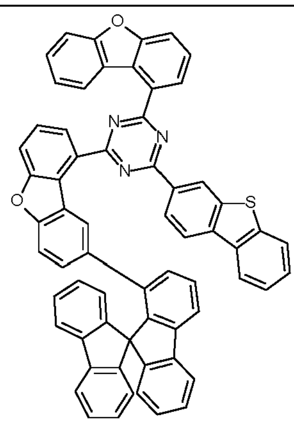 |

| | |
|---|---|
| 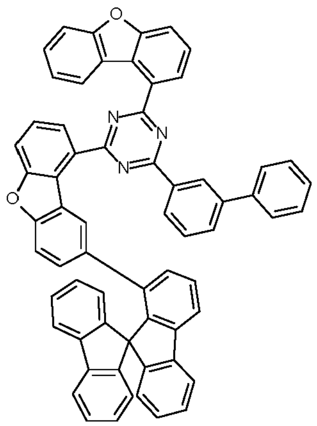 | 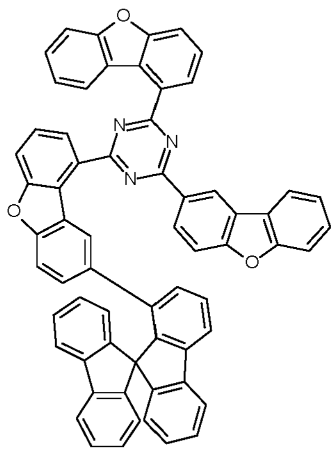 |
| 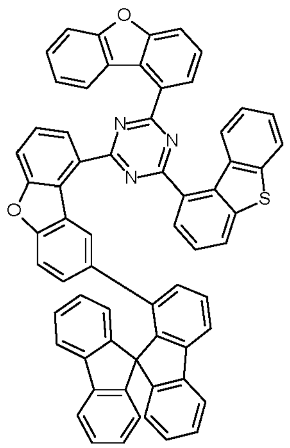 | 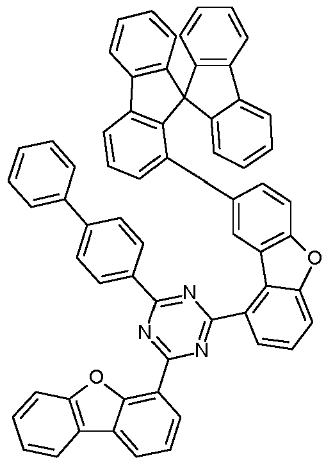 |
| 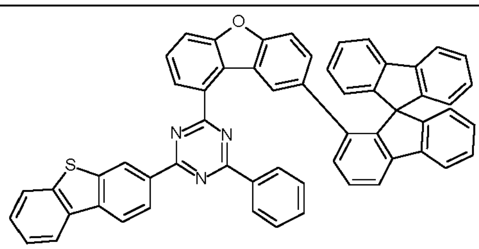 | 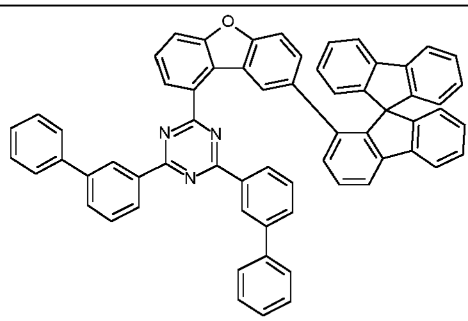 |
| 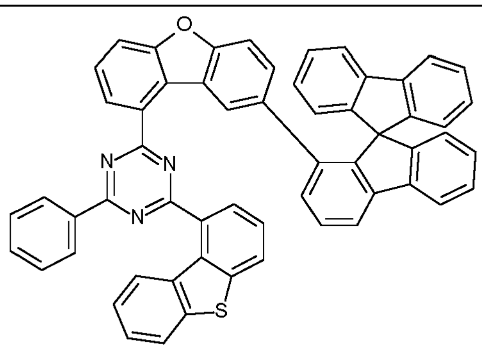 | 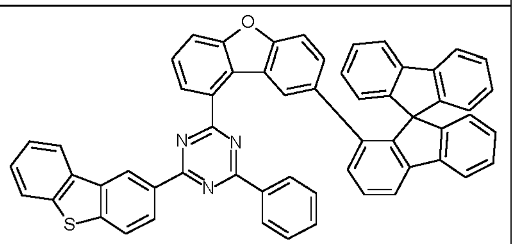 |

| | |
|---|---|
| 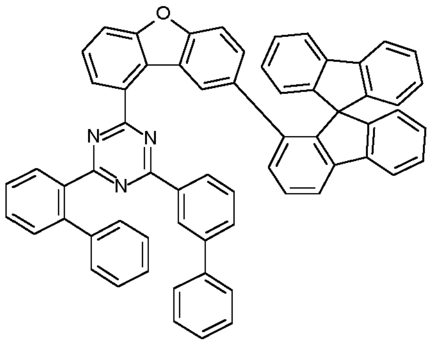 | 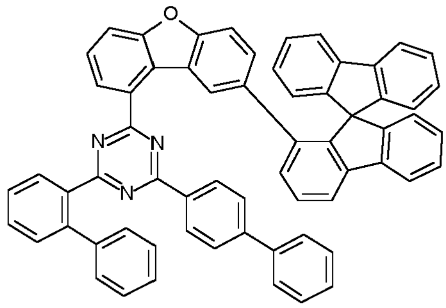 |
| 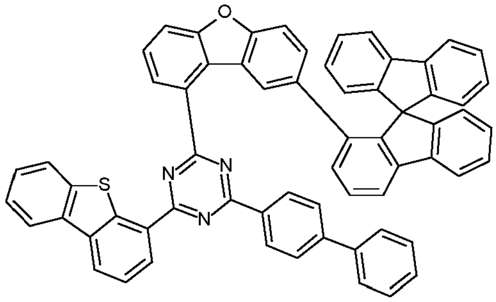 | 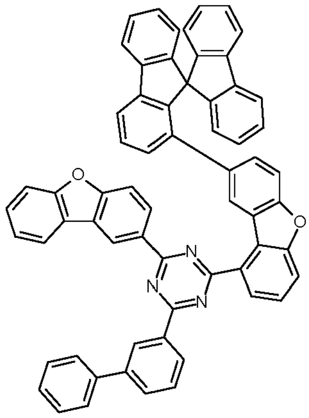 |
| 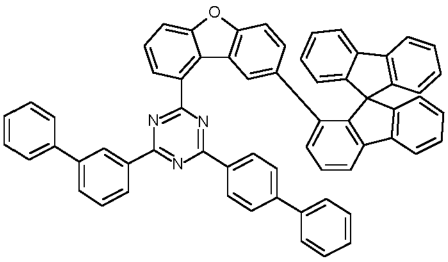 | 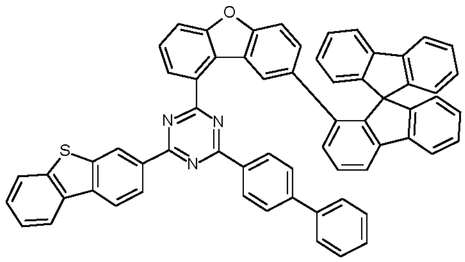 |
| 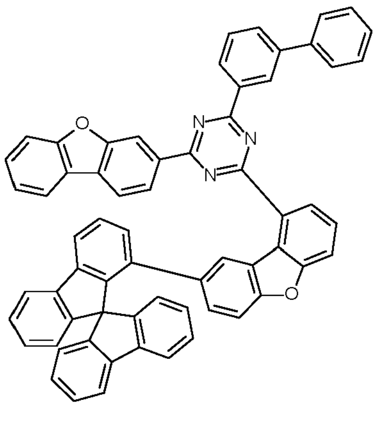 | 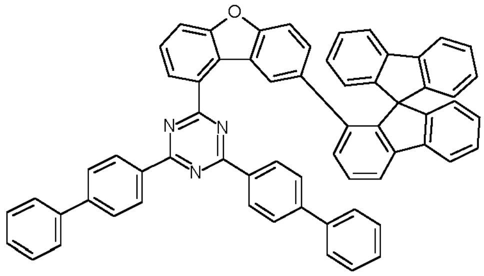 |

| | |
|---|---|
| 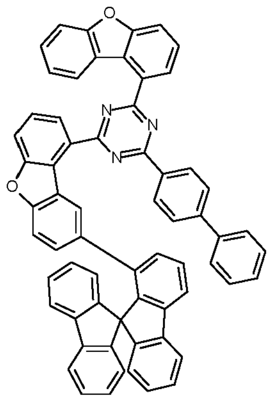 | 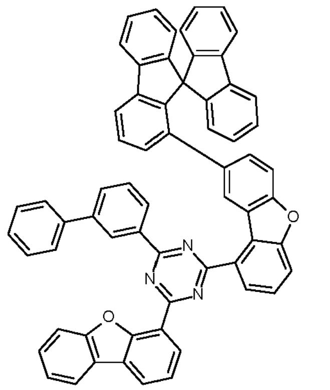 |
| 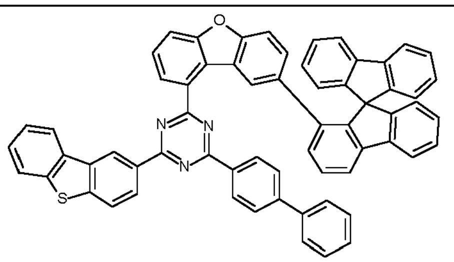 | 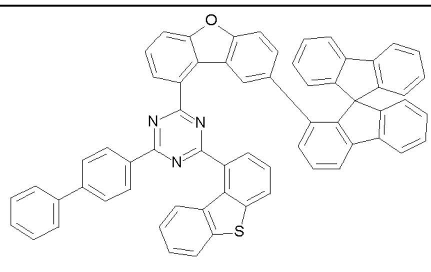 |
| 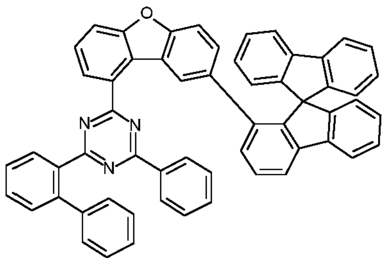 | 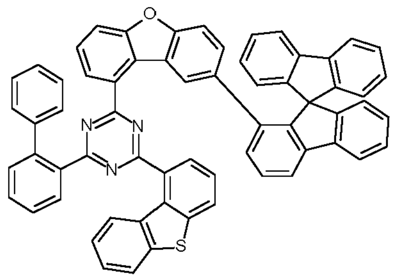 |
| 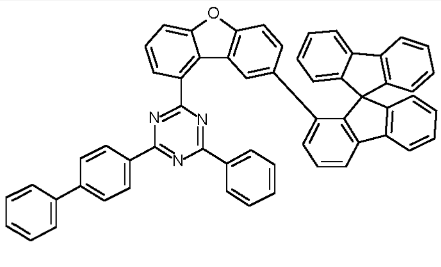 | 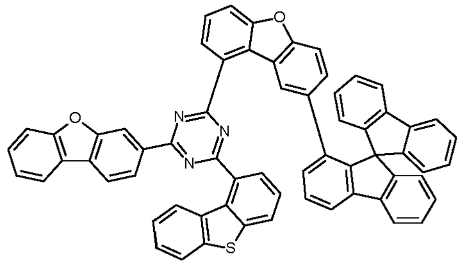 |
| 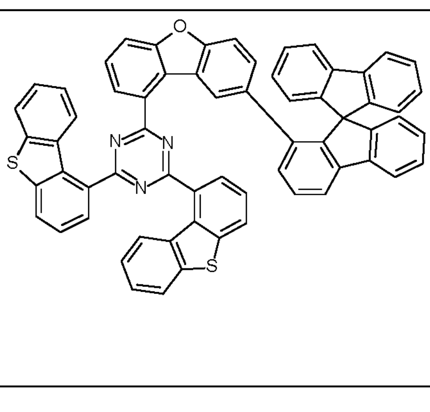 | 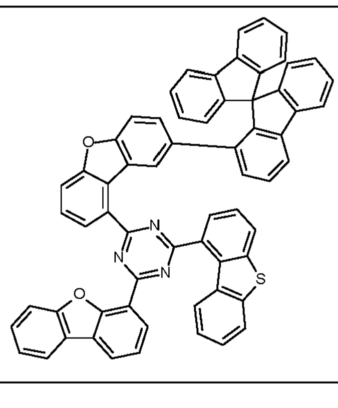 |

| | |
|---|---|
| 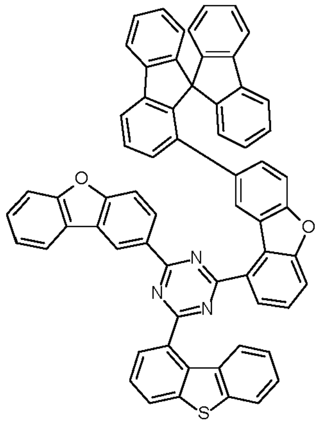 | 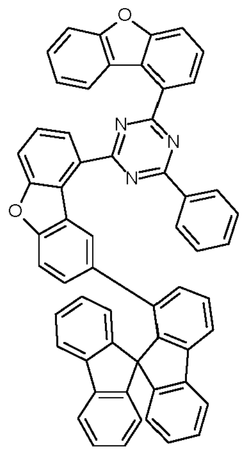 |
| 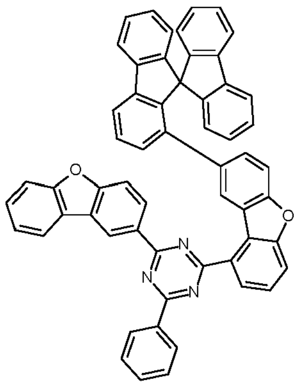 | 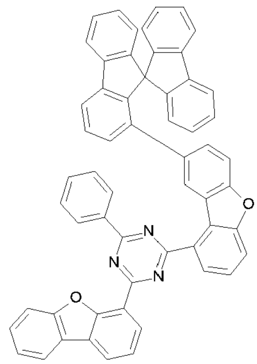 |
| 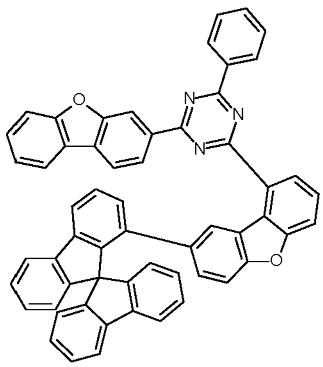 | 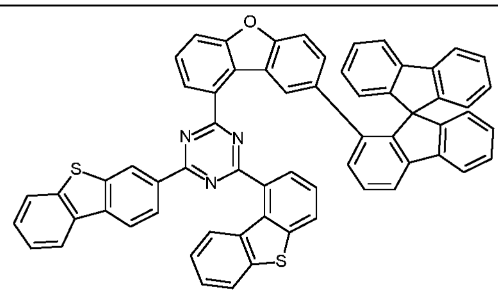 |
| 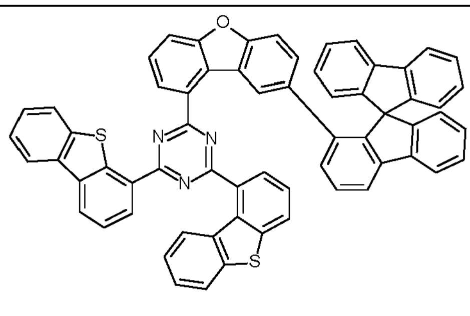 | 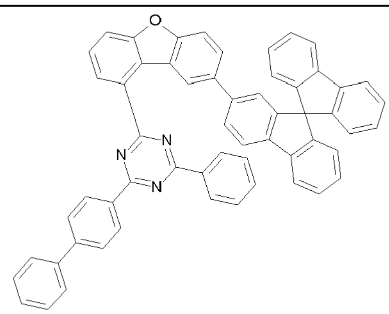 |

| | |
|---|---|
| 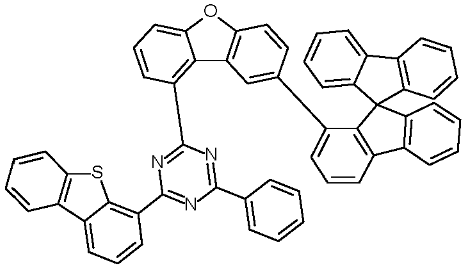 | 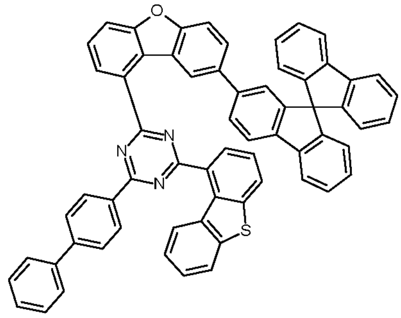 |
| 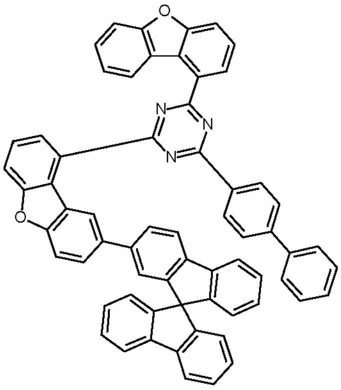 | 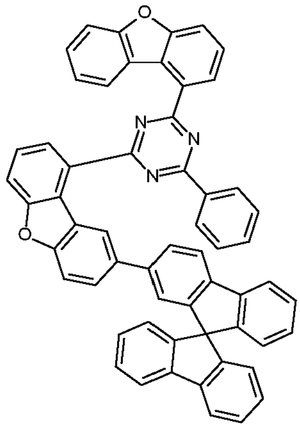 |
| 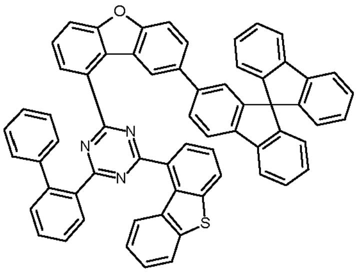 | 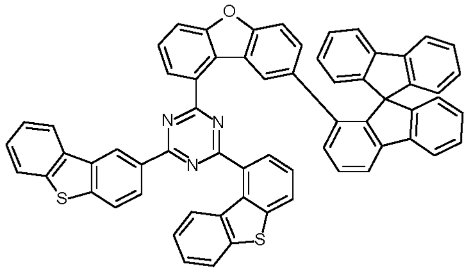 |
| 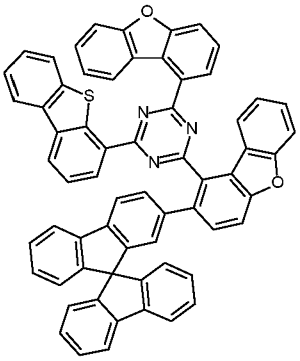 | 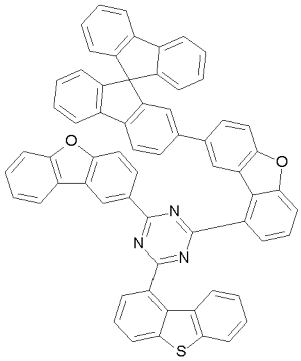 |

| | |
|---|---|
| 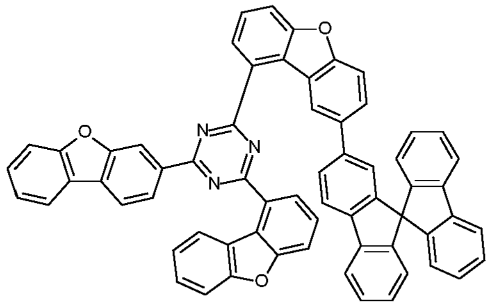 | 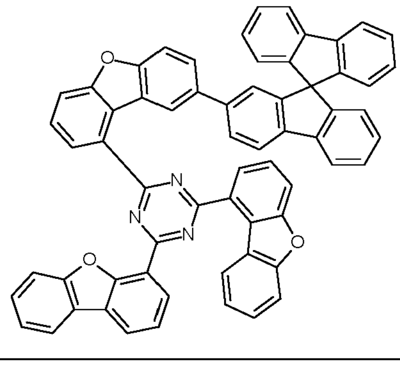 |
| 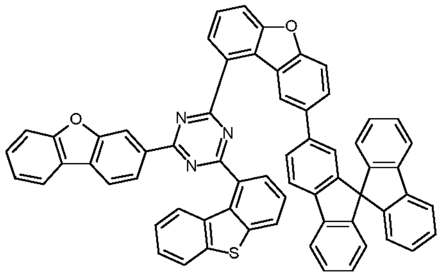 | 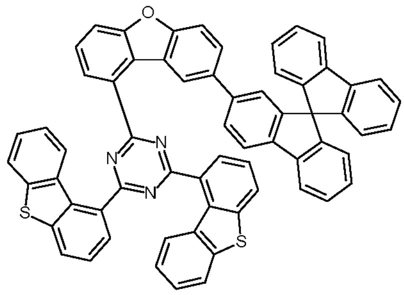 |
| 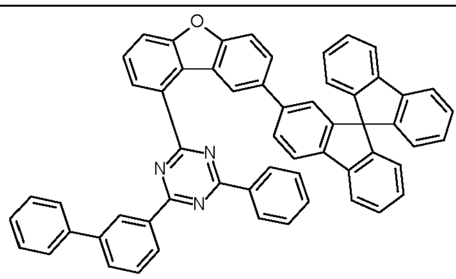 | 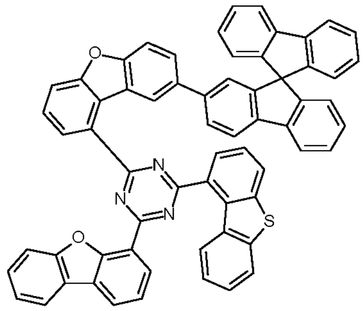 |
| 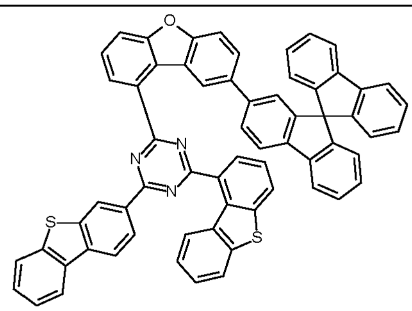 | 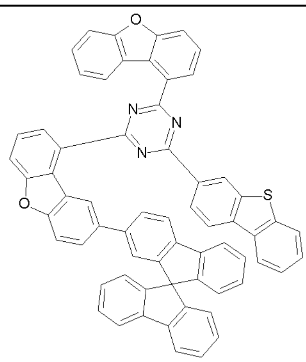 |
| 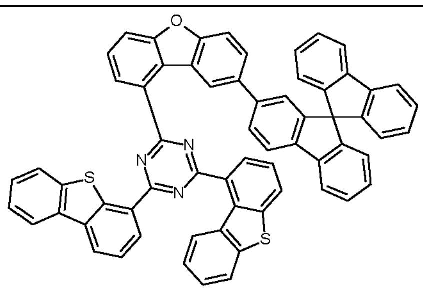 | 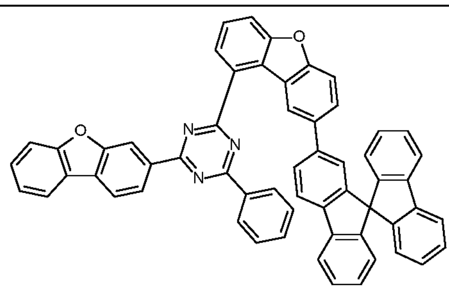 |

| | |
|---|---|
| 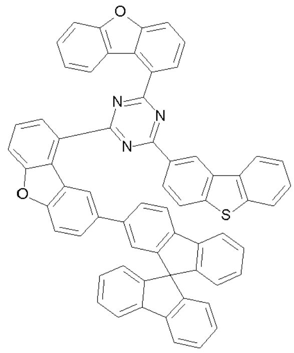 | 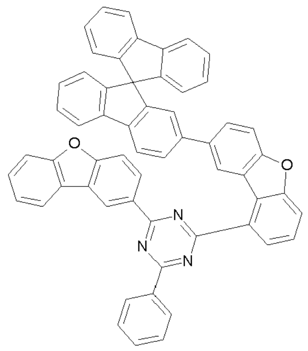 |
| 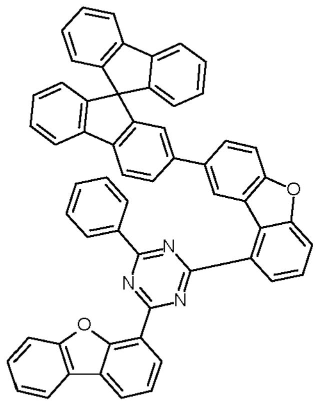 | 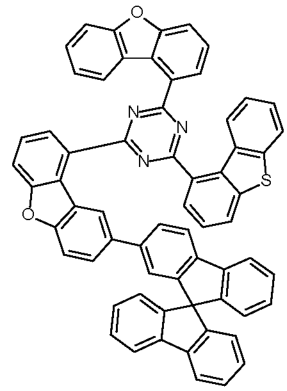 |
| 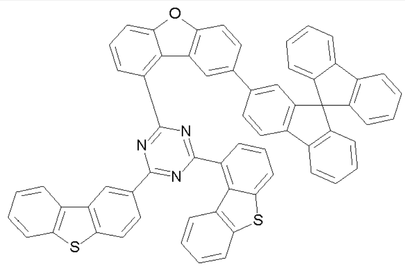 | 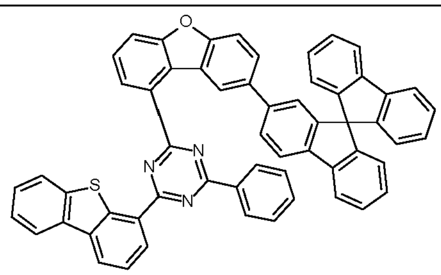 |
| 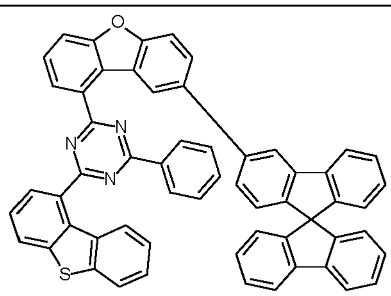 | 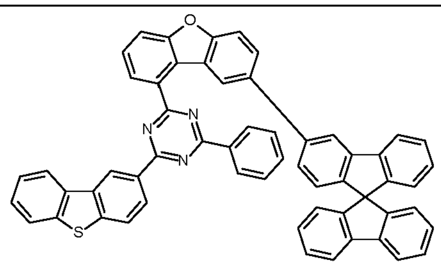 |
| 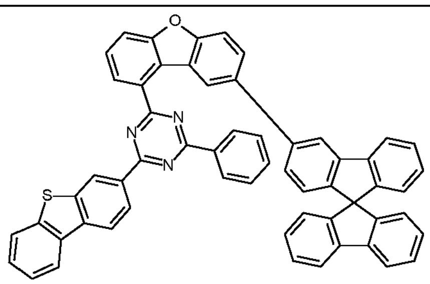 | 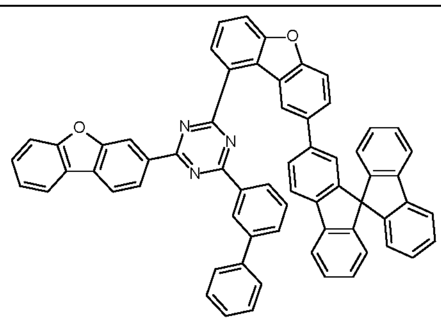 |

| | |
|---|---|
| 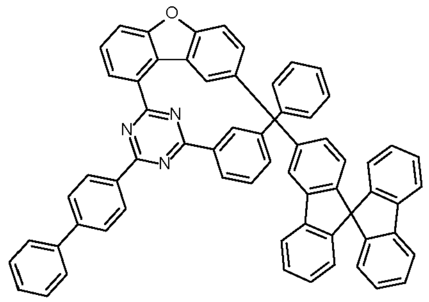 | 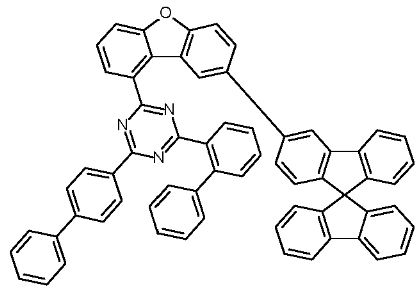 |
| 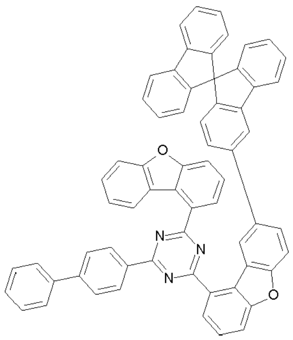 | 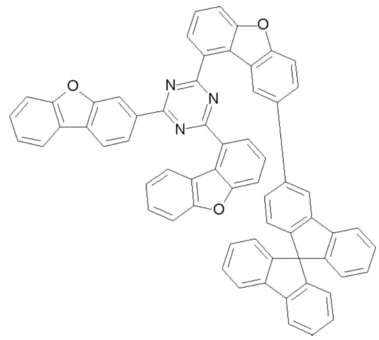 |
| 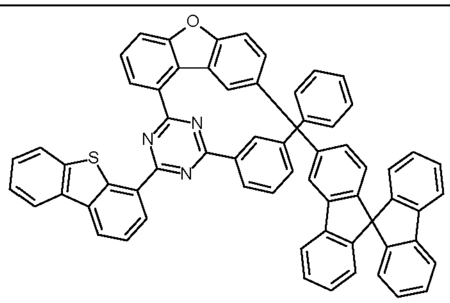 | 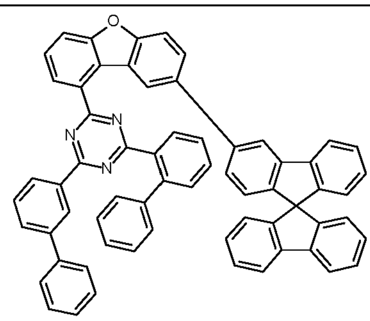 |
| 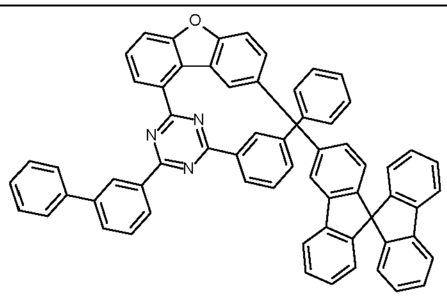 | 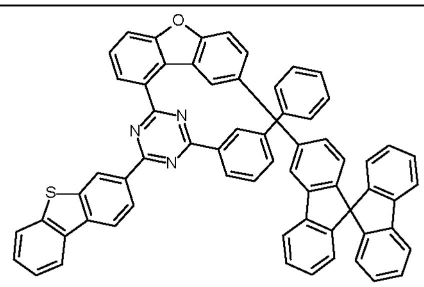 |
| 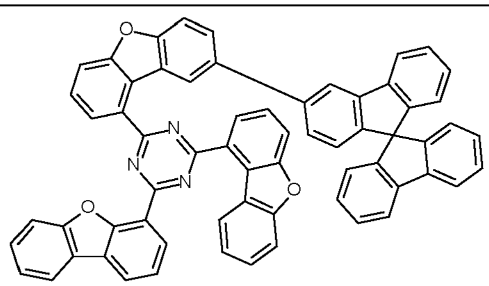 | 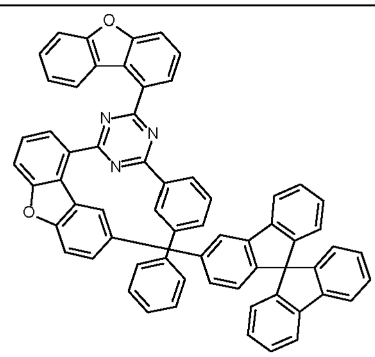 |

| | |
|---|---|
| 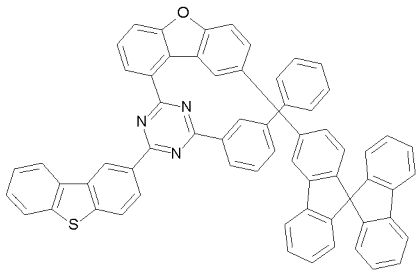 | 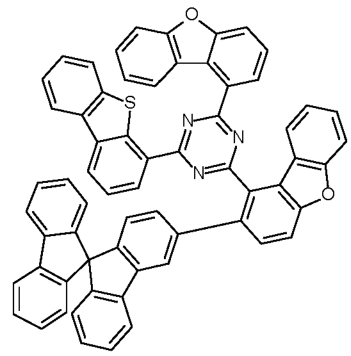 |
| 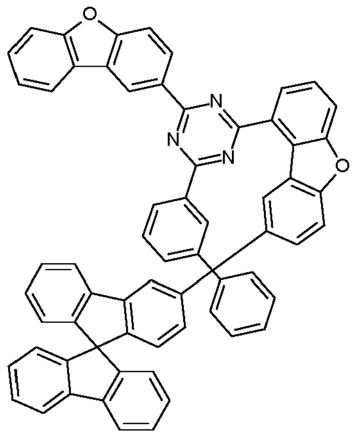 | 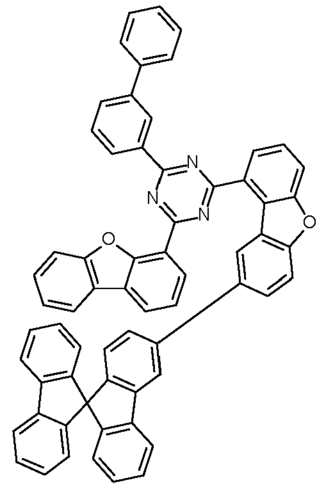 |
| 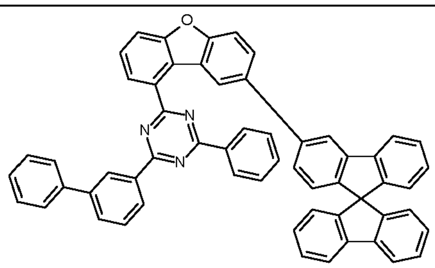 | 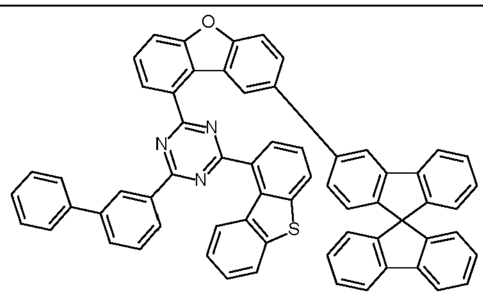 |
| 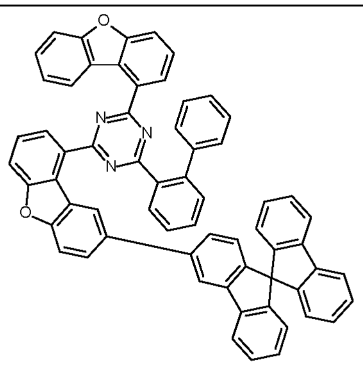 | 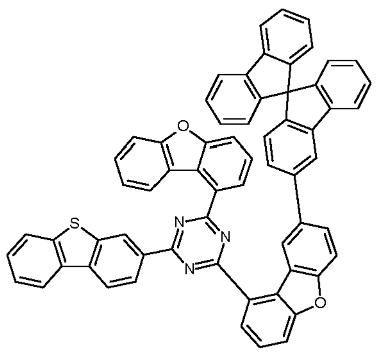 |

| | |
|---|---|
| 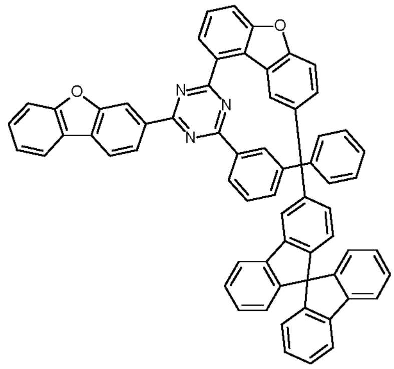 | 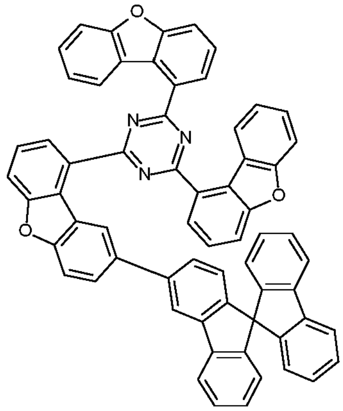 |
| 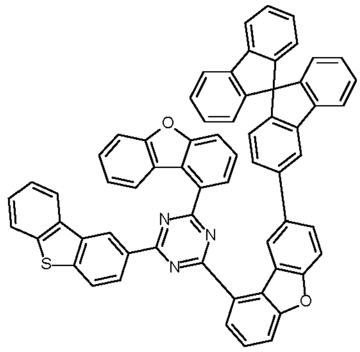 | 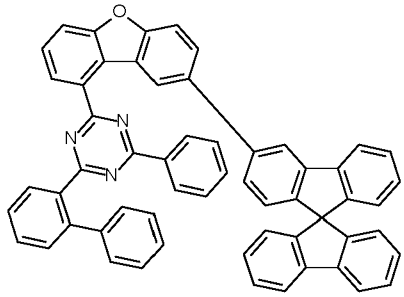 |
| 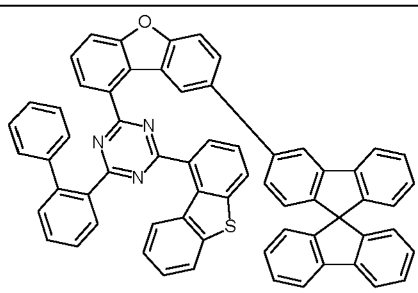 | 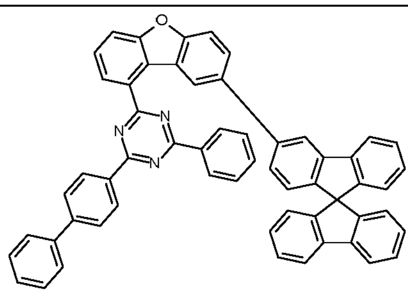 |
| 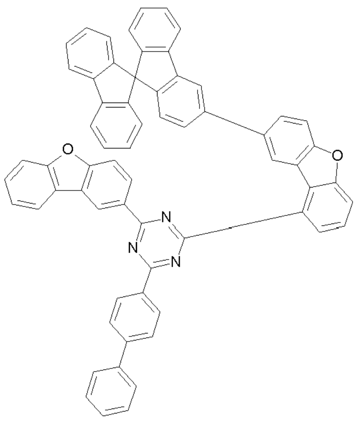 | 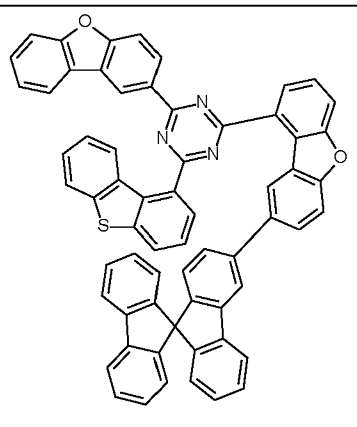 |

| | |
|---|---|
| 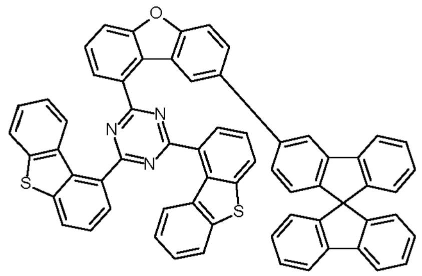 | 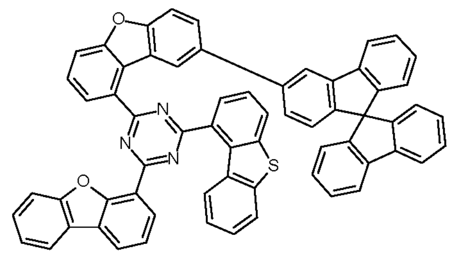 |
| 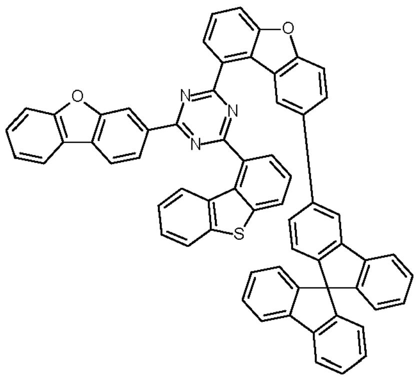 | 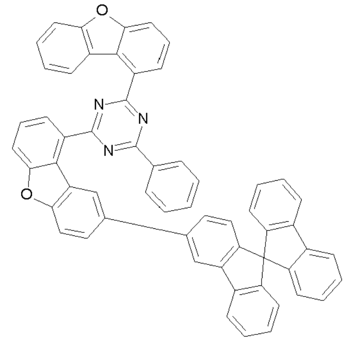 |
| 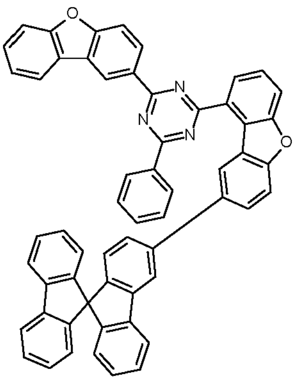 | 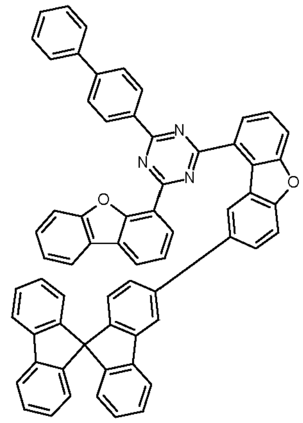 |
| 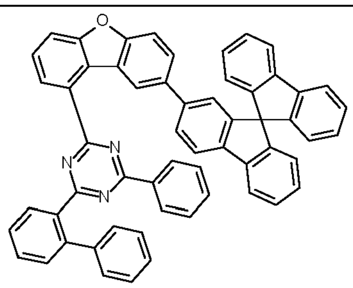 | 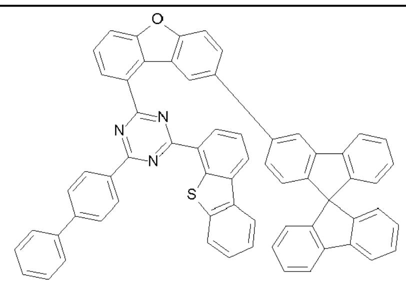 |

| | |
|---|---|
| 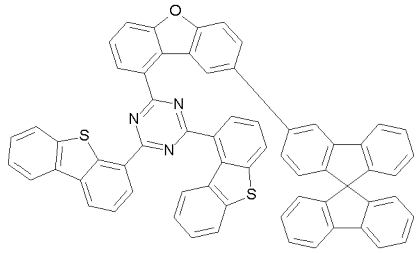 | 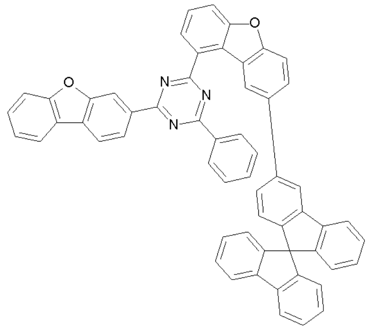 |
| 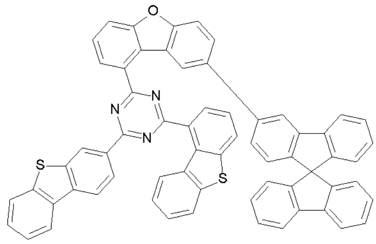 | 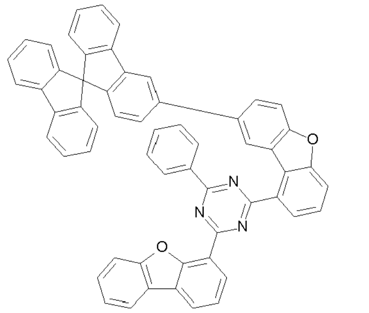 |
| 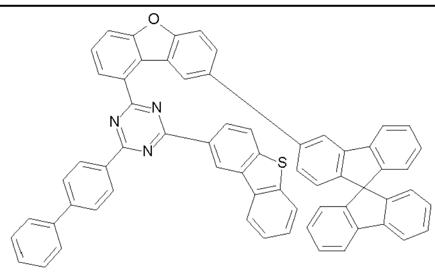 | 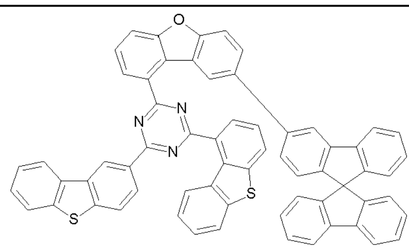 |
| 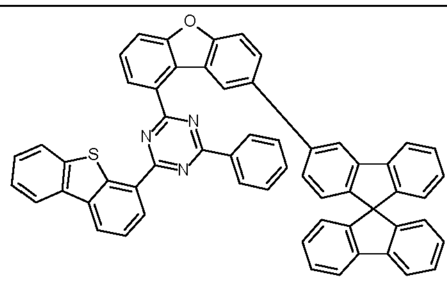 | 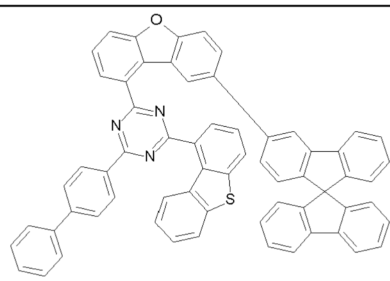 |
| 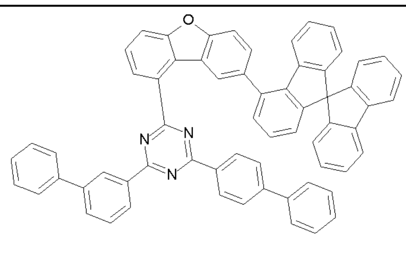 | 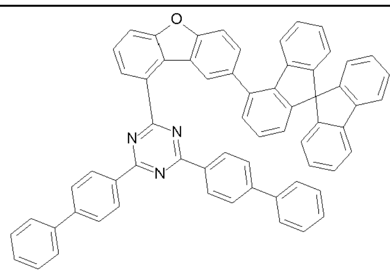 |

| | |
|---|---|
| 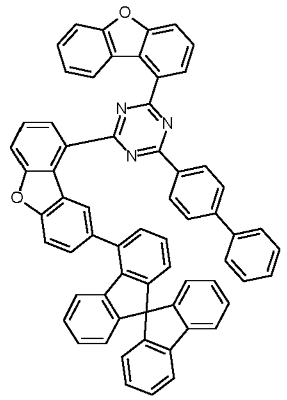 | 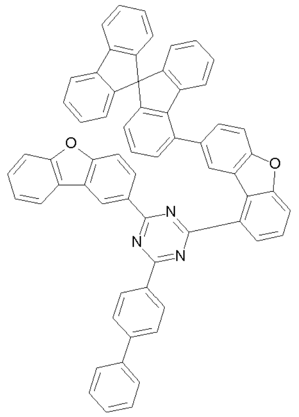 |
| 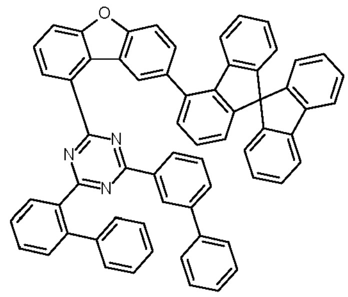 | 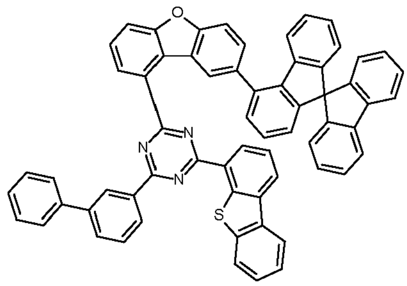 |
| 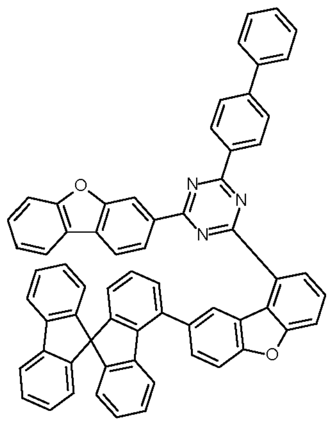 | 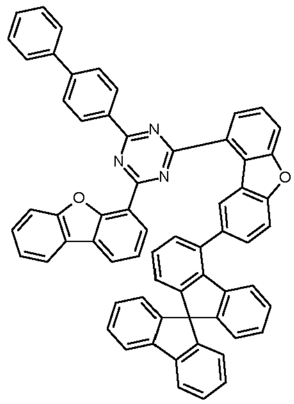 |
| 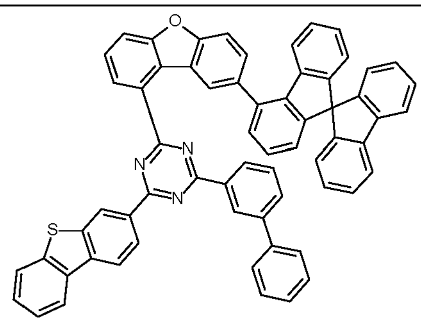 | 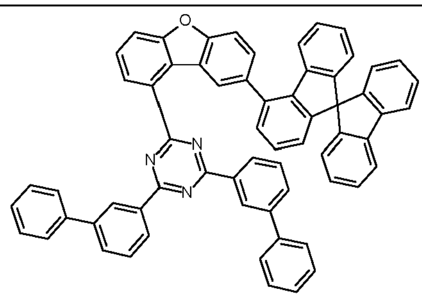 |

| | |
|---|---|
| 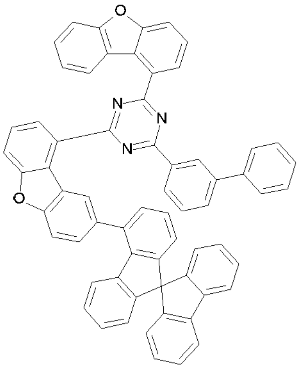 | 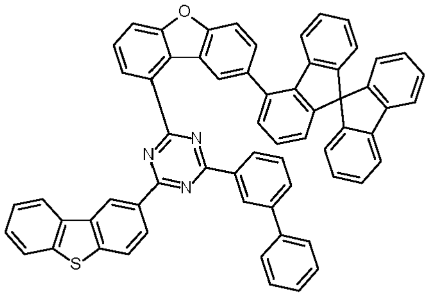 |
| 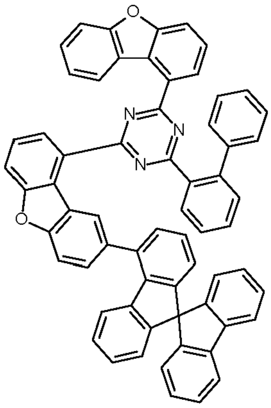 | 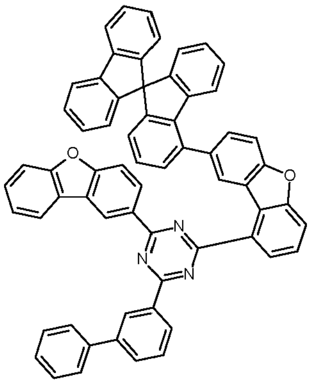 |
| 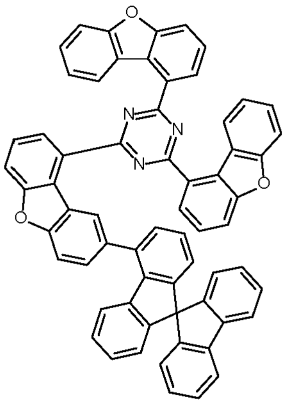 | 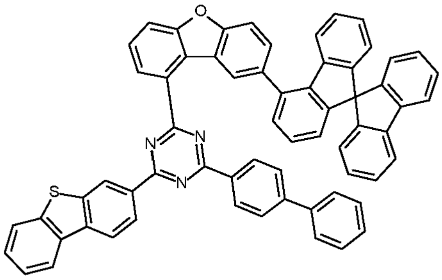 |
| 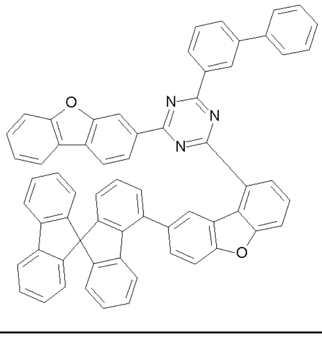 | 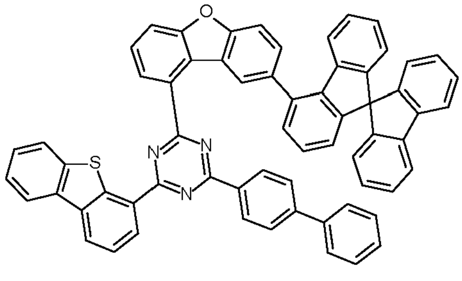 |

| | |
|---|---|
| 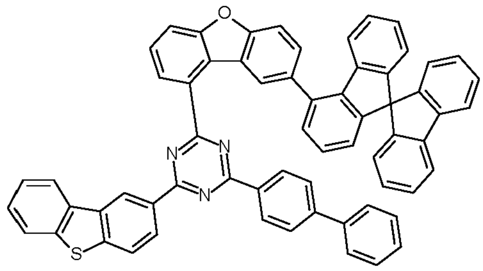 | 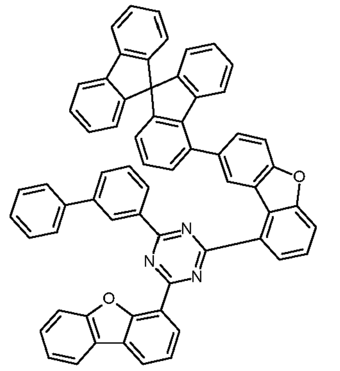 |
| 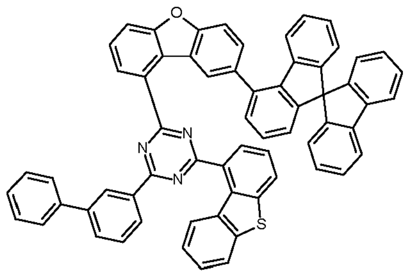 | 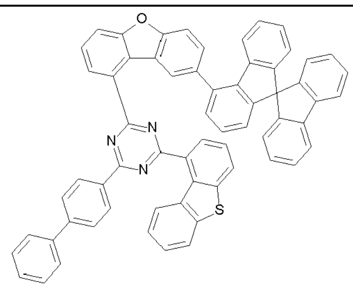 |
| 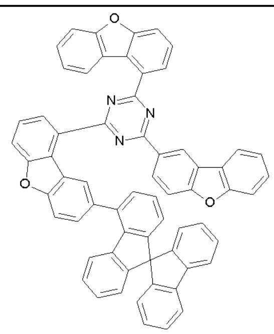 | 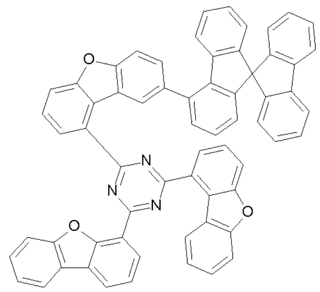 |
| 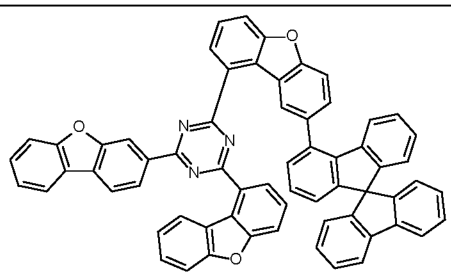 | 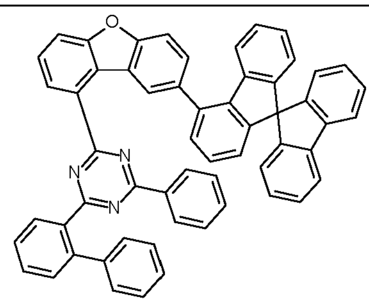 |
| 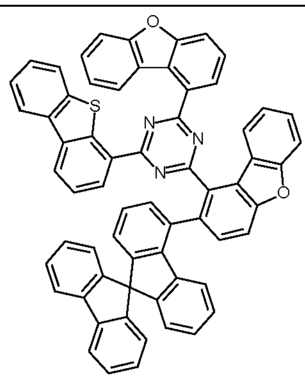 | 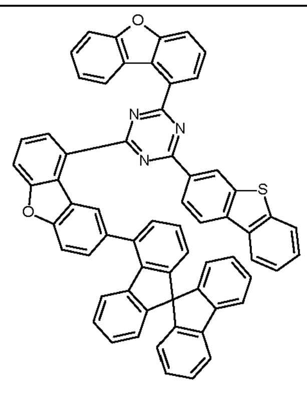 |

| | |
|---|---|
| 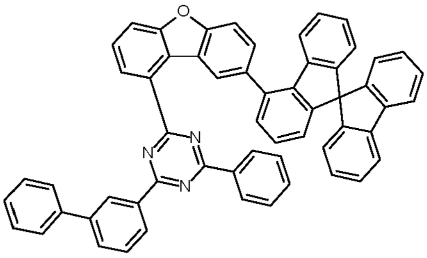 | 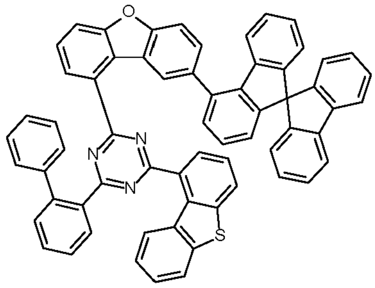 |
| 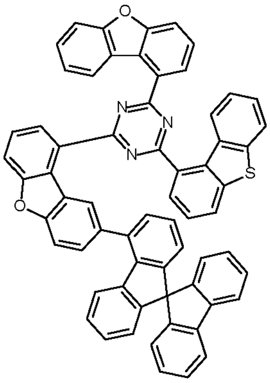 | 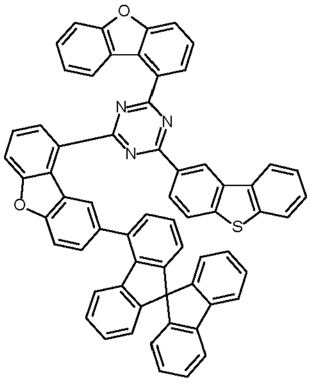 |
| 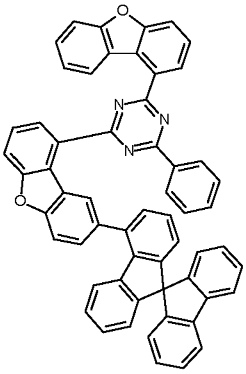 | 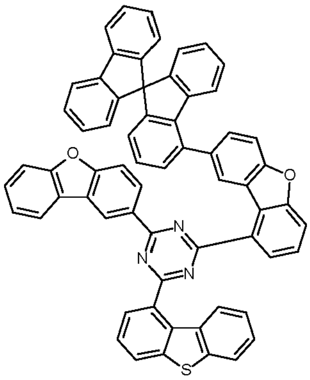 |
| 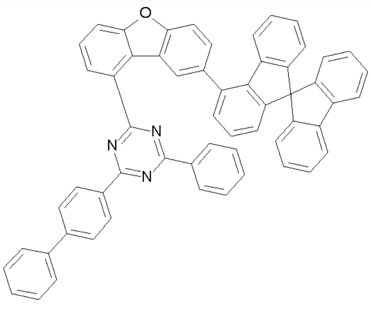 | 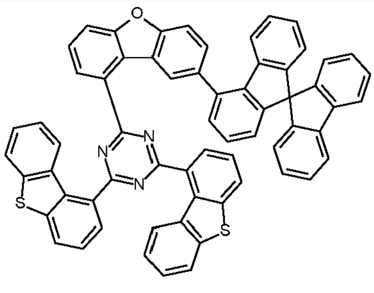 |

| | |
|---|---|
| 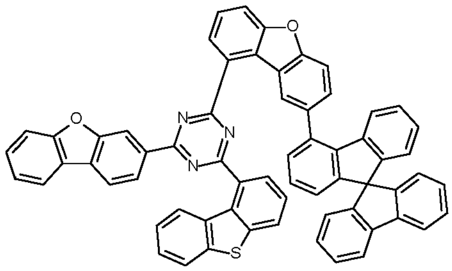 | 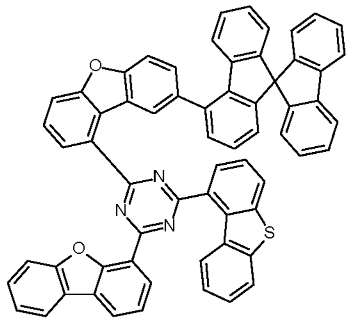 |
| 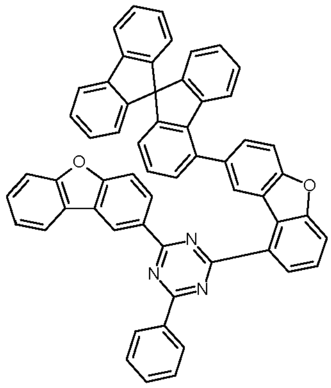 | 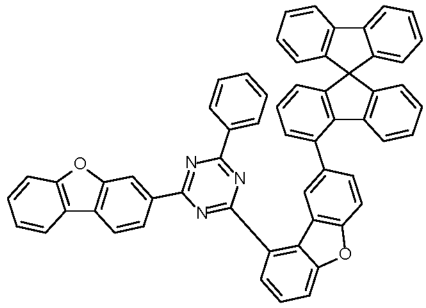 |
| 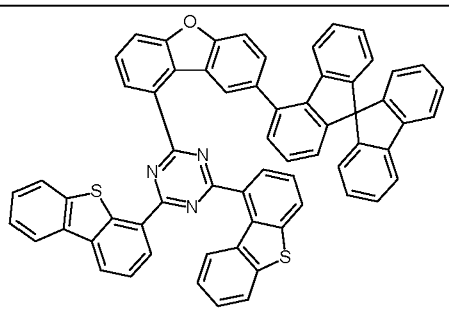 | 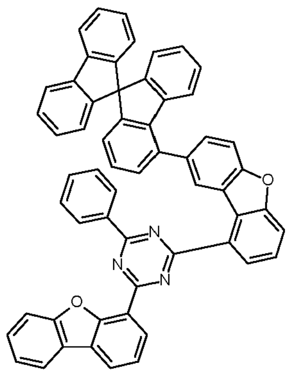 |
| 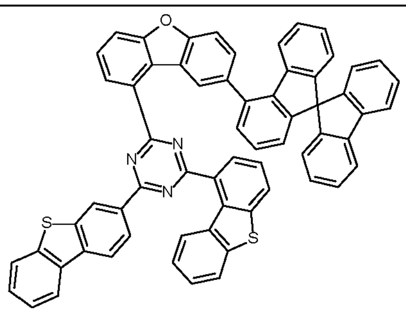 | 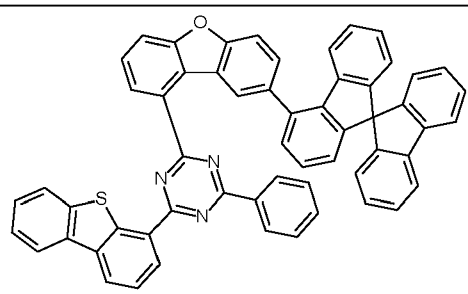 |

| | |
|---|---|
| 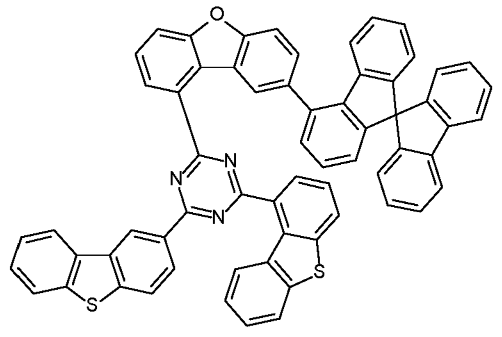 | 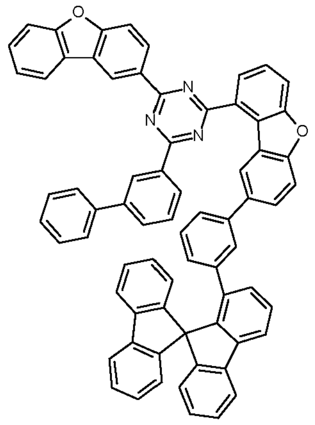 |
| 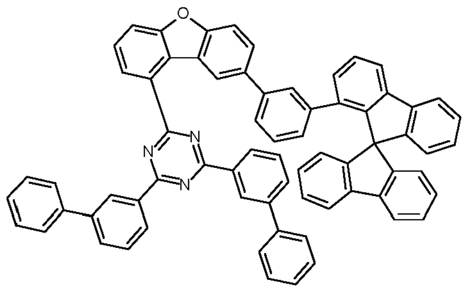 | 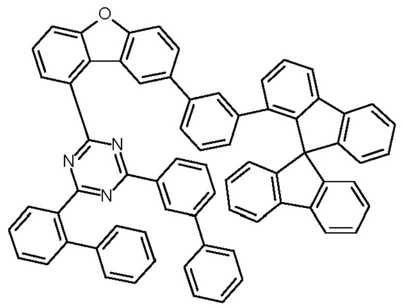 |
| 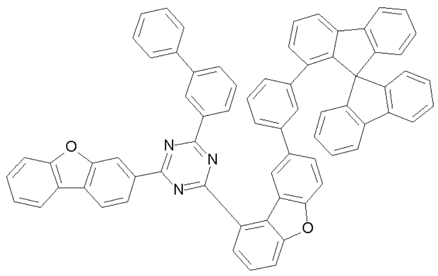 | 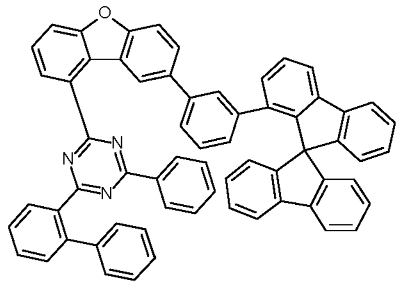 |
| 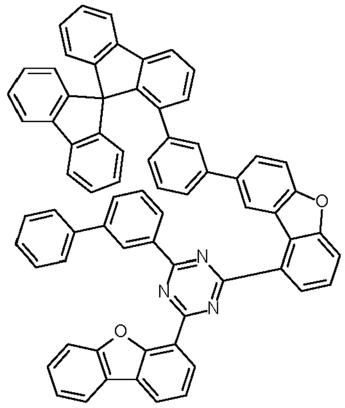 | 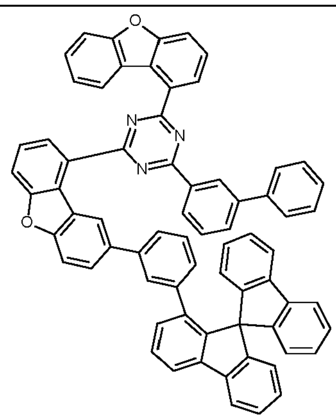 |

| | |
|---|---|
| 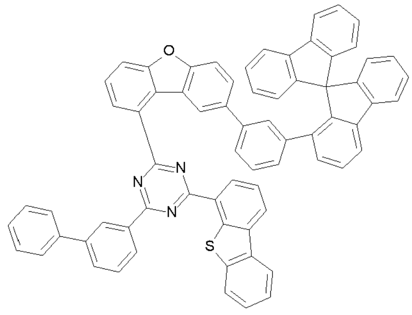 | 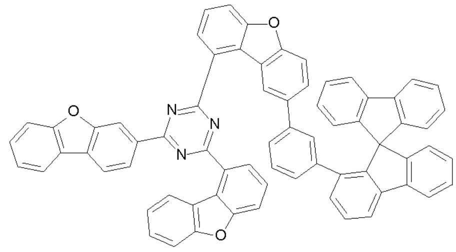 |
| 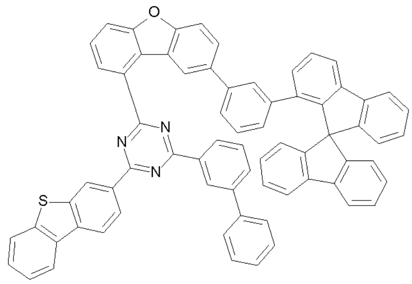 | 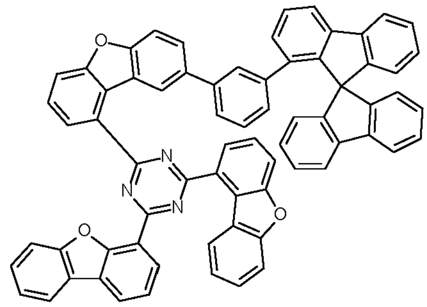 |
| 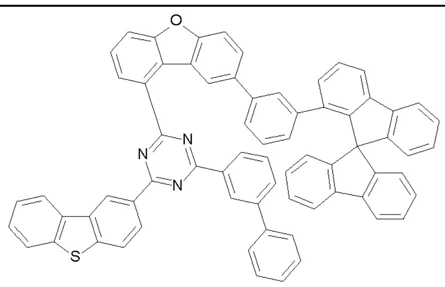 | 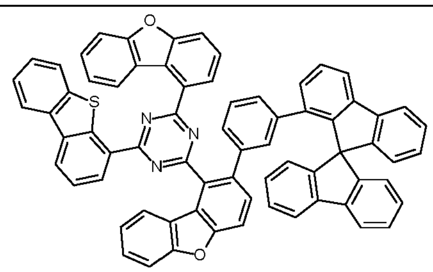 |
| 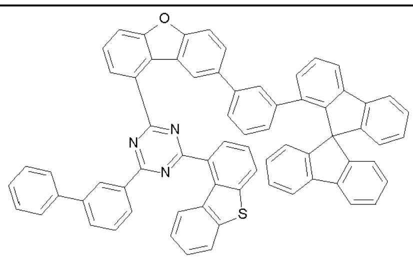 | 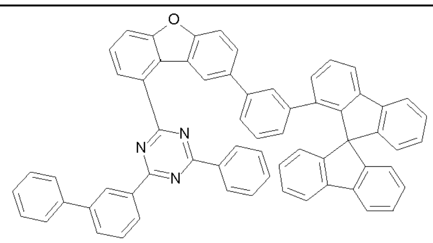 |
| 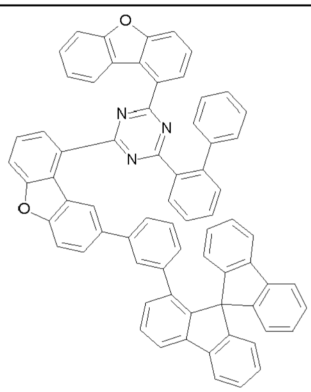 | 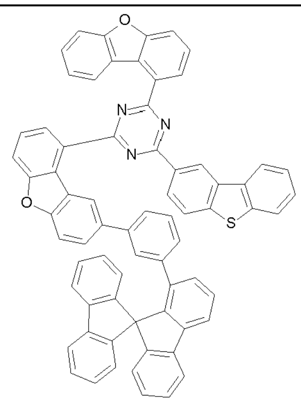 |

| | |
|---|---|
| 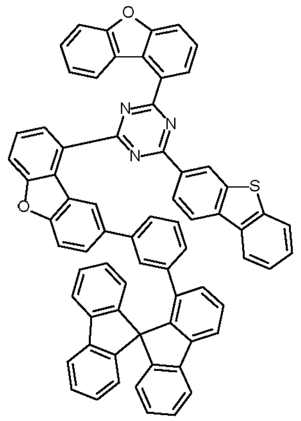 | 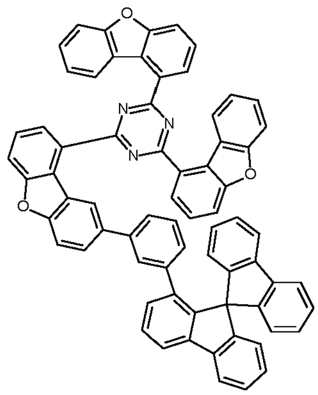 |
| 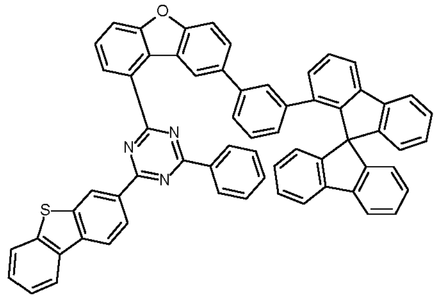 | 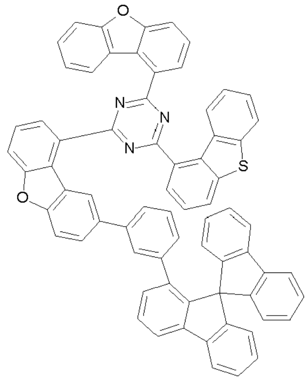 |
| 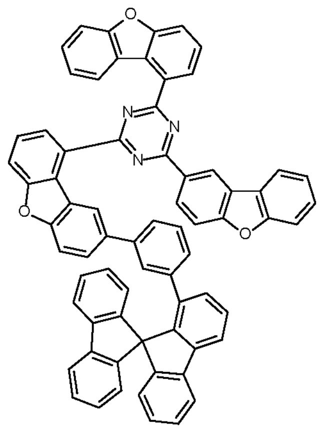 | 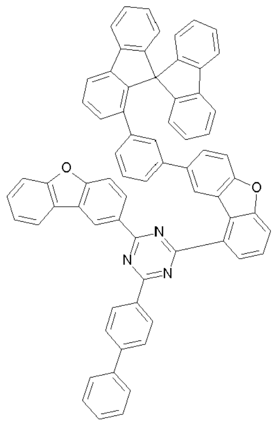 |
| 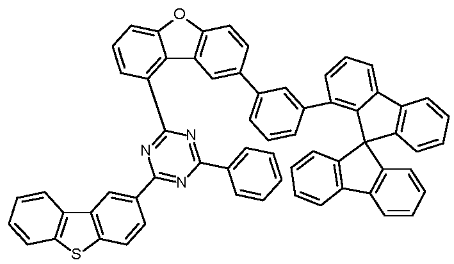 | 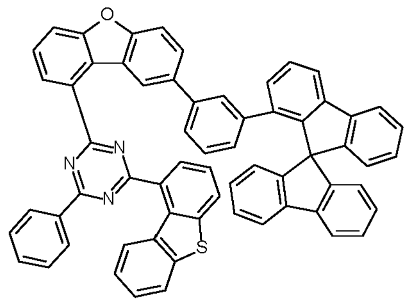 |

| | |
|---|---|
| 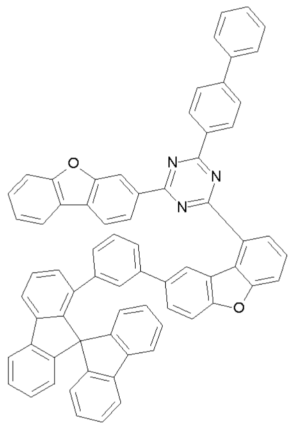 | 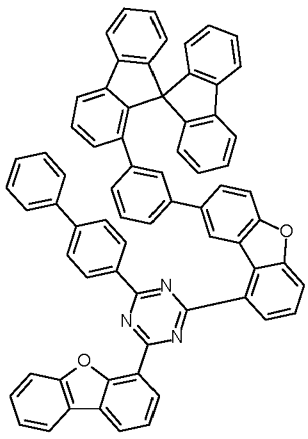 |
| 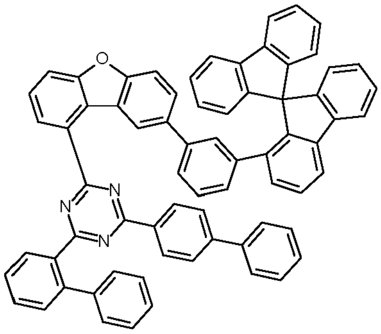 | 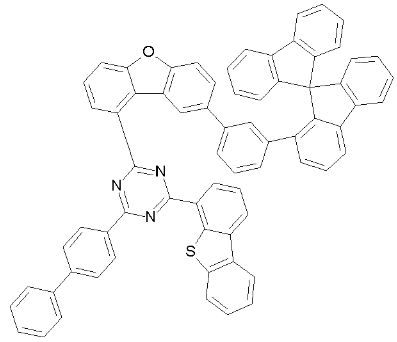 |
| 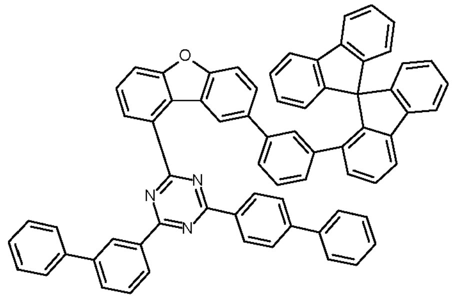 | 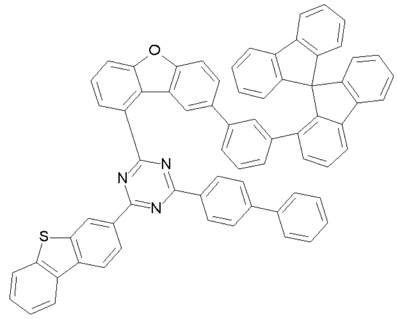 |
| 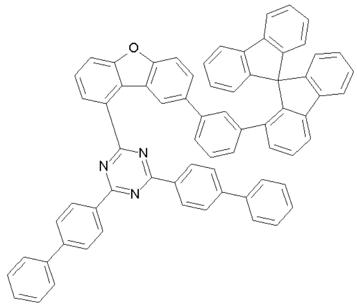 | 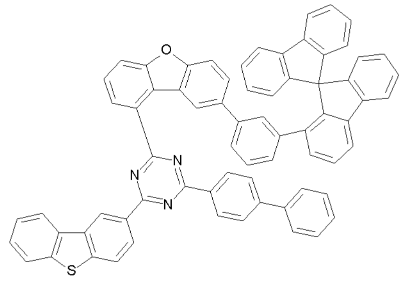 |

| | |
|---|---|
| 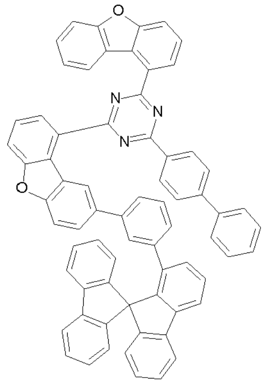 | 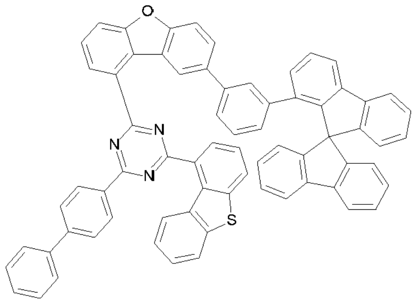 |
| 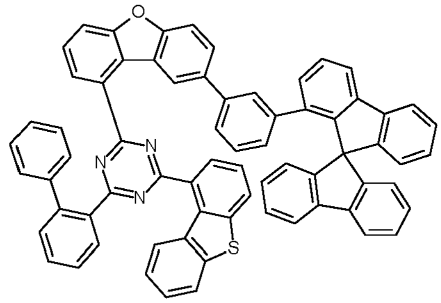 | 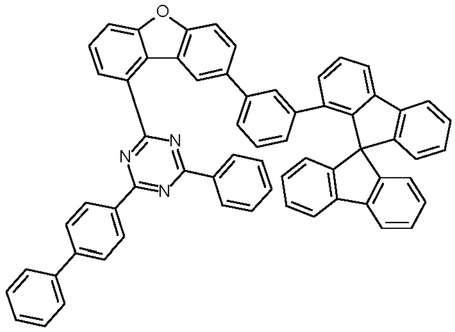 |
| 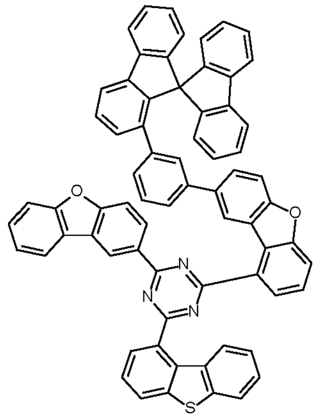 | 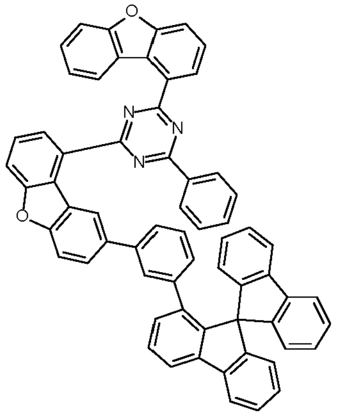 |
| 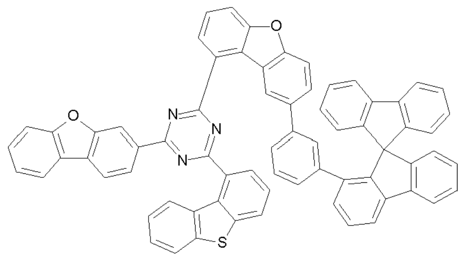 | 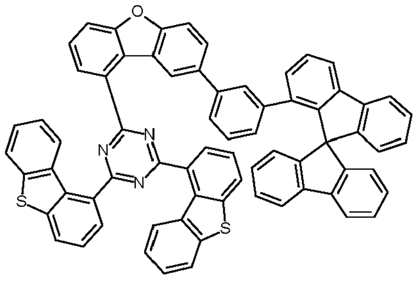 |

| | |
|---|---|
| 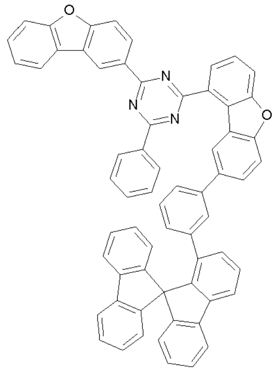 | 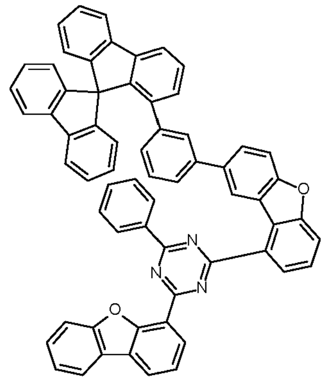 |
| 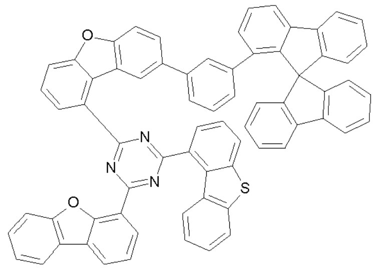 | 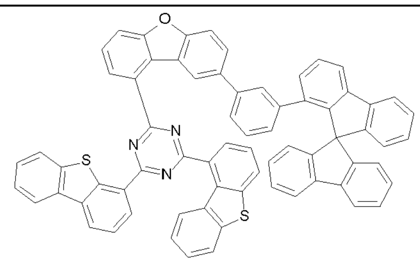 |
| 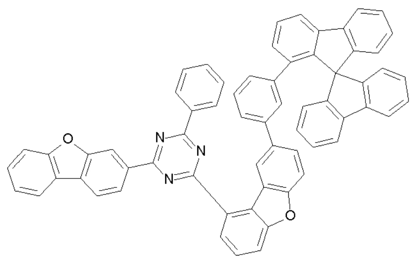 | 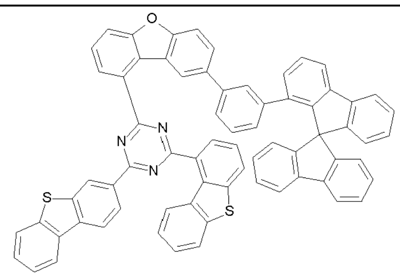 |
| 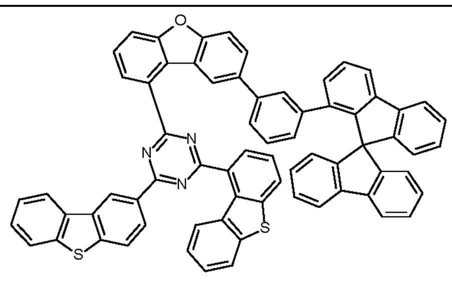 | 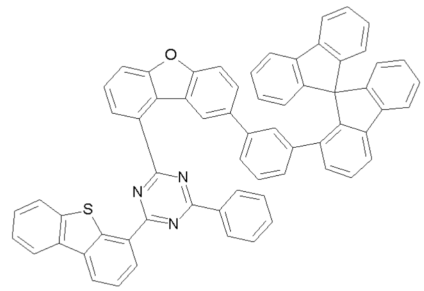 |
| 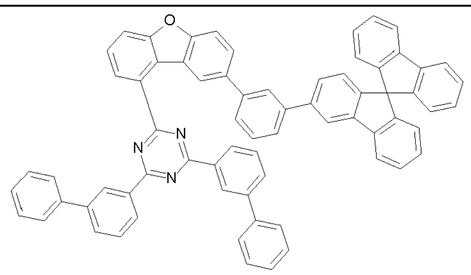 | 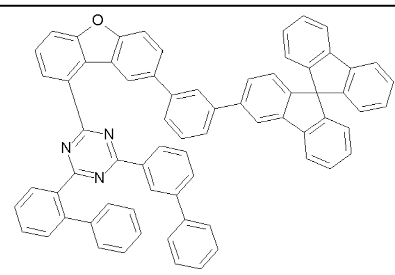 |

| | |
|---|---|
| 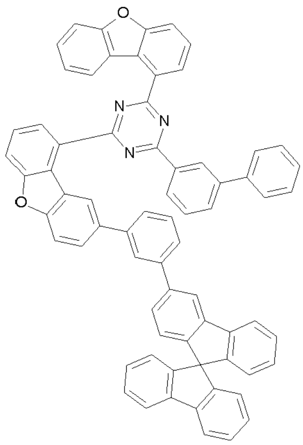 | 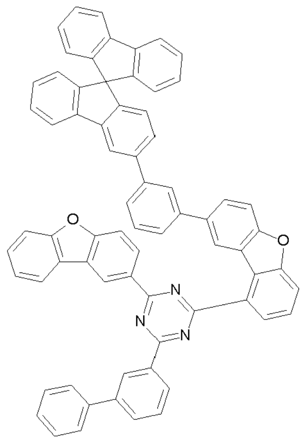 |
| 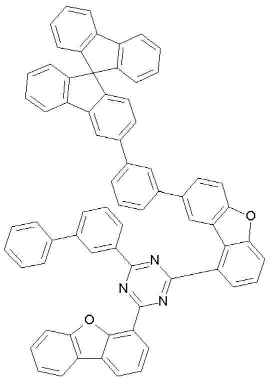 | 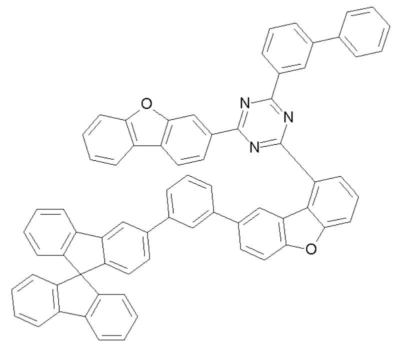 |
| 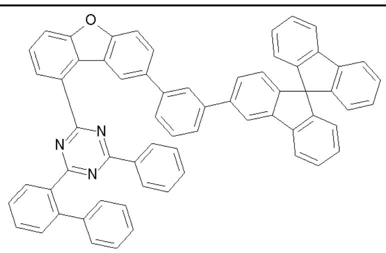 | 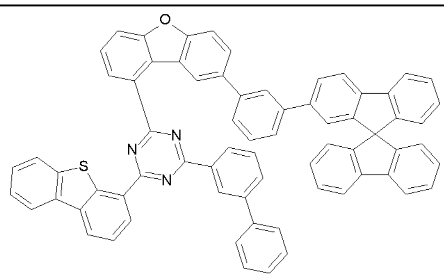 |
| 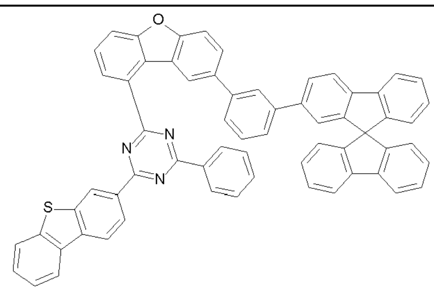 | 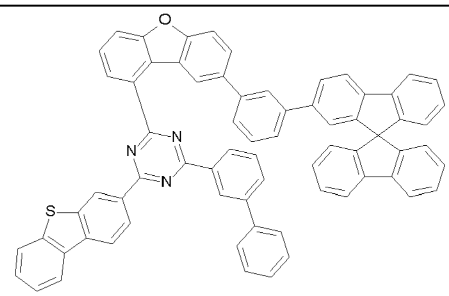 |
| 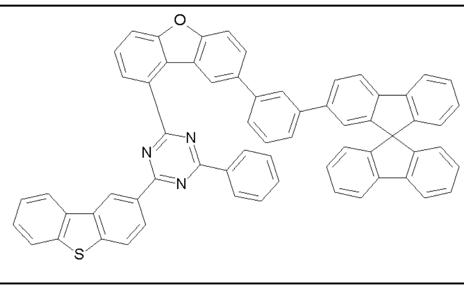 | 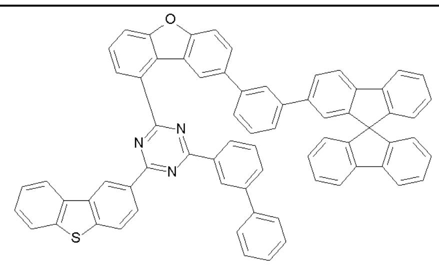 |

| | |
|---|---|
| 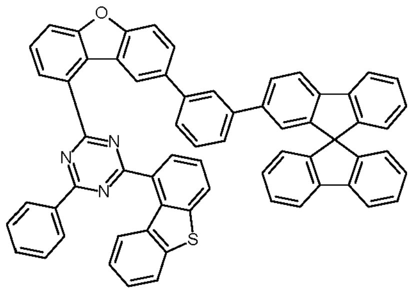 | 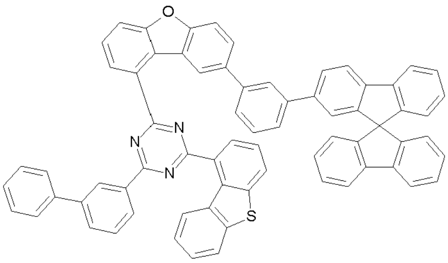 |
| 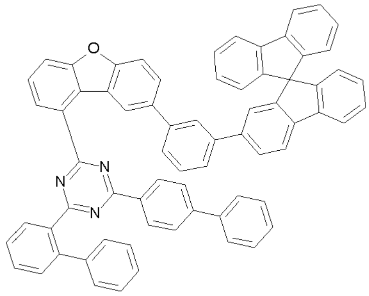 | 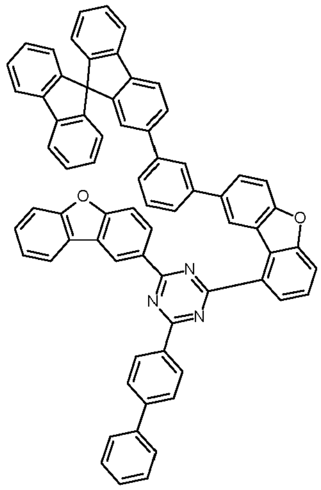 |
| 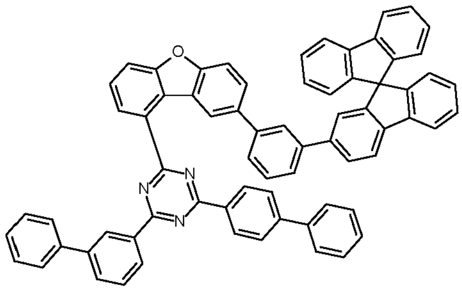 | 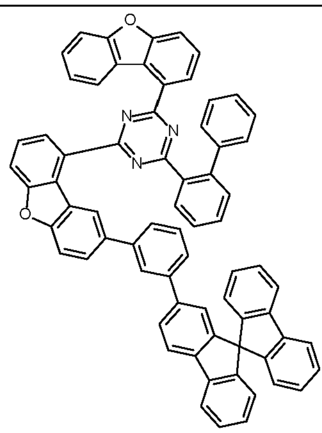 |
| 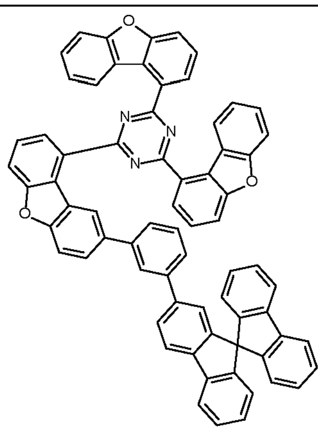 | 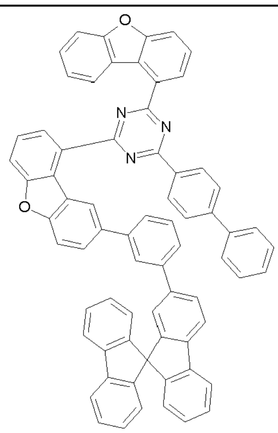 |

| | |
|---|---|
| 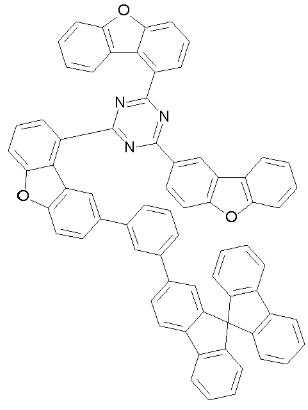 | 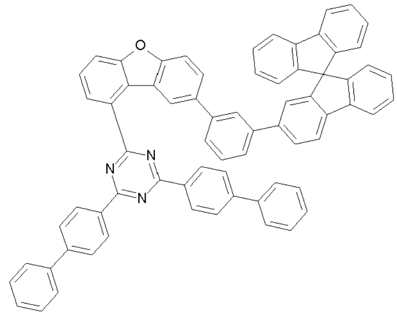 |
| 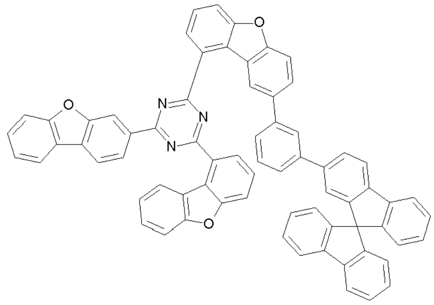 | 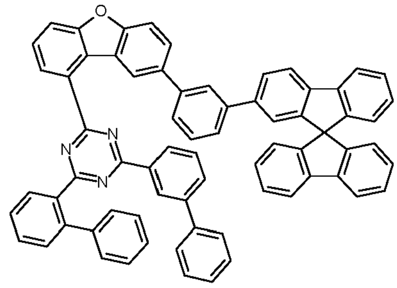 |
| 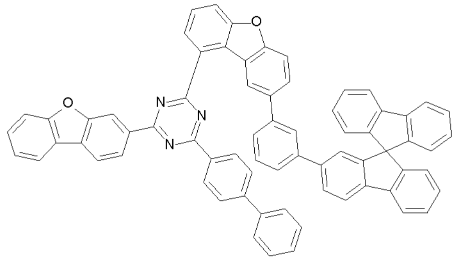 | 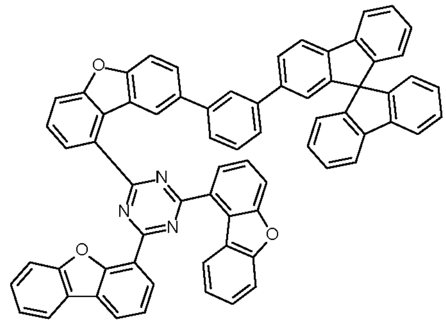 |
| 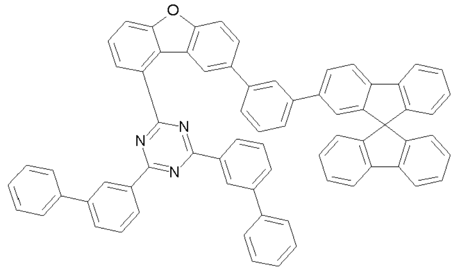 | 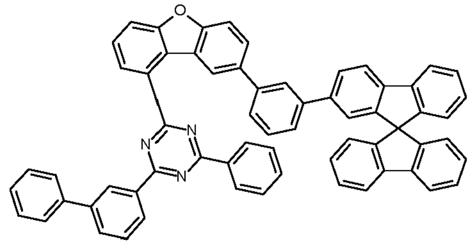 |
| 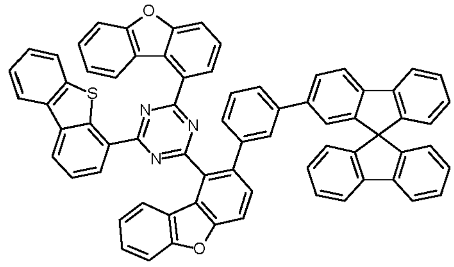 | 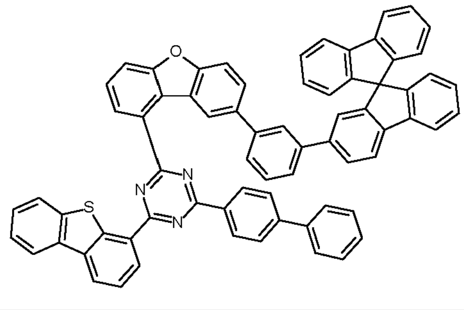 |

| | |
|---|---|
| 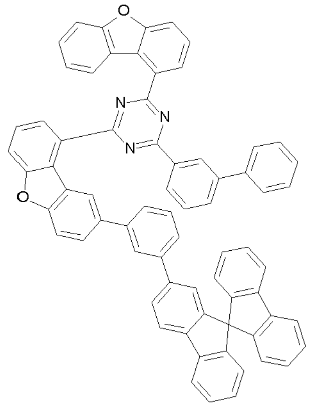 | 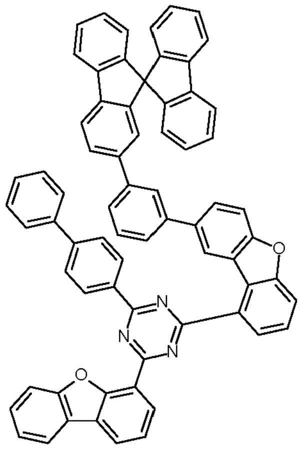 |
| 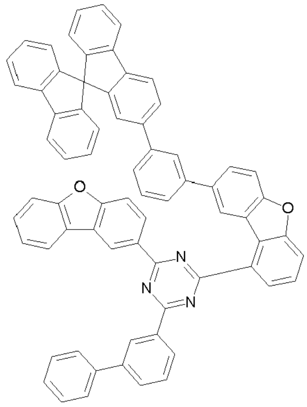 | 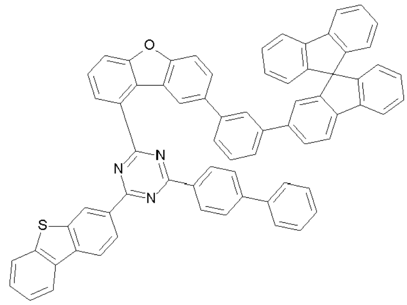 |
| 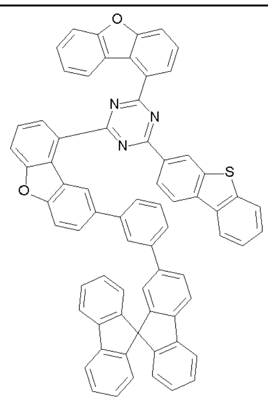 | 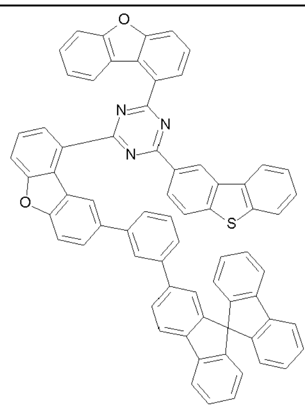 |
| 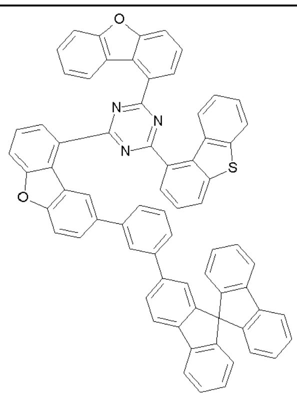 | 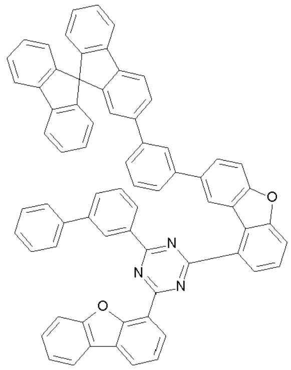 |

| | |
|---|---|
| 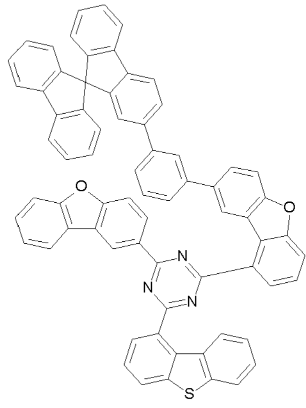 | 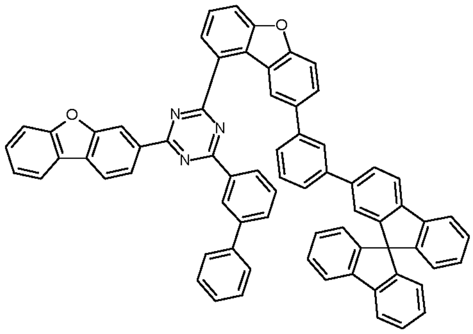 |
| 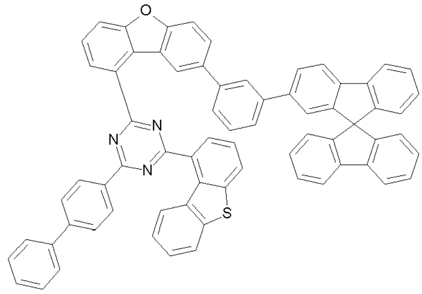 | 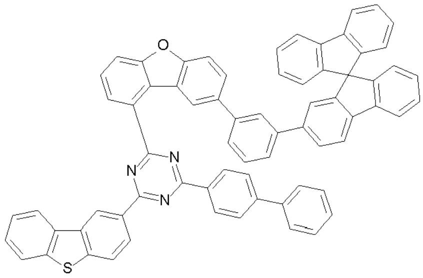 |
| 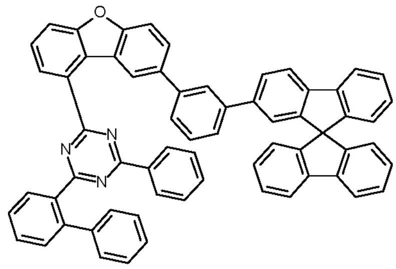 | 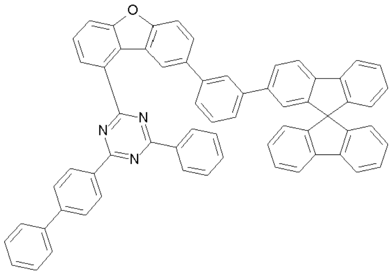 |
| 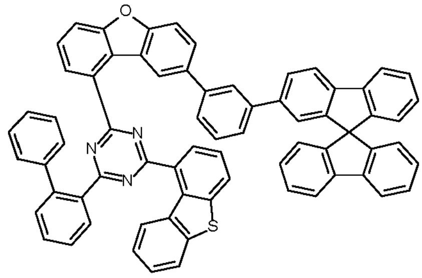 | 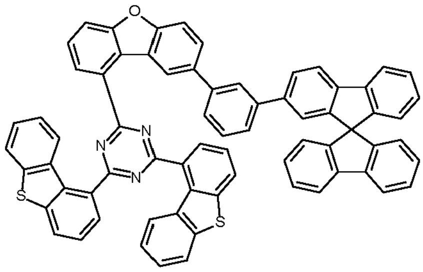 |
| 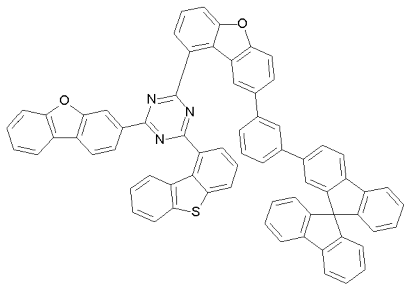 | 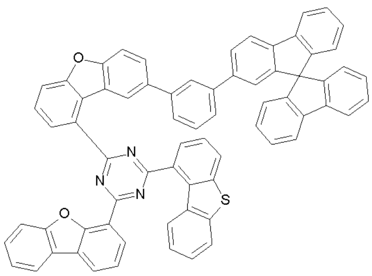 |

| | |
|---|---|
| 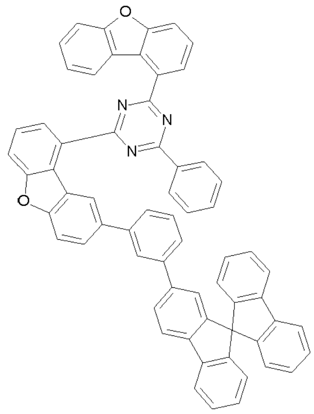 | 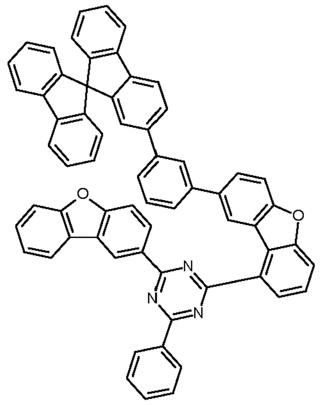 |
| 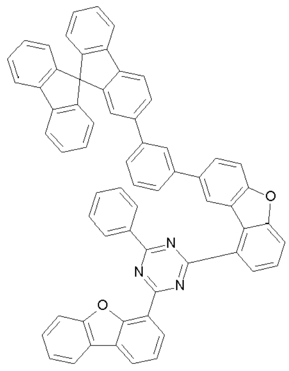 | 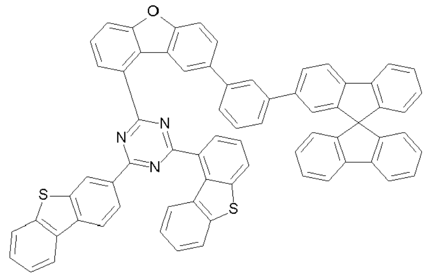 |
| 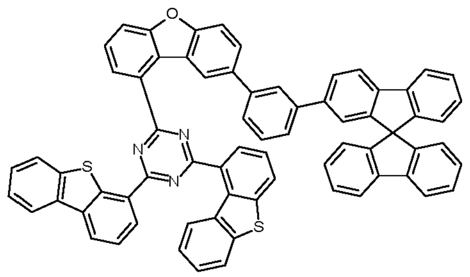 | 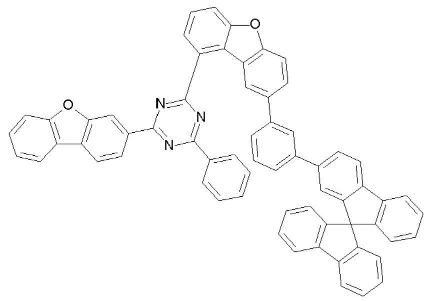 |
| 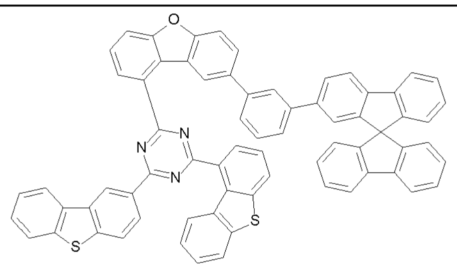 | 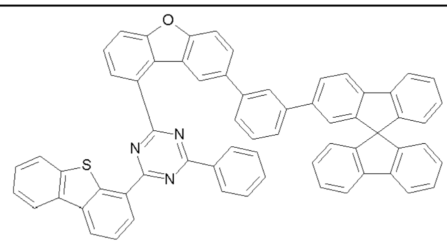 |
| 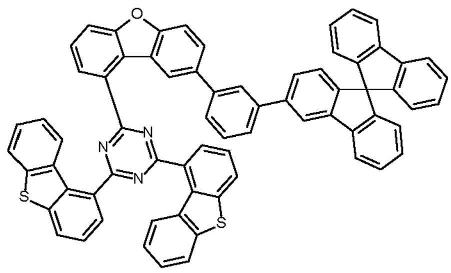 | 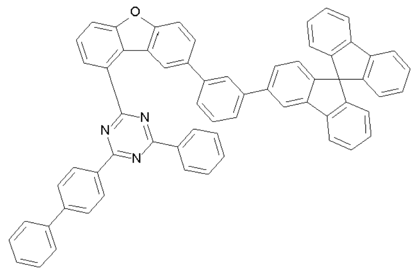 |

| | |
|---|---|
| 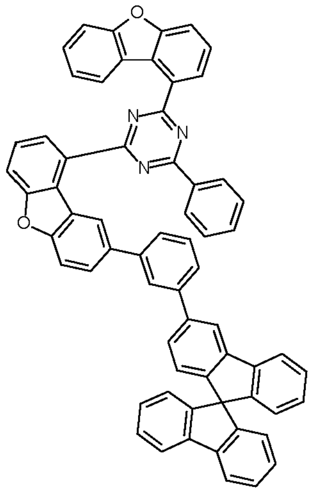 | 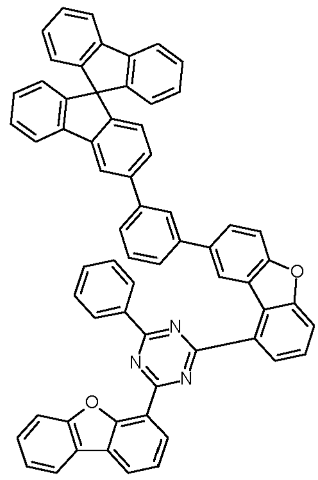 |
| 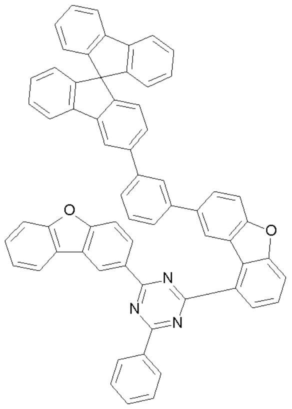 | 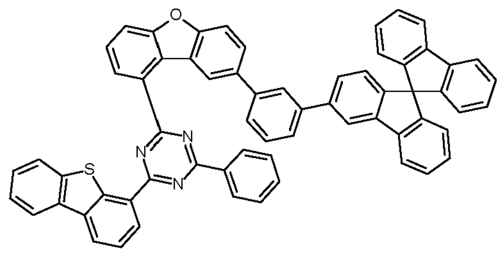 |
| 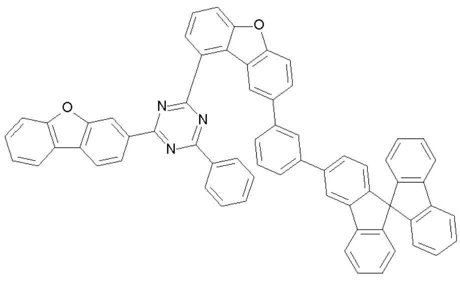 | 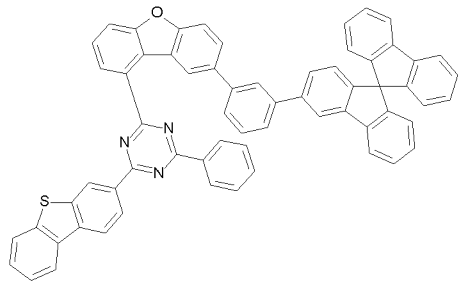 |
| 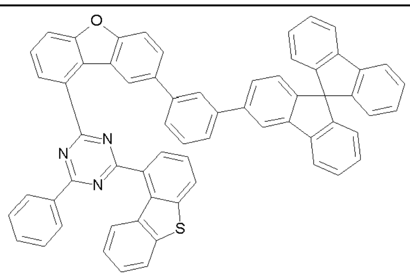 | 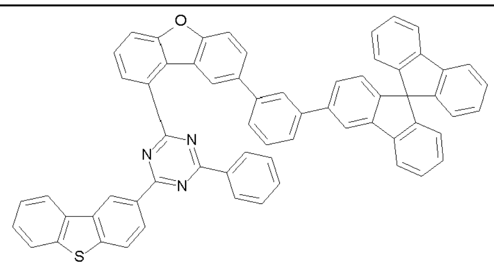 |

| | |
|---|---|
| 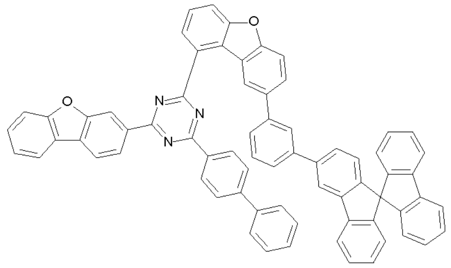 | 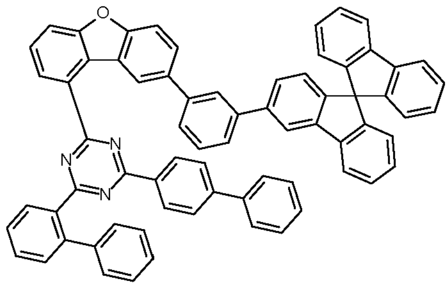 |
| 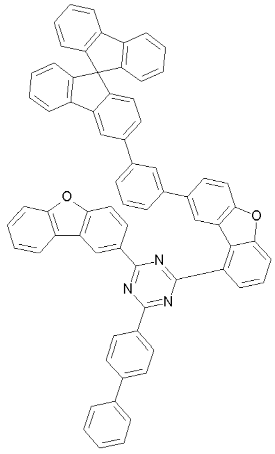 | 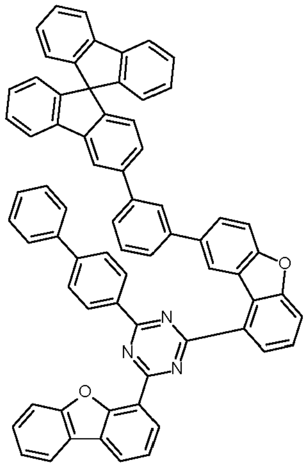 |
| 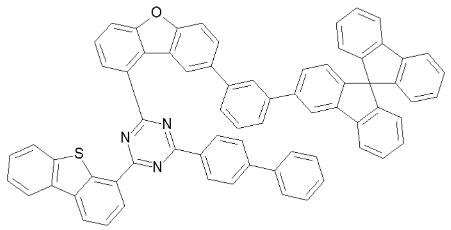 | 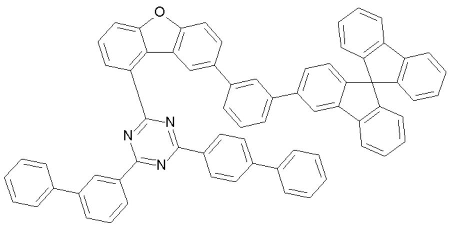 |
| 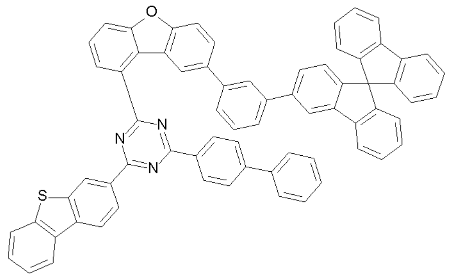 | 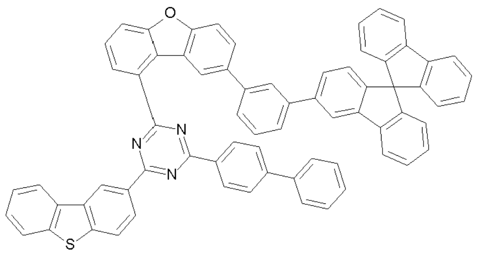 |
| 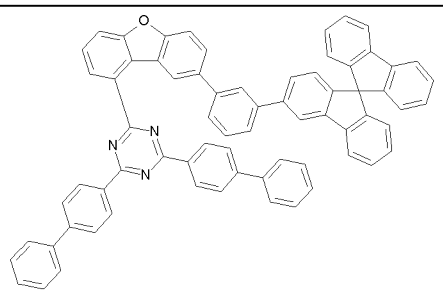 | 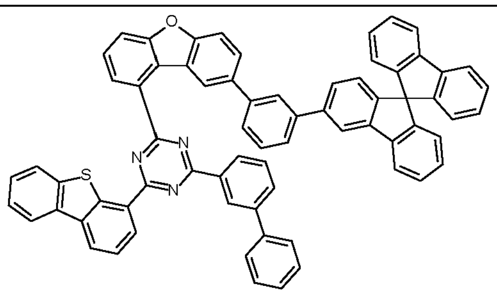 |

| | |
|---|---|
| 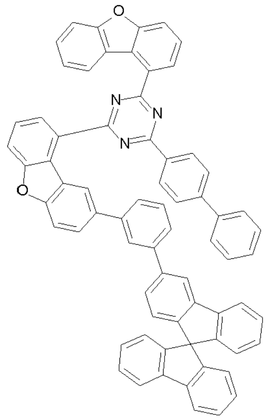 | 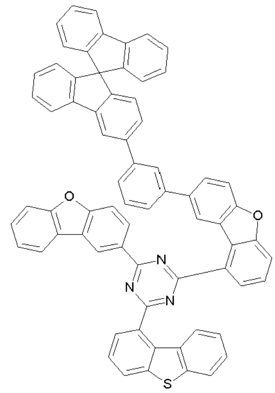 |
| 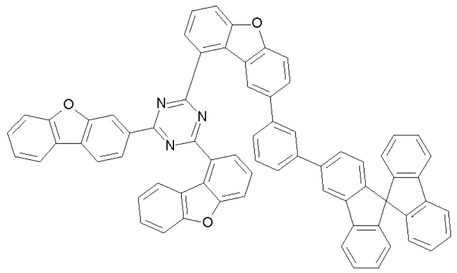 | 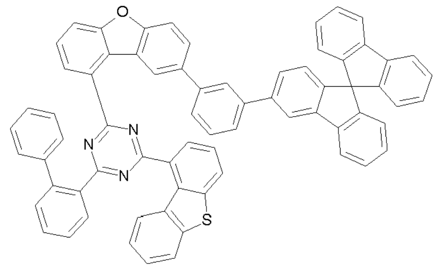 |
| 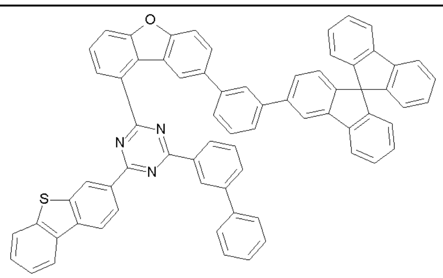 | 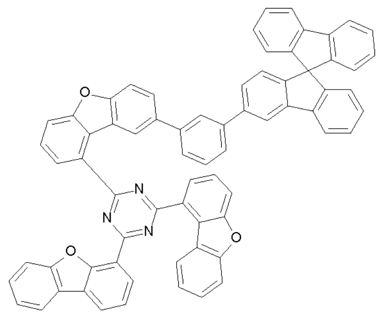 |
| 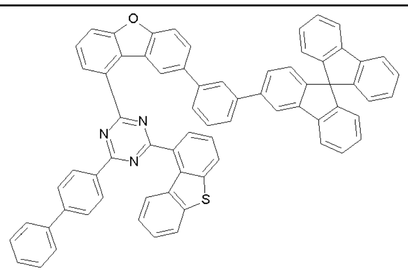 | 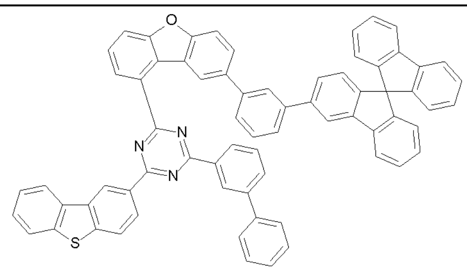 |
| 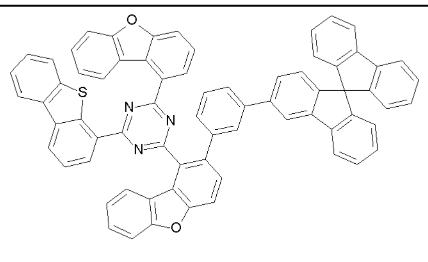 | 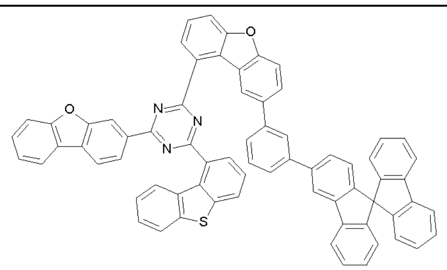 |

| | |
|---|---|
| 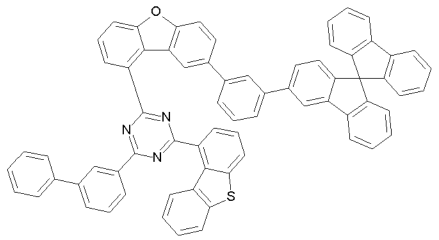 | 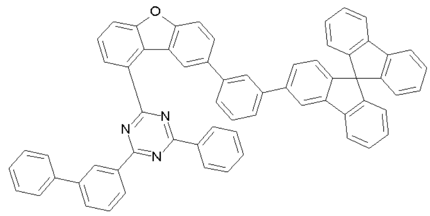 |
| 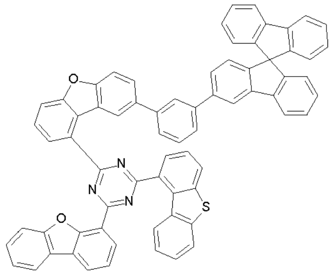 | 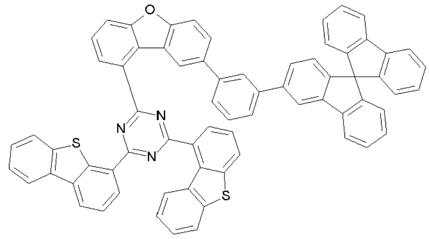 |
| 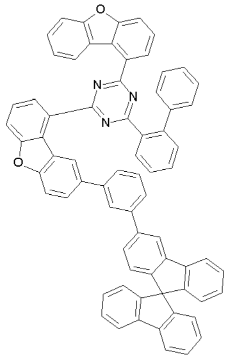 | 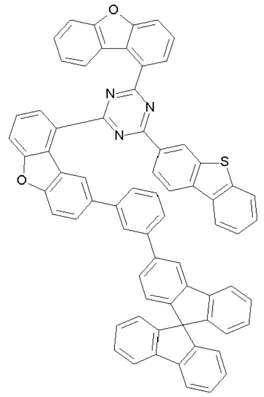 |
| 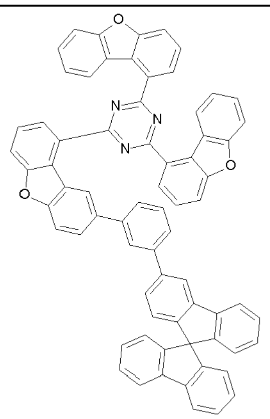 | 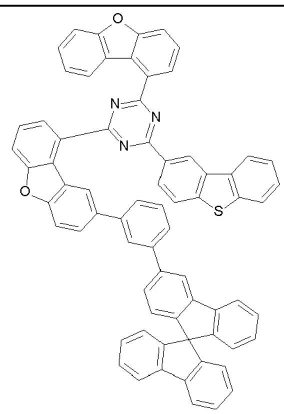 |
| 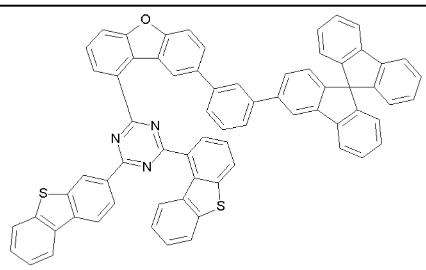 | 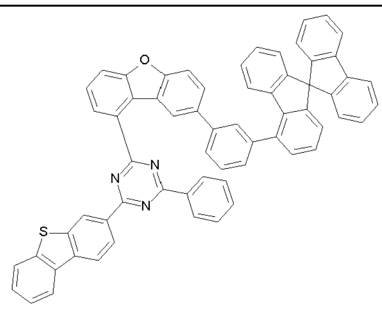 |

| | |
|---|---|
| 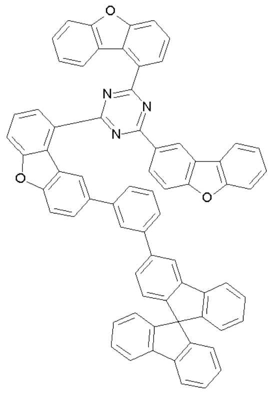 | 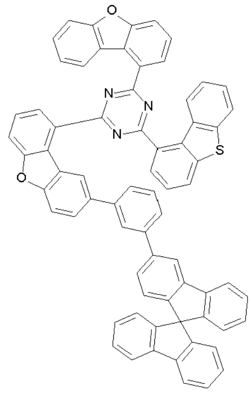 |
| 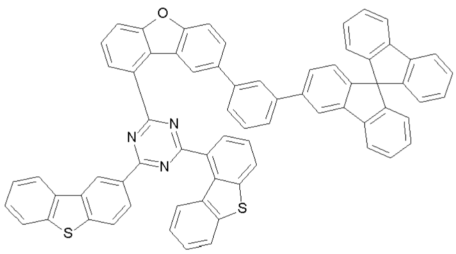 | 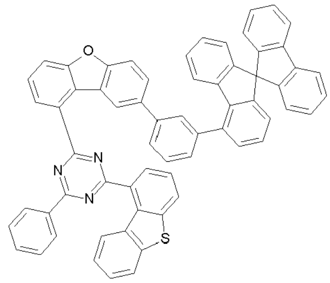 |
| 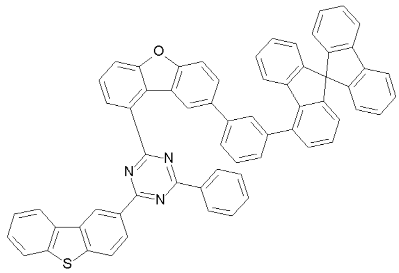 | 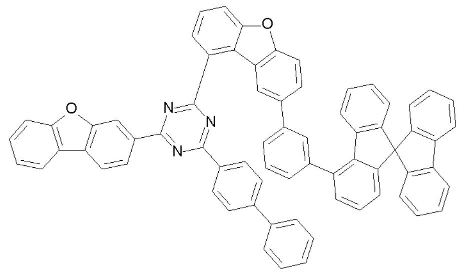 |
| 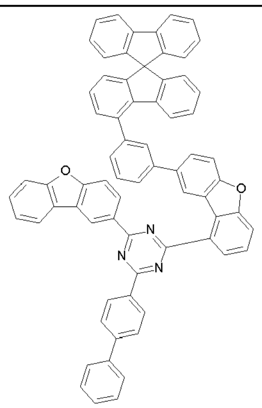 | 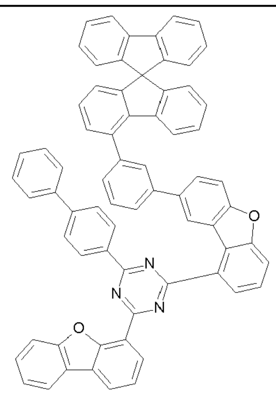 |
| 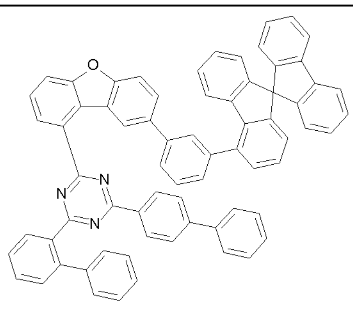 | 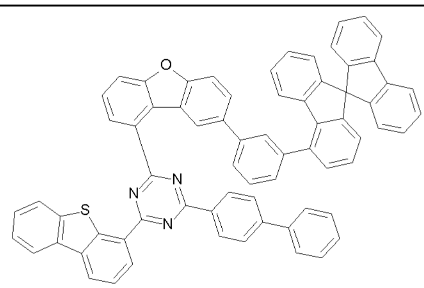 |

| | |
|---|---|
| 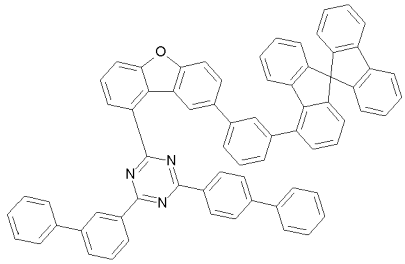 | 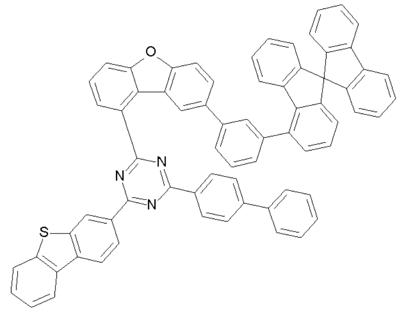 |
| 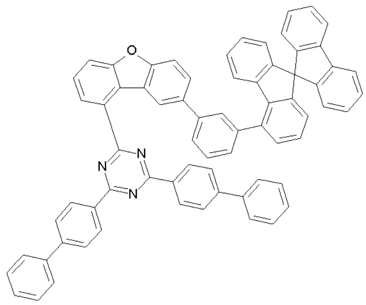 | 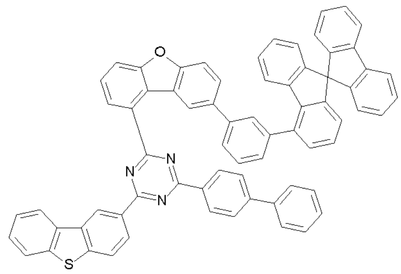 |
| 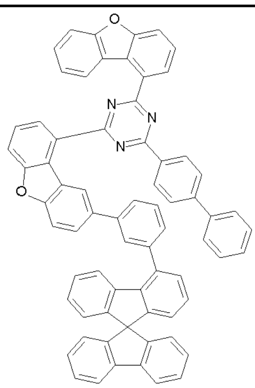 | 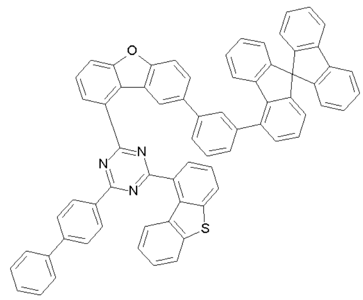 |
| 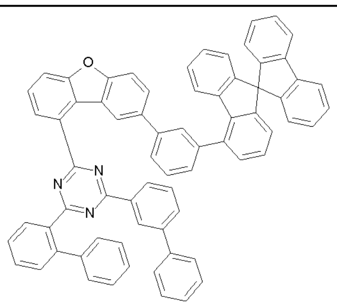 | 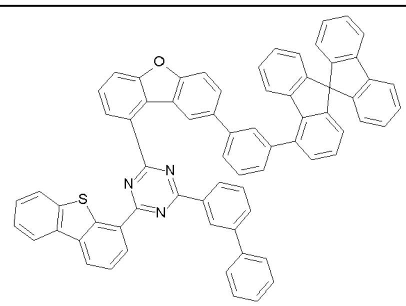 |
| 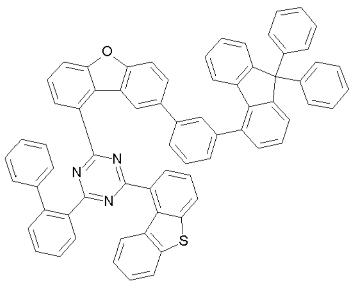 | 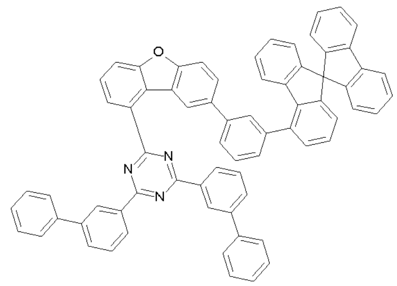 |

| | |
|---|---|
| 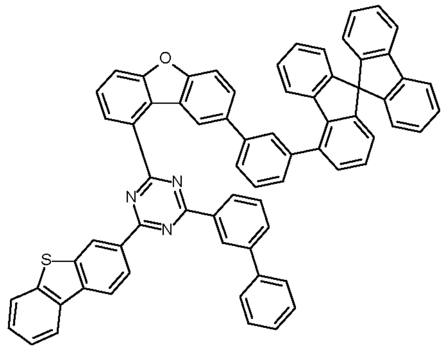 | 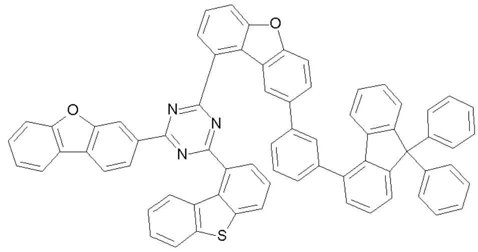 |
| 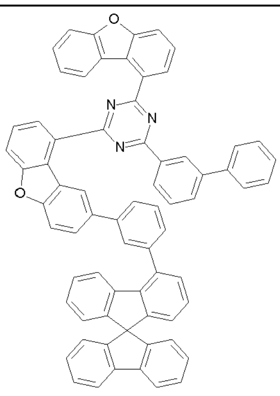 | 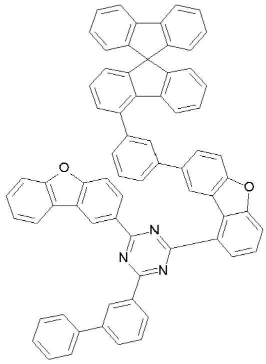 |
| 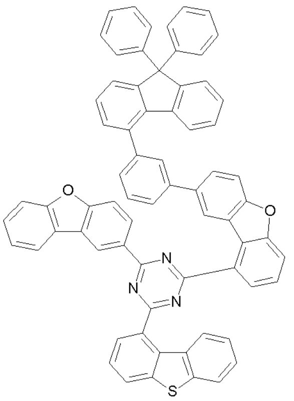 | 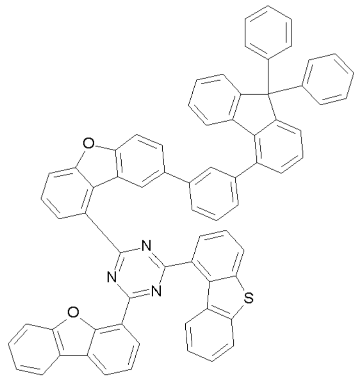 |
| 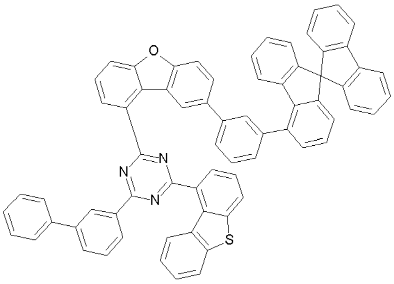 | 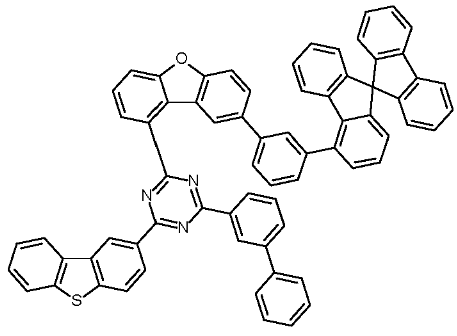 |

| | |
|---|---|
| 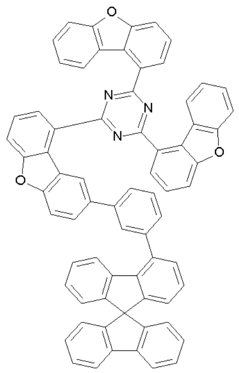 | 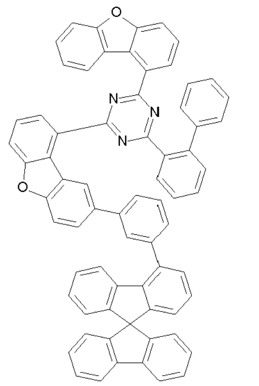 |
| 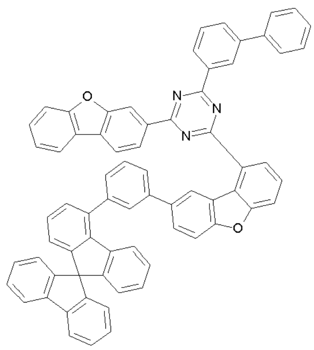 | 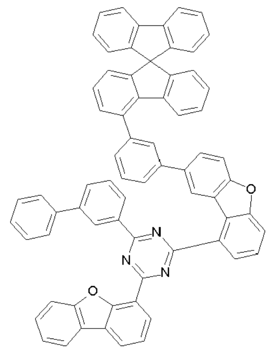 |
| 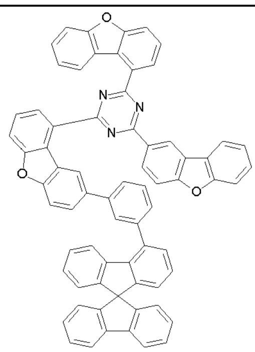 | 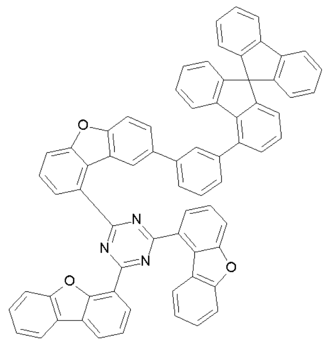 |
| 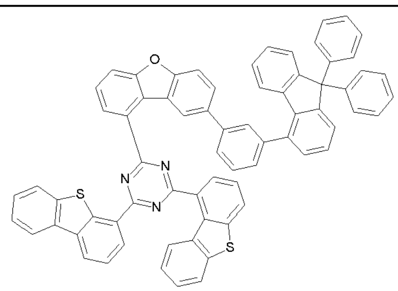 | 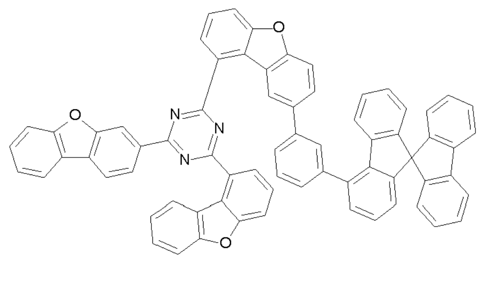 |
| 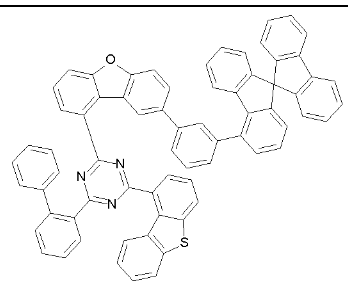 | 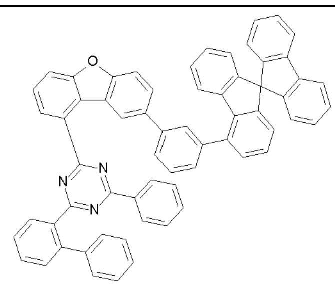 |

| | |
|---|---|
| 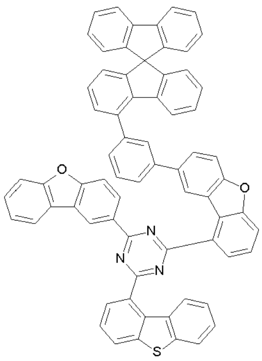 | 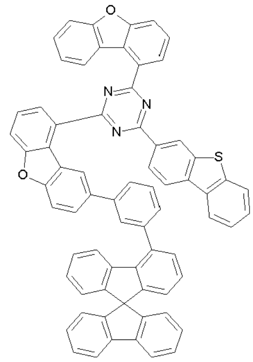 |
| 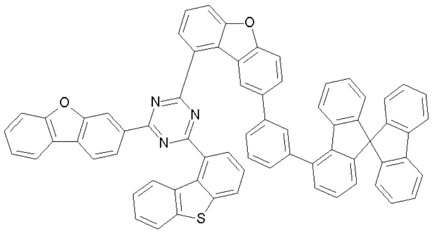 | 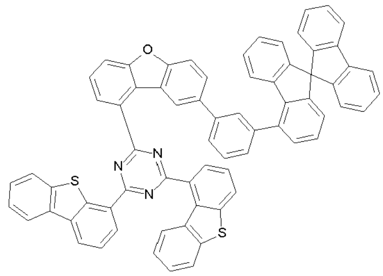 |
| 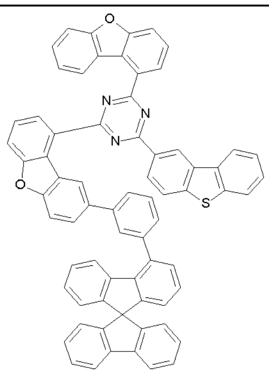 | 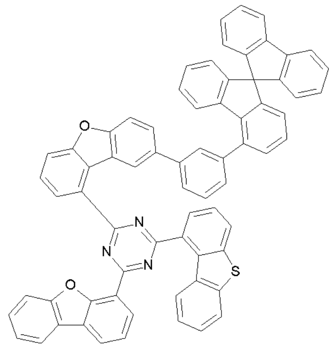 |
| 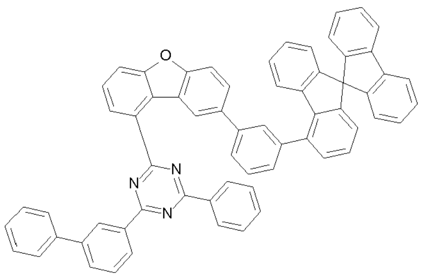 | 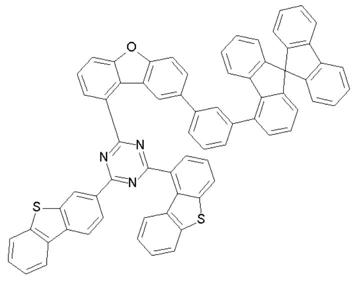 |
| 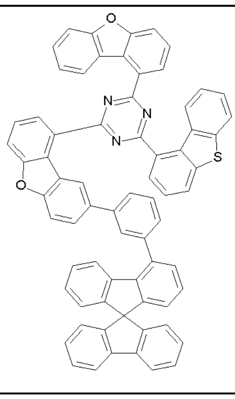 | 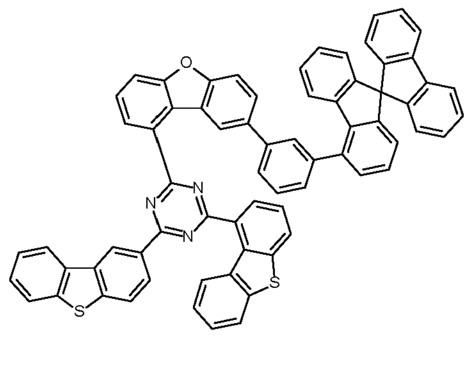 |

| | |
|---|---|
| 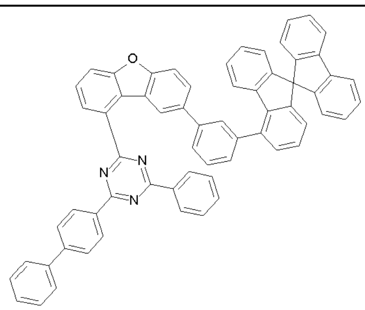 | 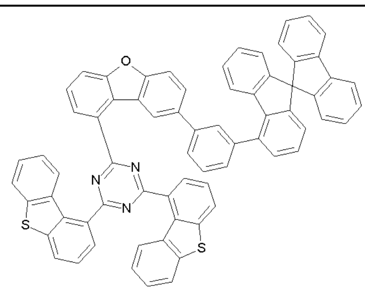 |
| 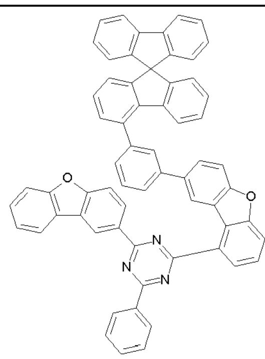 | 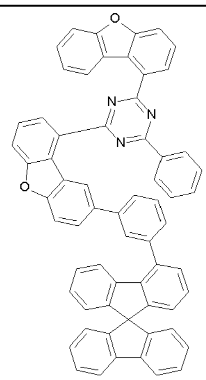 |
| 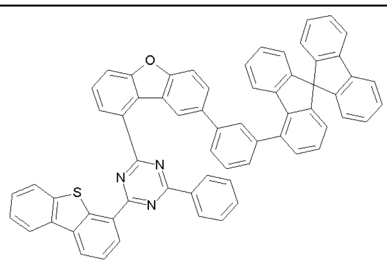 | 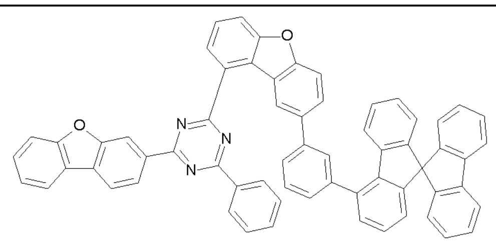 |
| 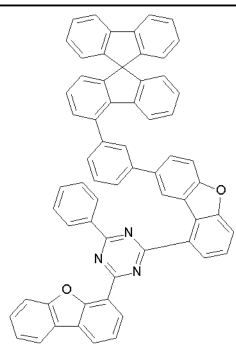 | 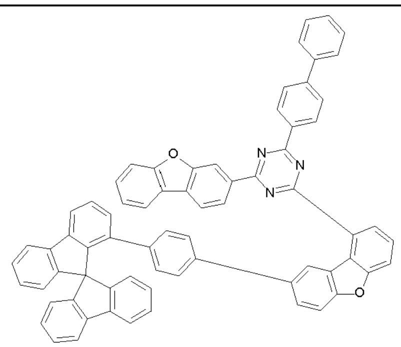 |
| 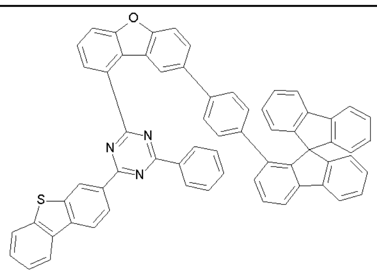 | 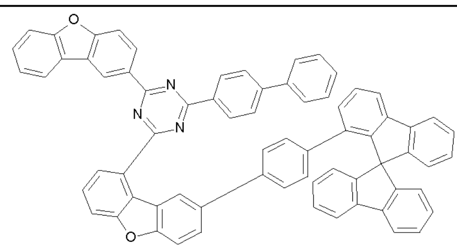 |

| | |
|---|---|
| 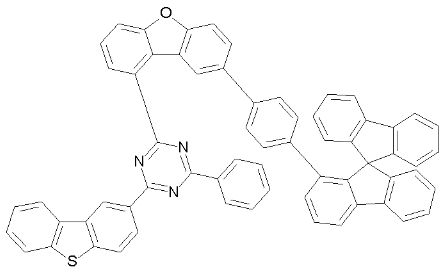 | 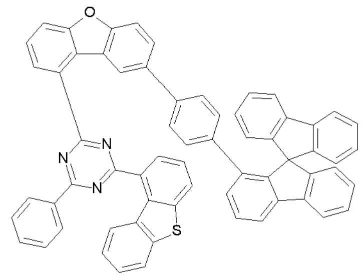 |
| 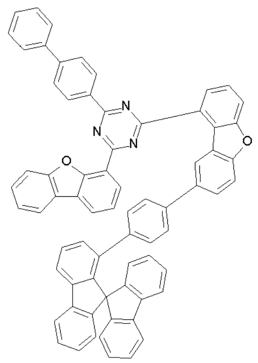 | 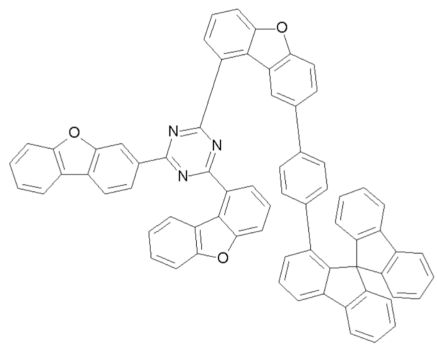 |
| 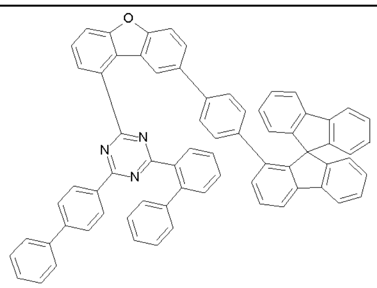 | 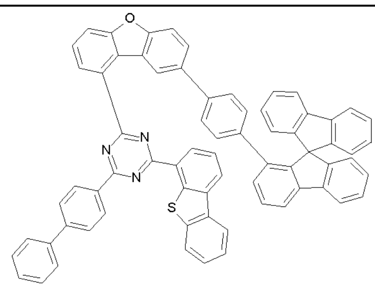 |
| 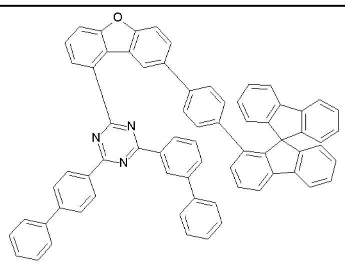 | 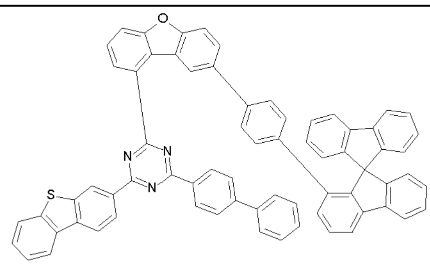 |
| 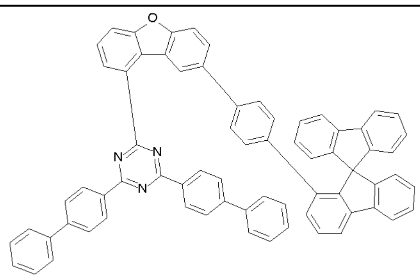 | 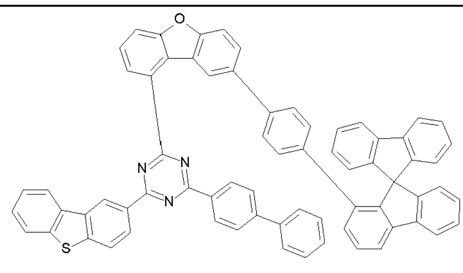 |

| | |
|---|---|
| 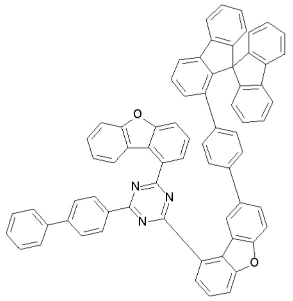 | 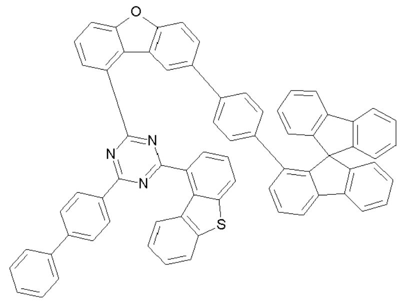 |
| 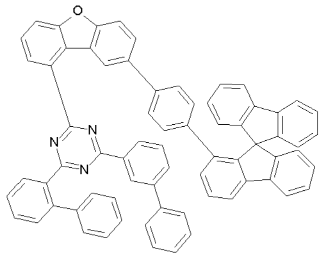 | 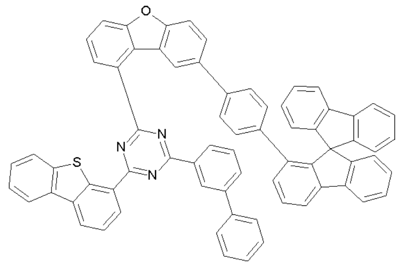 |
| 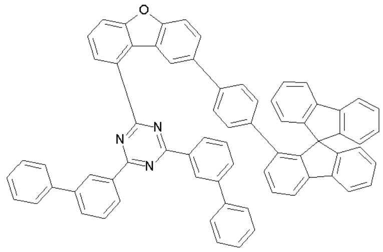 | 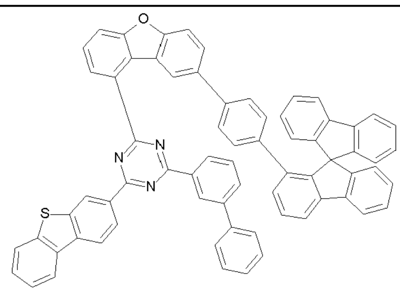 |
| 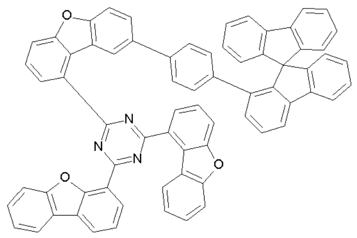 | 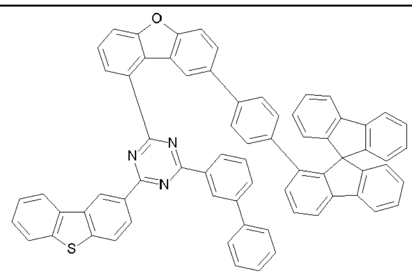 |
| 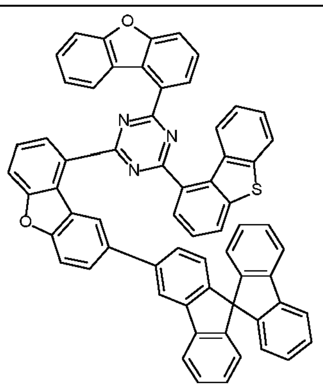 | 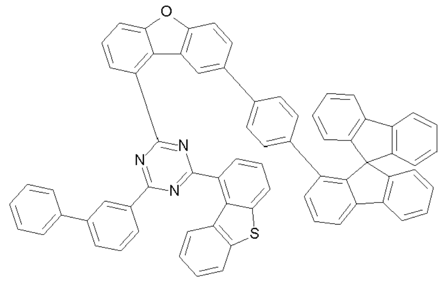 |

| | |
|---|---|
| 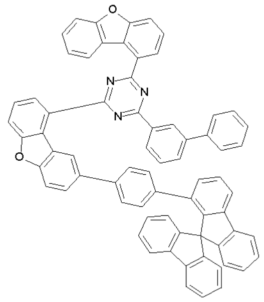 | 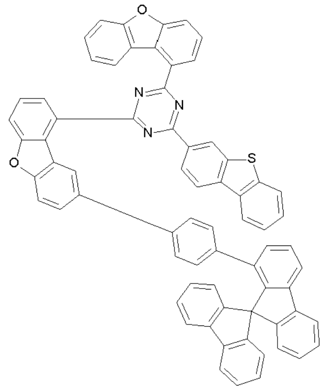 |
| 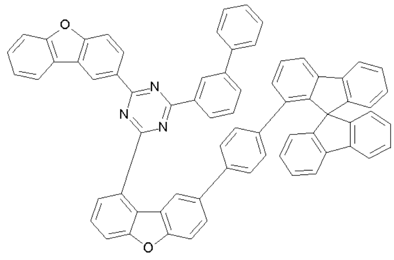 | 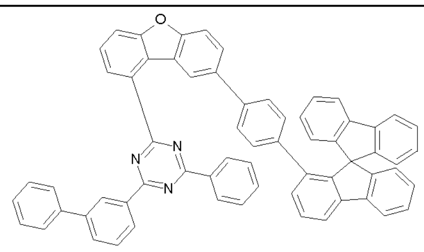 |
| 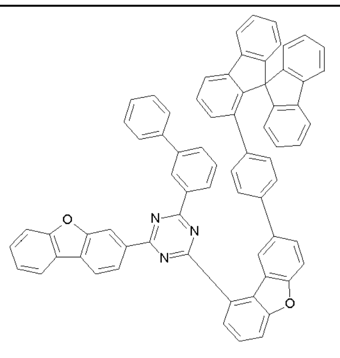 | 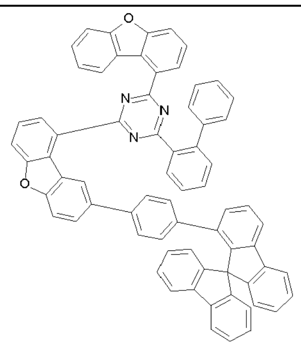 |
| 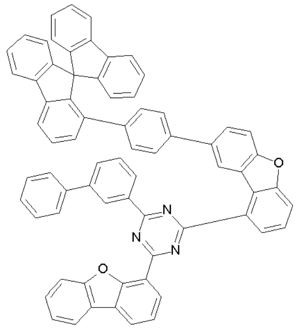 | 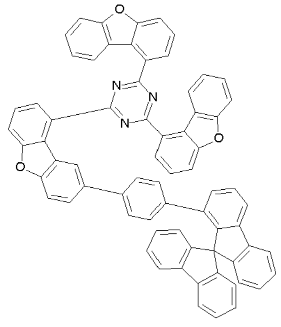 |
| 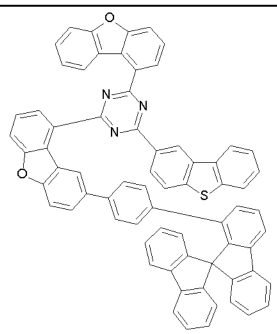 | 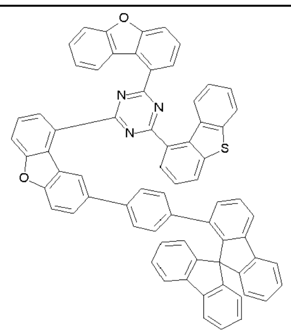 |

| | |
|---|---|
| 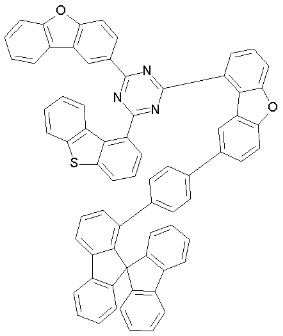 | 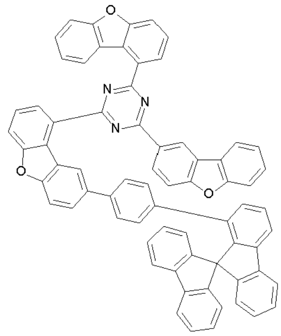 |
| 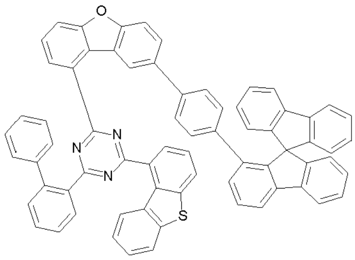 | 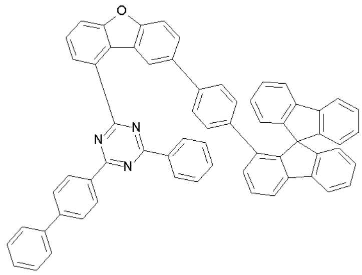 |
| 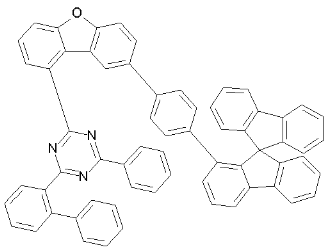 | 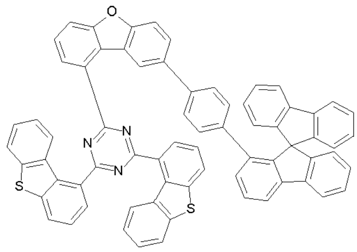 |
| 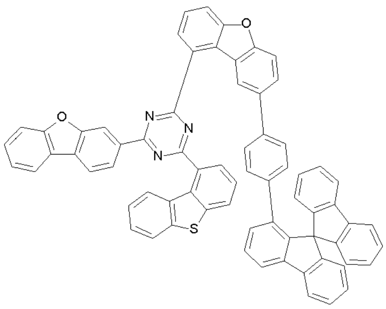 | 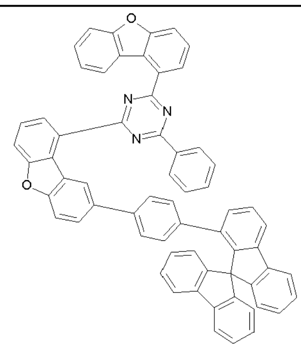 |
| 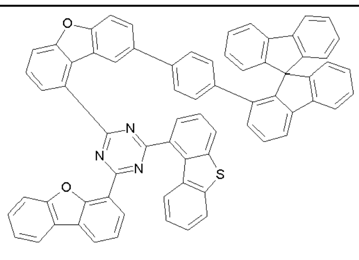 | 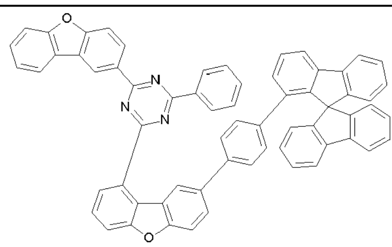 |

| | |
|---|---|
| 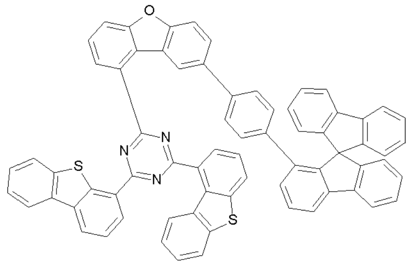 | 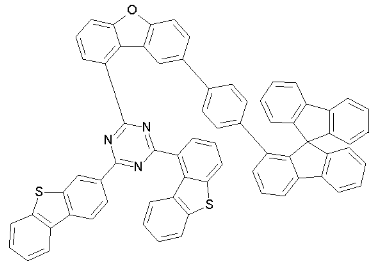 |
| 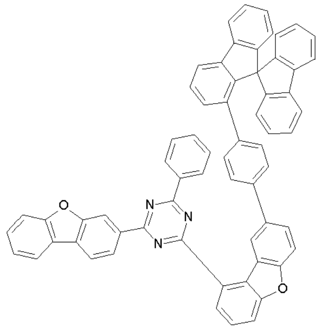 | 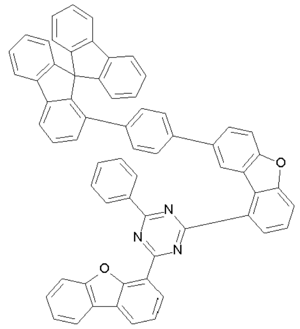 |
| 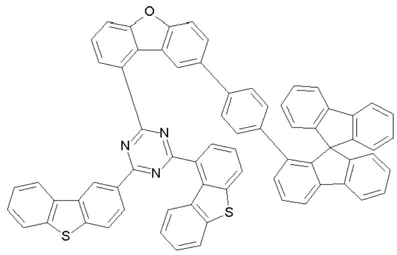 | 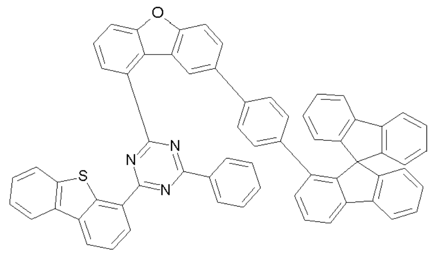 |
| 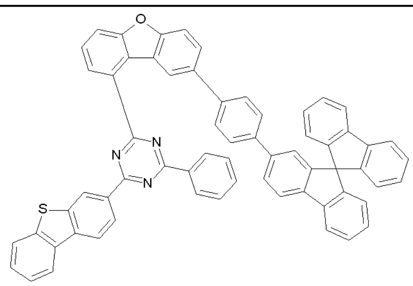 | 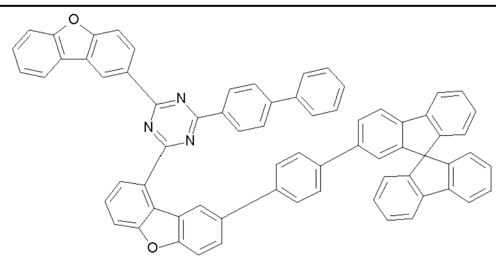 |
| 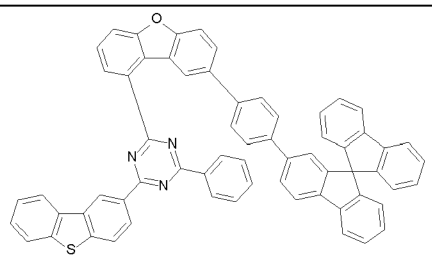 | 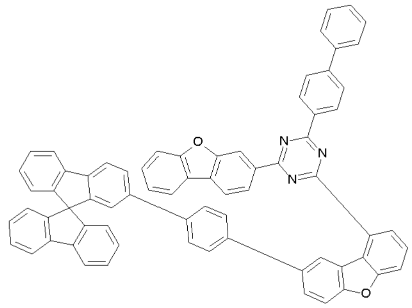 |

| | |
|---|---|
| 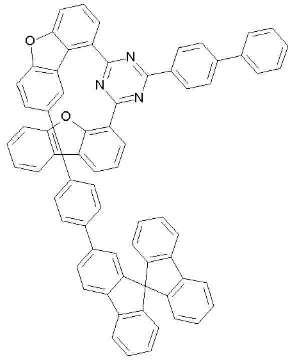 | 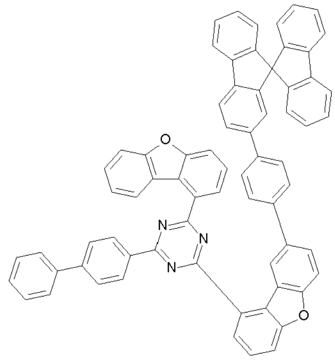 |
| 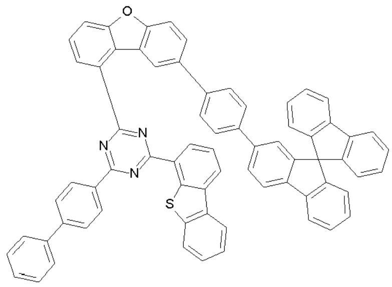 | 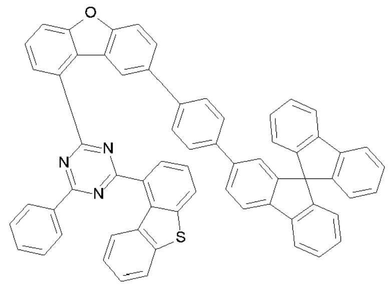 |
| 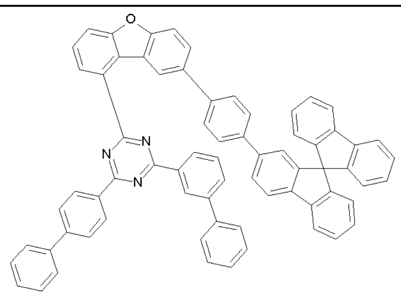 | 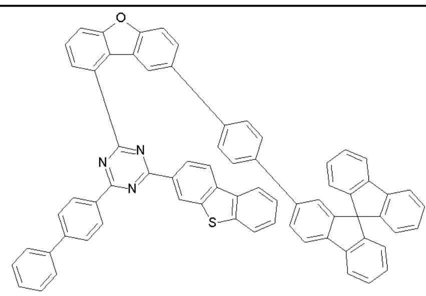 |
| 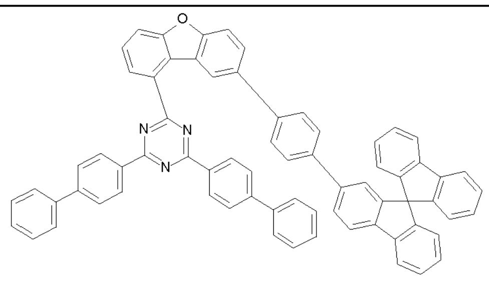 | 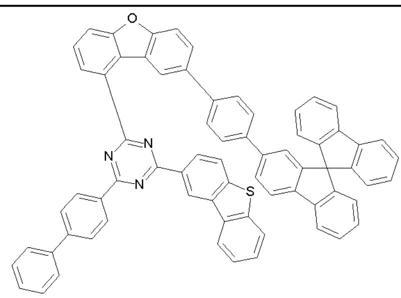 |
| 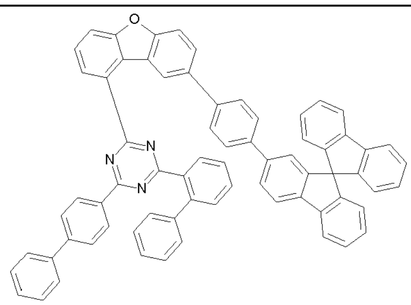 | 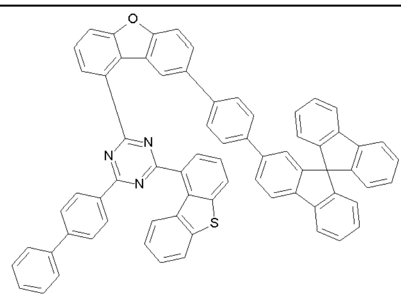 |

| | |
|---|---|
| 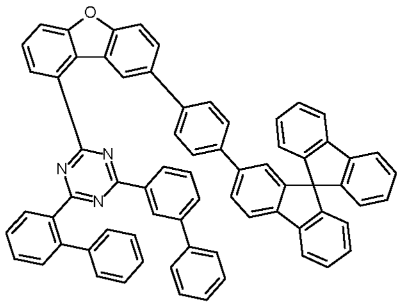 | 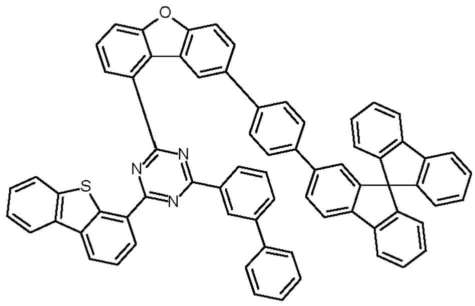 |
| 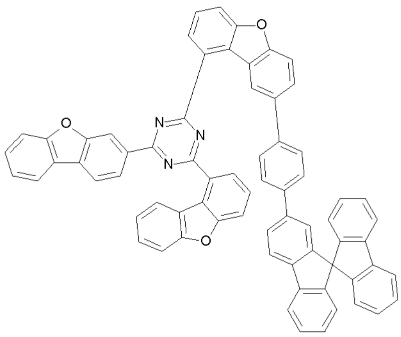 | 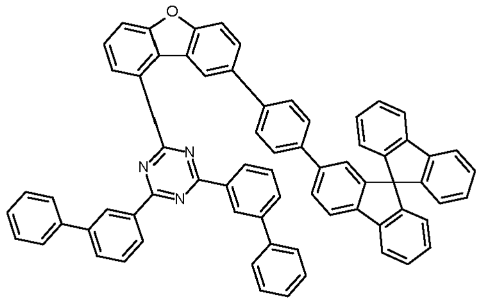 |
| 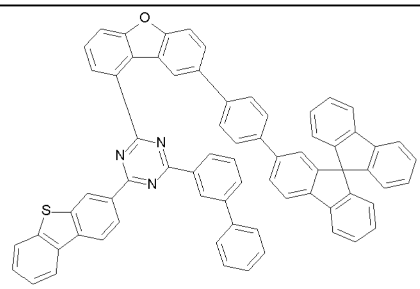 | 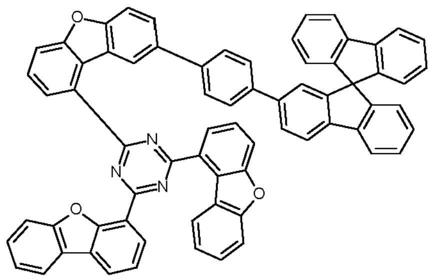 |
| 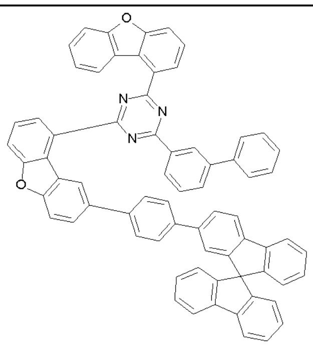 | 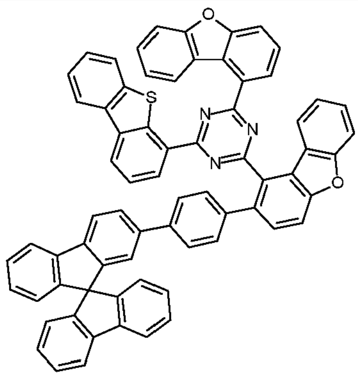 |
| 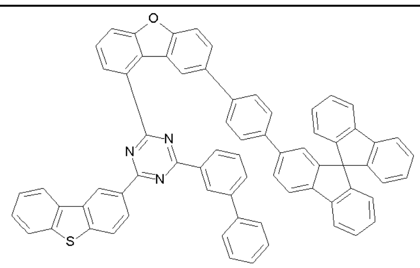 | 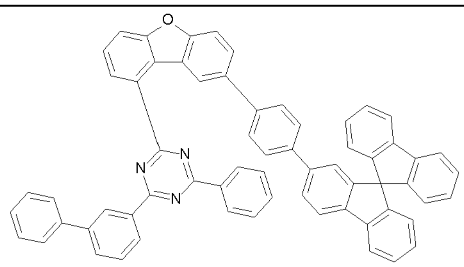 |

| | |
|---|---|
| 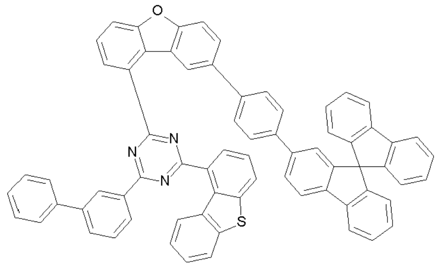 | 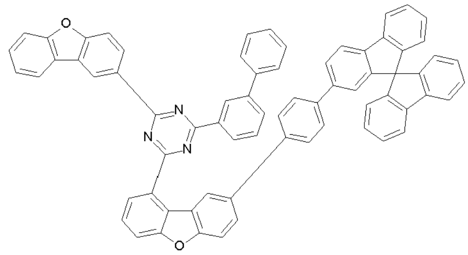 |
| 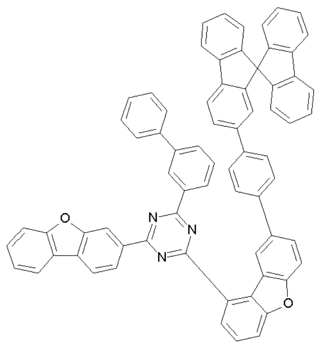 | 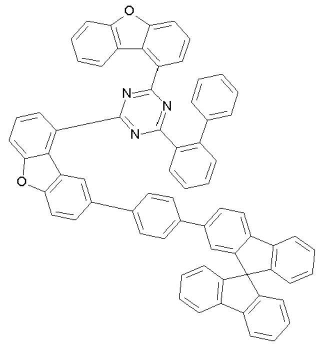 |
| 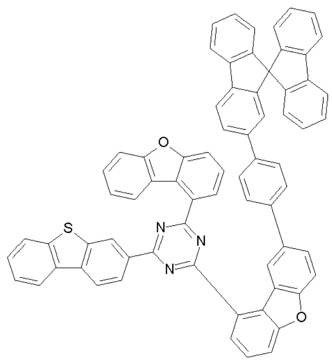 | 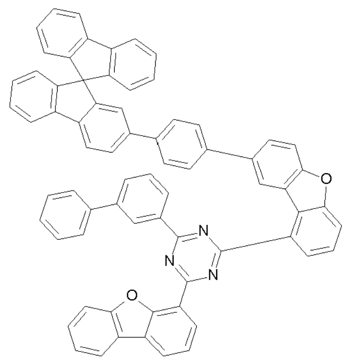 |
| 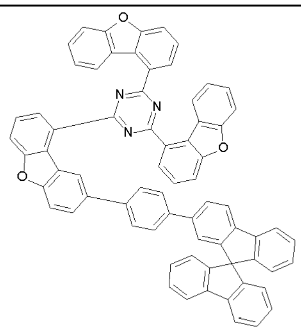 | 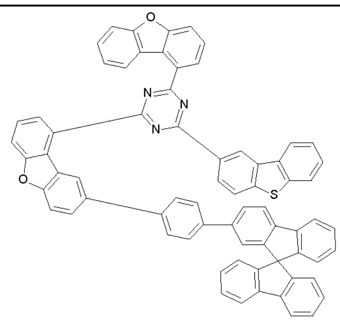 |
| 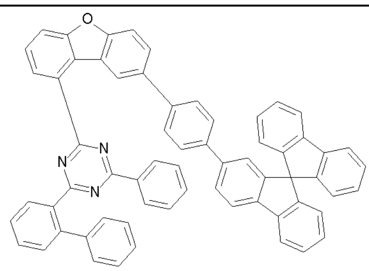 | 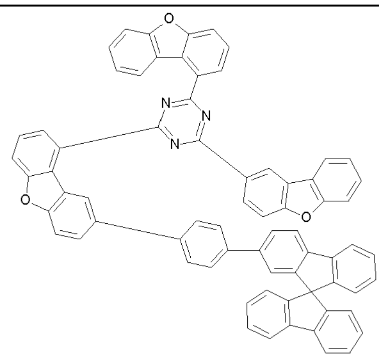 |

| | |
|---|---|
| 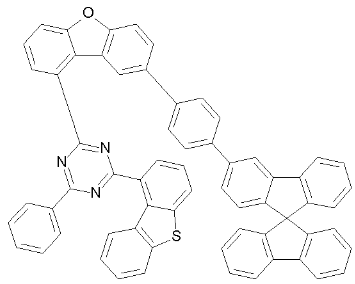 | 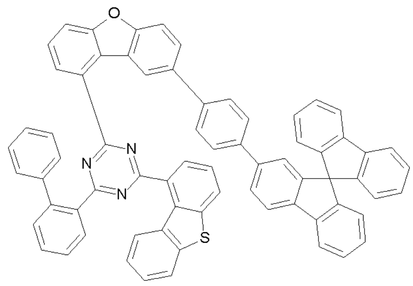 |
| 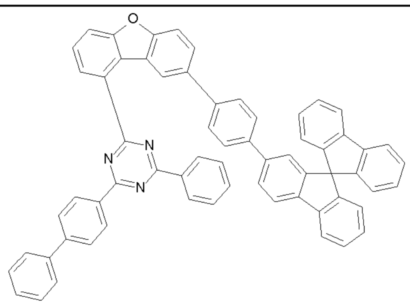 | 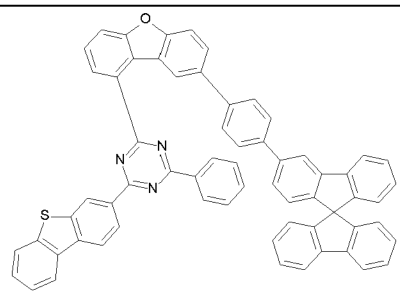 |
| 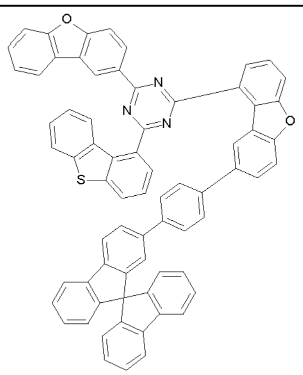 | 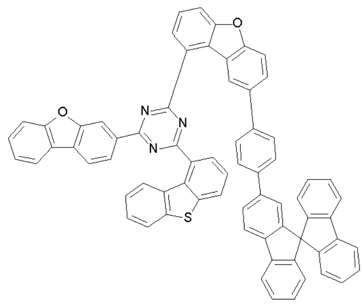 |
| 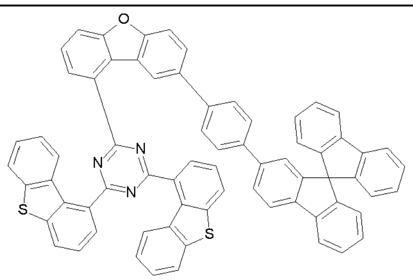 | 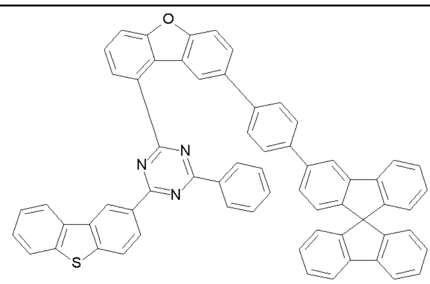 |
| 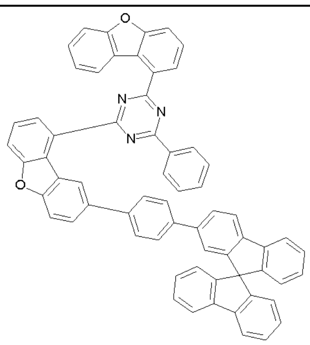 | 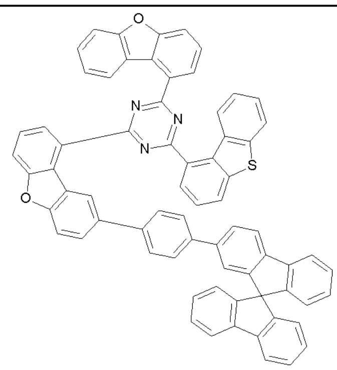 |

| | |
|---|---|
| 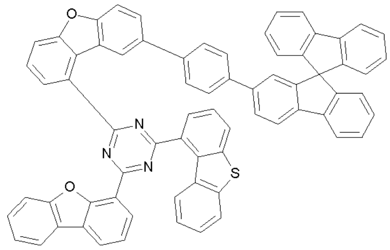 | 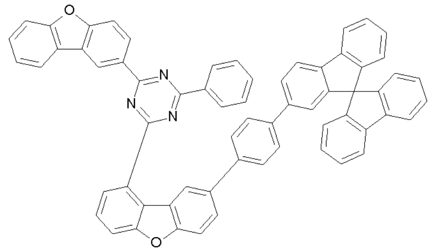 |
| 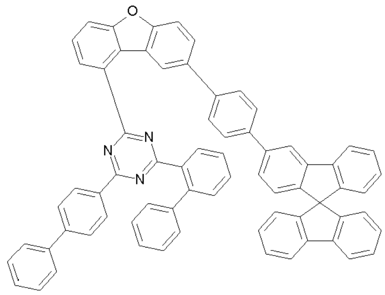 | 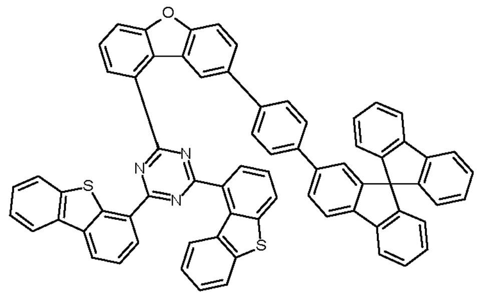 |
| 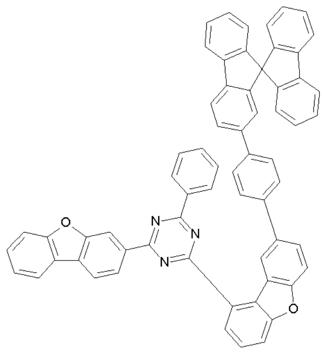 | 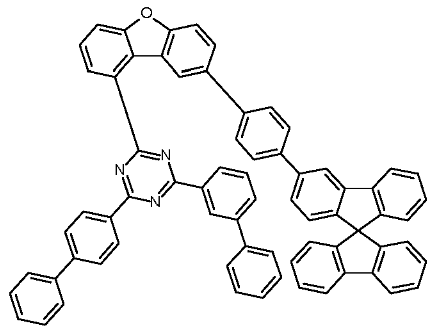 |
| 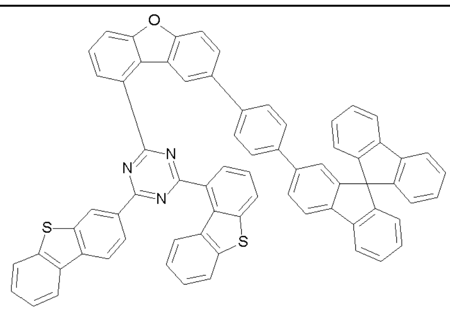 | 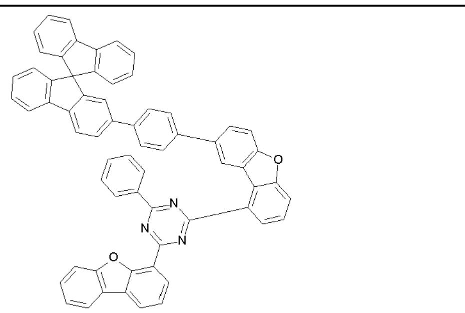 |
| 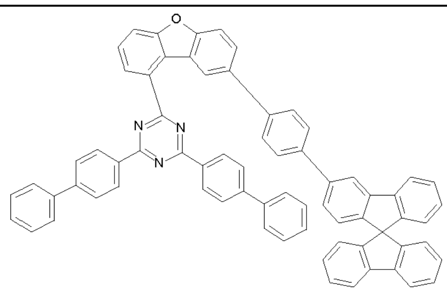 | 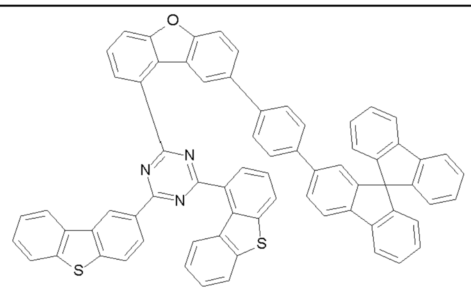 |

| | |
|---|---|
| 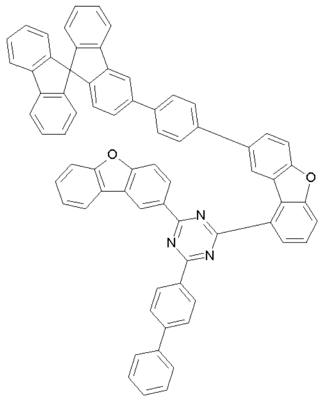 | 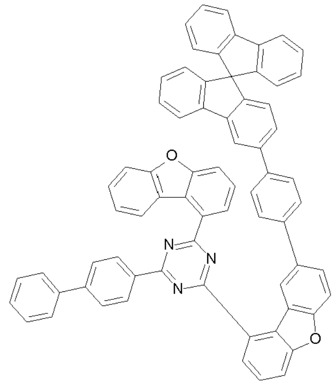 |
| 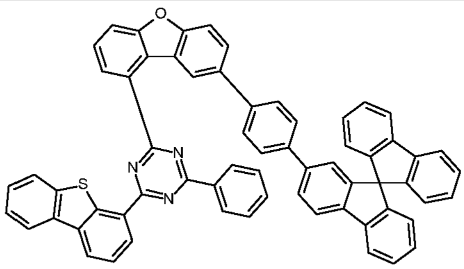 | 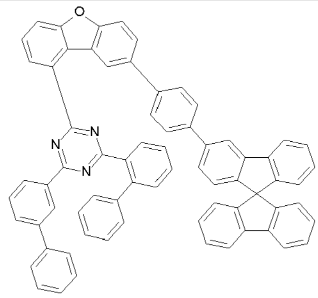 |
| 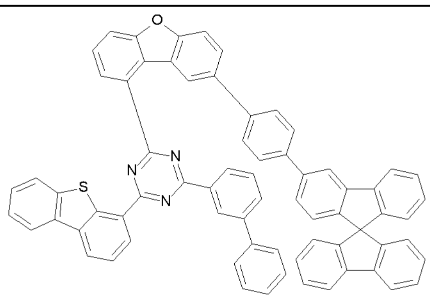 | 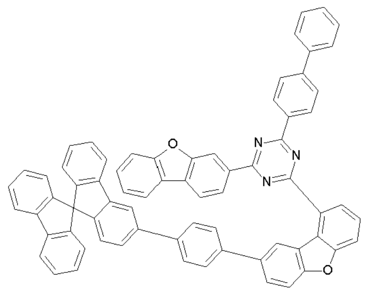 |
| 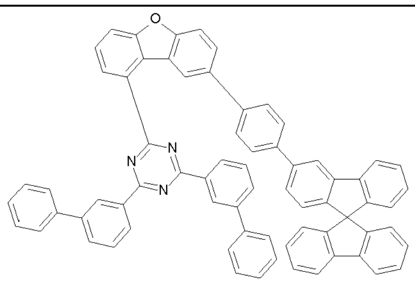 | 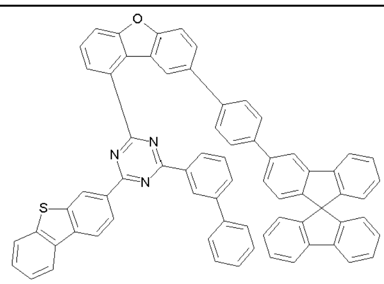 |
| 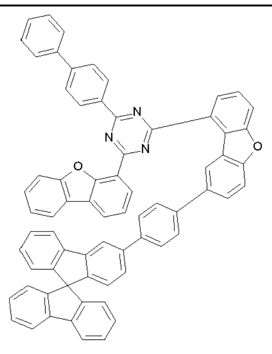 | 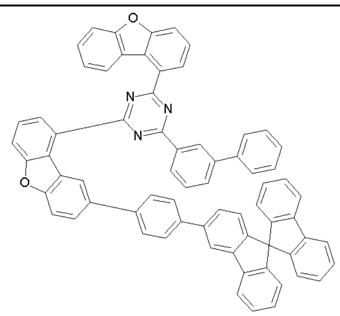 |

| | |
|---|---|
| 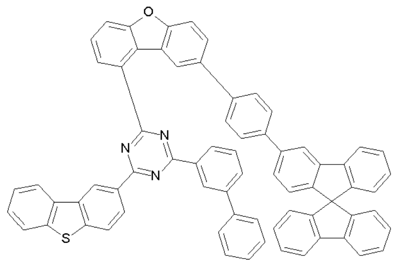 | 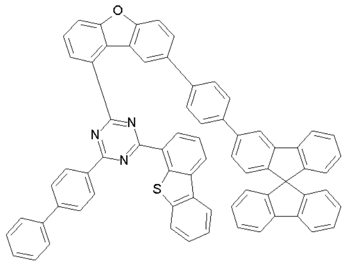 |
| 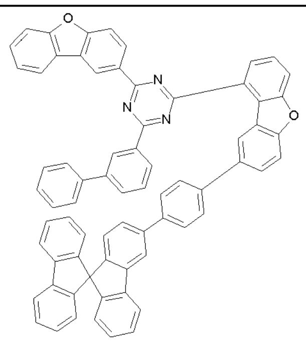 | 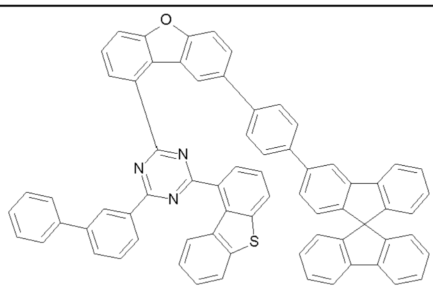 |
| 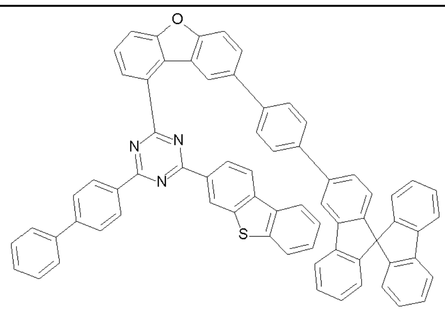 | 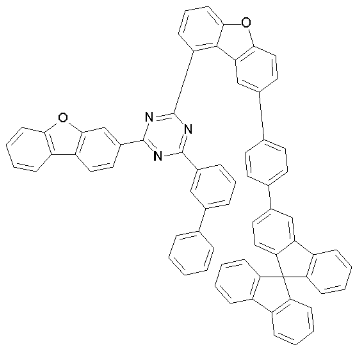 |
| 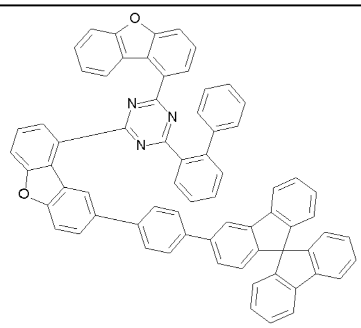 | 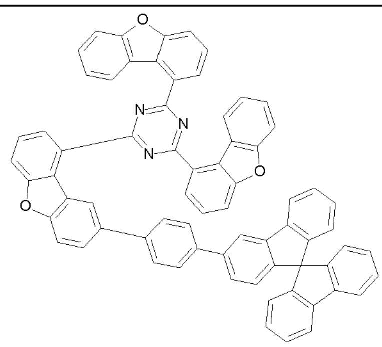 |
| 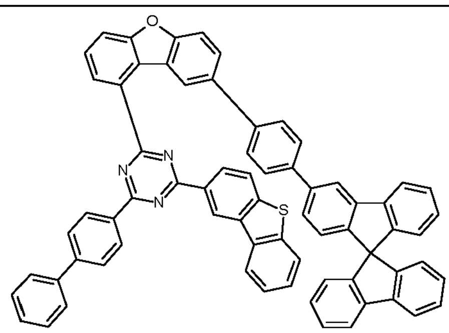 | 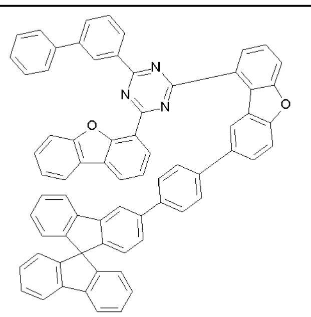 |

| | |
|---|---|
| 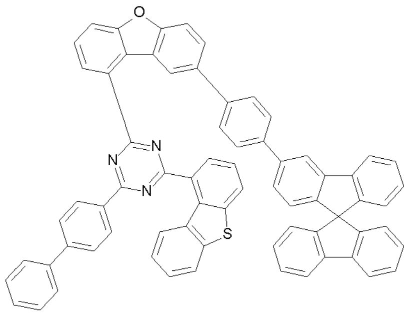 | 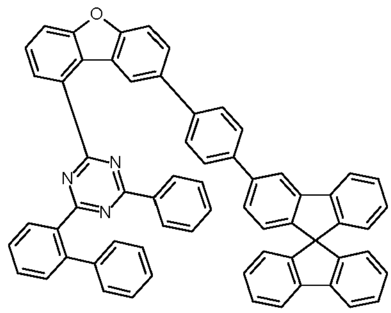 |
| 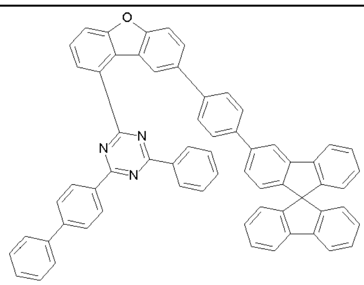 | 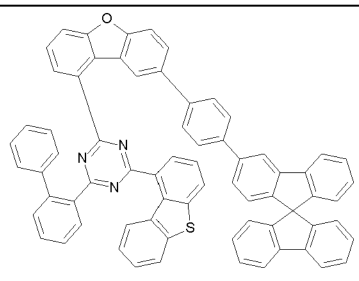 |
| 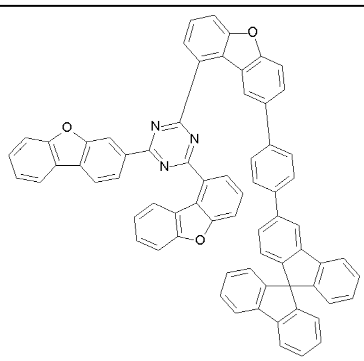 | 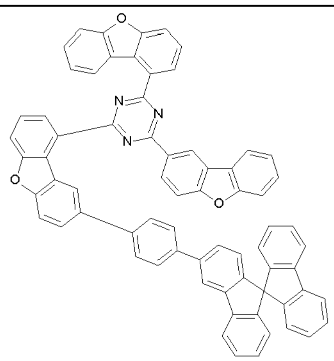 |
| 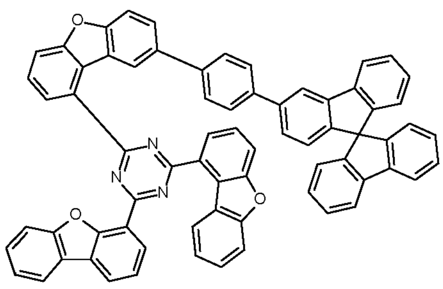 | 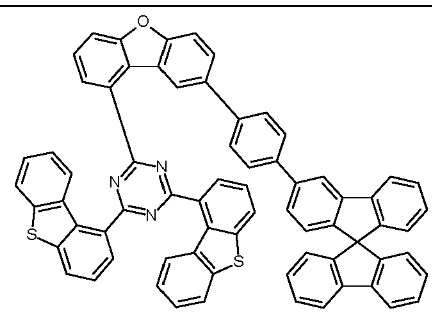 |
| 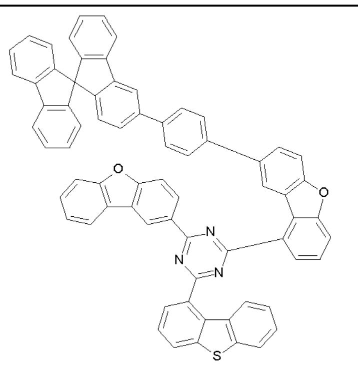 | 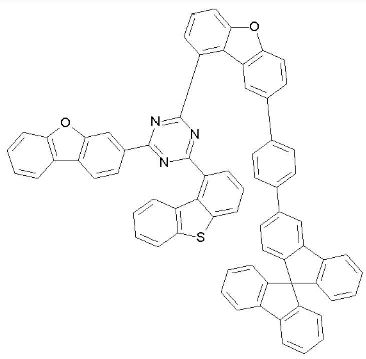 |

| | |
|---|---|
| 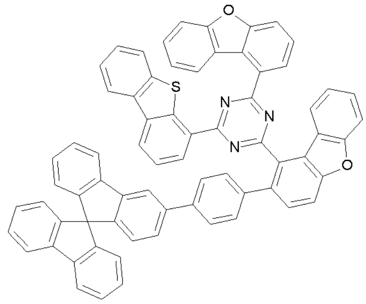 | 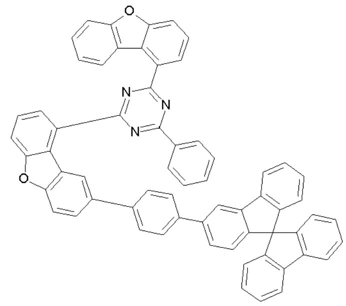 |
| 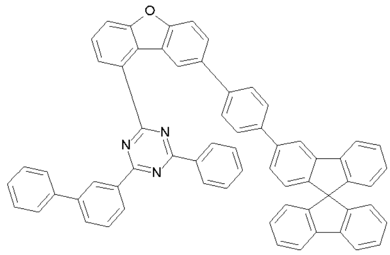 | 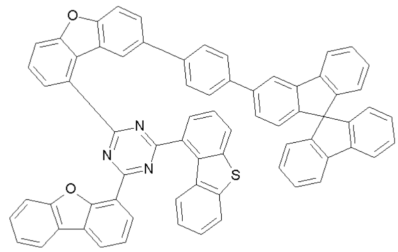 |
| 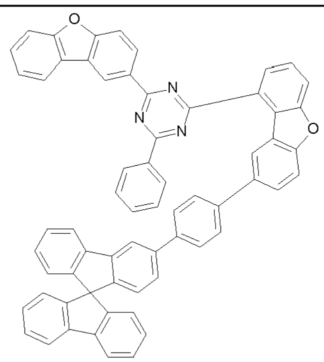 | 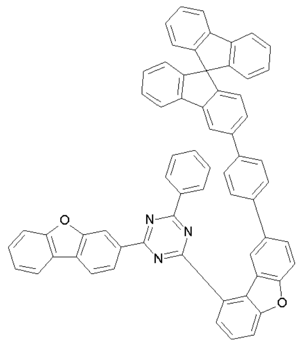 |
| 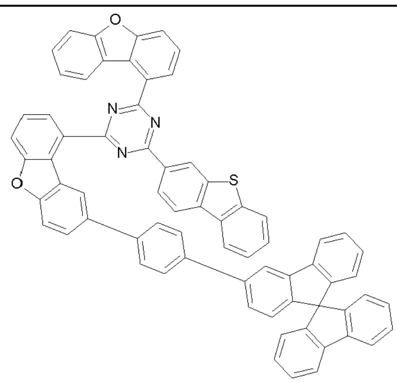 | 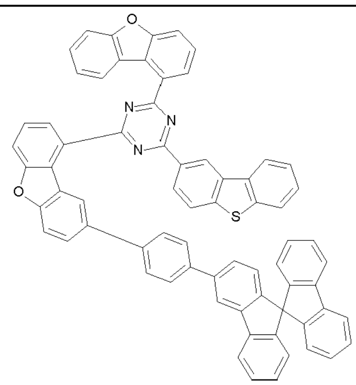 |
| 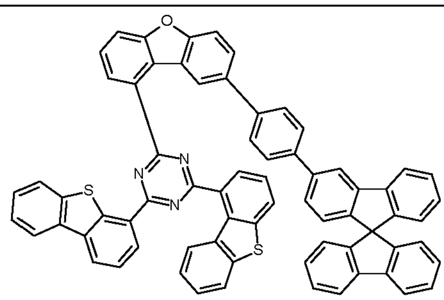 | 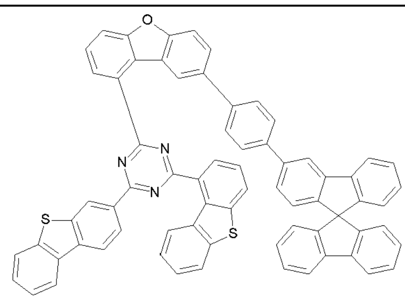 |

| | |
|---|---|
| 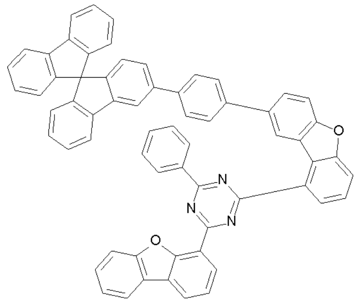 | 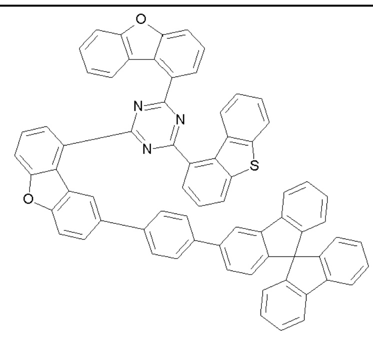 |
| 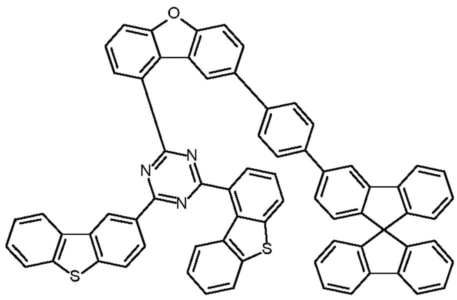 | 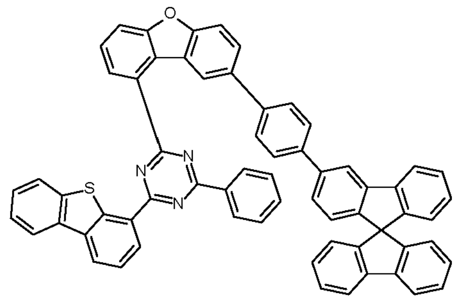 |
| 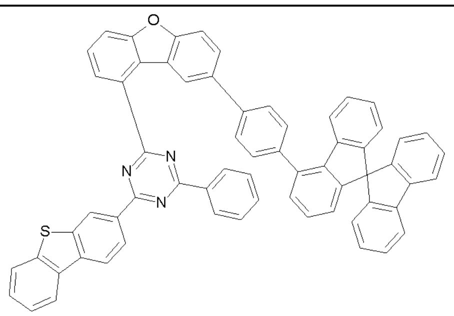 | 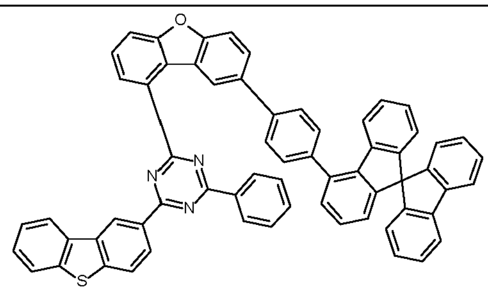 |
| 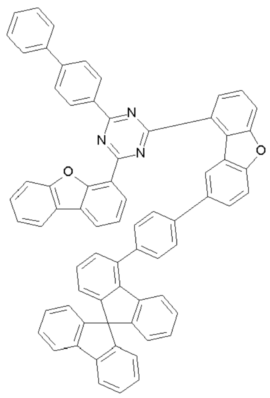 | 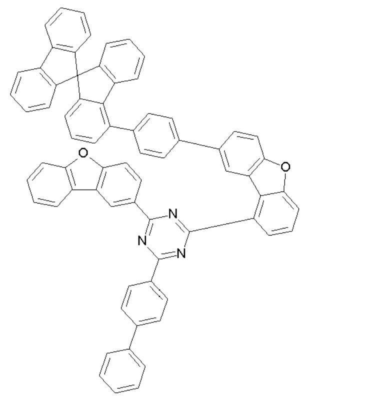 |

| | |
|---|---|
| 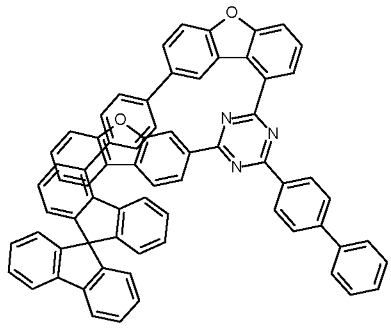 | 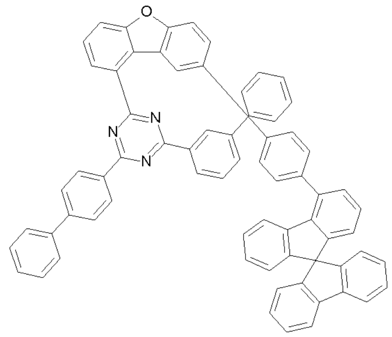 |
| 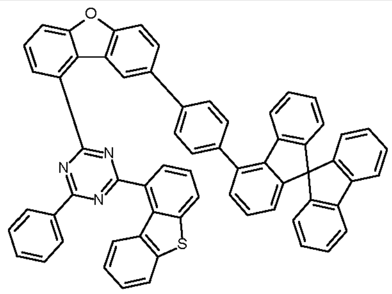 | 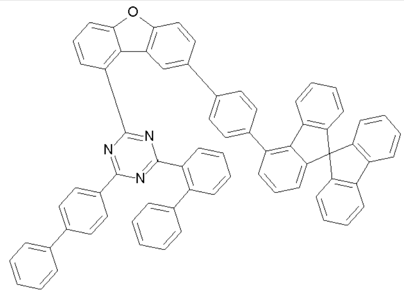 |
| 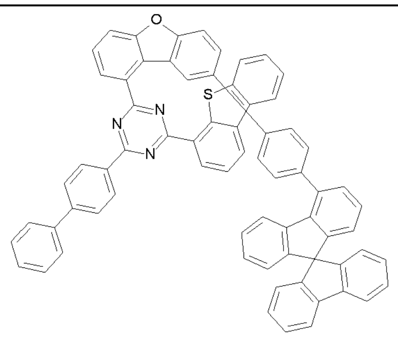 | 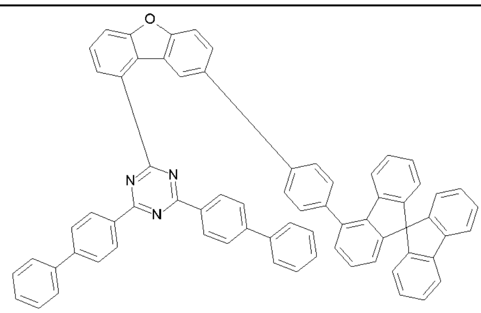 |
| 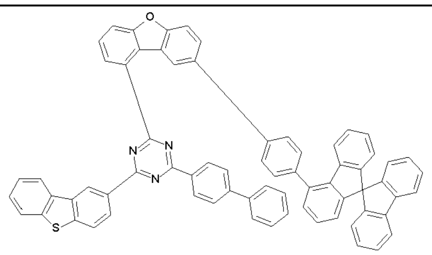 | 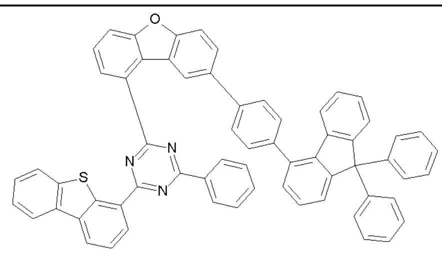 |
| 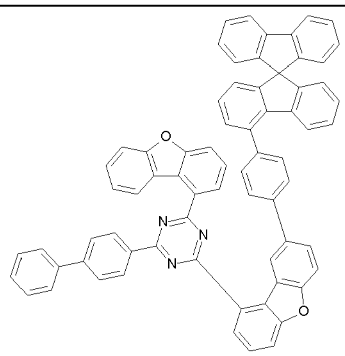 | 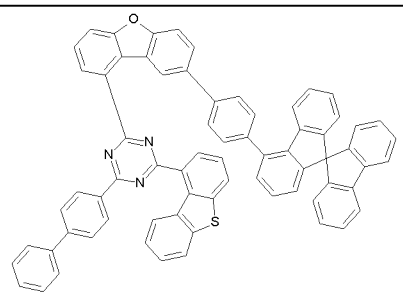 |

| | |
|---|---|
| 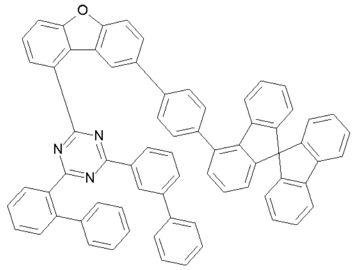 | 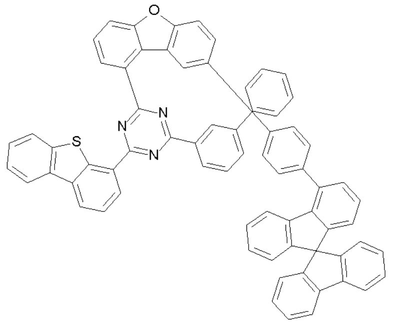 |
| 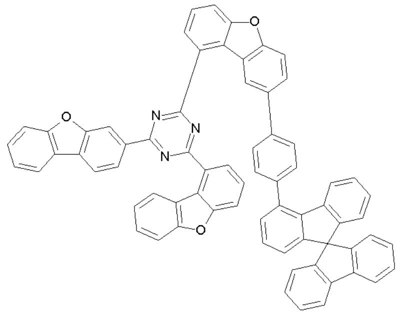 | 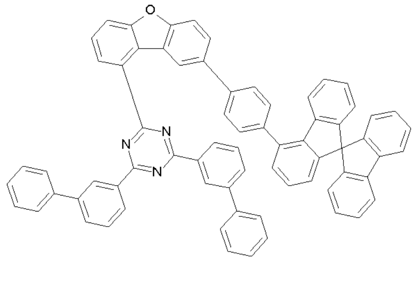 |
| 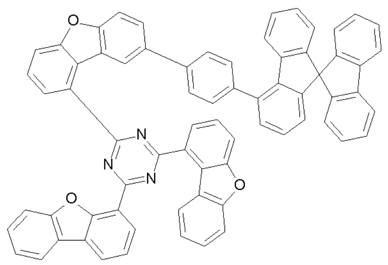 | 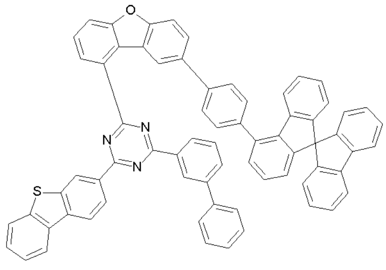 |
| 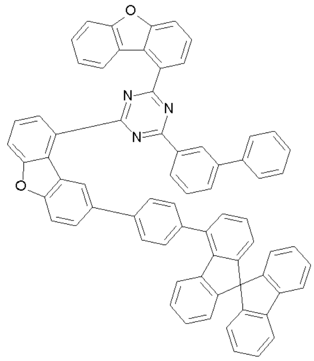 | 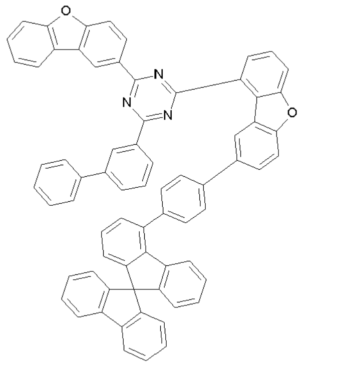 |
| 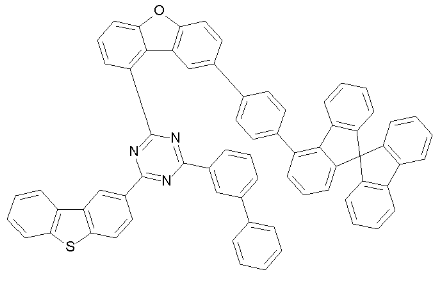 | 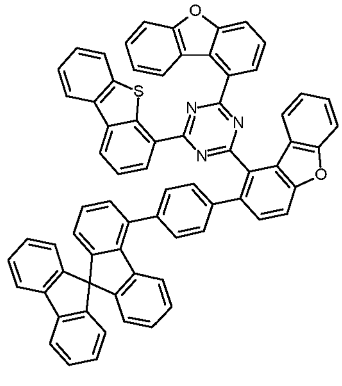 |

| | |
|---|---|
| 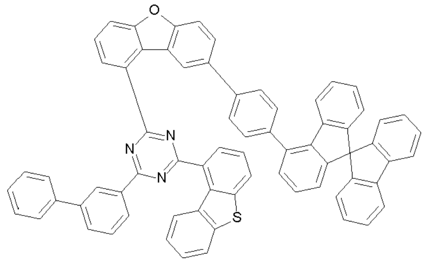 | 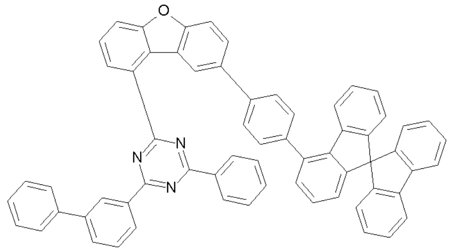 |
| 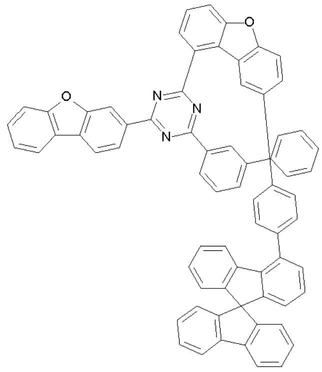 | 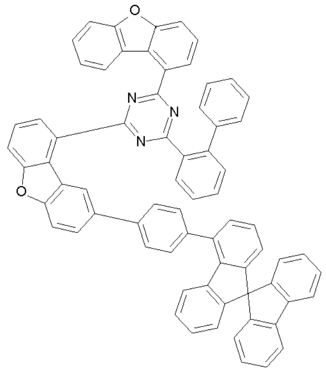 |
| 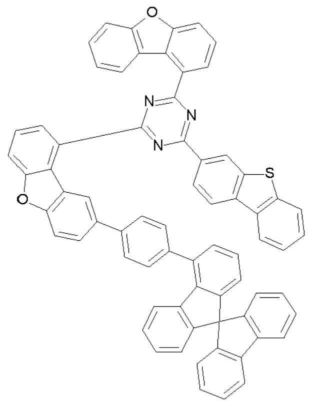 | 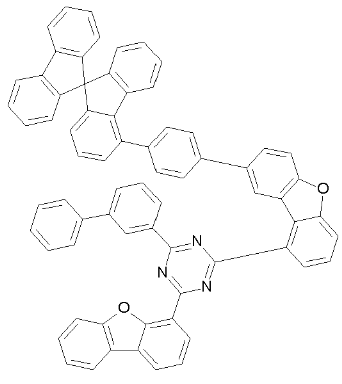 |
| 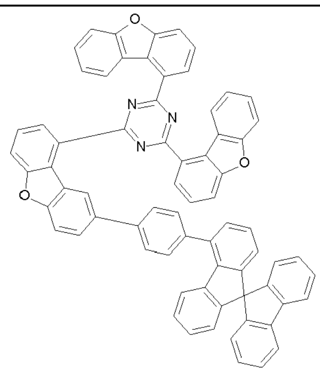 | 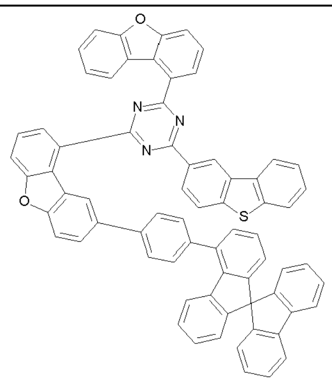 |

| | |
|---|---|
| 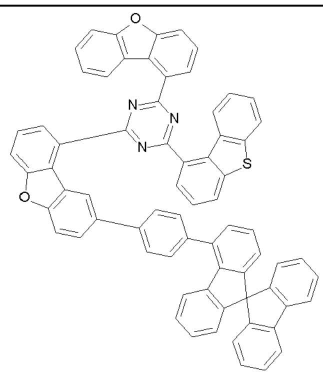 | 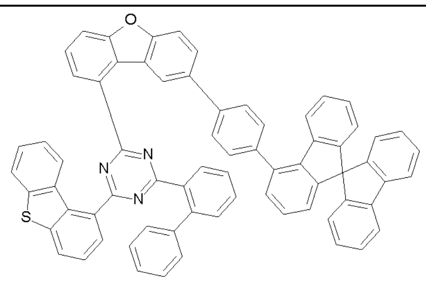 |
| 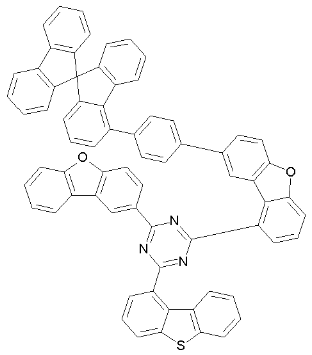 | 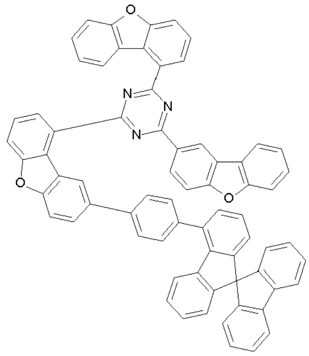 |
| 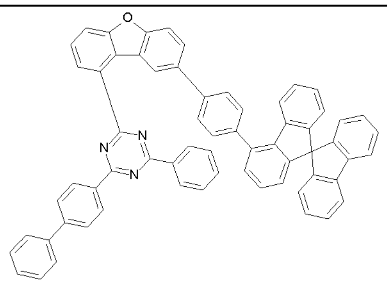 | 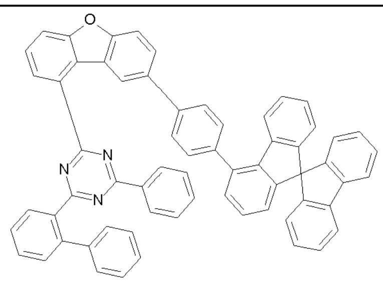 |
| 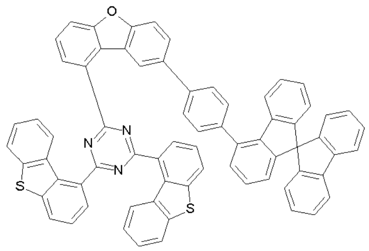 | 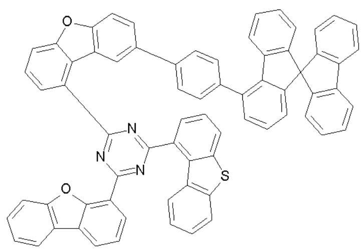 |
| 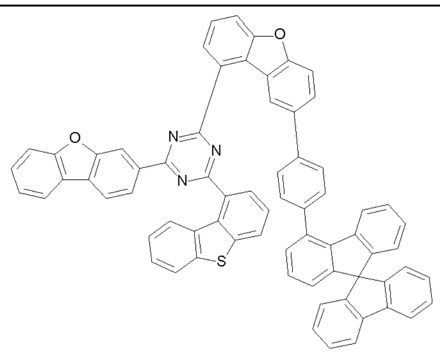 | 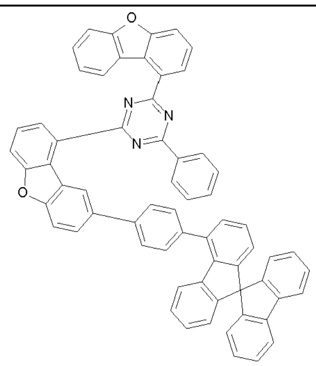 |

| | |
|---|---|
| 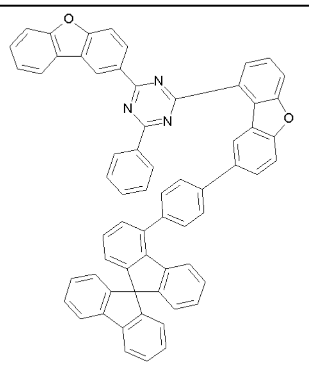 | 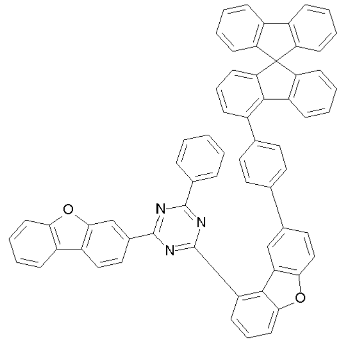 |
| 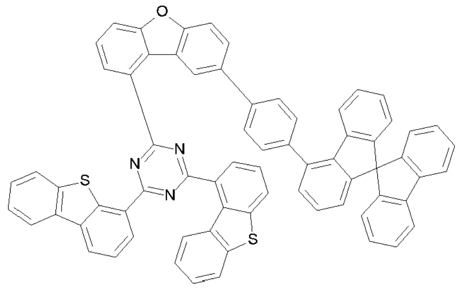 | 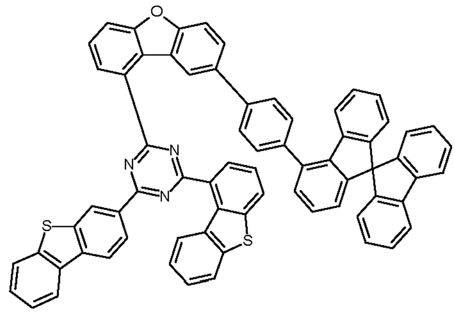 |
| 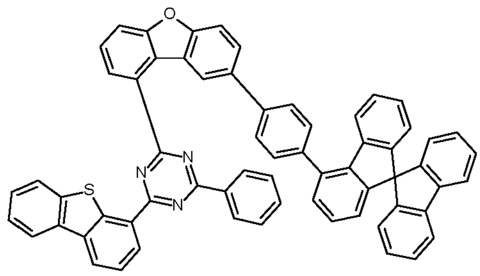 | 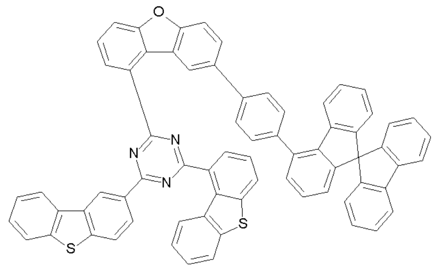 |
| 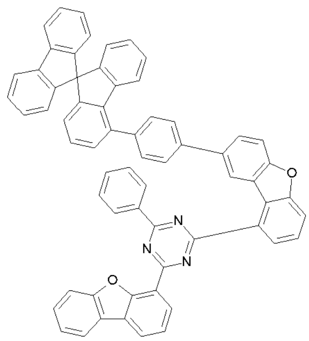 | 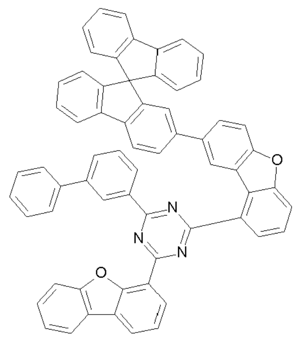 |
| 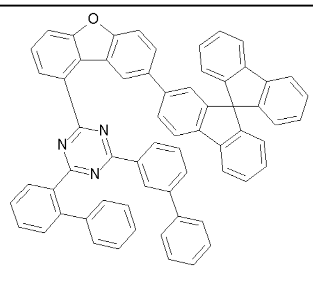 | 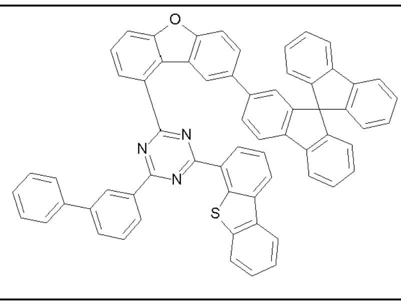 |

| | |
|---|---|
| 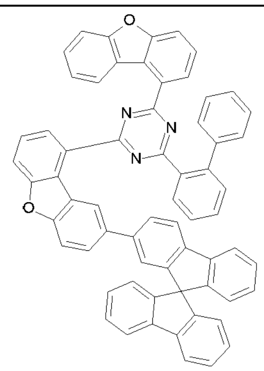 | 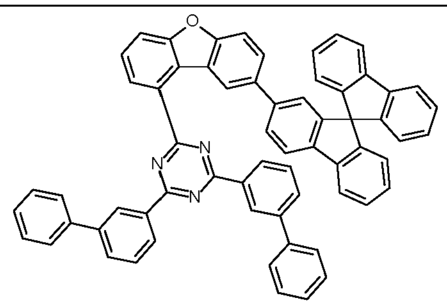 |
| 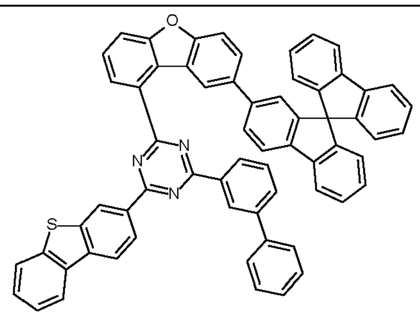 | 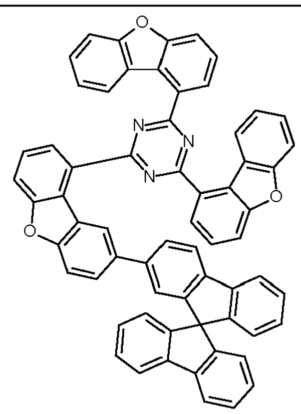 |
| 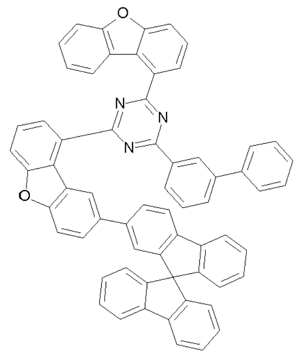 | 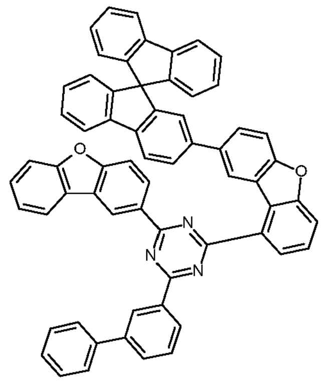 |
| 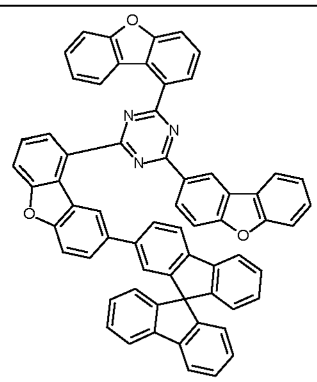 | 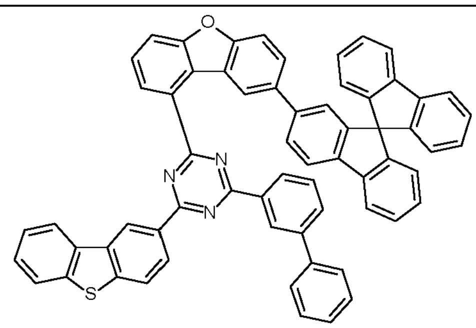 |

| | |
|---|---|
| 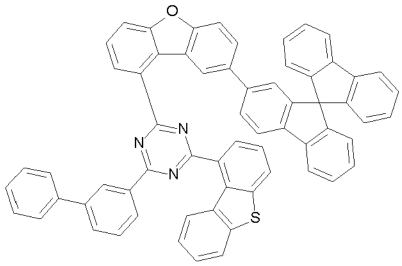 | 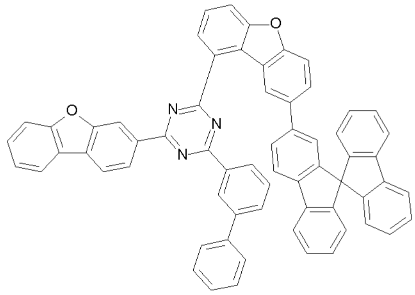 |
| 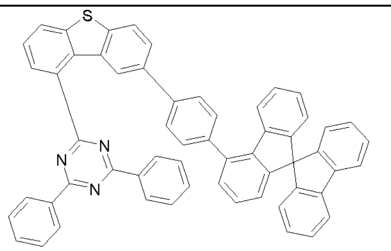 | 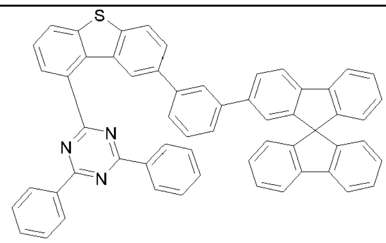 |
| 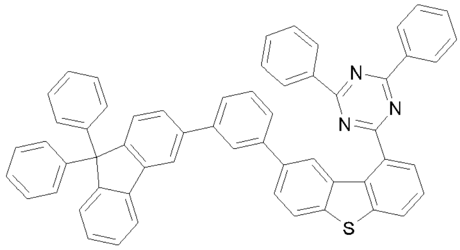 | 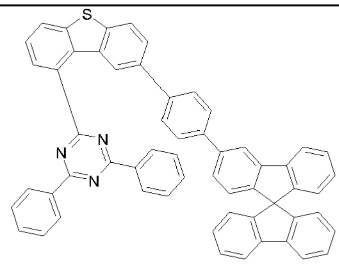 |
| 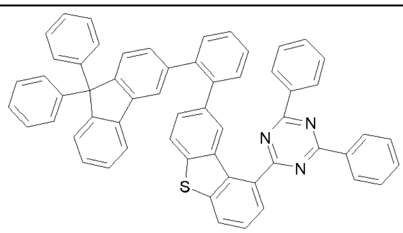 | 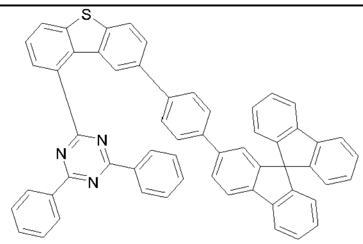 |
| 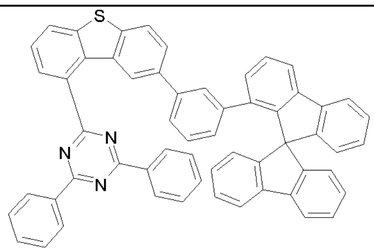 | 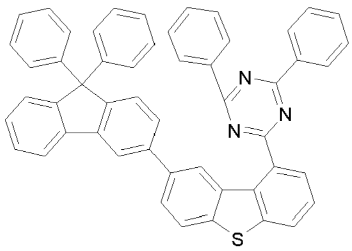 |
| 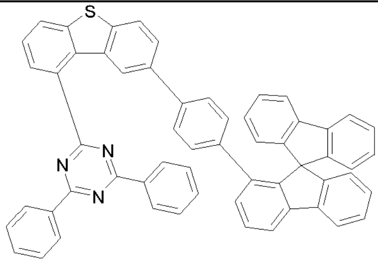 | 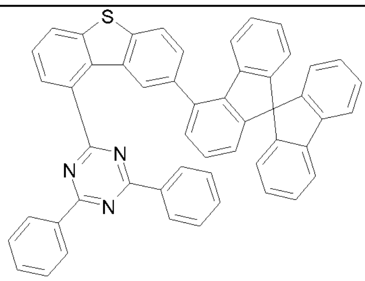 |

| | |
|---|---|
| 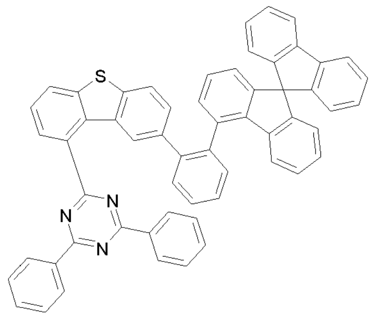 | 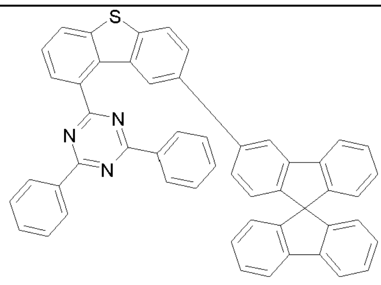 |
| 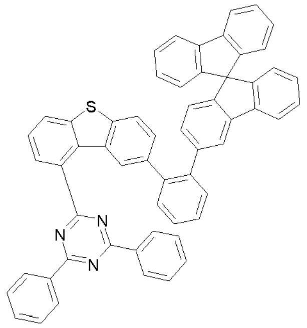 | 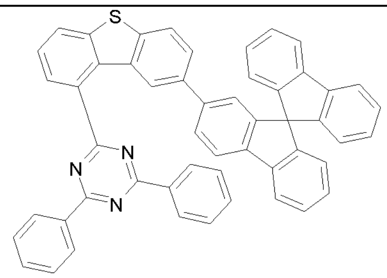 |
| 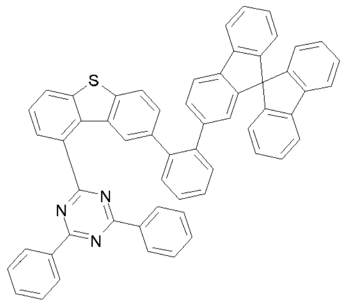 | 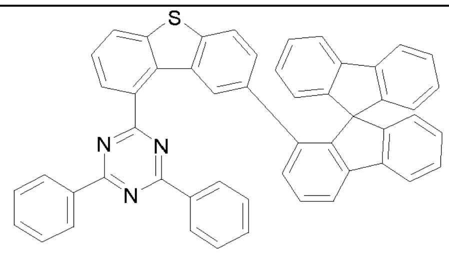 |
| 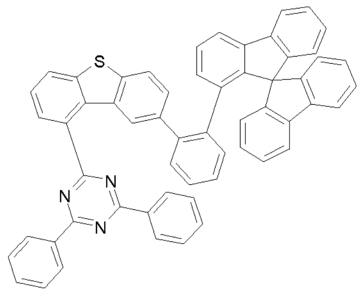 | 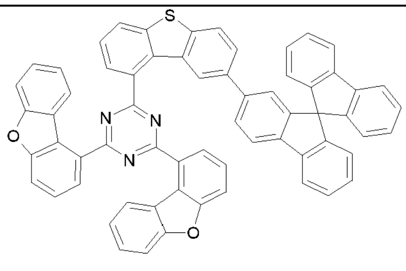 |
| 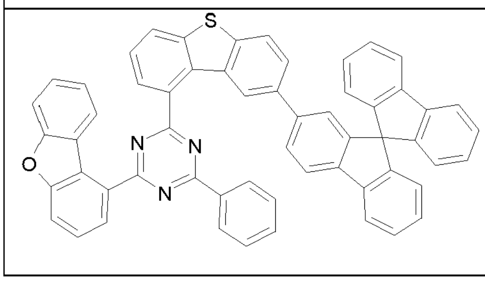 | 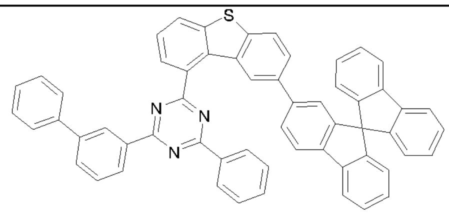 |

| | |
|---|---|
| 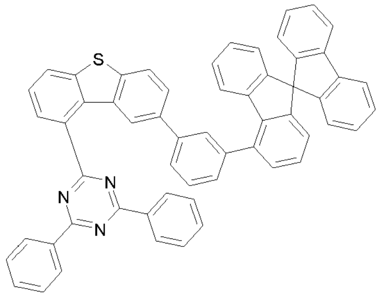 | 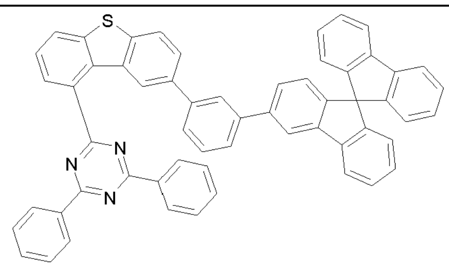 |
| 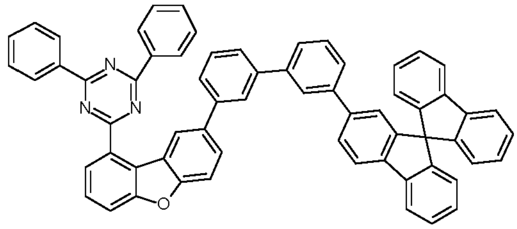 | 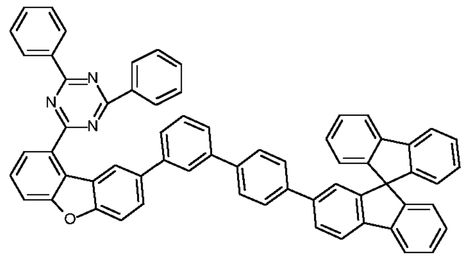 |
| 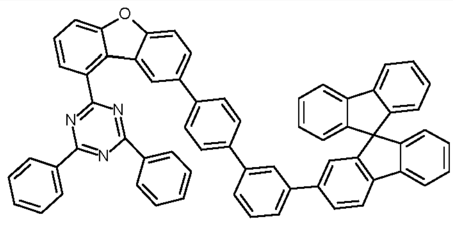 | 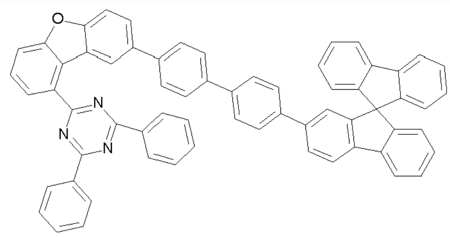 |
| 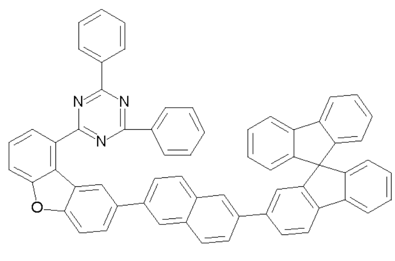 | 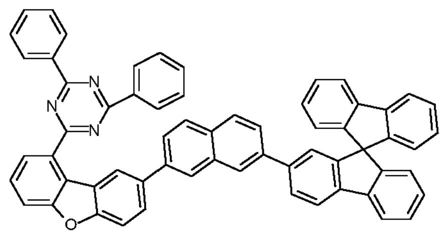 |
| 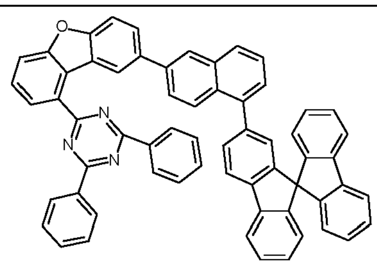 | 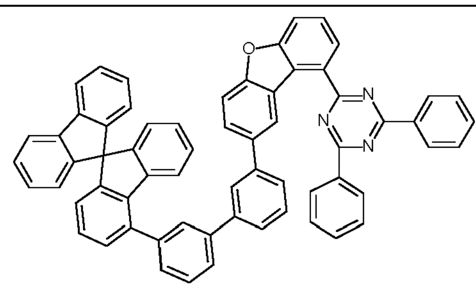 |
| 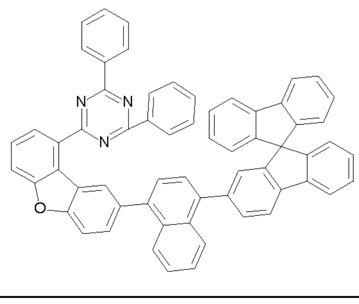 | 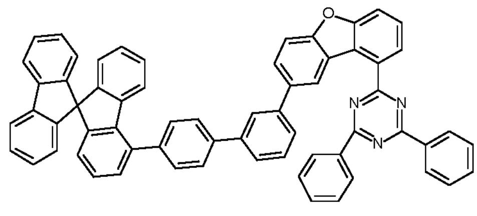 |

| | |
|---|---|
| 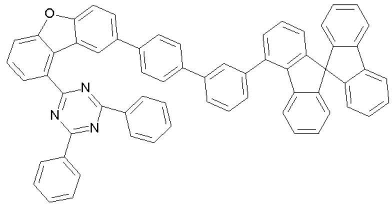 | 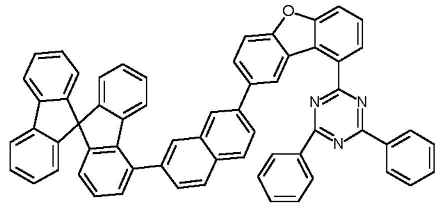 |
| 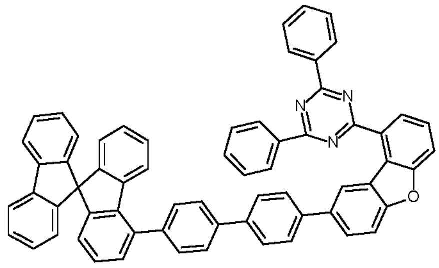 | 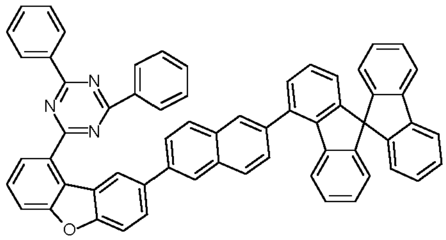 |
| 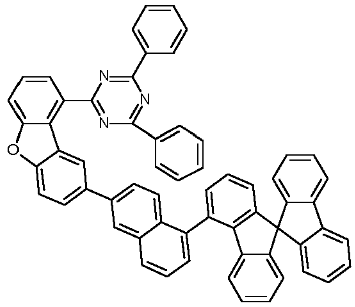 | 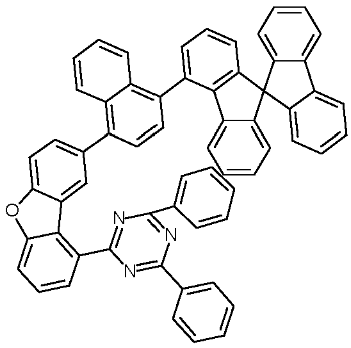 |

Besonders geeignete Verbindungen der Formeln (1), (1a), (1b) und/oder (1c) die bevorzugt in Kombination mit mindestens einer Verbindung der Formel (2) in der erfindungsgemäßen elektrolumineszierenden Vorrichtung verwendet werden, sind die Verbindungen **1** bis **10.**

Die Herstellung der Verbindungen der Formel (1) oder der bevorzugten Verbindungen der Tabelle 1 sowie der Verbindungen **1** bis **10** ist dem Fachmann bekannt. Die Verbindungen können nach dem Fachmann bekannten Syntheseschritten, wie z.B. Halogenierung, bevorzugt Bromierung, und einer sich anschließenden metallorganischen Kupplungsreaktion, z.B. Suzuki-Kupplung, Heck-Kupplung oder Hartwig-Buchwald-Kupplung, hergestellt werden. Die Herstellung der Verbindungen der Formel (1) oder der bevorzugten Verbindungen der Formel (1a) bis (1c) sowie der Verbindungen **1** bis **10** ist insbesondere aus WO2015169412 ableitbar, insbesondere Seite 63 sowie der Synthesebeispiele der Seiten 77 bis 114.

Die Herstellung der Verbindungen der Formeln (1) bis (1c), wobei L eine Einfachbindung bedeutet, kann nach folgendem Schema 1 erfolgen, wobei X, Y, $Ar_1$, $Ar_2$ eine der zuvor angegebenen Bedeutungen hat und R in Schema 1 eine Alkylgruppe mit 1 bis 4 C-Atomen bedeutet.

Schema 1:

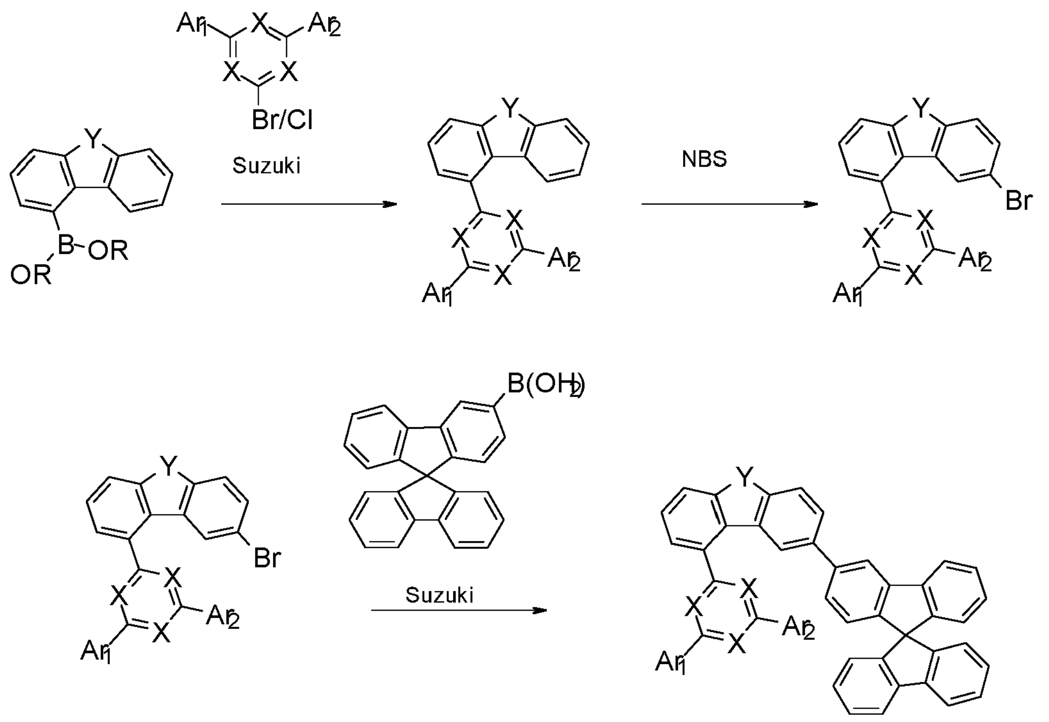


Die Herstellung der Verbindungen der Formeln (1) bis (1c), bei denen L eine Linkergruppe bedeutet, kann nach folgendem Schema 2 erfolgen, wobei X, Y, $Ar_1$, $Ar_2$ eine der zuvor angegebenen Bedeutungen hat.

Schema 2:

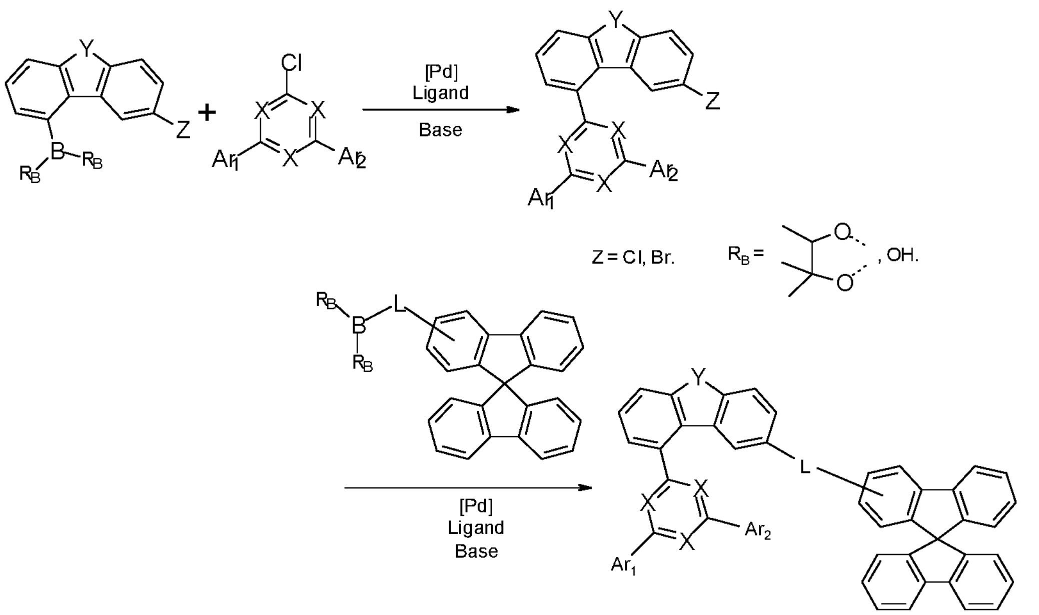


Im Folgenden wird das Hostmaterial 2 und dessen bevorzugte Ausführungsformen beschrieben, welches/welche in der erfindungsgemäßen Vorrichtung enthalten ist/sind. Die bevorzugten Ausführungsformen des Hostmaterials 2 der Formel (2) gelten auch für die erfindungsgemäße Mischung und/oder Formulierung.

Hostmaterial 2 ist mindestens eine Verbindung der Formel (2),

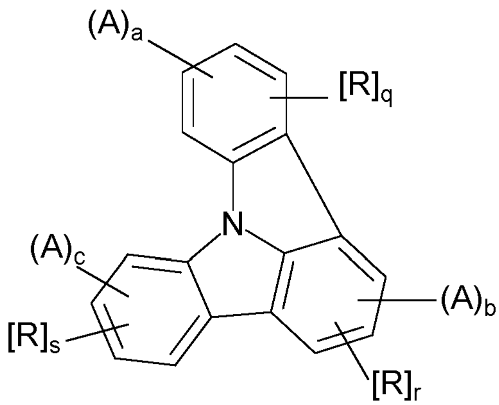

Formel (2) ,

wobei für die verwendeten Symbole und Indizes gilt:

R ist bei jedem Auftreten gleich oder verschieden ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen;

A ist bei jedem Auftreten unabhängig voneinander eine Gruppe der Formel (3) oder (4),

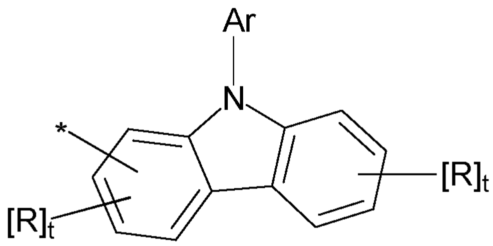

Formel (3) ,

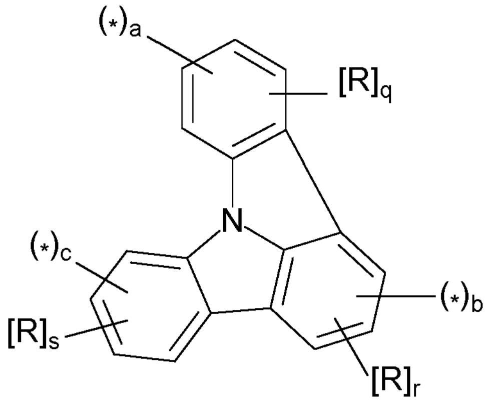

Formel (4) ;

Ar ist jeweils unabhängig voneinander bei jedem Auftreten eine Arylgruppe mit 6 bis 40 aromatischen Ringatomen, die mit einem oder mehreren Resten R substituiert sein können;

* kennzeichnet die Bindungsstelle an die Formel (2);

a, b, c bedeuten jeweils unabhängig voneinander bei jedem Auftreten 0 oder 1, wobei die Summe der Indizes bei jedem Auftreten a+b+c 1 bedeutet;

q, r, s, t bedeuten jeweils unabhängig bei jedem Auftreten 0 oder 1.

In einer Ausführungsform der Erfindung werden für die erfindungsgemäße Vorrichtung Verbindungen der Formel (2) ausgewählt, wie zuvor beschrieben, die mit Verbindungen der Formel (1), wie zuvor beschrieben oder bevorzugt beschrieben, oder mit den Verbindungen der Tabelle 1 oder den Verbindungen 1 bis 10, in der lichtemittierenden Schicht verwendet werden.

Verbindungen der Formel (2) können durch folgende Formeln (2a), (2b) und (2c) dargestellt werden,

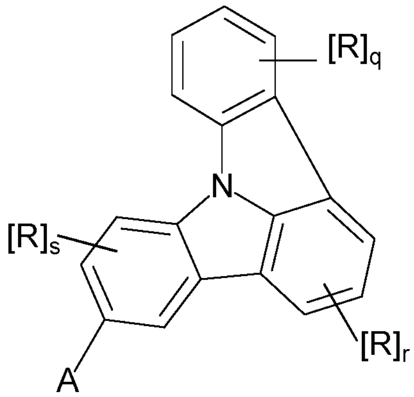

Formel (2a) ,

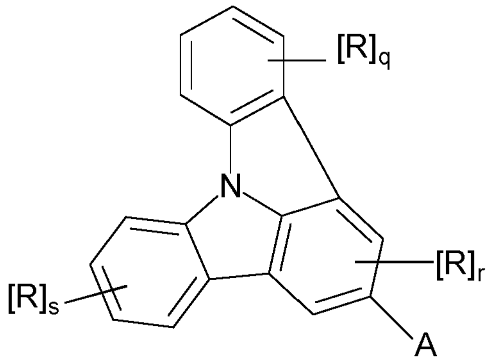

Formel (2b) ,

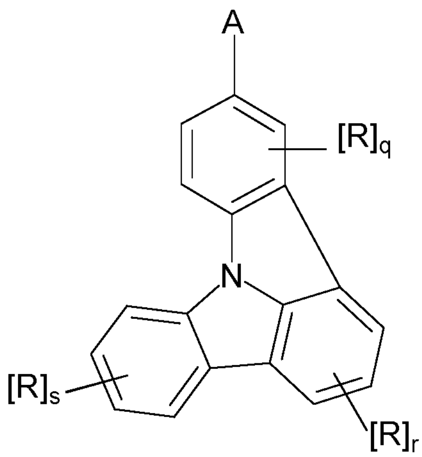

Formel (2c) ,

wobei A, R, q, r, s und t eine zuvor genannte oder nachfolgend genannte Bedeutung haben.

Ein weiterer Gegenstand der Erfindung ist demzufolge eine organische elektrolumineszierende Vorrichtung, wie zuvor beschrieben oder bevorzugt beschrieben, wobei das Hostmaterial 2 einer Verbindung der Formel (2a), (2b) oder (2c) entspricht.

R in Verbindungen der Formel (2) und der Formel (2a) bis (2c) oder bevorzugten Verbindungen der Formeln (2) und (2a) bis (2c), wie zuvor beschrieben, ist bei jedem Auftreten gleich oder verschieden ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen. Der Substituent R steht bei jedem Auftreten unabhängig voneinander bevorzugt für CN oder eine Arylgruppe mit 6 bis 40 C-Atomen, wie zuvor beschrieben. R ist bei jedem Auftreten unabhängig voneinander besonders bevorzugt Phenyl.

In Verbindungen der Formeln (2) oder (2a), (2b) oder (2c) ist die Summe der Indizes q+r+s bevorzugt 0 oder 1, wobei R eine zuvor angegebene Bedeutung hat.

In Verbindungen der Formeln (2) oder (2a), (2b) oder (2c) sind q, r und s bevorzugt 0 oder 1. Bevorzugt sind q, r und s 0.

In Formel (4) ist die Summe der Indizes q+r+s bevorzugt 0 oder 1, wobei R eine zuvor angegebene Bedeutung hat.

In Formel (4) sind q, r und s bevorzugt 0 oder 1. Bevorzugt sind q, r und s in Formel (4) 0.

In Formel (3) ist t jeweils unabhängig voneinander bevorzugt 0 oder 1. In Formel (3) ist t bevorzugt gleich und entspricht 0.

Ar ist jeweils unabhängig voneinander bei jedem Auftreten eine Arylgruppe mit 6 bis 40 aromatischen Ringatomen, die mit einem oder mehreren Resten R substituiert sein kann, oder eine Heterarylgruppe mit 5 bis 40 aromatischen Ringatomen, enthaltend O als Heteroatom, die mit einem oder mehreren Resten R substituiert sein kann, wobei der

Rest R eine zuvor für Formel (2) angegebene oder bevorzugt angegebene Bedeutung hat.

Ar ist bevorzugt bei jedem Auftreten eine Arylgruppe mit 6 bis 18 C-Atomen, die mit einem oder mehreren Resten R substituiert sein kann, wobei der Rest eine zuvor für Formel (2) angegebene oder bevorzugt angegebene Bedeutung hat oder Dibenzofuranyl. Ar ist besonders bevorzugt Phenyl, mit Dibenzofuran substituiertes Phenyl, mit Dibenzothiophen substituiertes Phenyl, 1,3-Biphenyl, 1-4-Biphenyl, Terphenyl, Quaterphenyl, Naphthyl, Fluorenyl, 9,9-Diphenyl-fluorenyl, Bispirofluorenyl, Triphenylenyl oder Dibenzofuranyl.

In einer bevorzugten Ausführungsform der Erfindung entspricht A der Formel (3), wie zuvor beschrieben oder wie bevorzugt beschrieben.

In Verbindungen der Formeln (2) oder (2a), (2b) oder (2c), wobei A der Formel (3) entspricht und q, r, s und t 0 bedeuten, können durch die Formeln (2d) und (2e) dargestellt werden,

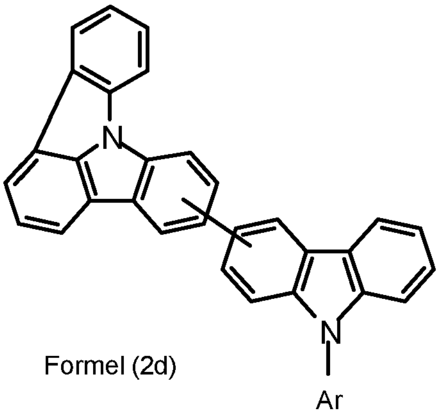
Formel (2d)
,
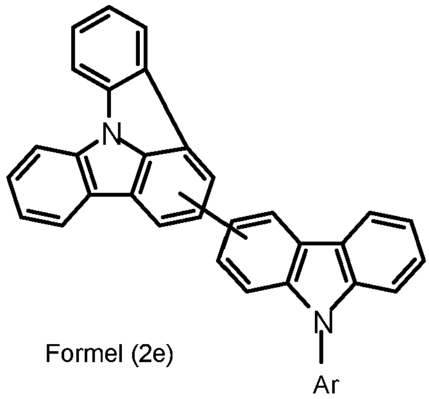
Formel (2e)
,

wobei Ar eine zuvor genannte oder bevorzugt genannte Bedeutung hat.

In einer bevorzugten Ausführungsform der Erfindung entspricht A der Formel (4), wie zuvor beschrieben oder wie bevorzugt beschrieben.

Ein weiterer Gegenstand der Erfindung ist demzufolge eine organische elektrolumineszierende Vorrichtung, wie zuvor beschrieben oder bevorzugt beschrieben, wobei die mindestens eine Verbindung der Formel (2) einer Verbindung der Formel (2d) oder der Formel (2e) entspricht.

In einer bevorzugten Ausführungsform der Verbindungen der (2), (2a), (2b), (2c), (2d) oder (2e) sind die Substituenten der Formeln (3) oder (4) jeweils in 2-Position oder 5-Position des Indolo[3,2,1-*jk*]carbazoles miteinander verknüpft, wie nachfolgend schematisch dargestellt, wobei die gestrichelte Linie die Verknüpfung an den Substituenten der Formel (3) bzw. (4) anzeigt:

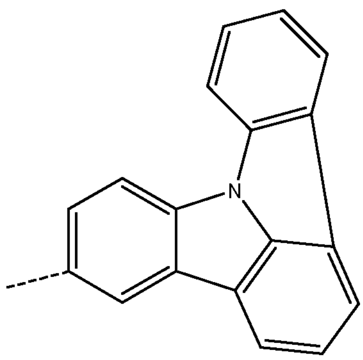

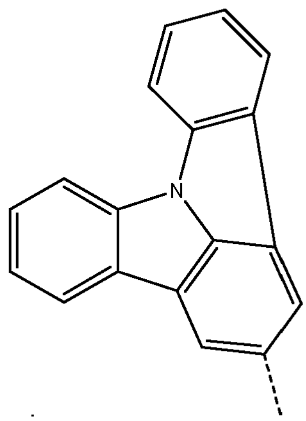
.

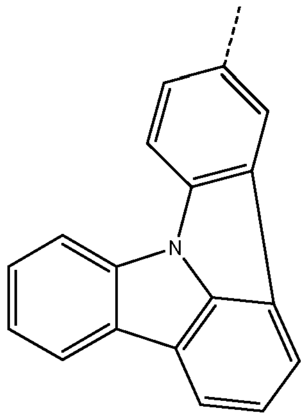

Beispiele für geeignete Hostmaterialien der Formel (2), (2a), (2b), (2c), (2d) und (2e), die erfindungsgemäß ausgewählt werden, und bevorzugt in Kombination mit mindestens einer Verbindung der Formel (1) in der erfindungsgemäßen elektrolumineszierenden Vorrichtung verwendet werden, sind die nachstehend genannten Strukturen der Tabelle 2.

Tabelle 2:

| | |
|---|---|
| 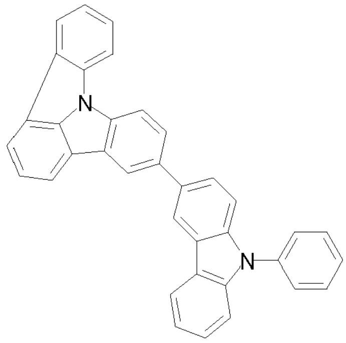 | 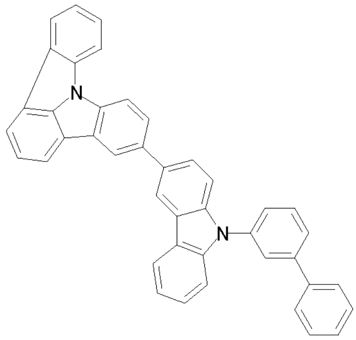 |
| 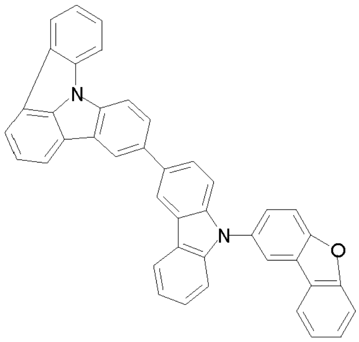 | 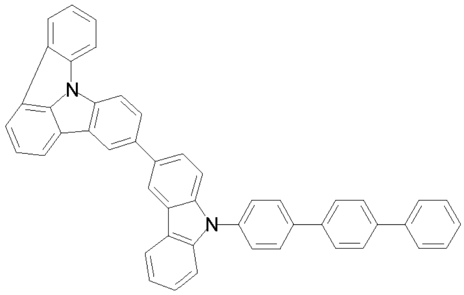 |
| 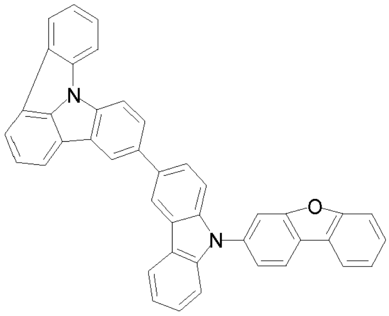 | 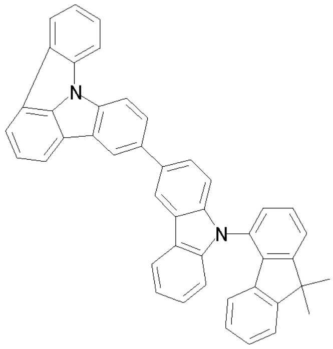 |

| | |
|---|---|
| 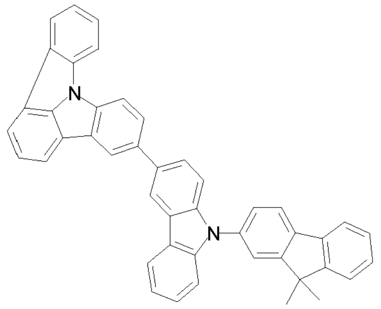 | 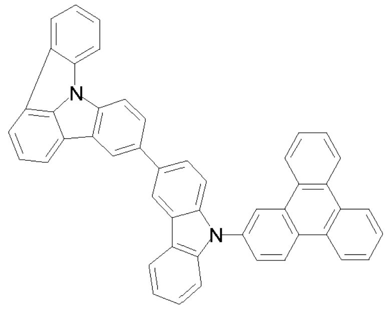 |
| 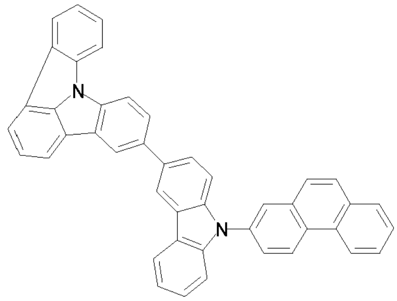 | 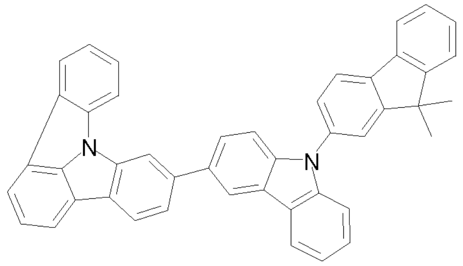 |
| 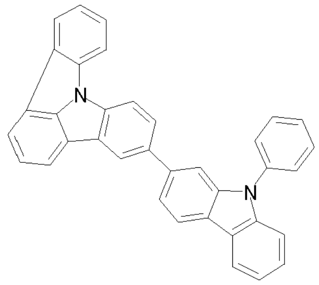 | 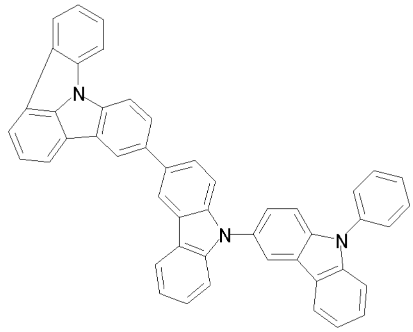 |
| 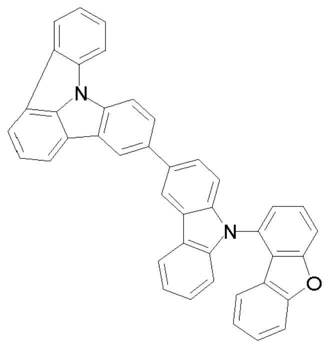 | 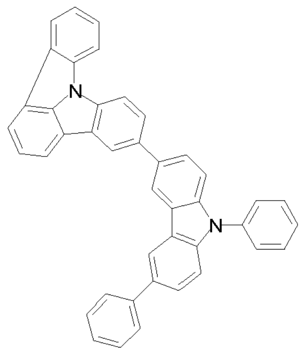 |

| | |
|---|---|
| 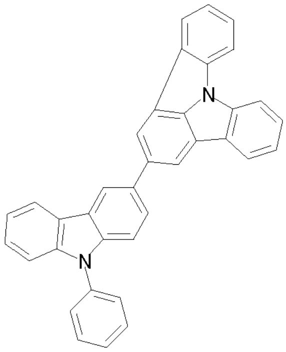 | 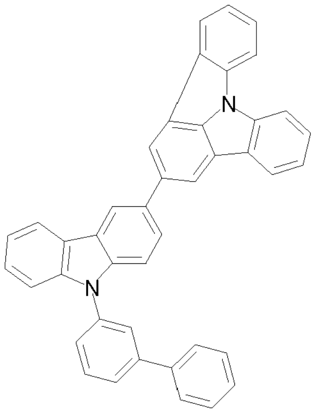 |
| 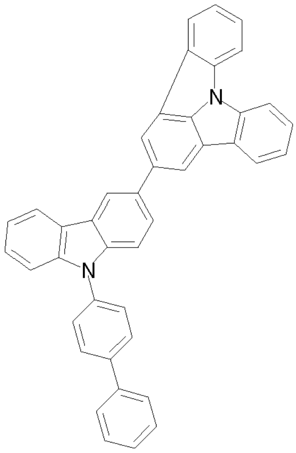 | 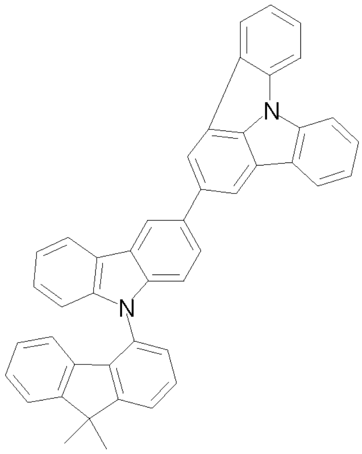 |
| 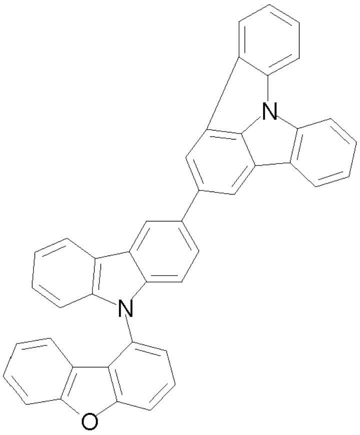 | 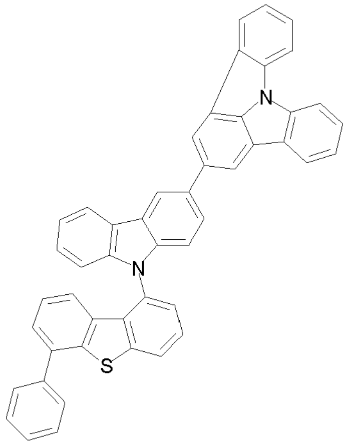 |

| | |
|---|---|
| 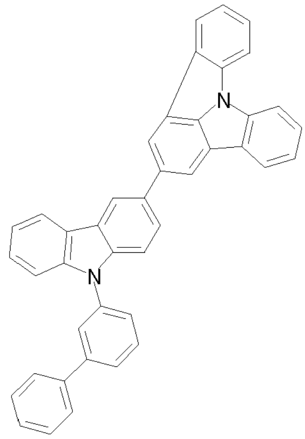 | 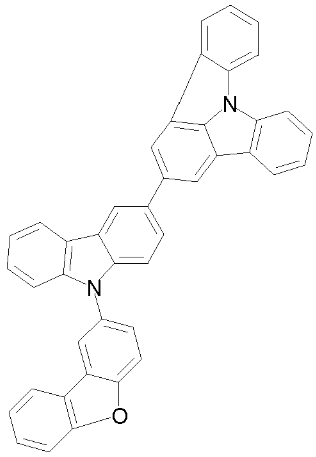 |
| 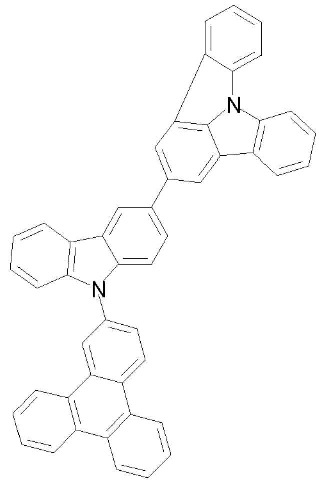 | 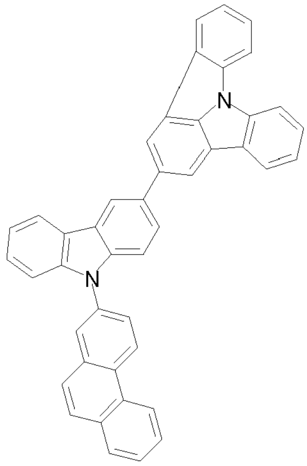 |
| 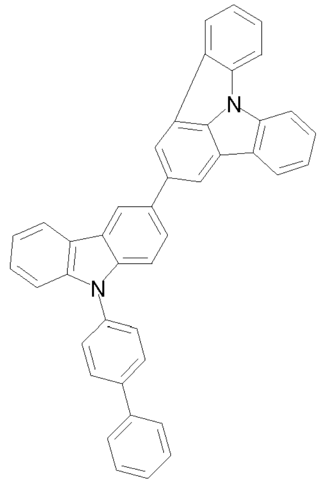 | 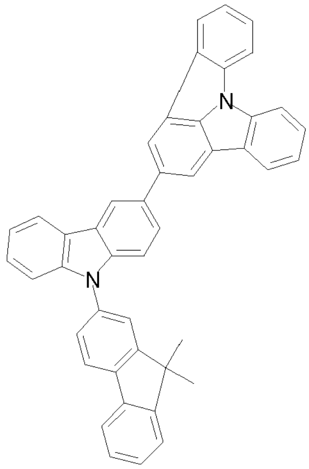 |

| | |
|---|---|
| 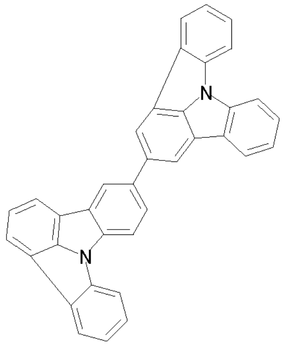 | 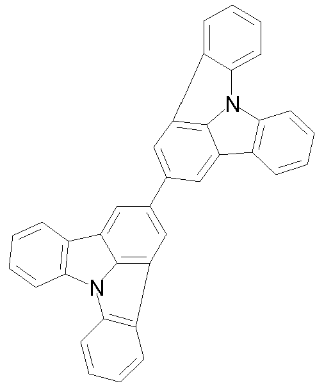 |
| 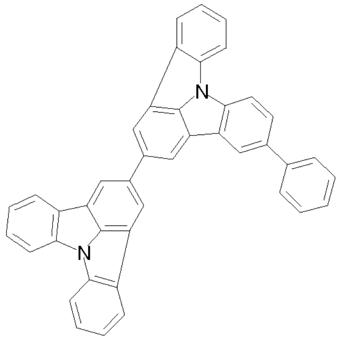 | 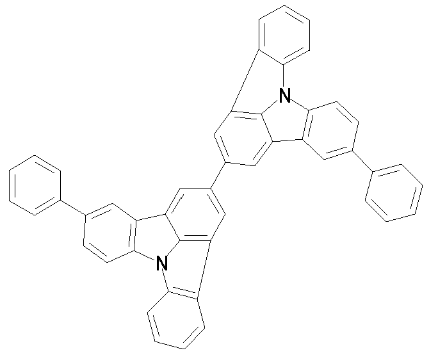 |
| 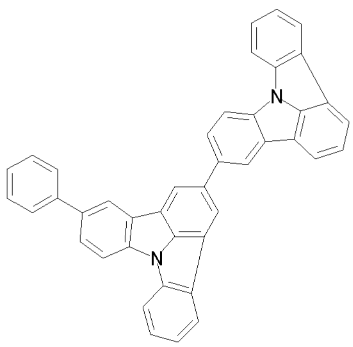 . | |

Besonders geeignete Verbindungen der Formel (2), die bevorzugt in Kombination mit mindestens einer Verbindung der Formel (1) in der erfindungsgemäßen elektrolumineszierenden Vorrichtung verwendet werden, sind die Verbindungen **11** bis **22:**

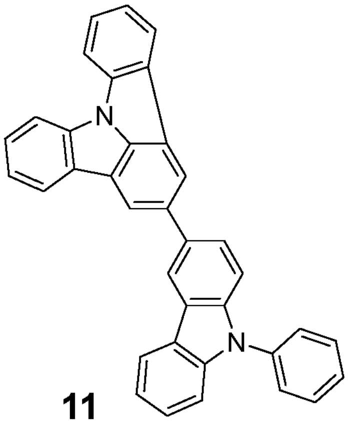

11

,

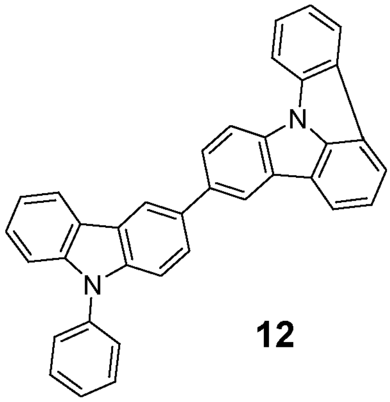

12

,

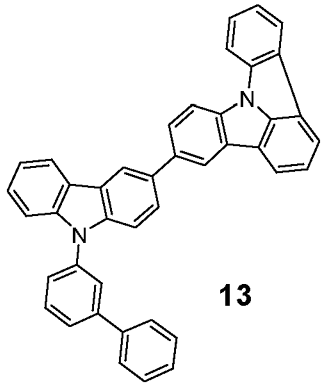

13

,

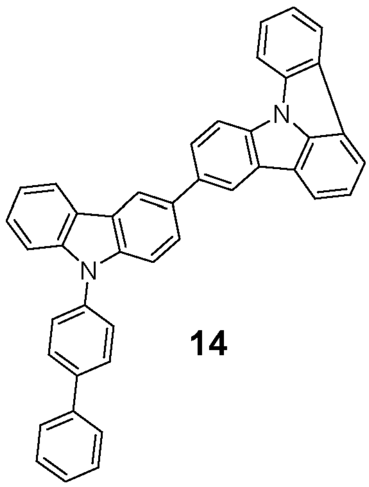

14

,

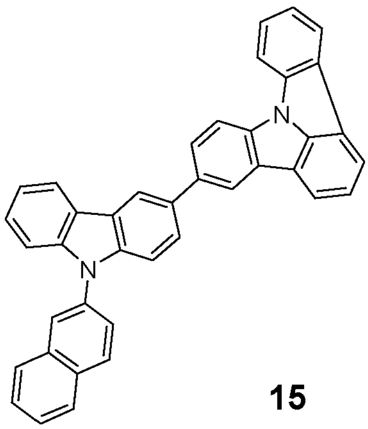

15

,

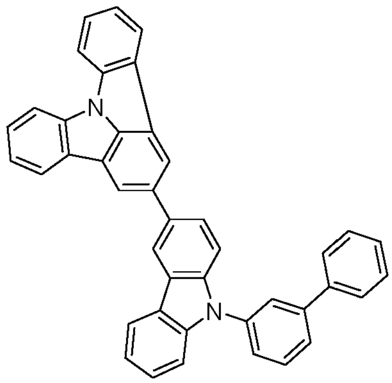

16

,

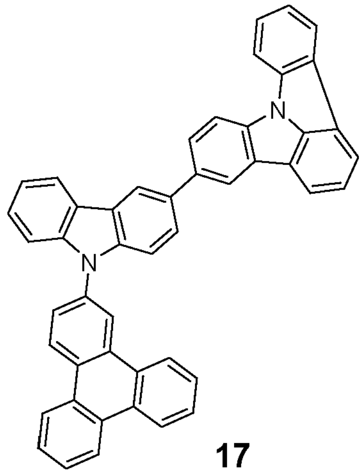

17

,

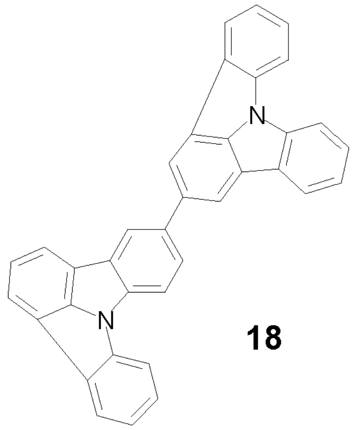

18

,

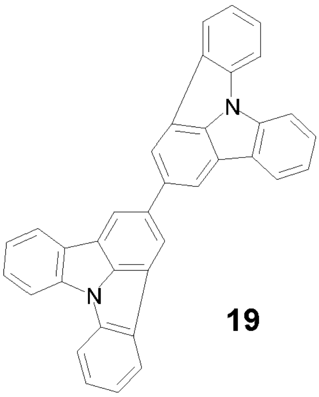

19

,.

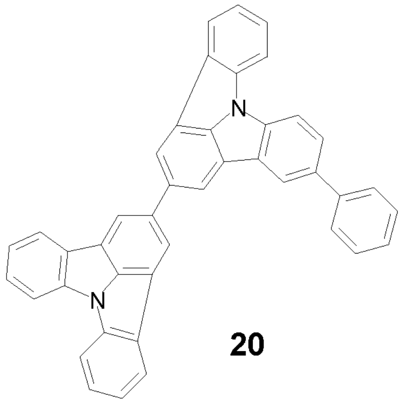

20

,

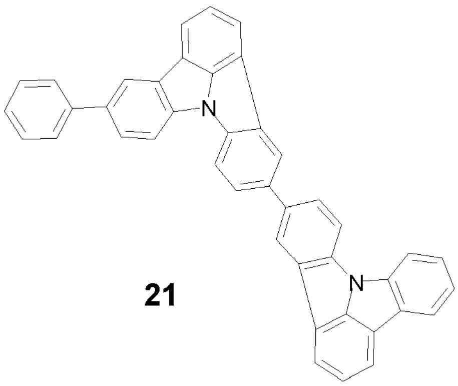

21

,

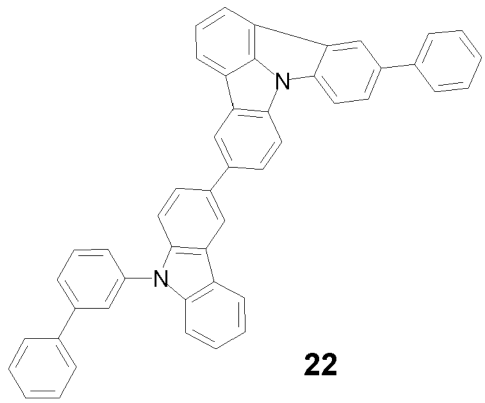

22

.

Die Herstellung der Verbindungen der Formel (2) oder der bevorzugten Verbindungen der Formel (2), (2a), (2b), (2c), (2d) und (2e), sowie der Verbindungen der Tabelle 2 und Verbindungen **11** bis **22** ist dem Fachmann bekannt. Die Verbindungen können nach dem Fachmann bekannten Syntheseschritten, wie z.B. Halogenierung, bevorzugt Bromierung, und einer sich anschließenden metallorganischen Kupplungsreaktion, z.B. Suzuki-Kupplung, Heck-Kupplung oder Hartwig-Buchwald-Kupplung, hergestellt werden. Die Synthese kann insbesondere aus der Offenbarung der WO2011088877 und KR20170113318 abgeleitet werden. Zum Teil sind die Verbindungen der Formel (2) kommerziell erhältlich.

Schema 3 beschreibt die Suszuki-Reaktion näher:

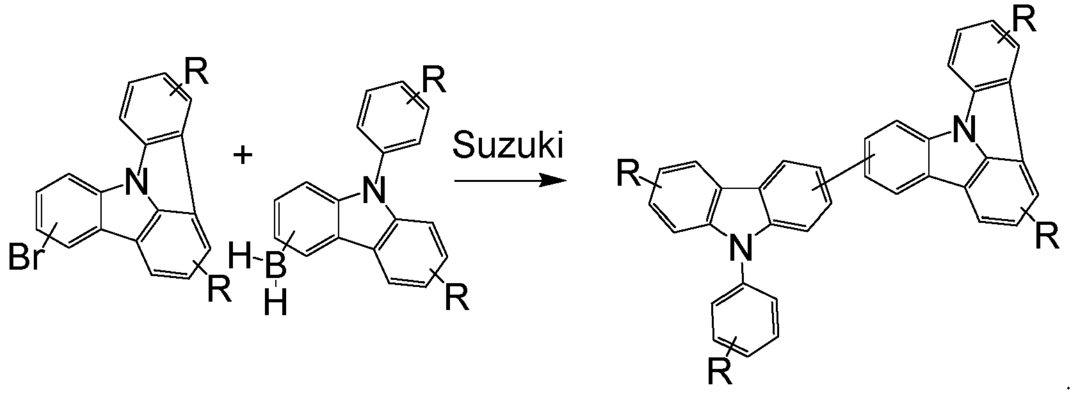


.

Die vorstehend genannten Hostmaterialien der Formel (1) sowie deren bevorzugt beschriebene Ausführungsformen oder die Verbindungen der Tabelle 1 und der Verbindungen **1** bis **10** können in der erfindungsgemäßen Vorrichtung beliebig mit den genannten Hostmaterialien der Formel (2), (2a), (2b), (2c), (2d) und (2e) sowie deren bevorzugt beschriebene Ausführungsformen oder den Verbindungen der Tabelle 2 oder den Verbindungen **11** bis **22** kombiniert werden.

Ein weiterer Gegenstand der Erfindung sind ebenfalls Mischungen enthaltend mindestens eine Verbindung der Formel (1) und mindestens eine Verbindung der Formel (2),

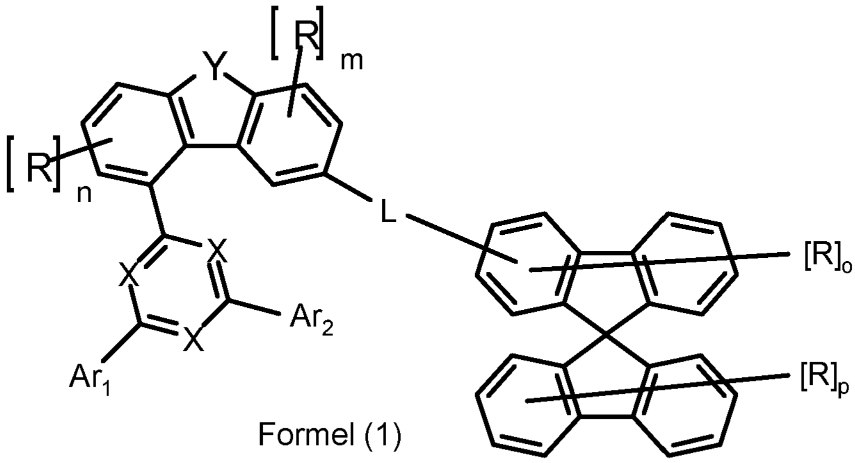

Formel (1)

,

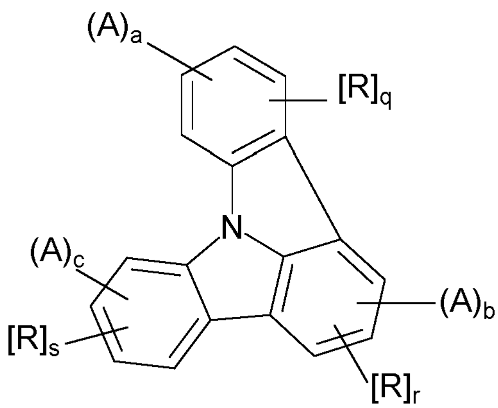

Formel (2)

,

wobei für die verwendeten Symbole und Indizes gilt:

X ist bei jedem Auftreten gleich oder verschieden $CR^0$ oder N, mit der Maßgabe, dass mindestens zwei Gruppen X für N stehen;

Y ist ausgewählt aus O oder S;

L ist bei jedem Auftreten gleich oder verschieden eine Einfachbindung oder ein Linker L-1 bis L-13,

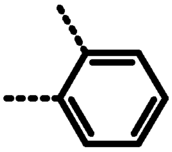 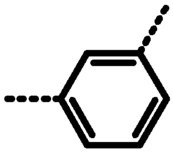 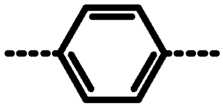

L-1 L-2 L-3

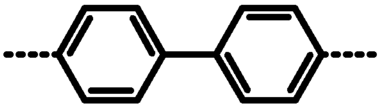 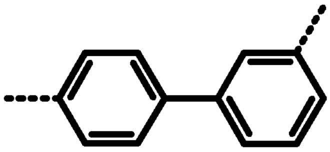

L-4 L-5

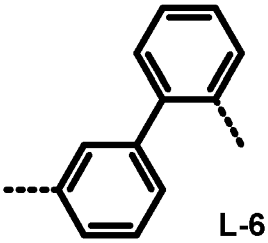 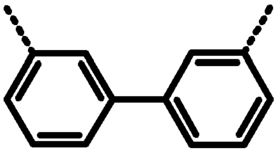 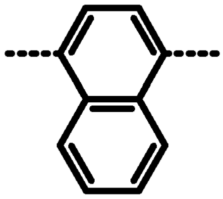

L-6 L-7 L-8

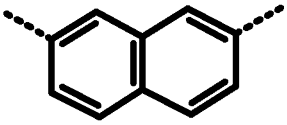 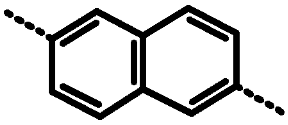 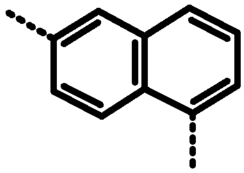

L-9 L-10 L-11

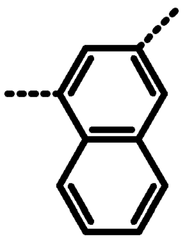 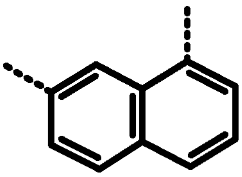

L-12 L-13 ,

wobei die Linker L-1 bis L-13 noch mit einem oder mehreren Substituenten R substituiert sein können und die gestrichelte Linie die jeweilige Bindung an den Rest der Formel (1) kennzeichnet;

R ist bei jedem Auftreten gleich oder verschieden ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen;

$Ar_1$, $Ar_2$ sind jeweils unabhängig voneinander bei jedem Auftreten eine Aryl- oder Heteroarylgruppe mit 5 bis 40 aromatischen Ringatomen, die mit einem oder mehreren Resten R substituiert sein können;

A ist bei jedem Auftreten unabhängig voneinander eine Gruppe der Formel (3) oder (4),

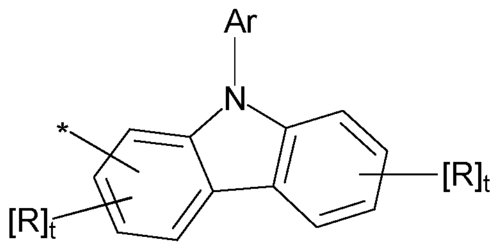

Formel (3) ,

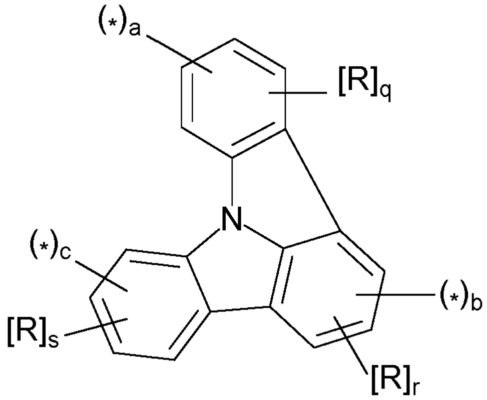

Formel (4) ;

Ar ist jeweils unabhängig voneinander bei jedem Auftreten eine Arylgruppe mit 6 bis 40 aromatischen Ringatomen, die mit einem oder mehreren Resten R substituiert sein können;

* kennzeichnet die Bindungsstelle an die Formel (2);

a, b, c bedeuten jeweils unabhängig voneinander bei jedem Auftreten 0 oder 1, wobei die Summe der Indizes bei jedem Auftreten a+b+c 1 bedeutet;

n und m bedeuten unabhängig voneinander bei jedem Auftreten 0, 1, 2 oder 3;

o bedeutet unabhängig bei jedem Auftreten 0, 1, 2, 3, 4, 5, 6 oder 7;

p bedeutet unabhängig bei jedem Auftreten 0, 1, 2, 3, 4, 5, 6, 7 oder 8;

q, r, s, t bedeuten jeweils unabhängig bei jedem Auftreten 0 oder 1;

$R^0$ ist bei jedem Auftreten unabhängig voneinander H oder ein unsubstituiertes oder teilweise oder vollständig deuteriertes aromatisches Ringsystem mit 6 bis 18 C-Atomen.

Die Ausführungen hinsichtlich der Hostmaterialien der Formeln (1) und (2) sowie deren bevorzugten Ausführungsformen gilt entsprechend auch für die erfindungsgemäße Mischung.

Besonders bevorzugte Mischungen der Hostmaterialien der Formel (1) mit den Hostmaterialien der Formel (2) für die erfindungsgemäße Vorrichtung erhält man durch Kombination der Verbindungen **1** bis **10** mit den Verbindungen der Tabelle 2.

Ganz besonders bevorzugte Mischungen der Hostmaterialien der Formel (1) mit den Hostmaterialien der Formel (2) für die erfindungsgemäße Vorrichtung erhält man durch Kombination der Verbindungen **1** bis **10** mit den Verbindungen **11** bis **22,** wie im Folgenden in Tabelle 3 gezeigt.

Tabelle 3:

| | | | | | | | | |
|---|---|---|---|---|---|---|---|---|
| M1 | **1** | **11** | M2 | **1** | **12** | M3 | **1** | **13** |
| M4 | **1** | **14** | M5 | **1** | **15** | M6 | **1** | **16** |
| M7 | **1** | **17** | M8 | **1** | **18** | M9 | **1** | **19** |
| M10 | **1** | **20** | M11 | **1** | **21** | M12 | **1** | **22** |
| M13 | **2** | **11** | M14 | **2** | **12** | M15 | **2** | **13** |
| M16 | **2** | **14** | M17 | **2** | **15** | M18 | **2** | **16** |

(fortgesetzt)

| | | | | | | | | |
|---|---|---|---|---|---|---|---|---|
| M19 | **2** | **17** | M20 | **2** | **18** | M21 | **2** | **19** |
| M22 | **2** | **20** | M23 | **2** | **21** | M24 | **2** | **22** |
| M25 | **3** | **11** | M26 | **3** | **12** | M27 | **3** | **13** |
| M28 | **3** | **14** | M29 | **3** | **15** | M30 | **3** | **16** |
| M31 | **3** | **17** | M32 | **3** | **18** | M33 | **3** | **19** |
| M34 | **3** | **20** | M35 | **3** | **21** | M36 | **3** | **22** |
| M37 | **4** | **11** | M38 | **4** | **12** | M39 | **4** | **13** |
| M40 | **4** | **14** | M41 | **4** | **15** | M42 | **4** | **16** |
| M43 | **4** | **17** | M44 | **4** | **18** | M45 | **4** | **19** |
| M46 | **4** | **20** | M47 | **4** | **21** | M48 | **4** | **22** |
| M49 | **5** | **11** | M50 | **5** | **12** | M51 | **5** | **13** |
| M52 | **5** | **14** | M53 | **5** | **15** | M54 | **5** | **16** |
| M55 | **5** | **17** | M56 | **5** | **18** | M57 | **5** | **19** |
| M58 | **5** | **20** | M59 | **5** | **21** | M60 | **5** | **22** |
| M61 | **6** | **11** | M62 | **6** | **12** | M63 | **6** | **13** |
| M64 | **6** | **14** | M65 | **6** | **15** | M66 | **6** | **16** |
| M67 | **6** | **17** | M68 | **6** | **18** | M69 | **6** | **19** |
| M70 | **6** | **20** | M71 | **6** | **21** | M72 | **6** | **22** |
| M73 | **7** | **11** | M74 | **7** | **12** | M75 | **7** | **13** |
| M76 | **7** | **14** | M77 | **7** | **15** | M78 | **7** | **16** |
| M79 | **7** | **17** | M80 | **7** | **18** | M81 | **7** | **19** |
| M82 | **7** | **20** | M83 | **7** | **21** | M84 | **7** | **22** |
| M85 | **8** | **11** | M86 | **8** | **12** | M87 | **8** | **13** |
| M88 | **8** | **14** | M89 | **8** | **15** | M90 | **8** | **16** |
| M91 | **8** | **17** | M92 | **8** | **18** | M93 | **8** | **19** |
| M94 | **8** | **20** | M95 | **8** | **21** | M96 | **8** | **22** |
| M97 | **9** | **11** | M98 | **9** | **12** | M99 | **9** | **13** |
| M100 | **9** | **14** | M101 | **9** | **15** | M102 | **9** | **16** |
| M103 | **9** | **17** | M104 | **9** | **18** | M105 | **9** | **19** |
| M106 | **9** | **20** | M107 | **9** | **21** | M108 | **9** | **22** |
| M109 | **10** | **11** | M110 | **10** | **12** | M111 | **10** | **13** |
| M112 | **10** | **14** | M113 | **10** | **15** | M114 | **10** | **16** |
| M115 | **10** | **17** | M116 | **10** | **18** | M117 | **10** | **19** |
| M118 | **10** | **20** | M119 | **10** | **21** | M120 | **10** | **22.** |

Die Konzentration des elektronentransportierenden Hostmaterials der Formel (1), wie zuvor beschrieben oder bevorzugt beschrieben, in der erfindungsgemäßen Mischung oder in der lichtemittierenden Schicht der erfindungsgemäßen Vorrichtung liegt im Bereich von 5 Gew.-% bis 90 Gew.-%, bevorzugt im Bereich von 10 Gew.-% bis 85 Gew.-%, mehr bevorzugt im Bereich von 20 Gew.-% bis 85 Gew.-%, noch mehr bevorzugt im Bereich von 30 Gew.-% bis 80 Gew.-%, ganz besonders bevorzugt im Bereich von 20 Gew.-% bis 60 Gew.-% und am meisten bevorzugt im Bereich von 30 Gew.-% bis 50 Gew.-%, bezogen auf die gesamte Mischung oder bezogen auf die gesamte Zusammensetzung

der lichtemittierenden Schicht.

Die Konzentration des lochtransportierenden Hostmaterials der Formel (2), wie zuvor beschrieben oder als bevorzugt beschrieben, in der erfindungsgemäßen Mischung oder in der lichtemittierenden Schicht der erfindungsgemäßen Vorrichtung liegt im Bereich von 10 Gew.-% bis 95 Gew.-%, bevorzugt im Bereich von 15 Gew.-% bis 90 Gew.-%, mehr bevorzugt im Bereich von 15 Gew.-% bis 80 Gew.-%, noch mehr bevorzugt im Bereich von 20 Gew.-% bis 70 Gew.-%, ganz besonders bevorzugt im Bereich von 40 Gew.-% bis 80 Gew.-% und am meisten bevorzugt im Bereich von 50 Gew.-% bis 70 Gew.-%, bezogen auf die gesamte Mischung oder bezogen auf die gesamte Zusammensetzung der lichtemittierenden Schicht.

Die vorliegende Erfindung betrifft auch eine Mischung, die neben den vorstehend genannten Hostmaterialien 1 und 2, wie zuvor beschrieben oder bevorzugt beschrieben, insbesondere Mischungen M1 bis M120, mindestens noch einen phosphoreszierenden Emitter enthält.

Die vorliegende Erfindung betrifft auch eine organische elektrolumineszierende Vorrichtung, wie zuvor beschrieben oder bevorzugt beschrieben, wobei die lichtemittierende Schicht neben den vorstehend genannten Hostmaterialien 1 und 2, wie zuvor beschrieben oder bevorzugt beschrieben, insbesondere den Materialkombinationen M1 bis M120, mindestens noch einen phosphoreszierenden Emitter enthält.

Vom Begriff phosphoreszierende Emitter sind typischerweise Verbindungen umfasst, bei denen die Lichtemission durch einen spin-verbotenen Übergang aus einem angeregten Zustand mit höherer Spinmultiplizität, also einem Spinzustand > 1, erfolgt, beispielsweise durch einen Übergang aus einem Triplett-Zustand oder einem Zustand mit einer noch höheren Spinquantenzahl, beispielsweise einem Quintett-Zustand. Bevorzugt wird hierbei ein Übergang aus einem Triplett-Zustand verstanden.

Als phosphoreszierende Emitter (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Emitter angesehen.

Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenzvorrichtungen bekannt sind.

Beispiele der oben beschriebenen Emitter können den Anmeldungen WO 2016/015815, WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815, WO 2016/124304, WO 2017/032439, WO 2015/036074, WO 2015/117718 und WO 2016/015815 entnommen werden.

Bevorzugte phosphoreszierende Emitter gemäß der vorliegenden Erfindung entsprechen der Formel (5),

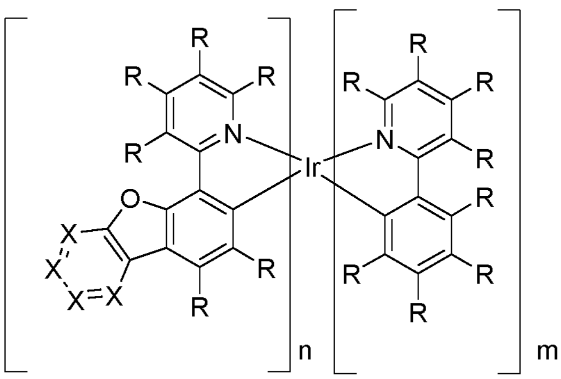

Formel (5)

,

wobei die Symbole und Indizes für diese Formel (5) die Bedeutung haben:

n+m ist 3, n ist 1 oder 2, m ist 2 oder 1,

X ist N oder CR,
R ist H, D oder eine verzweigte oder lineare Alkylgruppe mit 1 bis 10 C-Atomen oder eine teilweise oder vollständig deuterierte verzweigte oder lineare Alkylgruppe mit 1 bis 10 C-Atomen oder eine Cycloalkylgruppe mit 4 bis 7 C-Atomen, die teilweise oder vollständig mit Deuterium substituiert sein kann.

Ein weiterer Gegenstand der Erfindung ist demzufolge eine organische elektrolumineszierende Vorrichtung, wie zuvor beschrieben oder bevorzugt beschrieben, dadurch gekennzeichnet, dass die lichtemittierende Schicht neben den Hostmaterialien 1 und 2 mindestens einen phosphoreszierenden Emitter enthält, der der Formel (5) entspricht, wie zuvor beschrieben.

In Emittern der Formel (5) ist n bevorzugt 1 und m ist bevorzugt 2.

In Emittern der Formel (5) ist bevorzugt ein X ausgewählt aus N und die anderen X bedeuten CR.

In Emittern der Formel (5) ist mindestens ein R bevorzugt unterschiedlich von H. In Emittern der Formel (5) sind bevorzugt zwei R unterschiedlich von H und haben eine der sonst zuvor für die Emitter der Formel (5) angegebenen Bedeutungen.

Bevorzugte Beispiele von phosphoreszierenden Emittern sind in der folgenden Tabelle 4 aufgeführt.

Tabelle 4:

| | | |
|---|---|---|
| 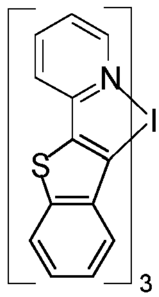 | 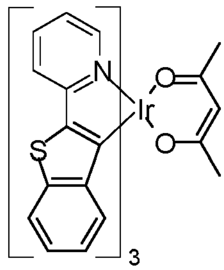 | 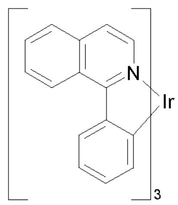 |
| 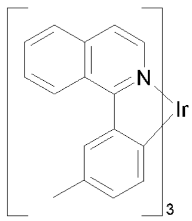 | 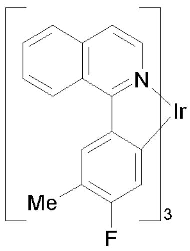 | 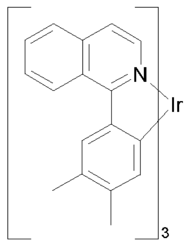 |
| 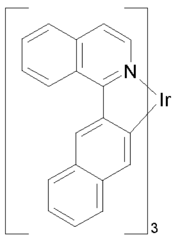 | 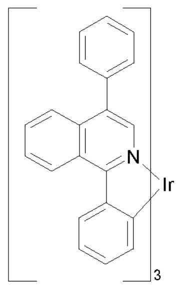 | 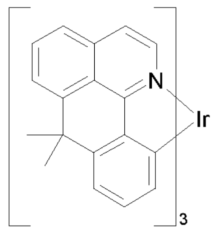 |
| 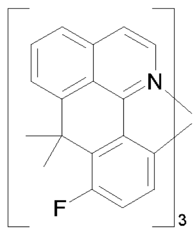 | 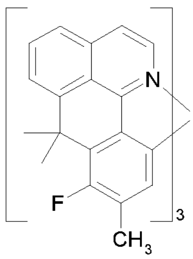 | 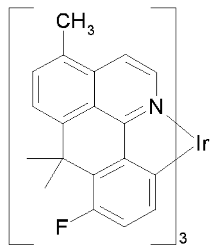 |

| | | |
|---|---|---|
| 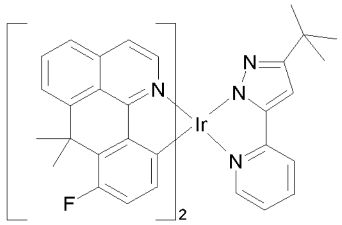 | 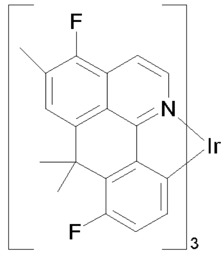 | 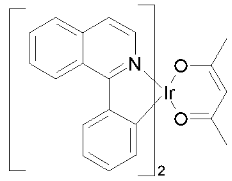 |
| 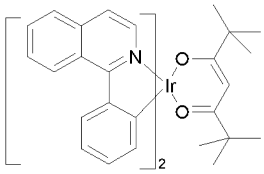 | 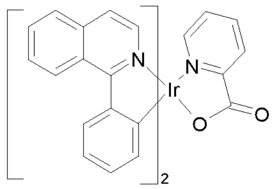 | 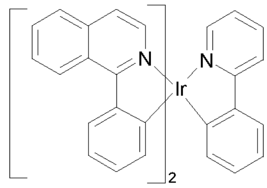 |
| 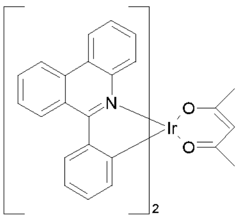 | 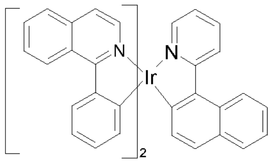 | 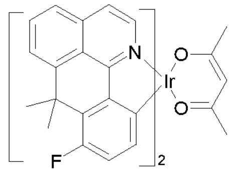 |
| 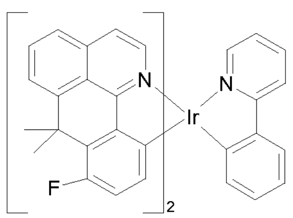 | 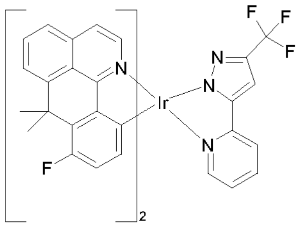 | 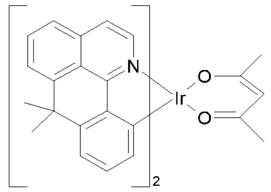 |
| 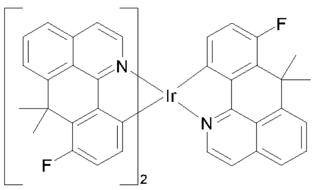 | 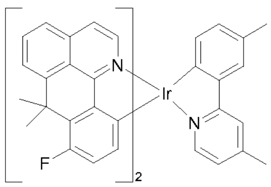 | 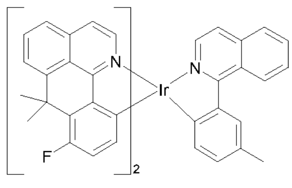 |
| 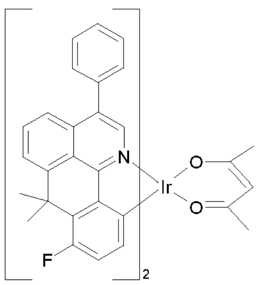 | 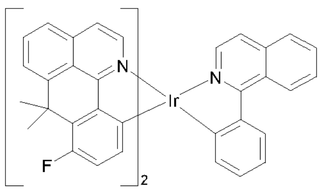 | 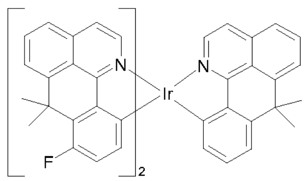 |
| 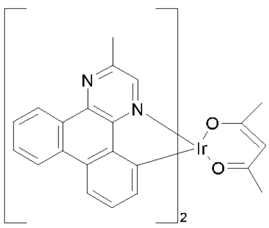 | 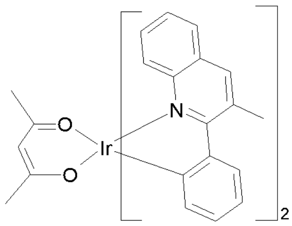 | 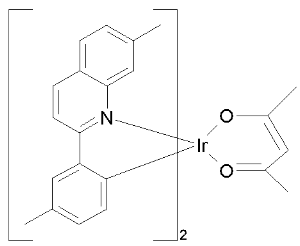 |

| | | |
|---|---|---|
| 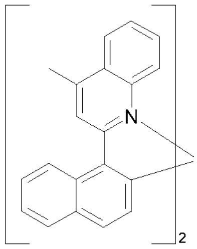 | 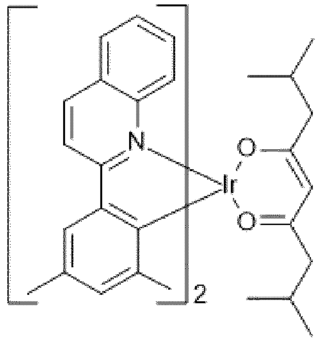 | 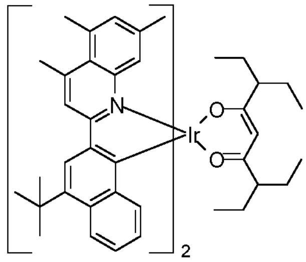 |
| 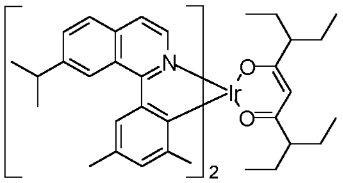 | 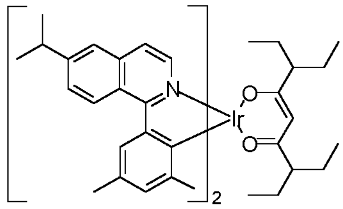 | 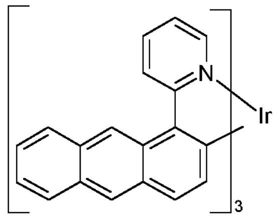 |
| 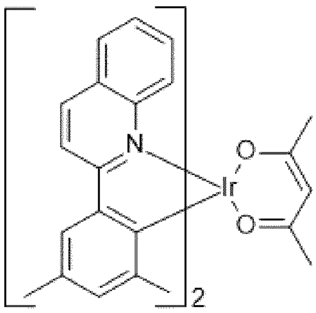 | 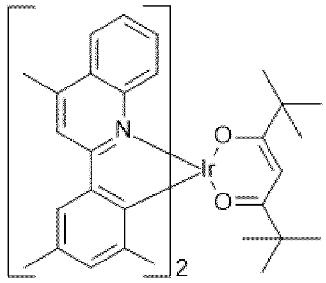 | 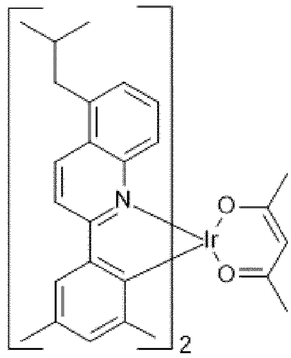 |
| 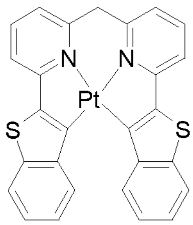 | 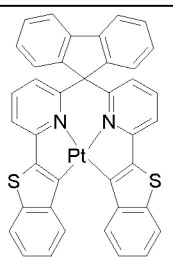 | 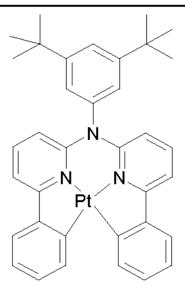 |
| 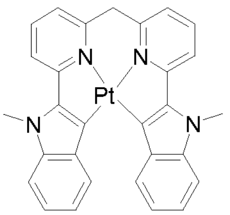 | 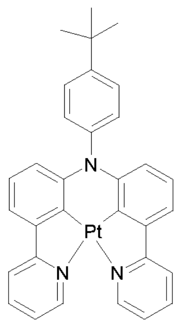 | 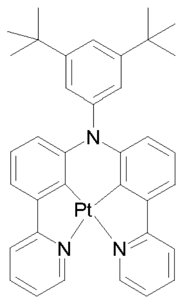 |

| | | |
|---|---|---|
| 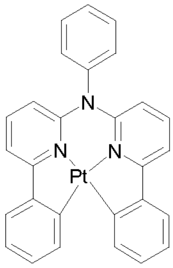 | 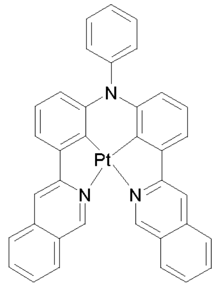 | 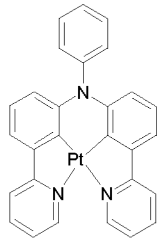 |
| 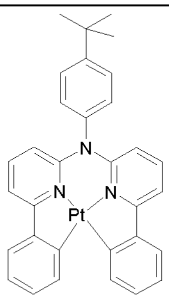 | 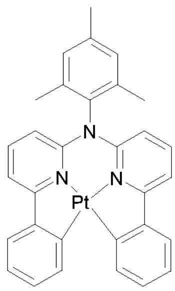 | 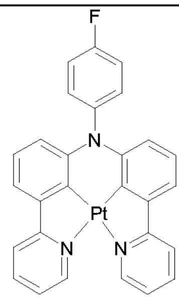 |
| 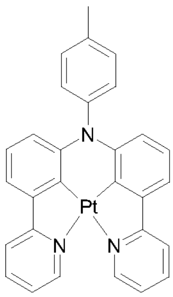 | 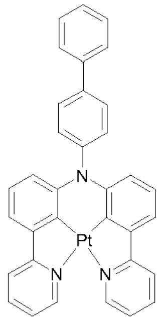 | 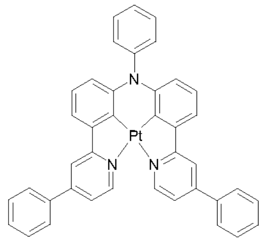 |
| 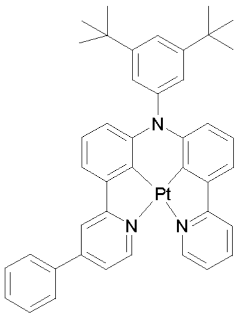 | 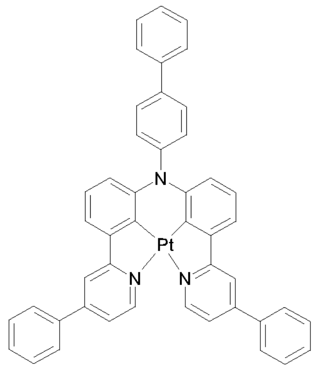 | 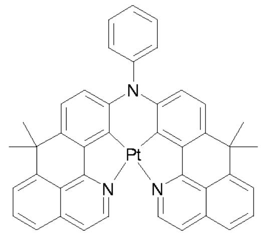 |
| 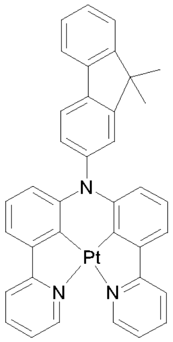 | 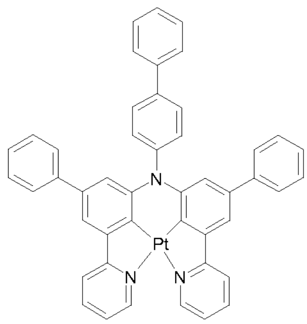 | 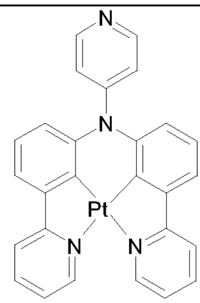 |

| | | |
|---|---|---|
| 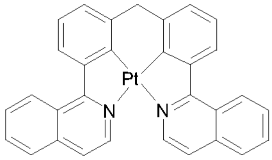 | 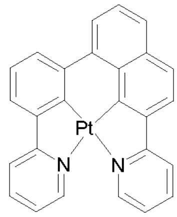 | 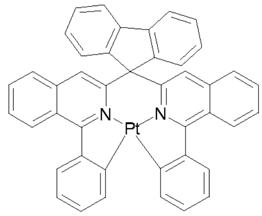 |
| 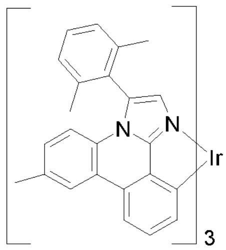 | 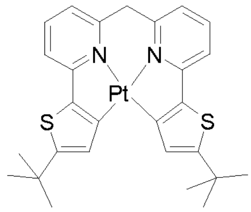 | 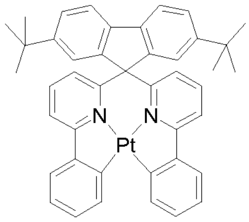 |
| 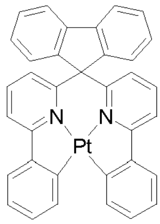 | 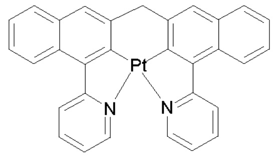 | 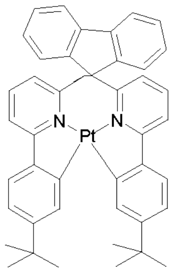 |
| 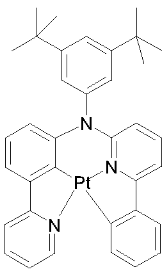 | 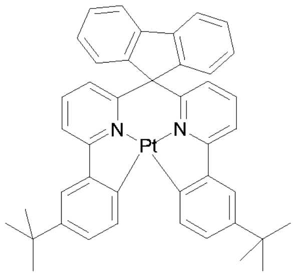 | 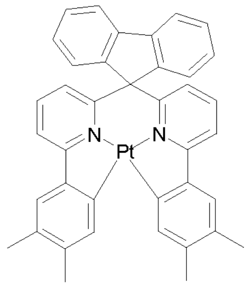 |
| 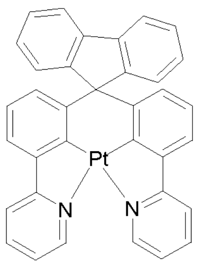 | 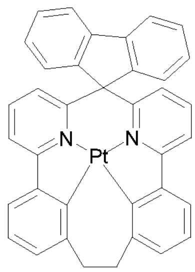 | 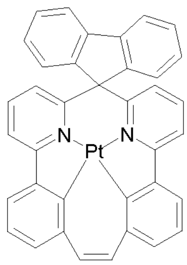 |
| 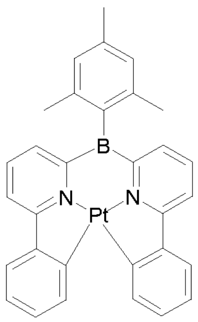 | 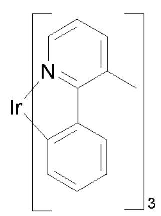 | 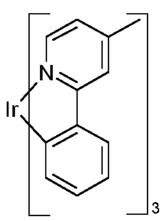 |

| | | |
|---|---|---|
| 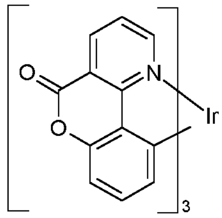 | 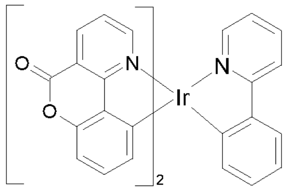 | 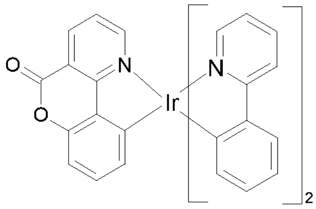 |
| 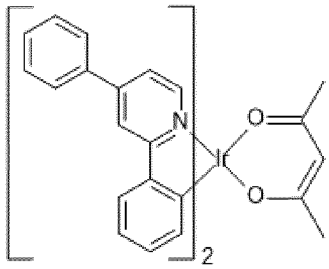 | 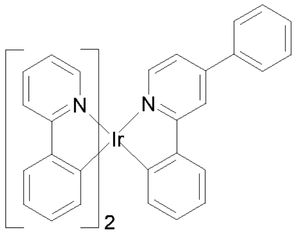 | 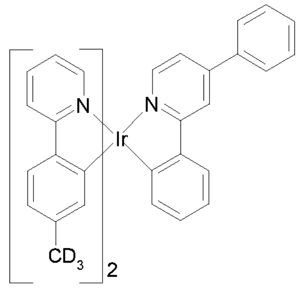 |
| 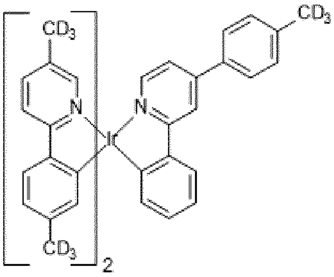 | 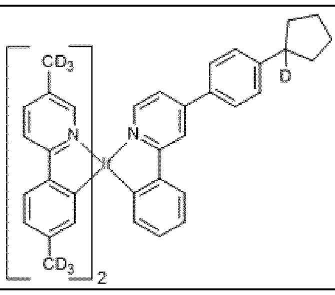 | 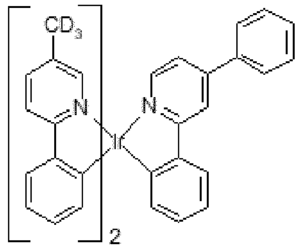 |
| 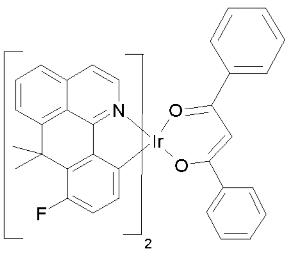 | 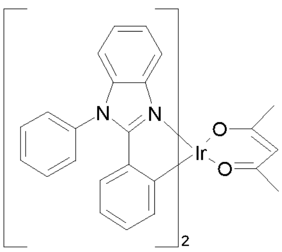 | 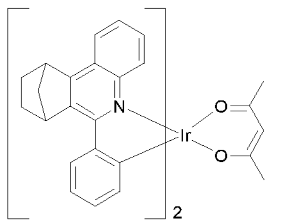 |
| 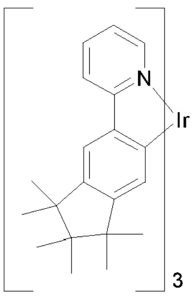 | 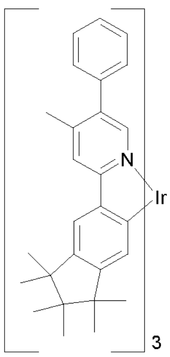 | 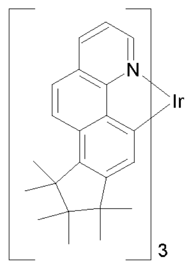 |

| | | |
|---|---|---|
| 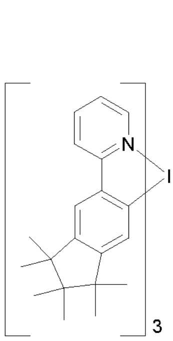 | 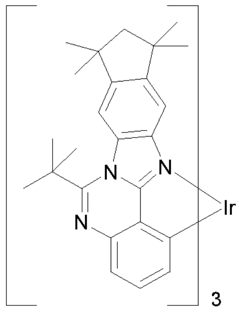 | 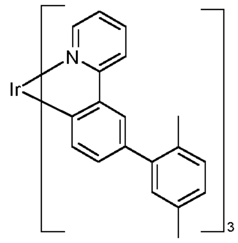 |
| 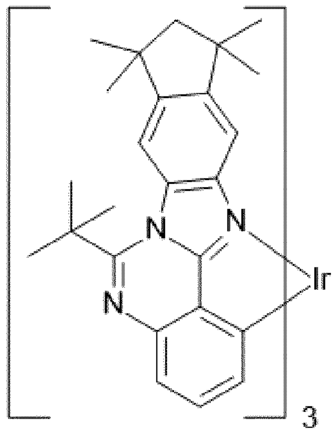 | 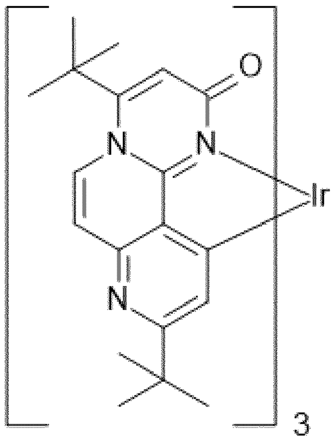 | 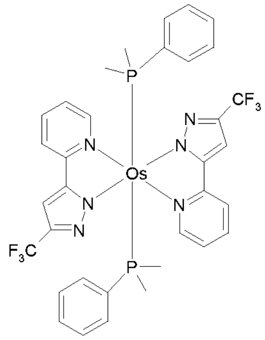 |
| 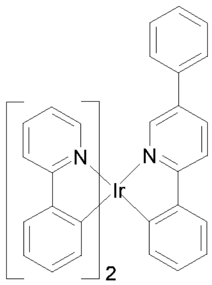 | 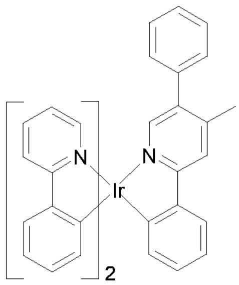 | 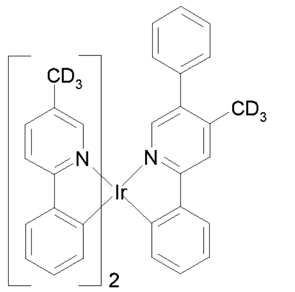 |
| 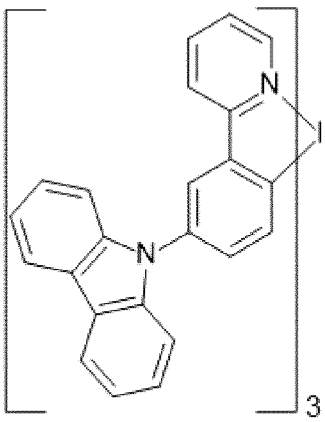 | 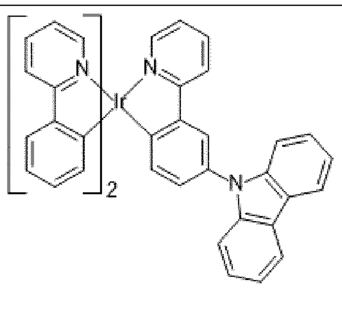 | 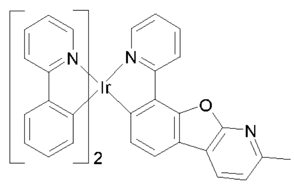 |

| | | |
|---|---|---|
| 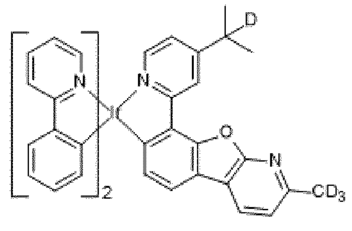 | 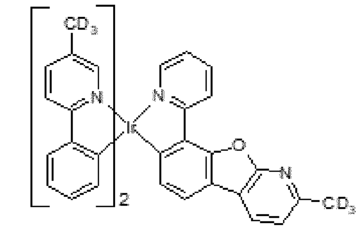 | 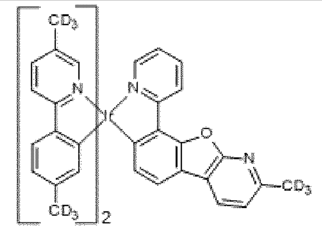 |
| 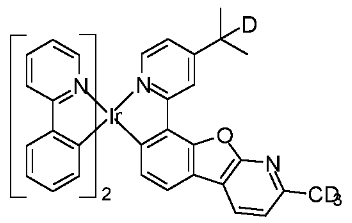 | 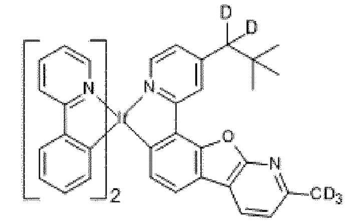 | 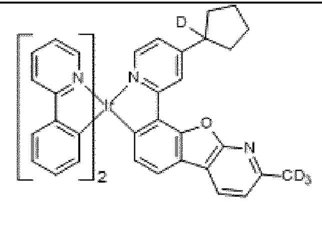 |
| 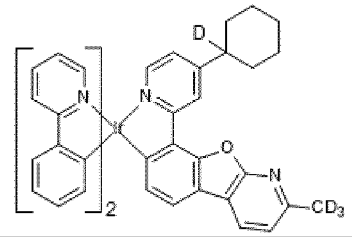 | 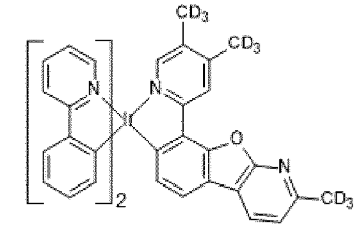 | 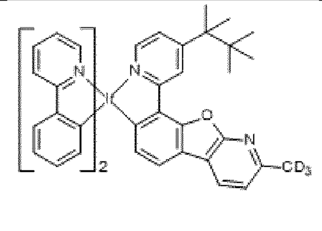 |
| 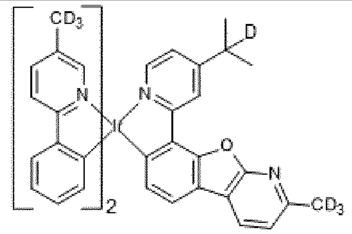 | 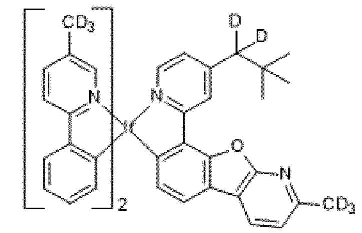 | 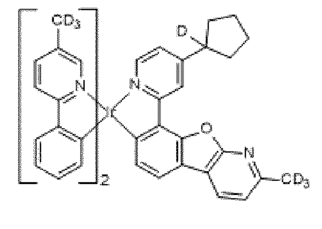 |
| 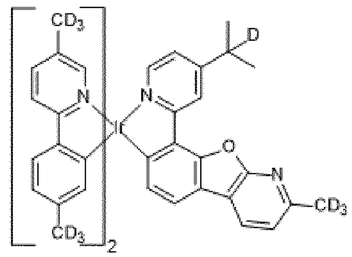 | 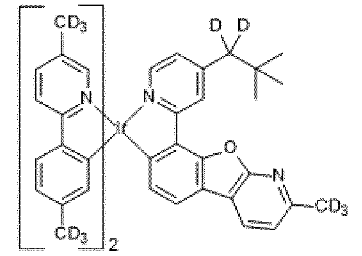 | 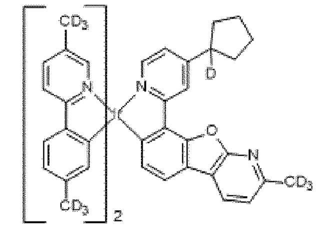 |
| 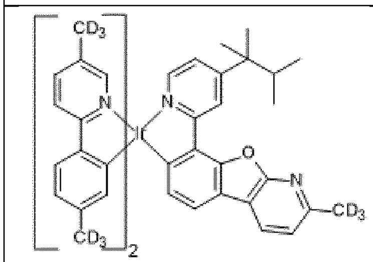 | 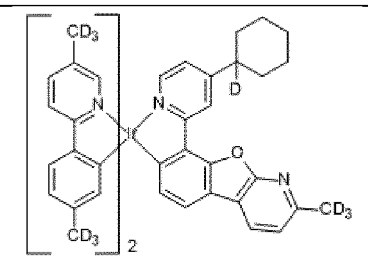 | 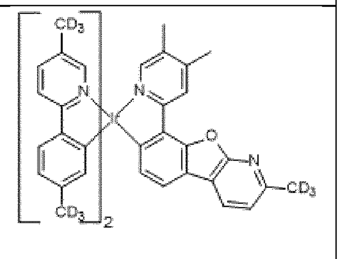 |
| 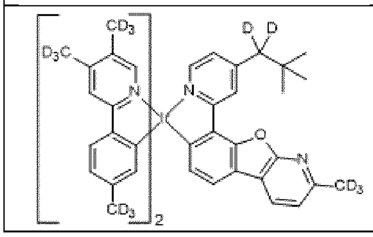 | 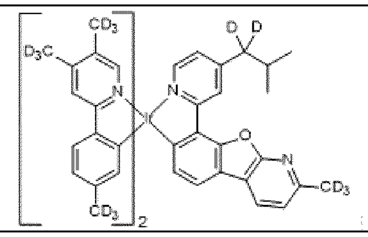 | 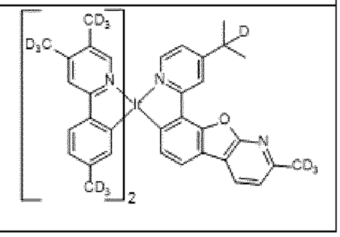 |

| | | |
|---|---|---|
| 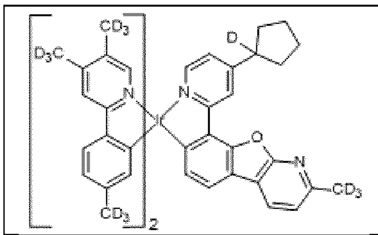 | 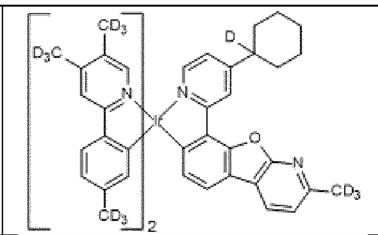 | 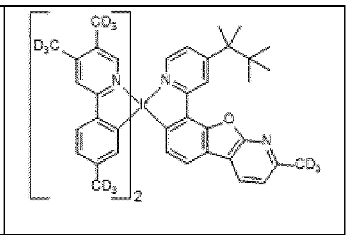 |
| 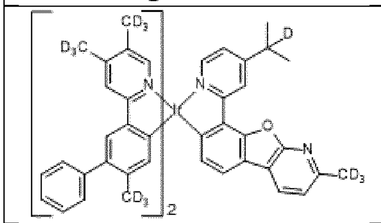 | 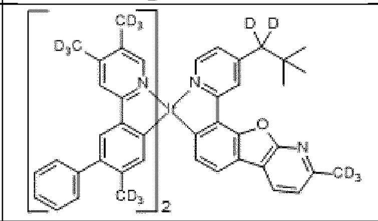 | 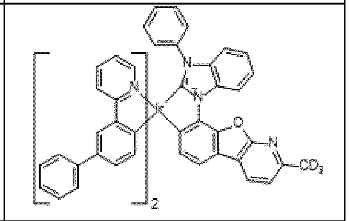 |
| 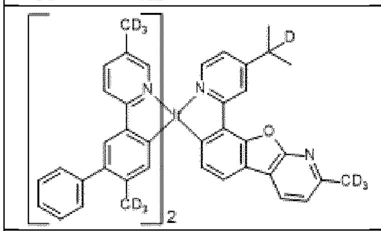 | 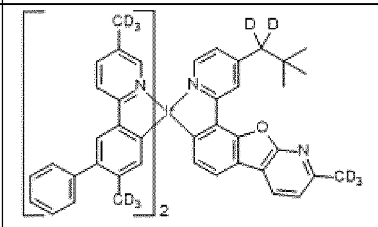 | 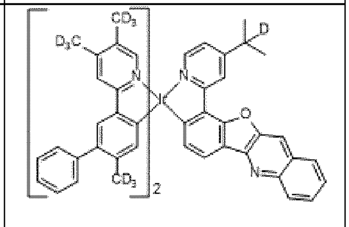 |
| 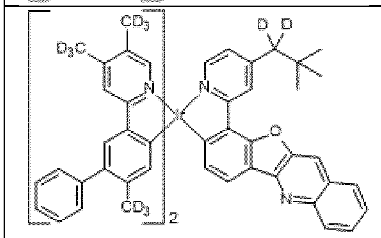 | 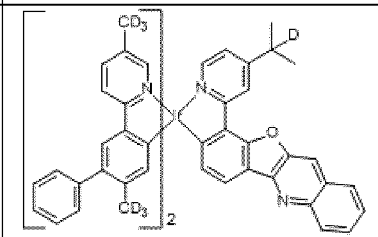 | 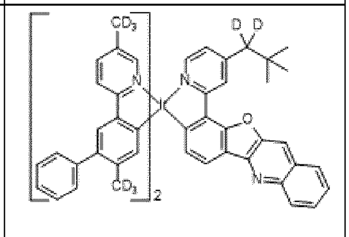 |
| 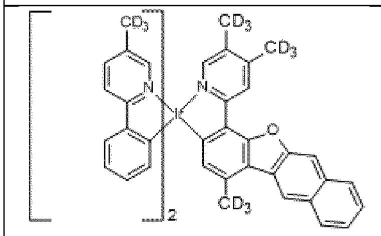 | 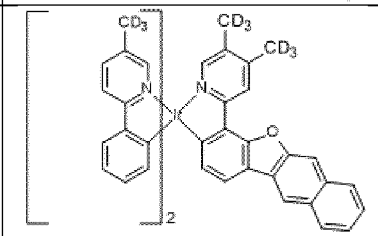 | 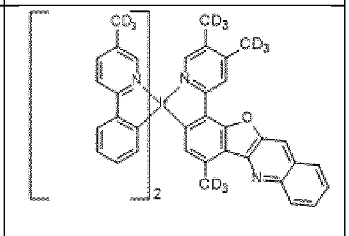 |
| 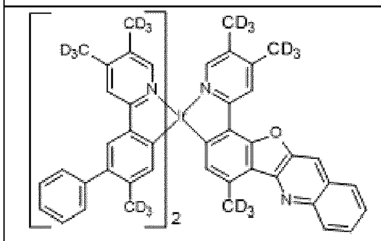 | 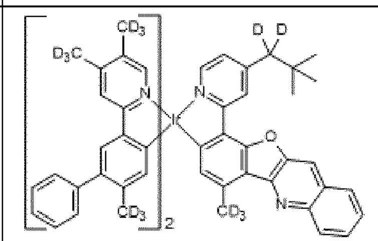 | 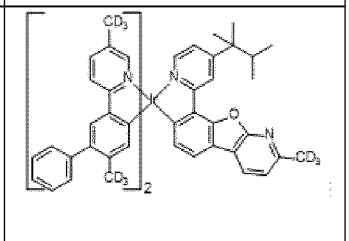 |
| 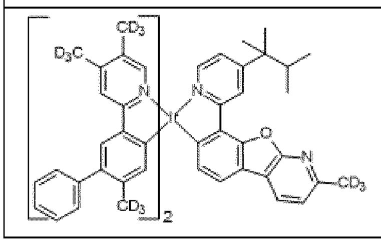 | 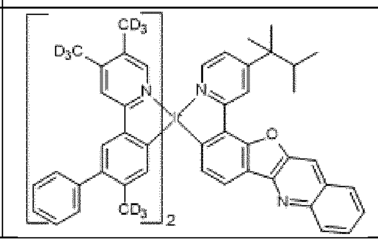 | 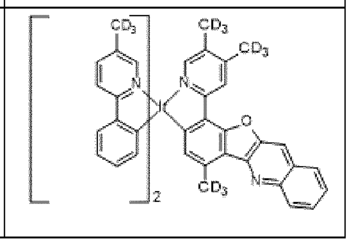 |

| | | |
|---|---|---|
| 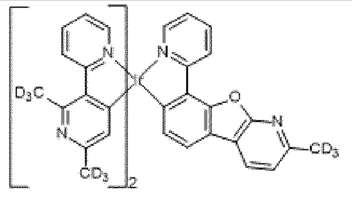 | 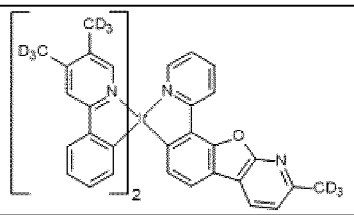 | 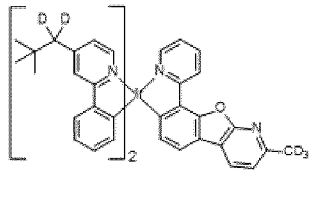 |
| 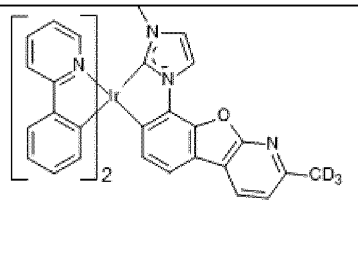 | 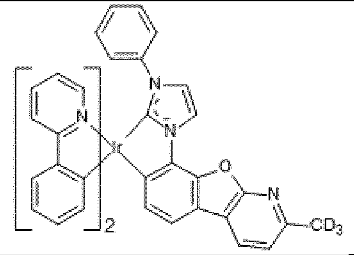 | 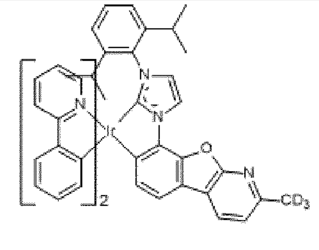 |
| 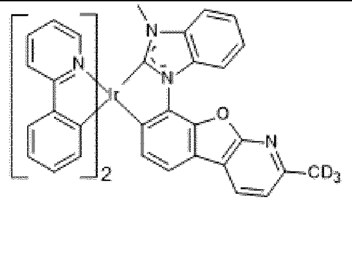 | 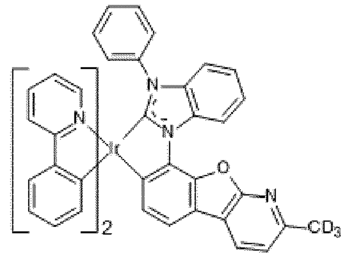 | 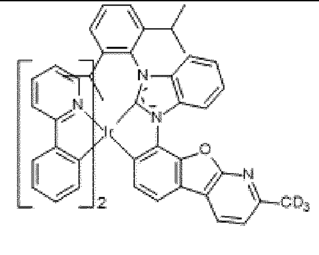 |
| 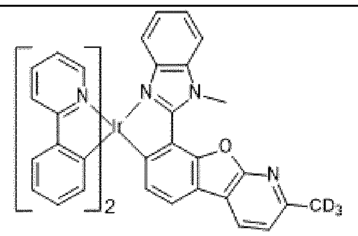 | 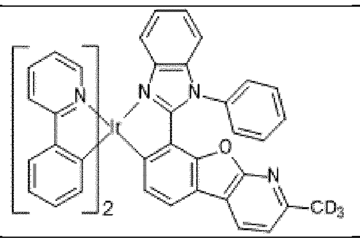 | 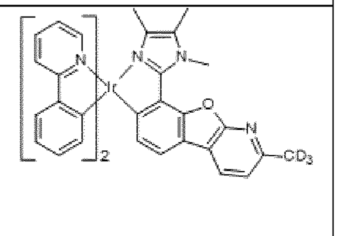 |
| 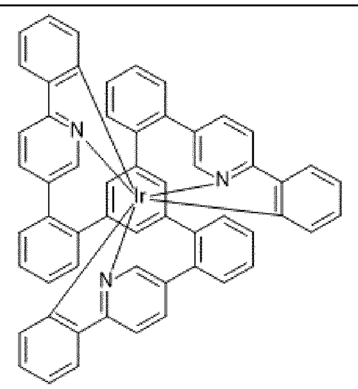 | 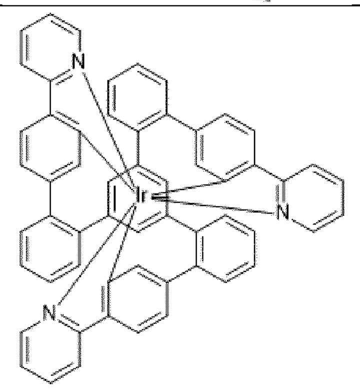 | 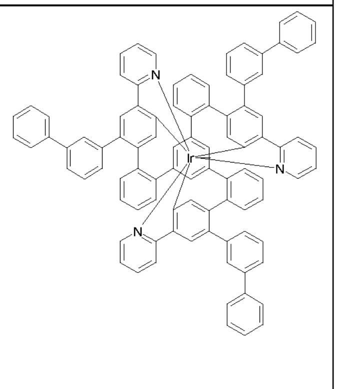 |
| 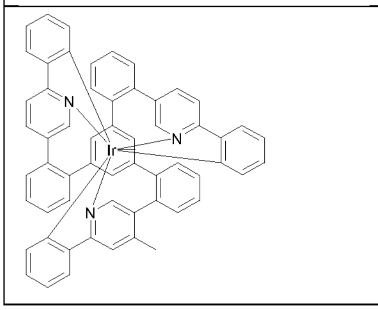 | 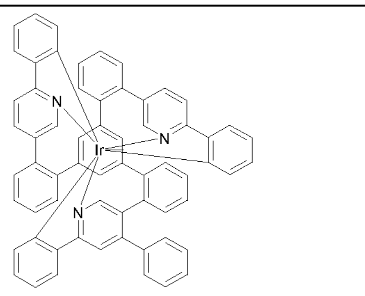 | 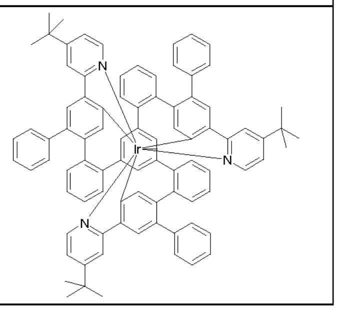 |

| | | |
|---|---|---|
| 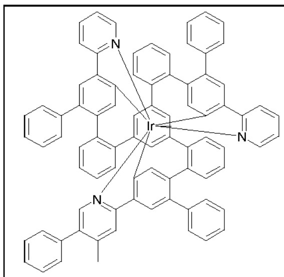 | 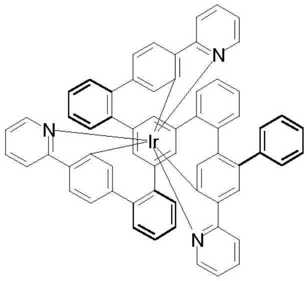 | 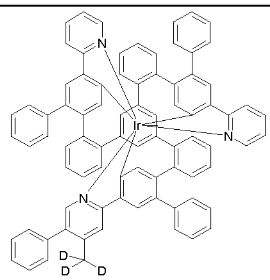 |
| 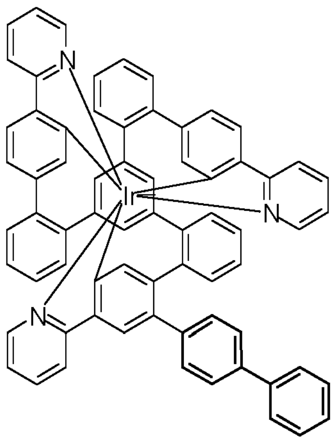 | 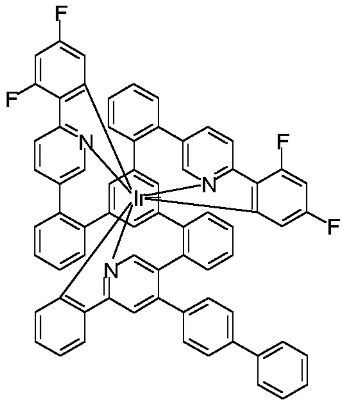 | 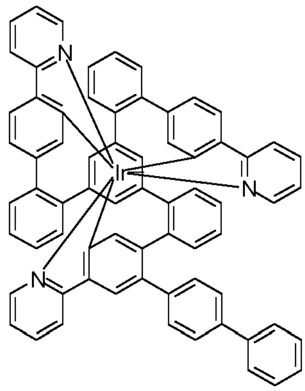 |
| 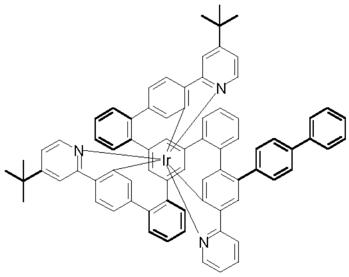 | 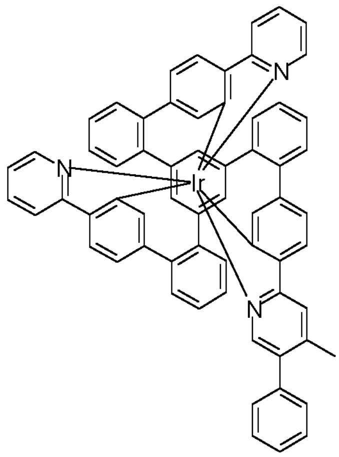 | 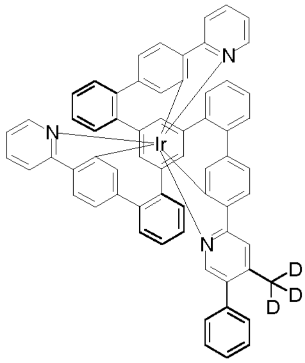 |
| 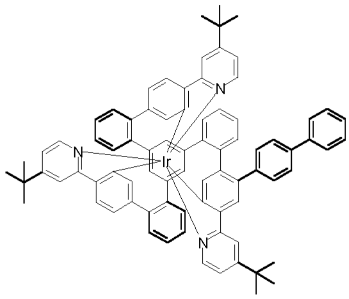 | 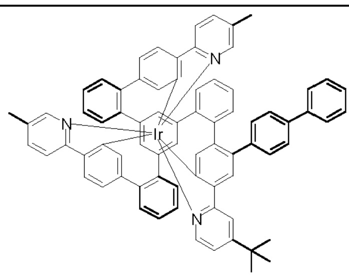 | 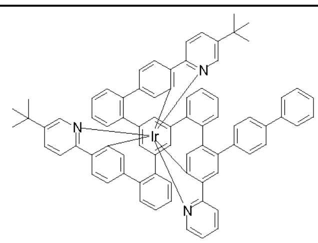 |

| | | |
|---|---|---|
| 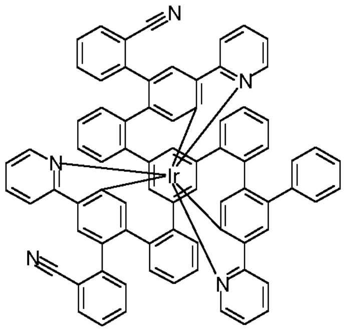 | 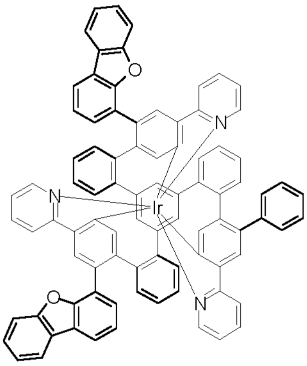 | 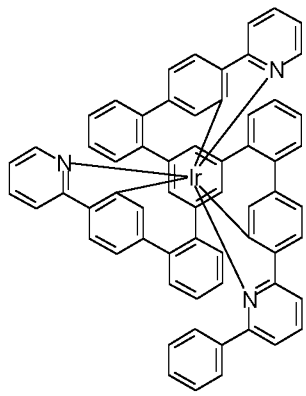 |
| 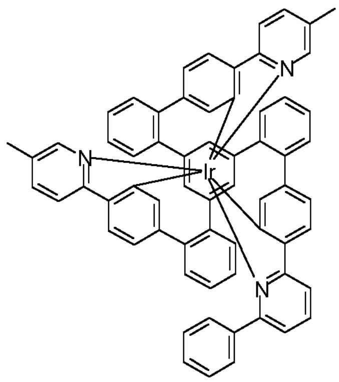 | 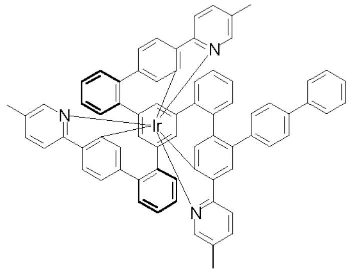 | 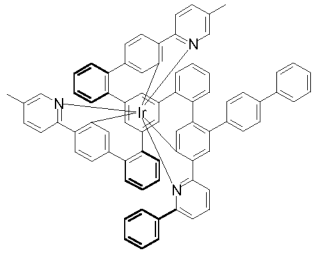 |
| 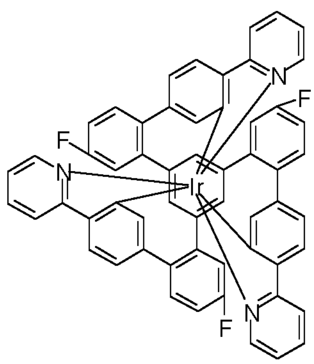 | 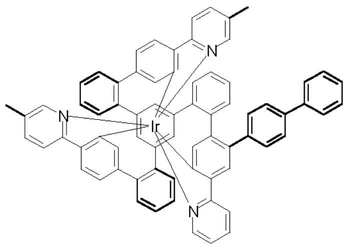 | 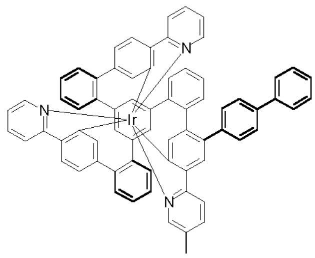 |
| 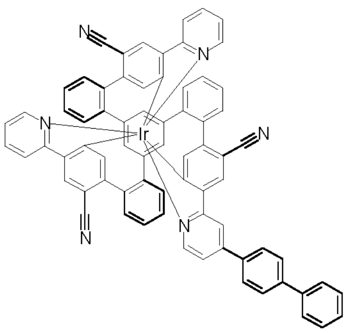 | 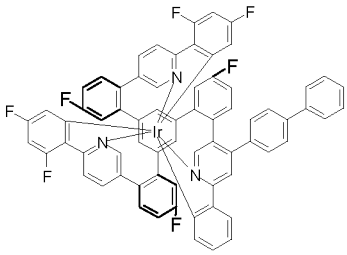 | 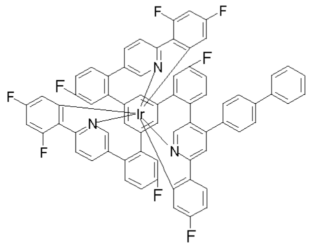 |

| | | |
|---|---|---|
| 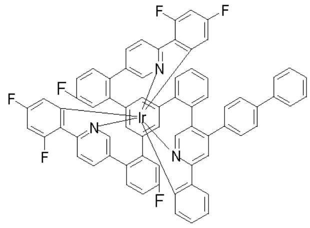 | 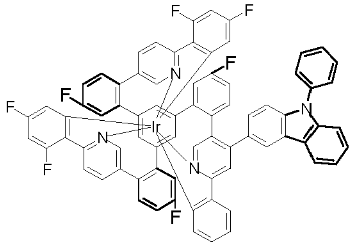 | 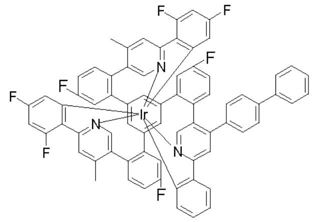 |
| 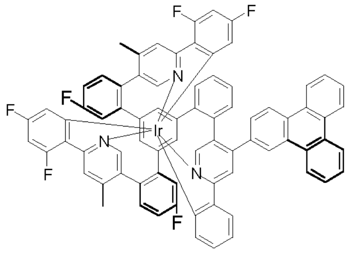 | 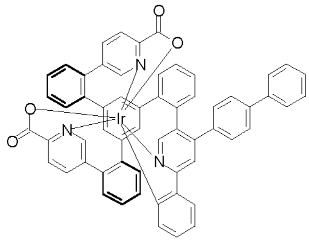 | 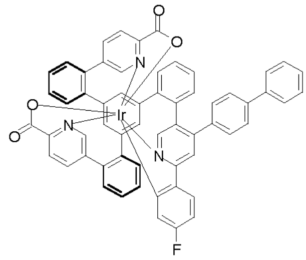 |
| 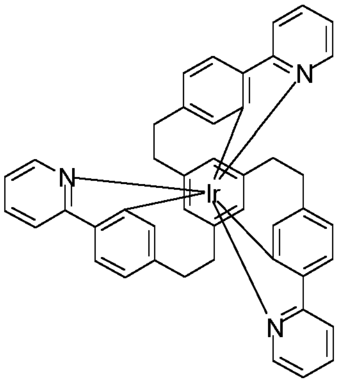 | 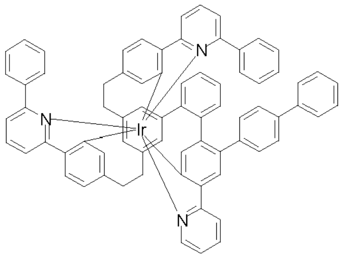 | 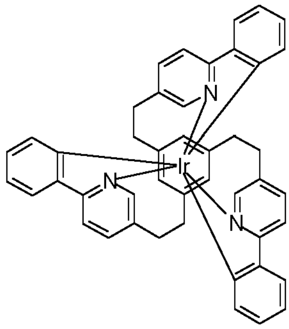 |
| 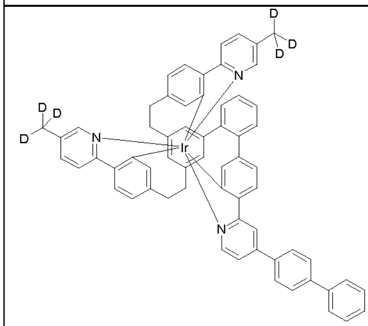 | 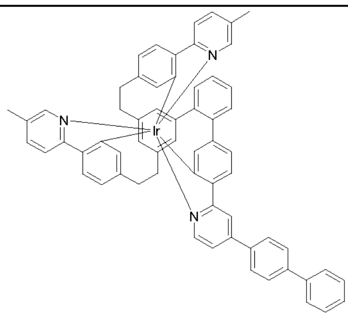 | 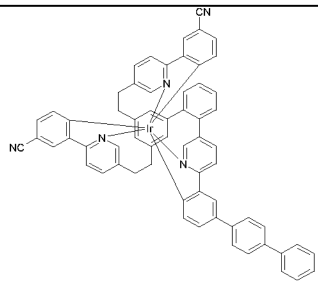 |
| 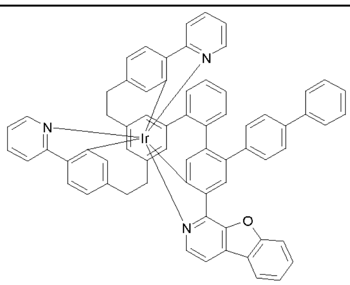 | 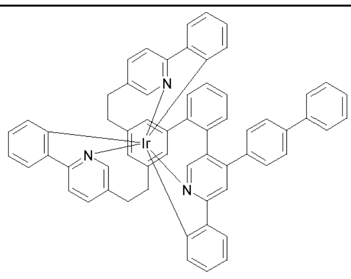 | 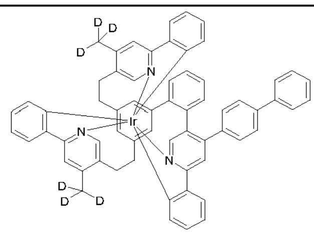 |

| | | |
|---|---|---|
| 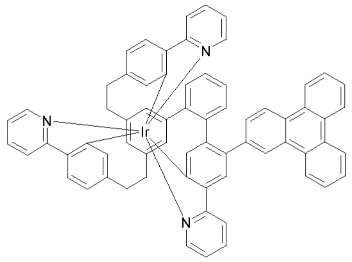 | 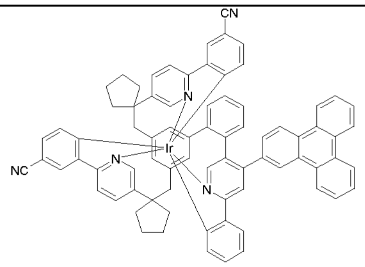 | 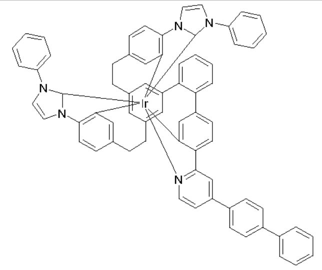 |
| 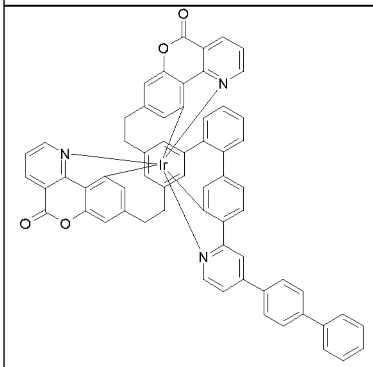 | 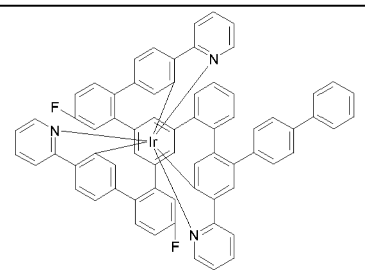 | 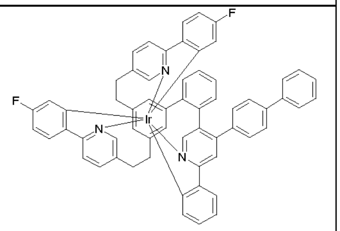 |
| 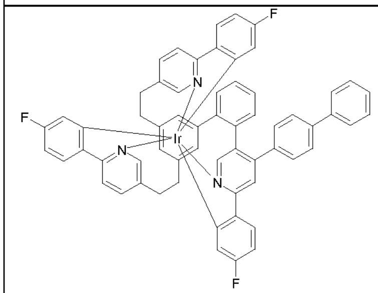 | 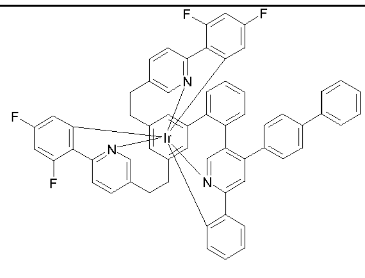 | 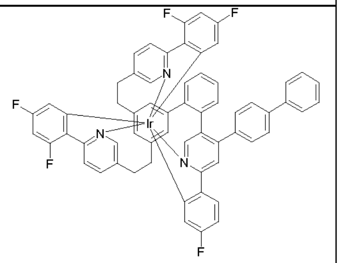 |
| 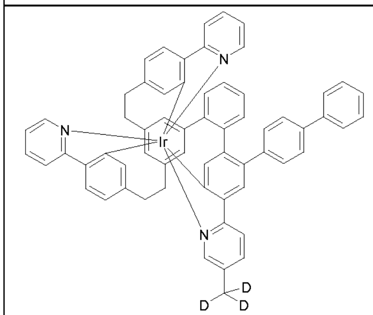 | 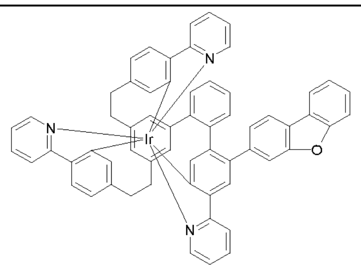 | 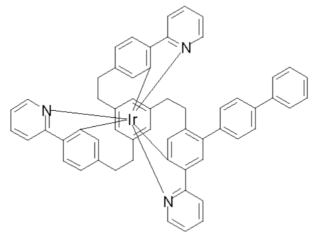 |
| 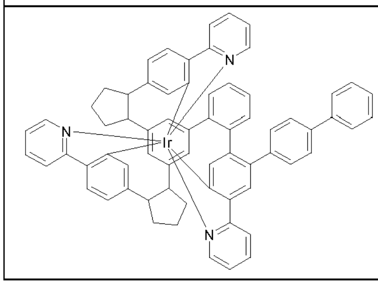 | 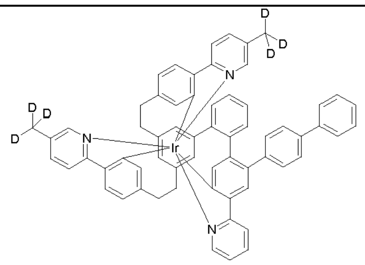 | 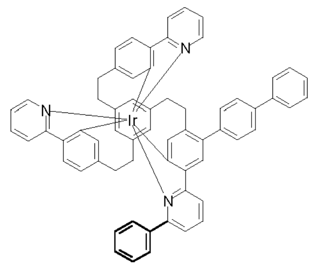 |

| | | |
|---|---|---|
| 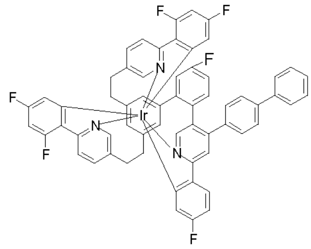 | 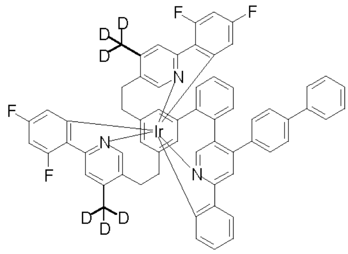 | 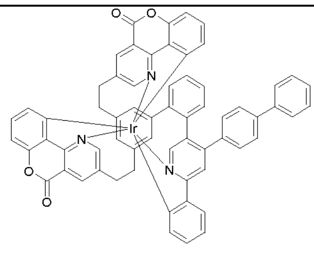 |
| 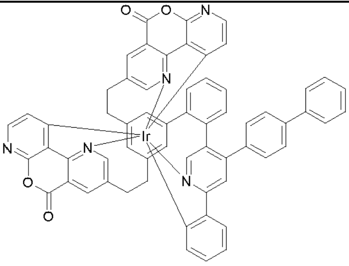 | 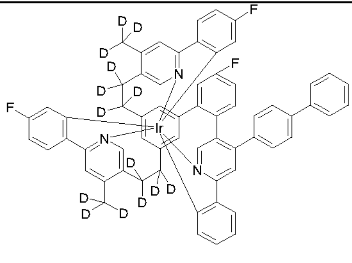 | 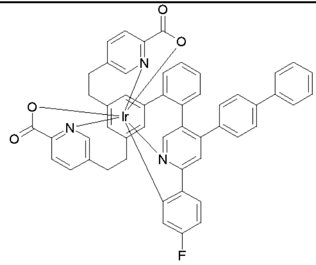 |
| 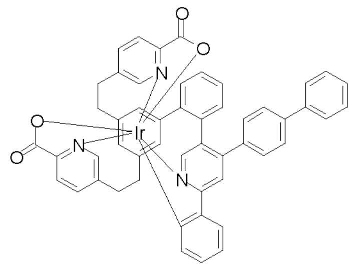 | 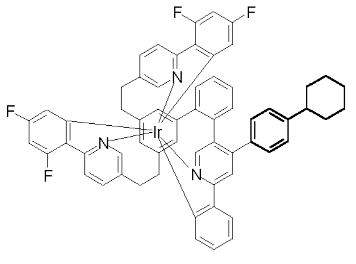 | 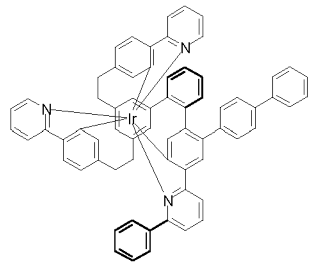 |
| 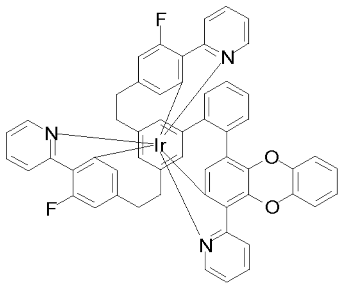 | 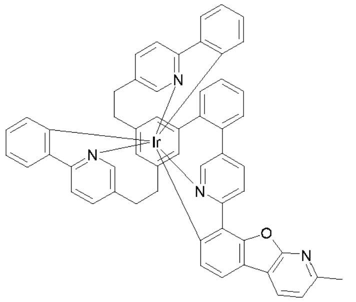 | 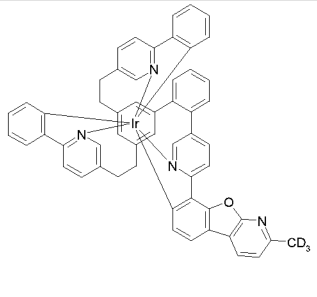 |
| 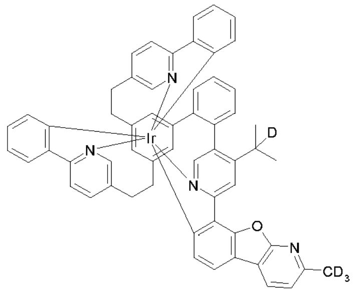 | 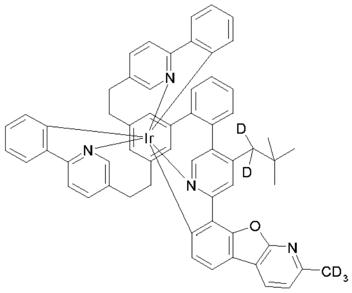 | 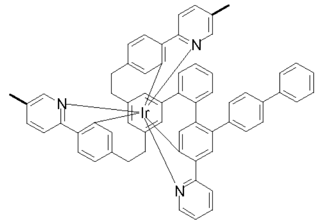 |
| 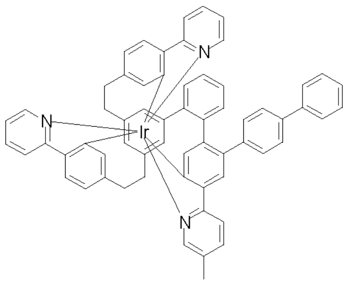 | 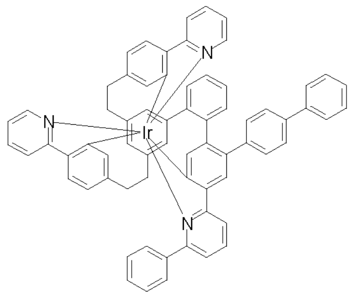 | 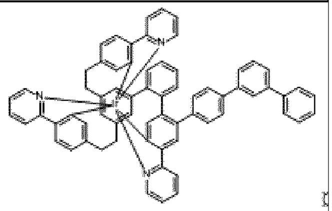 |

| | | |
|---|---|---|
| 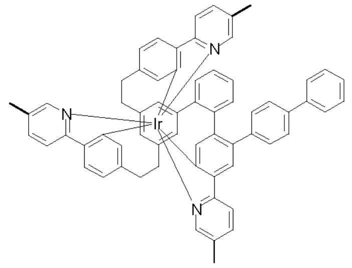 | 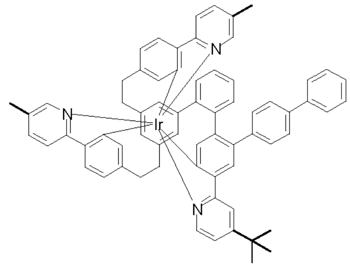 | 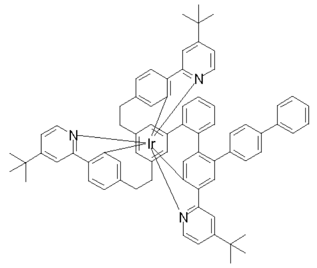 |
| 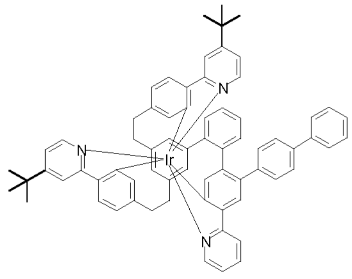 | 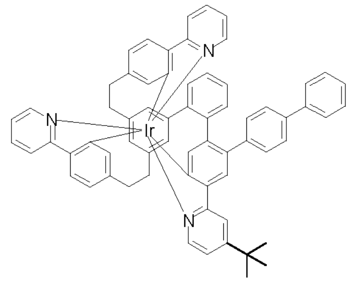 | 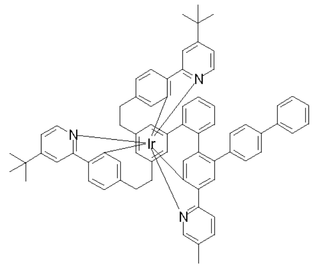 |
| 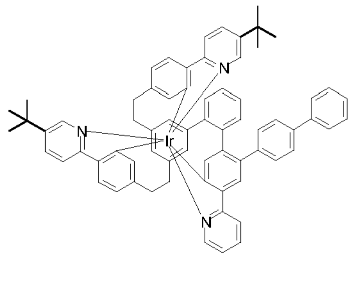 | 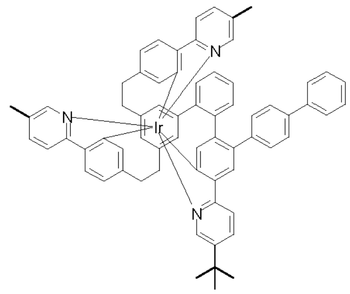 | 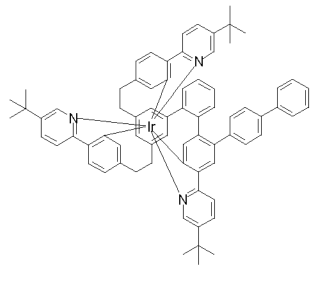 |
| 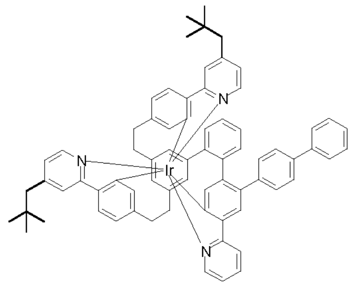 | 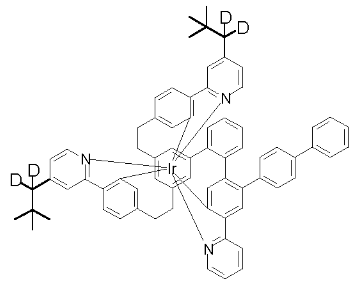 | 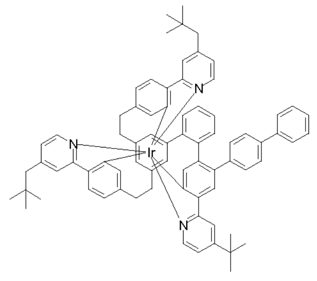 |
| 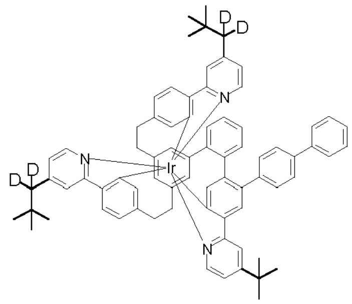 | 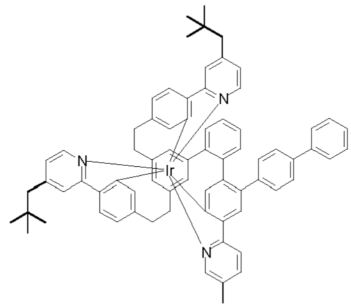 | 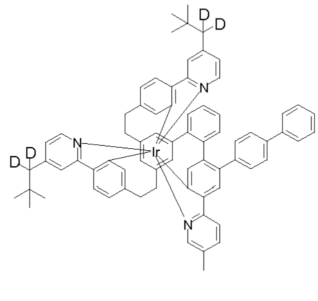 |
| 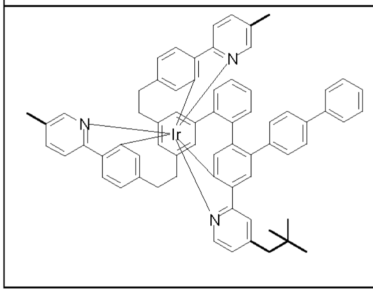 | 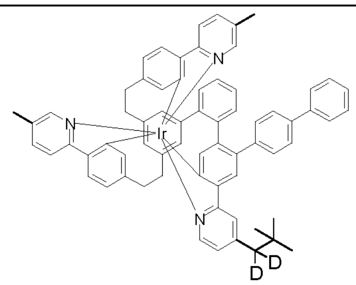 | 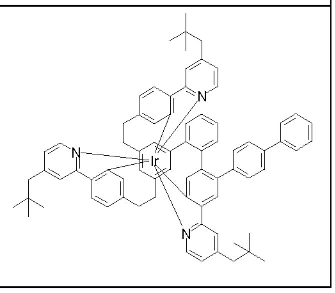 |

| | | |
|---|---|---|
| 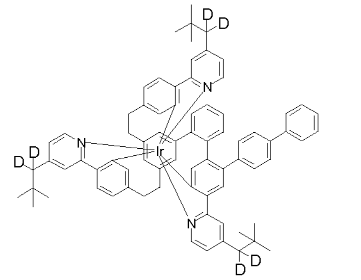 | 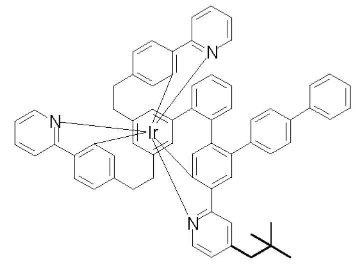 | 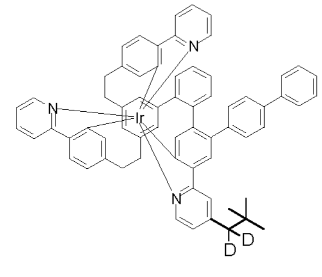 |
| 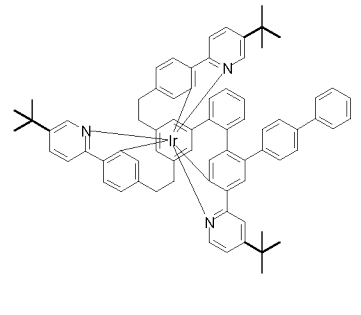 | 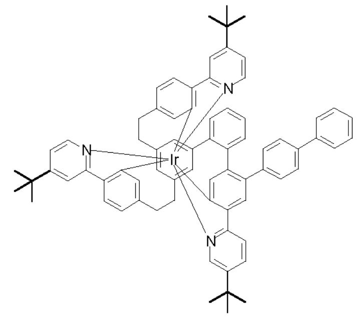 | 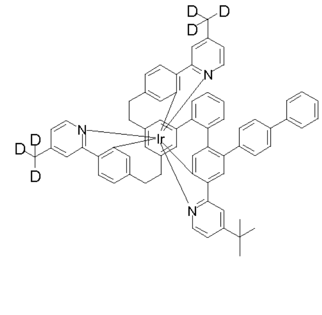 |
| 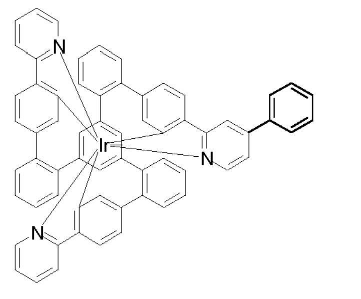 | 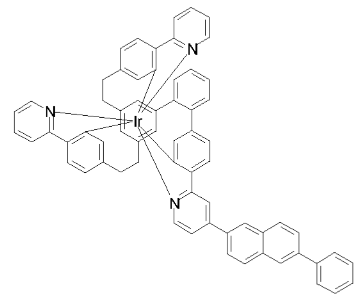 | 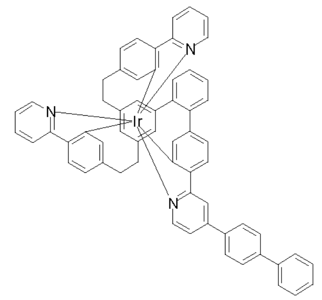 |
| 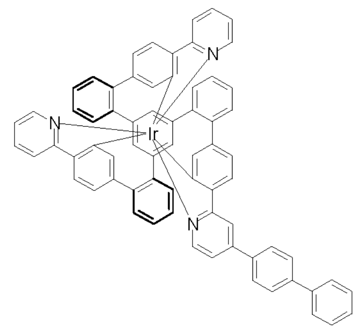 | 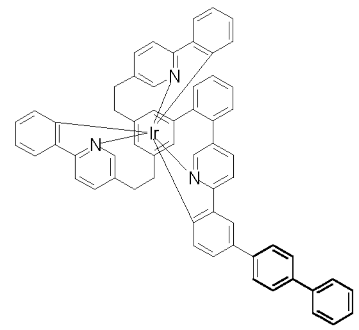 | 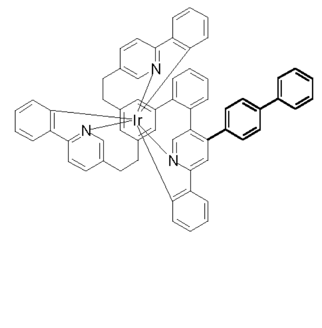 |
| 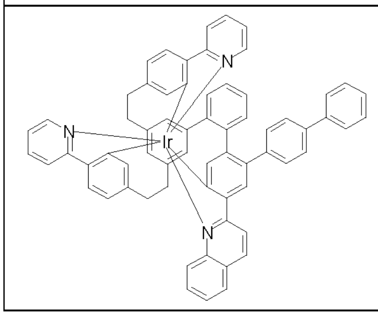 | 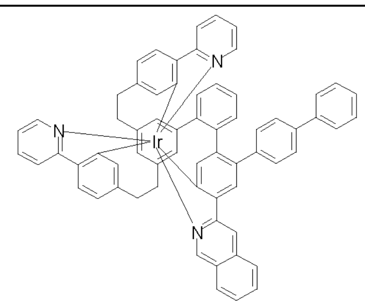 | 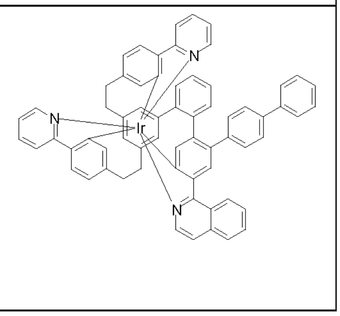 |

| | | |
|---|---|---|
| 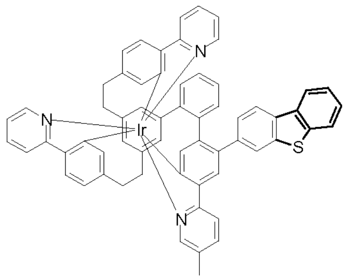 | 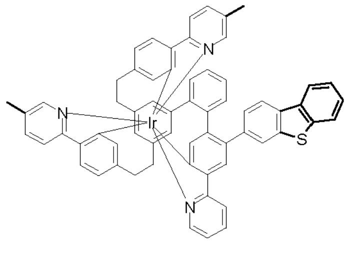 | 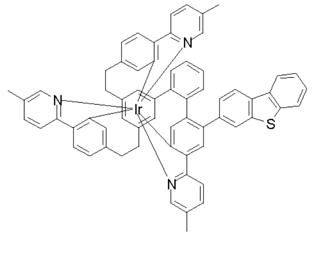 |
| 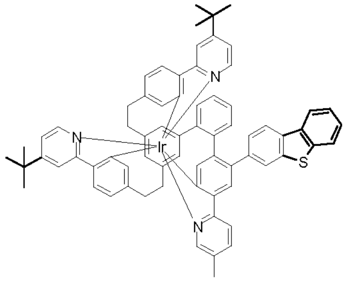 | 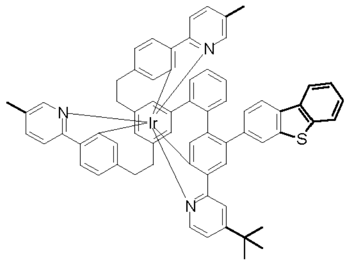 | 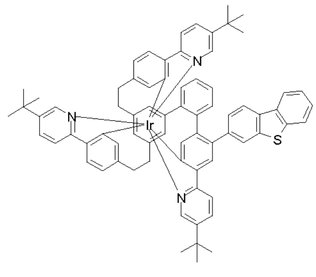 |
| 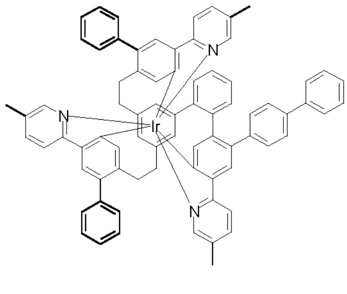 | 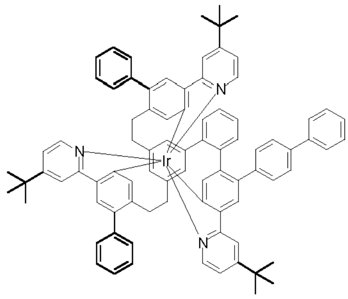 | 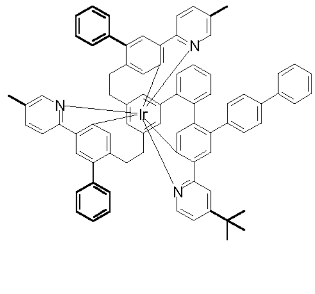 |
| 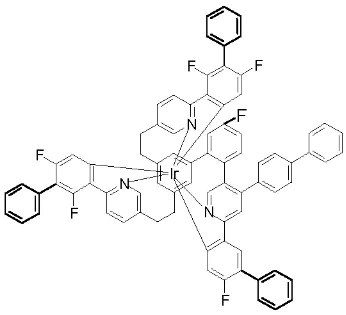 | 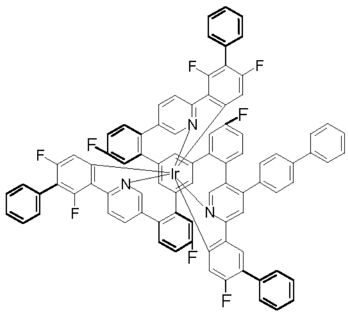 | 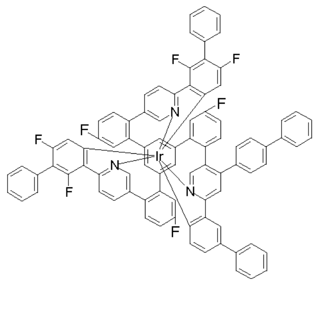 |
| 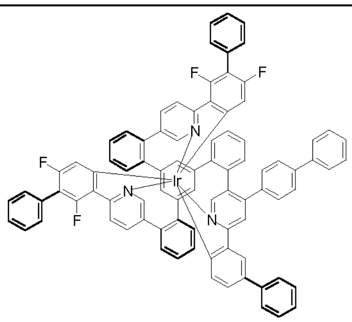 | 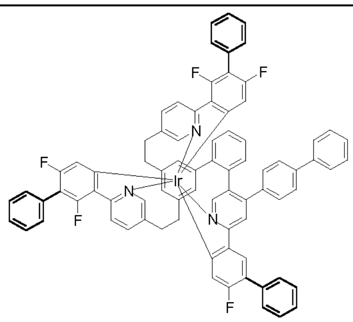 | 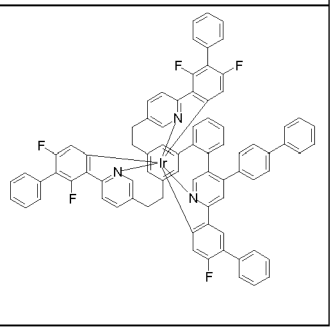 |

| | | |
|---|---|---|
| 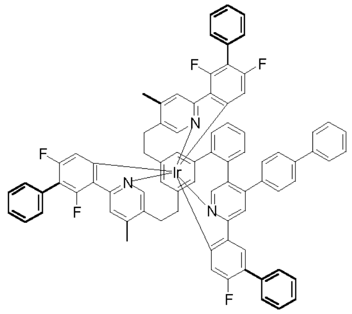 | 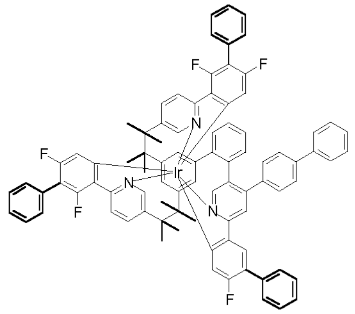 | 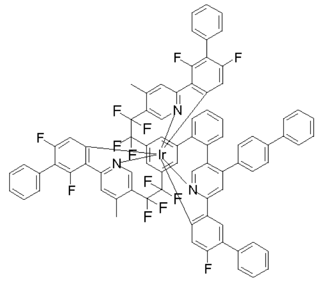 |

Bevorzugte Beispiele von phosphoreszierenden polypodalen Emittern sind in der folgenden Tabelle 5 aufgeführt.

Tabelle 5:

| | | | |
|---|---|---|---|
| CAS-1269508-30-6 | CAS-1989601-68-4 | CAS-1989602-19-8 | CAS-1989602-70-1 |
| CAS-1215692-34-4 | CAS-1989601-69-5 | CAS-1989602-20-1 | CAS-1989602-71-2 |
| CAS-1370364-40-1 | CAS-1989601-70-8 | CAS-1989602-21-2 | CAS-1989602-72-3 |
| CAS-1370364-42-3 | CAS-1989601-71-9 | CAS-1989602-22-3 | CAS-1989602-73-4 |
| CAS-1989600-74-9 | CAS-1989601-72-0 | CAS-1989602-23-4 | CAS-1989602-74-5 |
| CAS-1989600-75-0 | CAS-1989601-73-1 | CAS-1989602-24-5 | CAS-1989602-75-6 |
| CAS-1989600-77-2 | CAS-1989601-74-2 | CAS-1989602-25-6 | CAS-1989602-76-7 |
| CAS-1989600-78-3 | CAS-1989601-75-3 | CAS-1989602-26-7 | CAS-1989602-77-8 |
| CAS-1989600-79-4 | CAS-1989601-76-4 | CAS-1989602-27-8 | CAS-1989602-78-9 |
| CAS-1989600-82-9 | CAS-1989601-77-5 | CAS-1989602-28-9 | CAS-1989602-79-0 |
| CAS-1989600-83-0 | CAS-1989601-78-6 | CAS-1989602-29-0 | CAS-1989602-80-3 |
| CAS-1989600-84-1 | CAS-1989601-79-7 | CAS-1989602-30-3 | CAS-1989602-82-5 |
| CAS-1989600-85-2 | CAS-1989601-80-0 | CAS-1989602-31-4 | CAS-1989602-84-7 |
| CAS-1989600-86-3 | CAS-1989601-81-1 | CAS-1989602-32-5 | CAS-1989602-85-8 |
| CAS-1989600-87-4 | CAS-1989601-82-2 | CAS-1989602-33-6 | CAS-1989602-86-9 |
| CAS-1989600-88-5 | CAS-1989601-83-3 | CAS-1989602-34-7 | CAS-1989602-87-0 |
| CAS-1989600-89-6 | CAS-1989601-84-4 | CAS-1989602-35-8 | CAS-1989602-88-1 |
| CAS-1989601-11-7 | CAS-1989601-85-5 | CAS-1989602-36-9 | CAS-1989604-00-3 |
| CAS-1989601-23-1 | CAS-1989601-86-6 | CAS-1989602-37-0 | CAS-1989604-01-4 |
| CAS-1989601-26-4 | CAS-1989601-87-7 | CAS-1989602-38-1 | CAS-1989604-02-5 |
| CAS-1989601-28-6 | CAS-1989601-88-8 | CAS-1989602-39-2 | CAS-1989604-03-6 |
| CAS-1989601-29-7 | CAS-1989601-89-9 | CAS-1989602-40-5 | CAS-1989604-04-7 |
| CAS-1989601-33-3 | CAS-1989601-90-2 | CAS-1989602-41-6 | CAS-1989604-05-8 |
| CAS-1989601-40-2 | CAS-1989601-91-3 | CAS-1989602-42-7 | CAS-1989604-06-9 |
| CAS-1989601-41-3 | CAS-1989601-92-4 | CAS-1989602-43-8 | CAS-1989604-07-0 |
| CAS-1989601-42-4 | CAS-1989601-93-5 | CAS-1989602-44-9 | CAS-1989604-08-1 |
| CAS-1989601-43-5 | CAS-1989601-94-6 | CAS-1989602-45-0 | CAS-1989604-09-2 |
| CAS-1989601-44-6 | CAS-1989601-95-7 | CAS-1989602-46-1 | CAS-1989604-10-5 |
| CAS-1989601-45-7 | CAS-1989601-96-8 | CAS-1989602-47-2 | CAS-1989604-11-6 |

(fortgesetzt)

| | | | |
|---|---|---|---|
| CAS-1989601-46-8 | CAS-1989601-97-9 | CAS-1989602-48-3 | CAS-1989604-13-8 |
| CAS-1989601-47-9 | CAS-1989601-98-0 | CAS-1989602-49-4 | CAS-1989604-14-9 |
| CAS-1989601-48-0 | CAS-1989601-99-1 | CAS-1989602-50-7 | CAS-1989604-15-0 |
| CAS-1989601-49-1 | CAS-1989602-00-7 | CAS-1989602-51-8 | CAS-1989604-16-1 |
| CAS-1989601-50-4 | CAS-1989602-01-8 | CAS-1989602-52-9 | CAS-1989604-17-2 |
| CAS-1989601-51-5 | CAS-1989602-02-9 | CAS-1989602-53-0 | CAS-1989604-18-3 |
| CAS-1989601-52-6 | CAS-1989602-03-0 | CAS-1989602-54-1 | CAS-1989604-19-4 |
| CAS-1989601-53-7 | CAS-1989602-04-1 | CAS-1989602-55-2 | CAS-1989604-20-7 |
| CAS-1989601-54-8 | CAS-1989602-05-2 | CAS-1989602-56-3 | CAS-1989604-21-8 |
| CAS-1989601-55-9 | CAS-1989602-06-3 | CAS-1989602-57-4 | CAS-1989604-22-9 |
| CAS-1989601-56-0 | CAS-1989602-07-4 | CAS-1989602-58-5 | CAS-1989604-23-0 |
| CAS-1989601-57-1 | CAS-1989602-08-5 | CAS-1989602-59-6 | CAS-1989604-24-1 |
| CAS-1989601-58-2 | CAS-1989602-09-6 | CAS-1989602-60-9 | CAS-1989604-25-2 |
| CAS-1989601-59-3 | CAS-1989602-10-9 | CAS-1989602-61-0 | CAS-1989604-26-3 |
| CAS-1989601-60-6 | CAS-1989602-11-0 | CAS-1989602-62-1 | CAS-1989604-27-4 |
| CAS-1989601-61-7 | CAS-1989602-12-1 | CAS-1989602-63-2 | CAS-1989604-28-5 |
| CAS-1989601-62-8 | CAS-1989602-13-2 | CAS-1989602-64-3 | CAS-1989604-29-6 |
| CAS-1989601-63-9 | CAS-1989602-14-3 | CAS-1989602-65-4 | CAS-1989604-30-9 |
| CAS-1989601-64-0 | CAS-1989602-15-4 | CAS-1989602-66-5 | CAS-1989604-31-0 |
| CAS-1989601-65-1 | CAS-1989602-16-5 | CAS-1989602-67-6 | CAS-1989604-32-1 |
| CAS-1989601-66-2 | CAS-1989602-17-6 | CAS-1989602-68-7 | CAS-1989604-33-2 |
| CAS-1989601-67-3 | CAS-1989602-18-7 | CAS-1989602-69-8 | CAS-1989604-34-3 |
| CAS-1989604-35-4 | CAS-1989604-88-7 | CAS-1989605-52-8 | CAS-1989606-07-6 |
| CAS-1989604-36-5 | CAS-1989604-89-8 | CAS-1989605-53-9 | CAS-1989606-08-7 |
| CAS-1989604-37-6 | CAS-1989604-90-1 | CAS-1989605-54-0 | CAS-1989606-09-8 |
| CAS-1989604-38-7 | CAS-1989604-92-3 | CAS-1989605-55-1 | CAS-1989606-10-1 |
| CAS-1989604-39-8 | CAS-1989604-93-4 | CAS-1989605-56-2 | CAS-1989606-11-2 |
| CAS-1989604-40-1 | CAS-1989604-94-5 | CAS-1989605-57-3 | CAS-1989606-12-3 |
| CAS-1989604-41-2 | CAS-1989604-95-6 | CAS-1989605-58-4 | CAS-1989606-13-4 |
| CAS-1989604-42-3 | CAS-1989604-96-7 | CAS-1989605-59-5 | CAS-1989606-14-5 |
| CAS-1989604-43-4 | CAS-1989604-97-8 | CAS-1989605-61-9 | CAS-1989606-15-6 |
| CAS-1989604-45-6 | CAS-1989605-09-5 | CAS-1989605-62-0 | CAS-1989606-16-7 |
| CAS-1989604-46-7 | CAS-1989605-10-8 | CAS-1989605-63-1 | CAS-1989606-17-8 |
| CAS-1989604-47-8 | CAS-1989605-11-9 | CAS-1989605-64-2 | CAS-1989606-18-9 |
| CAS-1989604-48-9 | CAS-1989605-13-1 | CAS-1989605-65-3 | CAS-1989606-19-0 |
| CAS-1989604-49-0 | CAS-1989605-14-2 | CAS-1989605-66-4 | CAS-1989606-20-3 |
| CAS-1989604-50-3 | CAS-1989605-15-3 | CAS-1989605-67-5 | CAS-1989606-21-4 |
| CAS-1989604-52-5 | CAS-1989605-16-4 | CAS-1989605-68-6 | CAS-1989606-22-5 |
| CAS-1989604-53-6 | CAS-1989605-17-5 | CAS-1989605-69-7 | CAS-1989606-23-6 |

(fortgesetzt)

| | | | |
|---|---|---|---|
| CAS-1989604-54-7 | CAS-1989605-18-6 | CAS-1989605-70-0 | CAS-1989606-24-7 |
| CAS-1989604-55-8 | CAS-1989605-19-7 | CAS-1989605-71-1 | CAS-1989606-26-9 |
| CAS-1989604-56-9 | CAS-1989605-20-0 | CAS-1989605-72-2 | CAS-1989606-27-0 |
| CAS-1989604-57-0 | CAS-1989605-21-1 | CAS-1989605-73-3 | CAS-1989606-28-1 |
| CAS-1989604-58-1 | CAS-1989605-22-2 | CAS-1989605-74-4 | CAS-1989606-29-2 |
| CAS-1989604-59-2 | CAS-1989605-23-3 | CAS-1989605-75-5 | CAS-1989606-30-5 |
| CAS-1989604-60-5 | CAS-1989605-24-4 | CAS-1989605-76-6 | CAS-1989606-31-6 |
| CAS-1989604-61-6 | CAS-1989605-25-5 | CAS-1989605-77-7 | CAS-1989606-32-7 |
| CAS-1989604-62-7 | CAS-1989605-26-6 | CAS-1989605-78-8 | CAS-1989606-33-8 |
| CAS-1989604-63-8 | CAS-1989605-27-7 | CAS-1989605-79-9 | CAS-1989606-34-9 |
| CAS-1989604-64-9 | CAS-1989605-28-8 | CAS-1989605-81-3 | CAS-1989606-35-0 |
| CAS-1989604-65-0 | CAS-1989605-29-9 | CAS-1989605-82-4 | CAS-1989606-36-1 |
| CAS-1989604-66-1 | CAS-1989605-30-2 | CAS-1989605-83-5 | CAS-1989606-37-2 |
| CAS-1989604-67-2 | CAS-1989605-31-3 | CAS-1989605-84-6 | CAS-1989606-38-3 |
| CAS-1989604-68-3 | CAS-1989605-32-4 | CAS-1989605-85-7 | CAS-1989606-39-4 |
| CAS-1989604-69-4 | CAS-1989605-33-5 | CAS-1989605-86-8 | CAS-1989606-40-7 |
| CAS-1989604-70-7 | CAS-1989605-34-6 | CAS-1989605-87-9 | CAS-1989606-41-8 |
| CAS-1989604-71-8 | CAS-1989605-35-7 | CAS-1989605-88-0 | CAS-1989606-42-9 |
| CAS-1989604-72-9 | CAS-1989605-36-8 | CAS-1989605-89-1 | CAS-1989606-43-0 |
| CAS-1989604-73-0 | CAS-1989605-37-9 | CAS-1989605-90-4 | CAS-1989606-44-1 |
| CAS-1989604-74-1 | CAS-1989605-38-0 | CAS-1989605-91-5 | CAS-1989606-45-2 |
| CAS-1989604-75-2 | CAS-1989605-39-1 | CAS-1989605-92-6 | CAS-1989606-46-3 |
| CAS-1989604-76-3 | CAS-1989605-40-4 | CAS-1989605-93-7 | CAS-1989606-48-5 |
| CAS-1989604-77-4 | CAS-1989605-41-5 | CAS-1989605-94-8 | CAS-1989606-49-6 |
| CAS-1989604-78-5 | CAS-1989605-42-6 | CAS-1989605-95-9 | CAS-1989606-53-2 |
| CAS-1989604-79-6 | CAS-1989605-43-7 | CAS-1989605-96-0 | CAS-1989606-55-4 |
| CAS-1989604-80-9 | CAS-1989605-44-8 | CAS-1989605-97-1 | CAS-1989606-56-5 |
| CAS-1989604-81-0 | CAS-1989605-45-9 | CAS-1989605-98-2 | CAS-1989606-61-2 |
| CAS-1989604-82-1 | CAS-1989605-46-0 | CAS-1989605-99-3 | CAS-1989606-62-3 |
| CAS-1989604-83-2 | CAS-1989605-47-1 | CAS-1989606-00-9 | CAS-1989606-63-4 |
| CAS-1989604-84-3 | CAS-1989605-48-2 | CAS-1989606-01-0 | CAS-1989606-67-8 |
| CAS-1989604-85-4 | CAS-1989605-49-3 | CAS-1989606-04-3 | CAS-1989606-69-0 |
| CAS-1989604-86-5 | CAS-1989605-50-6 | CAS-1989606-05-4 | CAS-1989606-70-3 |
| CAS-1989604-87-6 | CAS-1989605-51-7 | CAS-1989606-06-5 | CAS-1989606-74-7 |
| CAS-1989658-39-0 | CAS-2088184-56-7 | CAS-2088185-07-1 | CAS-2088185-66-2 |
| CAS-1989658-41-4 | CAS-2088184-57-8 | CAS-2088185-08-2 | CAS-2088185-67-3 |
| CAS-1989658-43-6 | CAS-2088184-58-9 | CAS-2088185-09-3 | CAS-2088185-68-4 |
| CAS-1989658-47-0 | CAS-2088184-59-0 | CAS-2088185-10-6 | CAS-2088185-69-5 |
| CAS-1989658-49-2 | CAS-2088184-60-3 | CAS-2088185-11-7 | CAS-2088185-70-8 |

(fortgesetzt)

| | | | |
|---|---|---|---|
| CAS-2088184-07-8 | CAS-2088184-61-4 | CAS-2088185-12-8 | CAS-2088185-71-9 |
| CAS-2088184-08-9 | CAS-2088184-62-5 | CAS-2088185-13-9 | CAS-2088185-72-0 |
| CAS-2088184-09-0 | CAS-2088184-63-6 | CAS-2088185-14-0 | CAS-2088185-73-1 |
| CAS-2088184-10-3 | CAS-2088184-64-7 | CAS-2088185-15-1 | CAS-2088185-74-2 |
| CAS-2088184-11-4 | CAS-2088184-65-8 | CAS-2088185-16-2 | CAS-2088185-75-3 |
| CAS-2088184-13-6 | CAS-2088184-66-9 | CAS-2088185-17-3 | CAS-2088185-76-4 |
| CAS-2088184-14-7 | CAS-2088184-67-0 | CAS-2088185-18-4 | CAS-2088185-77-5 |
| CAS-2088184-15-8 | CAS-2088184-68-1 | CAS-2088185-19-5 | CAS-2088185-78-6 |
| CAS-2088184-16-9 | CAS-2088184-69-2 | CAS-2088185-20-8 | CAS-2088185-79-7 |
| CAS-2088184-17-0 | CAS-2088184-70-5 | CAS-2088185-21-9 | CAS-2088185-80-0 |
| CAS-2088184-18-1 | CAS-2088184-71-6 | CAS-2088185-22-0 | CAS-2088185-81-1 |
| CAS-2088184-19-2 | CAS-2088184-72-7 | CAS-2088185-23-1 | CAS-2088185-82-2 |
| CAS-2088184-20-5 | CAS-2088184-73-8 | CAS-2088185-32-2 | CAS-2088185-83-3 |
| CAS-2088184-21-6 | CAS-2088184-74-9 | CAS-2088185-33-3 | CAS-2088185-84-4 |
| CAS-2088184-22-7 | CAS-2088184-75-0 | CAS-2088185-34-4 | CAS-2088185-85-5 |
| CAS-2088184-23-8 | CAS-2088184-76-1 | CAS-2088185-35-5 | CAS-2088185-86-6 |
| CAS-2088184-24-9 | CAS-2088184-77-2 | CAS-2088185-36-6 | CAS-2088185-87-7 |
| CAS-2088184-25-0 | CAS-2088184-78-3 | CAS-2088185-37-7 | CAS-2088185-88-8 |
| CAS-2088184-26-1 | CAS-2088184-79-4 | CAS-2088185-38-8 | CAS-2088185-89-9 |
| CAS-2088184-27-2 | CAS-2088184-80-7 | CAS-2088185-39-9 | CAS-2088185-90-2 |
| CAS-2088184-28-3 | CAS-2088184-81-8 | CAS-2088185-40-2 | CAS-2088185-91-3 |
| CAS-2088184-29-4 | CAS-2088184-82-9 | CAS-2088185-41-3 | CAS-2088185-92-4 |
| CAS-2088184-30-7 | CAS-2088184-83-0 | CAS-2088185-42-4 | CAS-2088185-93-5 |
| CAS-2088184-32-9 | CAS-2088184-84-1 | CAS-2088185-43-5 | CAS-2088185-94-6 |
| CAS-2088184-34-1 | CAS-2088184-85-2 | CAS-2088185-44-6 | CAS-2088185-95-7 |
| CAS-2088184-35-2 | CAS-2088184-86-3 | CAS-2088185-45-7 | CAS-2088185-96-8 |
| CAS-2088184-36-3 | CAS-2088184-87-4 | CAS-2088185-46-8 | CAS-2088185-97-9 |
| CAS-2088184-37-4 | CAS-2088184-88-5 | CAS-2088185-47-9 | CAS-2088185-98-0 |
| CAS-2088184-38-5 | CAS-2088184-89-6 | CAS-2088185-48-0 | CAS-2088185-99-1 |
| CAS-2088184-39-6 | CAS-2088184-90-9 | CAS-2088185-49-1 | CAS-2088186-00-7 |
| CAS-2088184-40-9 | CAS-2088184-91-0 | CAS-2088185-50-4 | CAS-2088186-01-8 |
| CAS-2088184-41-0 | CAS-2088184-92-1 | CAS-2088185-51-5 | CAS-2088186-02-9 |
| CAS-2088184-42-1 | CAS-2088184-93-2 | CAS-2088185-52-6 | CAS-2088195-88-2 |
| CAS-2088184-43-2 | CAS-2088184-94-3 | CAS-2088185-53-7 | CAS-2088195-89-3 |
| CAS-2088184-44-3 | CAS-2088184-95-4 | CAS-2088185-54-8 | CAS-2088195-90-6 |
| CAS-2088184-45-4 | CAS-2088184-96-5 | CAS-2088185-55-9 | CAS-2088195-91-7 |
| CAS-2088184-46-5 | CAS-2088184-97-6 | CAS-2088185-56-0 | CAS-861806-70-4 |
| CAS-2088184-47-6 | CAS-2088184-98-7 | CAS-2088185-57-1 | CAS-1269508-30-6 |
| CAS-2088184-48-7 | CAS-2088184-99-8 | CAS-2088185-58-2 | |

(fortgesetzt)

| | | | |
|---|---|---|---|
| CAS-2088184-49-8 | CAS-2088185-00-4 | CAS-2088185-59-3 | |
| CAS-2088184-50-1 | CAS-2088185-01-5 | CAS-2088185-60-6 | |
| CAS-2088184-51-2 | CAS-2088185-02-6 | CAS-2088185-61-7 | |
| CAS-2088184-52-3 | CAS-2088185-03-7 | CAS-2088185-62-8 | |
| CAS-2088184-53-4 | CAS-2088185-04-8 | CAS-2088185-63-9 | |
| CAS-2088184-54-5 | CAS-2088185-05-9 | CAS-2088185-64-0 | |
| CAS-2088184-55-6 | CAS-2088185-06-0 | CAS-2088185-65-1 | |

In den erfindungsgemäßen Mischungen oder in der lichtemittierenden Schicht der erfindungsgemäßen Vorrichtung wird bevorzugt jede Mischung M1, M2, M3, M4, M5, M6, M7, M8, M9, M10, M11, M12, M13, M14, M15, M16, M17, M18, M19, M20, M21, M22, M23, M24, M25, M26, M27, M28, M29, M30, M31, M32, M33, M34, M35, M36, M37, M38, M39, M40, M41, M42, M43, M44, M45, M46, M47, M48, M49, M50, M51, M52, M53, M54, M55, M56, M57, M58, M59, M60, M61, M62, M63, M64, M65, M66, M67, M68, M69, M70, M71, M72, M73, M74, M75, M76, M77, M78, M79, M80, M81, M82, M83, M84, M85, M86, M87, M88, M89, M90, M91, M92, M93, M94, M95, M96, M97, M98, M99, M100, M101, M102, M103, M104, M105, M106, M107, M108, M109, M110, M111, M112, M113, M114, M115, M116, M117, M118, M119, M120, mit einer Verbindung der Formel (5) oder einer Verbindung aus Tabelle 4 oder 5 kombiniert.

Die lichtemittierende Schicht in der erfindungsgemäßen organischen elektrolumineszierenden Vorrichtung enthaltend mindestens einen phosphoreszierenden Emitter ist bevorzugt eine infra-rot emittierende, gelb, orange, rot, grün, blau oder ultra-violett emittierende Schicht, besonders bevorzugt eine gelb oder grün emittierende Schicht und ganz besonders bevorzugt eine grün emittierende Schicht.

Dabei wird unter einer gelb emittierenden Schicht eine Schicht verstanden, deren Photolumineszenzmaximum im Bereich von 540 bis 570 nm liegt. Unter einer orange emittierenden Schicht wird eine Schicht verstanden, deren Photolumineszenzmaximum im Bereich von 570 bis 600 nm liegt. Unter einer rot emittierenden Schicht wird eine Schicht verstanden, deren Photolumineszenzmaximum im Bereich von 600 bis 750 nm liegt. Unter einer grün emittierenden Schicht wird eine Schicht verstanden, deren Photolumineszenzmaximum im Bereich von 490 bis 540 nm liegt. Unter einer blau emittierenden Schicht wird eine Schicht verstanden, deren Photolumineszenzmaximum im Bereich von 440 bis 490 nm liegt. Dabei wird das Photolumineszenzmaximum der Schicht durch Messung des Photolumineszenzspektrums der Schicht mit einer Schichtdicke von 50 nm bei Raumtemperatur bestimmt, wobei die Schicht die erfindungsgemäße Kombination der Hostmaterialien der Formeln (1) und (2) und den entsprechenden Emitter enthält.

Die Aufnahme des Photolumineszenzspektrums der Schicht erfolgt beispielsweise mit einem handelsüblichen Photolumineszenzspektrometer.

Das Photolumineszenzspektrum des gewählten Emitters wird in der Regel in Sauerstoff-freier Lösung, 10-5 molar, gemessen, wobei die Messung bei Raumtemperatur erfolgt und jedes Lösemittel geeignet ist, in dem sich der gewählte Emitter in der genannten Konzentration löst. Besonders geeignete Lösemittel sind üblicherweise Toluol oder 2-Methyl-THF, aber auch Dichlormethan. Gemessen wird mit einem handelsüblichen Photolumineszenzspektrometer. Die Triplettenergie T1 in eV wird aus den Photolumineszenzspektren der Emitter bestimmt. Es wird zunächst das Peakmaximum Plmax. (in nm) des Photolumineszenzspektrums bestimmt. Das Peakmaximum Plmax. (in nm) wird dann in eV umgerechnet gemäß:

$$E(T1 \text{ in eV}) = 1240 / E(T1 \text{ in nm}) = 1240 / PLmax. (\text{in nm}).$$

Bevorzugte phosphoreszierende Emitter sind demzufolge infra-rote Emitter, vorzugsweise der Formel (5) oder aus Tabelle 4 oder 5, deren Triplettenergie $T_1$ bevorzugt bei ~1.9 eV bis ~1.0 eV liegt.

Bevorzugte phosphoreszierende Emitter sind demzufolge rote Emitter, vorzugsweise der Formel (5) oder aus Tabelle 4 oder 5, deren Triplettenergie $T_1$ bevorzugt bei ~2.1 eV bis ~1.9 eV liegt.

Bevorzugte phosphoreszierende Emitter sind demzufolge gelbe Emitter, vorzugsweise der Formel (5) oder aus Tabelle 4 oder 5, deren Triplettenergie $T_1$ bevorzugt bei ~2.3 eV bis ~2.1 eV liegt.

Bevorzugte phosphoreszierende Emitter sind demzufolge grüne Emitter, vorzugsweise der Formel (5) oder aus Tabelle 4 oder 5, deren Triplettenergie $T_1$ bevorzugt bei ~2.5 eV bis ~2.3 eV liegt.

Bevorzugte phosphoreszierende Emitter sind demzufolge blaue Emitter, vorzugsweise der Formel (5) oder aus Tabelle 4 oder 5, deren Triplettenergie $T_1$ bevorzugt bei ~3.1 eV bis ~2.5 eV liegt.

Bevorzugte phosphoreszierende Emitter sind demzufolge ultra-violette Emitter, der Formel (5) oder aus Tabelle

4 oder 5, deren Triplettenergie $T_1$ bevorzugt bei ~4.0 eV bis ~3.1 eV liegt.

Besonders bevorzugte phosphoreszierende Emitter sind demzufolge grüne oder gelbe Emitter, vorzugsweise der Formel (5) oder aus Tabelle 4 oder 5, wie zuvor beschrieben.

Ganz besonders bevorzugte phosphoreszierende Emitter sind demzufolge grüne Emitter, vorzugsweise der Formel (5) oder aus Tabelle 4 oder 5, deren Triplettenergie $T_1$ bevorzugt bei ~2.5 eV bis ~2.3 eV liegt.

Ganz besonders bevorzugt werden grüne Emitter, vorzugsweise der Formel (5) oder aus Tabelle 4 oder 5, wie zuvor beschrieben, für die erfindungsgemäße Zusammensetzung bzw. erfindungsgemäße emittierende Schicht ausgewählt.

In der lichtemittierenden Schicht der erfindungsgemäßen Vorrichtung können auch fluoreszierende Emitter enthalten sein.

Bevorzugte fluoreszierende Emitter sind ausgewählt aus der Klasse der Arylamine. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, besonders bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird eine Verbindung verstanden, in der zwei Diarylaminogruppen direkt an eine Anthracengruppe gebunden sind, vorzugsweise in 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind. Weitere bevorzugte fluoreszierende Emitter sind Indenofluorenamine bzw. -diamine, beispielsweise gemäß WO 2006/108497 oder WO 2006/122630, Benzoindenofluorenamine bzw. -diamine, beispielsweise gemäß WO 2008/006449, und Dibenzoindenofluorenamine bzw. -diamine, beispielsweise gemäß WO 2007/140847, sowie die in WO 2010/012328 offenbarten Indenofluorenderivate mit kondensierten Arylgruppen.

In einer weiteren bevorzugten Ausführungsform der Erfindung kann die mindestens eine lichtemittierende Schicht der organischen elektrolumineszierenden Vorrichtung neben den Hostmaterialien 1 und 2, wie zuvor beschrieben oder als bevorzugt beschrieben, weitere Hostmaterialien oder Matrixmaterialien umfassen, sogenannte Mixed-Matrix-Systeme. Die Mixed-Matrix-Systeme umfassen bevorzugt drei oder vier verschiedene Matrixmaterialien, besonders bevorzugt drei verschiedene Matrixmaterialien (das heißt, eine weitere Matrixkomponente zusätzlich zu den Hostmaterialien 1 und 2, wie zuvor beschrieben). Besonders geeignete Matrixmaterialien, welche in Kombination als Matrixkomponente eines Mixed-Matrix-Systems verwendet werden können, sind ausgewählt aus *wide-band-gap*-Materialien, bipolaren Hostmaterialien, Elektronentransportmaterialien (ETM) und Lochtransportmaterialien (HTM).

Unter *wide-band-gap*-Material wird hierin ein Material im Sinne der Offenbarung von US 7,294,849 verstanden, das durch eine Bandlücke von mindestens 3.5 eV charakterisiert ist, wobei unter Bandlücke der Abstand zwischen HOMO und LUMO-Energie eines Materials verstanden wird.

Gemäß einer Ausführungsform der vorliegenden Erfindung enthält die Mischung neben den Bestandteilen elektronentransportierendes Hostmaterial der Formel (1) und lochtransportierendes Hostmaterial der Formel (2) keine weiteren Bestandteile, das heißt, funktionellen Materialien. Es handelt sich um Materialmischungen, die als solches zur Herstellung der lichtemittierenden Schicht, verwendet werden. Man bezeichnet diese Mischungen auch als Premix-Systeme, die als einzige Materialquelle bei der Aufdampfung der Hostmaterialien für die lichtemittierende Schicht verwendet wird und die ein konstantes Mischungsverhältnis bei der Aufdampfung haben. Dadurch lässt sich auf einfache und schnelle Art und Weise das Aufdampfen einer Schicht mit gleichmäßiger Verteilung der Komponenten erreichen, ohne dass eine präzise Ansteuerung einer Vielzahl an Materialquellen notwendig ist.

Gemäß einer alternativen Ausführungsform der vorliegenden Erfindung enthält die Mischung neben den Bestandteilen elektronentransportierendes Hostmaterial der Formel (1) und lochtransportierendes Hostmaterial der Formel (2) noch den phosphoreszierenden Emitter, wie zuvor beschrieben. Bei geeignetem Mischungsverhältnis bei der Aufdampfung kann auch diese Mischung als einzige Materialquelle verwendet werden, wie zuvor beschrieben.

Die Komponenten bzw. Bestandteile der lichtemittierenden Schicht der erfindungsgemäßen Vorrichtung können somit durch Aufdampfen oder aus Lösung prozessiert werden. Die Materialkombination der Hostmaterialien 1 und 2, wie zuvor beschrieben oder bevorzugt beschrieben, gegebenenfalls mit dem phosphoreszierenden Emitter, wie zuvor beschrieben oder bevorzugt beschrieben, werden dazu in einer Formulierung bereitgestellt, die mindestens ein Lösemittel enthält. Diese Formulierungen können beispielsweise Lösungen, Dispersionen oder Emulsionen sein. Es kann bevorzugt sein, hierfür Mischungen aus zwei oder mehr Lösemitteln zu verwenden.

Ein weiterer Gegenstand der vorliegenden Erfindung ist daher eine Formulierung enthaltend eine erfindungsgemäße Mischung an Hostmaterialien 1 und 2, wie zuvor beschrieben, gegebenenfalls in Kombination mit einem phosphoreszierenden Emitter, wie zuvor beschrieben oder bevorzugt beschrieben, und mindestens ein Lösemittel.

Geeignete und bevorzugte Lösemittel sind beispielsweise Toluol, Anisol, o-, m- oder p-Xylol, Methylbenzoat,

Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, $\alpha$-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Die Formulierung kann dabei auch mindestens eine weitere organische oder anorganische Verbindung enthalten, die ebenfalls in der lichtemittierenden Schicht der erfindungsgemäßen Vorrichtung eingesetzt wird, insbesondere eine weitere emittierende Verbindung und/oder ein weiteres Matrixmaterial.

Die lichtemittierende Schicht in der erfindungsgemäßen Vorrichtung gemäß den bevorzugten Ausführungsformen und der emittierenden Verbindung enthält vorzugsweise zwischen 99,9 und 1 Vol.-%, weiter vorzugsweise zwischen 99 und 10 Vol.-%, besonders bevorzugt zwischen 98 und 60 Vol.-%, ganz besonders bevorzugt zwischen 97 und 80 Vol.-% an Matrixmaterial aus mindestens einer Verbindung der Formel (1) und mindestens einer Verbindung der Formel (2) gemäß den bevorzugten Ausführungsformen, bezogen auf die gesamte Zusammensetzung aus Emitter und Matrixmaterial. Entsprechend enthält die lichtemittierende Schicht in der erfindungsgemäßen Vorrichtung vorzugsweise zwischen 0,1 und 99 Vol.-%, weiter vorzugsweise zwischen 1 und 90 Vol.-%, besonders bevorzugt zwischen 2 und 40 Vol.-%, ganz besonders bevorzugt zwischen 3 und 20 Vol.-% des Emitters bezogen auf die gesamte Zusammensetzung der aus Emitter und Matrixmaterial bestehenden lichtemittierenden Schicht. Werden die Verbindungen aus Lösung verarbeitet, so werden statt der oben angegebenen Mengen in Vol.-% bevorzugt die entsprechenden Mengen in Gew.-% verwendet.

Die lichtemittierende Schicht in der erfindungsgemäßen Vorrichtung gemäß den bevorzugten Ausführungsformen und der emittierenden Verbindung enthält vorzugsweise das Matrixmaterial der Formel (1) und das Matrixmaterial der Formel (2) in einem Volumenprozentverhältnis zwischen 3:1 und 1:3, bevorzugt zwischen 1:2.5 und 1:1, besonders bevorzugt zwischen 1:2 und 1:1. Werden die Verbindungen aus Lösung verarbeitet, so werden statt des oben angegebenen Verhältnisses in Vol.-% bevorzugt das entsprechende Verhältnis in Gew.-% verwendet.

Die Abfolge der Schichten in der erfindungsgemäßen organischen Elektrolumineszenzvorrichtung ist bevorzugt die folgende:
Anode / Lochinjektionsschicht / Lochtransportschicht / emittierende Schicht / Elektronentransportschicht / Elektroneninjektionsschicht / Kathode.

Diese Abfolge der Schichten ist eine bevorzugte Abfolge.

Dabei soll erneut darauf hingewiesen werden, dass nicht alle der genannten Schichten vorhanden sein müssen, und/oder dass zusätzlich weitere Schichten vorhanden sein können.

Die erfindungsgemäße organische Elektrolumineszenzvorrichtung kann mehrere emittierende Schichten enthalten. Mindestens eine der emittierenden Schichten ist die erfindungsgemäße lichtemittierende Schicht enthaltend mindestens eine Verbindung der Formel (1) als Hostmaterial 1 und mindestens eine Verbindung der Formel (2) als Hostmaterial 2, wie zuvor beschrieben. Besonders bevorzugt weisen diese Emissionsschichten in diesem Fall insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues oder gelbes oder orangefarbenes oder rotes Licht emittieren. Insbesondere bevorzugt sind Dreischichtsysteme, also Systeme mit drei emittierenden Schichten, wobei die drei Schichten blaue, grüne und orange oder rote Emission zeigen (für den prinzipiellen Aufbau siehe z. B.

WO 2005/011013). Es soll angemerkt werden, dass sich für die Erzeugung von weißem Licht anstelle mehrerer farbig emittierender Emitterverbindungen auch eine einzeln verwendete Emitterverbindung eignen kann, welche in einem breiten Wellenlängenbereich emittiert.

Geeignete Ladungstransportmaterialien, wie sie in der Lochinjektions- bzw. Lochtransportschicht bzw. Elektronenblockierschicht oder in der Elektronentransportschicht der erfindungsgemäßen organischen Elektrolumineszenzvorrichtung verwendet werden können, sind beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Als Materialien für die Elektronentransportschicht können alle Materialien verwendet werden, wie sie gemäß dem Stand der Technik als Elektronentransportmaterialien in der Elektronentransportschicht verwendet werden. Insbesondere eignen sich Aluminiumkomplexe, beispielsweise $Alq_3$, Zirkoniumkomplexe, beispielsweise $Zrq_4$, Benzimidazolderviate, Triazinderivate, Pyrimidinderivate, Pyridinderivate, Pyrazinderivate, Chinoxalinderivate, Chinolinderivate, Oxadiazolderivate, aromatische Ketone, Lactame, Borane, Diazaphospholderivate und Phosphinoxidderivate. Weiterhin geeignete Materialien sind Derivate der oben genannten Verbindungen, wie sie in JP 2000/053957, WO 2003/060956,

WO 2004/028217, WO 2004/080975 und WO 2010/072300 offenbart werden.

Als Lochtransportmaterialien sind insbesondere Materialien bevorzugt, die in einer Lochtransport-, Lochinjektions- oder Elektronenblockierschicht verwendet werden können, wie Indenofluorenamin-Derivate (z. B. gemäß WO 06/122630 oder WO 06/100896), die in EP 1661888 offenbarten Aminderivate, Hexaazatriphenylenderivate (z. B. gemäß WO 01/049806), Aminderivate mit kondensierten Aromaten (z. B. gemäß US 5,061,569), die in WO 95/09147 offenbarten Aminderivate, Monobenzoindenofluorenamine (z. B. gemäß WO 08/006449), Dibenzoindenofluorenamine (z. B. gemäß WO 07/140847), Spirobifluoren-Amine (z. B. gemäß WO 2012/034627 oder der noch nicht offengelegten EP 12000929.5), Fluoren-Amine (z. B. gemäß WO 2014/015937, WO 2014/015938 und WO 2014/015935), Spiro-Dibenzopyran-Amine (z. B. gemäß WO 2013/083216) und Dihydroacridin-Derivate (z. B. WO 2012/150001).

Als Kathode der erfindungsgemäßen Vorrichtung eignen sich Metalle mit geringer Austrittsarbeit, Metalllegierungen oder mehrlagige Strukturen aus verschiedenen Metallen, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die erfindungsgemäße organische elektrolumineszierende Vorrichtung wird bei der Herstellung entsprechend (je nach Anwendung) strukturiert, kontaktiert und schließlich versiegelt, da sich die Lebensdauer der erfindungsgemäßen Vorrichtungen bei Anwesenheit von Wasser und/oder Luft verkürzt.

Die Herstellung der erfindungsgemäßen Vorrichtung ist hierbei nicht eingeschränkt. Es ist möglich, dass eine oder mehrere organische Schichten, auch die lichtemittierende Schicht, mit einem Sublimationsverfahren beschichtet werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Dabei ist es jedoch auch möglich, dass der Anfangsdruck noch geringer ist, beispielsweise kleiner $10^{-7}$ mbar.

Bevorzugt ist die erfindungsgemäße organische Elektrolumineszenzvorrichtung dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist die erfindungsgemäße organische Elektrolumineszenzvorrichtung dadurch gekennzeichnet, dass eine oder mehrere organische Schichten enthaltend die erfindungsgemäße Zusammensetzung aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Hostmaterialien 1 und 2 und phosphoreszierende Emitter nötig. Das Verarbeiten aus Lösung hat den Vorteil, dass beispielsweise die lichtemittierende Schicht sehr einfach und kostengünstig aufgebracht werden kann. Diese Technik eignet sich insbesondere für die Massenproduktion organischer elektrolumineszierender Vorrichtungen.

Weiterhin sind Hybridverfahren möglich, bei denen beispielsweise eine oder mehrere Schichten aus Lösung aufgebracht werden und eine oder mehrere weitere Schichten aufgedampft werden.

Diese Verfahren sind dem Fachmann generell bekannt und können auf organische Elektrolumineszenzvorrichtungen angewandt werden.

Ein weiterer Gegenstand der Erfindung ist daher ein Verfahren zur Herstellung der erfindungsgemäßen organischen elektrolumineszierenden Vorrichtung, wie zuvor beschrieben oder bevorzugt beschrieben, dadurch gekennzeichnet, dass die lichtemittierende Schicht durch Gasphasenabscheidung, insbesondere mit einem Sublimationsverfahren und/oder mit einem OVPD (Organic Vapour Phase Deposition) Verfahren und/oder mit Hilfe einer Trägergassublimation, oder aus Lösung, insbesondere durch Spincoating oder mit einem Druckverfahren, aufgebracht wird.

Bei der Herstellung mittels Gasphasenabscheidung bestehen grundsätzlich zwei Möglichkeiten, wie die erfindungsgemäße lichtemittierende Schicht, auf ein beliebiges Substrat bzw. die vorherige Schicht aufgebracht bzw. aufgedampft werden kann. Zum einen können die verwendeten Materialien jeweils in einer Materialquelle vorgelegt und schließlich aus den verschiedenen Materialquellen verdampft werden ("co-evaporation"). Zum anderen können die verschiedenen Materialien vorgemischt ("premixed", Premix-Systeme) und das Gemisch in einer einzigen Materialquelle vorgelegt werden, aus der es schließlich verdampft wird ("premixevaporation"). Dadurch lässt sich auf einfache und schnelle Art und Weise das Aufdampfen der lichtemittierenden Schicht mit gleichmäßiger Verteilung der Komponenten erreichen, ohne dass eine präzise Ansteuerung einer Vielzahl an Materialquellen notwendig ist.

Ein weiterer Gegenstand der Erfindung ist demzufolge ein Verfahren zur Herstellung der erfindungsgemäßen Vorrichtung, dadurch gekennzeichnet, dass die mindestens eine Verbindung der Formel (1), wie zuvor beschrieben oder als bevorzugt beschrieben, und die mindestens eine Verbindung der Formel (2), wie zuvor beschrieben oder als bevorzugt beschrieben, nacheinander oder gleichzeitig aus mindestens zwei Materialquellen, gegebenenfalls mit dem mindestens einen phosphoreszierenden Emitter, wie zuvor beschrieben oder bevorzugt beschrieben, aus der Gasphase abgeschieden werden und die lichtemittierende Schicht bilden.

In einer bevorzugten Ausführungsform der vorliegenden Erfindung wird die lichtemittierende Schicht mittels Gasphasenabscheidung aufgebracht, wobei die Bestandeile der Zusammensetzung vorgemischt und aus einer einzigen Materialquelle verdampft werden.

Ein weiterer Gegenstand der Erfindung ist demzufolge ein Verfahren zur Herstellung der erfindungsgemäßen Vorrichtung, dadurch gekennzeichnet, dass die mindestens eine Verbindung der Formel (1) und die mindestens eine Verbindung der Formel (2) als Mischung, nacheinander oder gleichzeitig mit dem mindestens einen phosphoreszierenden Emitter, aus der Gasphase abgeschieden werden und die lichtemittierende Schicht bilden.

Ein weiterer Gegenstand der Erfindung ist ein Verfahren zur Herstellung der erfindungsgemäßen Vorrichtung, wie zuvor beschrieben oder bevorzugt beschrieben, dadurch gekennzeichnet, dass die mindestens eine Verbindung der Formel (1) und die mindestens eine Verbindung der Formel (2), wie zuvor beschrieben oder bevorzugt beschrieben, zusammen mit dem mindestens einen phosphoreszierenden Emitter, aus Lösung aufgebracht werden, um die lichtemittierende Schicht zu bilden.

Die erfindungsgemäßen Vorrichtungen zeichnen sich durch folgende überraschende Vorteile gegenüber dem Stand der Technik aus:
Die Verwendung der beschriebenen Materialkombination der Hostmaterialien 1 und 2, wie zuvor beschrieben, führt insbesondere zu einer Steigerung der Lebensdauer der Vorrichtungen.

Wie im nachfolgend angegebenen Beispiel ersichtlich, lässt sich durch Vergleich der Daten für OLEDs mit Kombinationen aus dem Stand der Technik feststellen, dass die erfindungsgemäßen Kombinationen an Matrixmaterialien in der EML zu Vorrichtungen führen, deren Lebensdauer um ca. 20 bis 240% erhöht sind, unabhängig von der Emitterkonzentration.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbarte Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Die mit der vorliegenden Erfindung offengelegte Lehre zum technischen Handeln kann abstrahiert und mit anderen Beispielen kombiniert werden. Die Erfindung wird durch die nachfolgenden Beispiele näher erläutert, ohne sie dadurch einschränken zu wollen.

Allgemeine Methoden:
In allen quantenchemischen Berechnungen wird das Programmpaket Gaussian16 (Rev. B.01) verwendet. Der neutrale Singulettgrundzustand wird auf dem B3LYP/6-31G(d)-Niveau optimiert. HOMO- und LUMO-Werte werden auf dem B3LYP/6-31G(d)-Niveau für die mit B3LYP/6-31G(d) optimierte Grundzustandsenergie bestimmt. Daraufhin werden TD-DFT-Singulett- und Triplettanregungen (vertikale Anregungen) mit der gleichen Methode (B3LYP/6-31G(d)) und der optimierten Grundzustandsgeometrie berechnet. Die Standardeinstellungen für SCF- und Gradientenkonvergenz werden verwendet.

Aus der Energierechnung erhält man das HOMO als das letzte mit zwei Elektronen besetze Orbital (Alpha occ. eigenvalues) und LUMO als das erste unbesetzte Orbital (Alpha virt. eigenvalues) in Hartree-Einheiten, wobei HEh und LEh für die HOMO Energie in Hartree-Einheiten beziehungsweise für die LUMO-Energie in Hartree-Einheiten steht. Daraus wird der anhand von Cyclovoltammetriemessungen kalibrierte HOMO- und LUMO-Wert in Elektronenvolt wie folgt bestimmt:

$$\text{HOMOcorr} = 0.90603 * \text{HOMO} - 0.84836$$

$$\text{LUMOcorr} = 0.99687 * \text{LUMO} - 0.72445$$

Das Triplett-Niveau T1 eines Materials ist definiert als die relative Anregungsenergie (in eV) des Triplettzustands mit der niedrigsten Energie, der sich aus der quantenchemischen Energierechnung ergibt.

Das Singulett-Niveau S1 eines Materials ist definiert als die relative Anregungsenergie (in eV) des Singulettzustands mit der zweitniedrigsten Energie, der sich aus der quantenchemischen Energierechnung ergibt. Der energetisch niedrigste Singulettzustand wird als S0 bezeichnet.

Die hierin beschriebene Methode ist unabhängig von dem verwendeten Softwarepaket und liefert immer dieselben Ergebnisse. Beispiele oft benutzter Programme für diesen Zweck sind "Gaussian09" (Gaussian Inc.) und Q-Chem 4.1 (Q-Chem, Inc.). Vorliegend wird zur Berechnung der Energien das Programmpaket "Gaussian16 (Rev. B.01)" verwendet.

Beispiel 1: **Herstellung der OLEDs**

In den folgenden Beispielen V1 bis Ex24 (siehe Tabellen 6 und 7) wird der Einsatz der erfindungsgemäßen Materialkombinationen in OLEDs in Vergleich zu Materialkombinationen aus dem Stand der Technik vorgestellt.

**Vorbehandlung für die Beispiele V1 bis Ex24:** Glasplättchen, die mit strukturiertem ITO (Indium Zinn Oxid) der Dicke 50 nm beschichtet sind, werden vor der Beschichtung zunächst mit einem Sauerstoffplasma, gefolgt von einem Argonplasma, behandelt. Diese mit Plasma behandelten Glasplättchen bilden die Substrate, auf welche die OLEDs aufgebracht werden.

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 6 zu entnehmen. Die zur Herstellung der OLEDs benötigten Materialien sind in Tabelle 8 gezeigt. Die Device Daten der OLEDs sind in Tabelle 7 aufgelistet. Die Beispiele V1 und V4 sind Vergleichsbeispiele mit einem elektronentransportierenden Host gemäß dem Stand der Technik WO2011088877.

Die Beispiele V2, V3 und V5, V6 sind Vergleichsbeispiele mit einem elektronentransportierenen Host gemäß dem Stand der Technik, beispielsweise bekannt aus US20180337348.

Die Beispiele Ex1 bis Ex24 zeigen Daten von erfindungsgemäßen OLEDs.

Alle Materialien werden in einer Vakuumkammer thermisch aufgedampft. Dabei besteht die Emissionsschicht immer aus mindestens zwei Matrixmaterialien und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie SoA1:CoH1:TEG1 (45%:45%:10%) bedeutet hierbei, dass das Material SoA1 in einem Volumenanteil von 45%, CoH1 in einem Anteil von 45% und TEG1 in einem Anteil von 10% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren und Strom-Spannungs-Leuchtdichte-Kennlinien (IUL-Kennlinien) gemessen. EQE und die Stromeffizienz SE (in cd/A) werden daraus berechnet. Die Berechnung der SE erfolgt unter Annahme einer lambertschen Abstrahlcharakteristik.

Als Lebensdauer LD wird die Zeit definiert, nach der die Leuchtdichte gemessen in cd/$m^2$ in Vorwärtsrichtung, bei Betrieb mit konstanter Stromdichte $j_0$ von der Startleuchtdichte auf einen gewissen Anteil L1 absinkt. Eine Angabe L1=80% in Tabelle 7 bedeutet, dass die in Spalte LD angegebene Lebensdauer der Zeit entspricht, nach der die Leuchtdichte in cd/$m^2$ auf 80% ihres Anfangswertes absinkt.

**Verwendung von erfindungsgemäßen Mischungen in OLEDs**

Die erfindungsgemäßen Materialkombinationen können in der Emissionsschicht in phosphoreszierenden grünen OLEDs eingesetzt werden. Die erfindungsgemäßen Kombinationen der Verbindungen Eg1 bis Eg6 in Kombination

mit den Verbindungen CoH1 und CoH3 werden in den Beispielen Ex1 bis Ex12 als Matrixmaterial in der Emissionsschicht eingesetzt.

Die dazu entsprechenden Vergleichsbeispiele V1 bis V6 beziehen sich auf die Verbindungen SoA1 bis SoA3 in Kombination mit den Verbindungen CoH1 und CoH3, welche in den Beispielen V1 bis V6 als Matrixmaterial in der Emissionsschicht eingesetzt werden.

Beim Vergleich der erfindungsgemäßen Beispiele mit den entsprechenden Vergleichsbeispielen (s.o.), ist deutlich ersichtlich, dass die erfindungsgemäßen Beispiele jeweils einen deutlichen Vorteil in der Device Lebensdauer aufzeigen.

Tabelle 6: Aufbau der OLEDs

| Bsp | HIL Dicke | HTL Dicke | EBL Dicke | EML Dicke | HBL Dicke | ETL Dicke | EIL Dicke |
|---|---|---|---|---|---|---|---|
| V1 | HATCN 5nm | SpMA1 230nm | SpMA2 20nm | SoA1:CoH1:TEG1 (44%: 44%:12%) 30nm | ST2 10nm | ST2:LiQ (50%: 50%) 30nm | LiQ 1nm |
| V2 | HATCN 5nm | SpMA1 230nm | SpMA2 20nm | SoA2:CoH1:TEG1 (44%: 44%:12%) 30nm | ST2 10nm | ST2:LiQ (50%: 50%) 30nm | LiQ 1nm |
| V3 | HATCN 5nm | SpMA1 230nm | SpMA2 20nm | SoA3:CoH1:TEG1 (44%: 44%:12%) 30nm | ST2 10nm | ST2:LiQ (50%: 50%) 30nm | LiQ 1nm |
| Ex1 | HATCN 5nm | SpMA1 230nm | SpMA2 20nm | Eg1:CoH1:TEG1 (44%: 44%:12%) 30nm | ST2 10nm | ST2:LiQ (50%: 50%) 30nm | LiQ 1nm |
| Ex2 | HATCN 5nm | SpMA1 230nm | SpMA2 20nm | Eg2:CoH1:TEG1 (44%: 44%:12%) 30nm | ST2 10nm | ST2:LiQ (50%: 50%) 30nm | LiQ 1nm |
| Ex3 | HATCN 5nm | SpMA1 230nm | SpMA2 20nm | Eg3:CoH1:TEG1 (44%: 44%:12%) 30nm | ST2 10nm | ST2:LiQ (50%: 50%) 30nm | LiQ 1nm |
| Ex4 | HATCN 5nm | SpMA1 230nm | SpMA2 20nm | Eg4:CoH1:TEG1 (44%: 44%:12%) 30nm | ST2 10nm | ST2:LiQ (50%: 50%) 30nm | LiQ 1nm |
| Ex5 | HATCN 5nm | SpMA1 230nm | SpMA2 20nm | Eg5:CoH1:TEG1 (44%: 44%:12%) 30nm | ST2 10nm | ST2:LiQ (50%: 50%) 30nm | LiQ 1nm |
| Ex6 | HATCN 5nm | SpMA1 230nm | SpMA2 20nm | Eg6:CoH1:TEG1 (44%: 44%:12%) 30nm | ST2 10nm | ST2:LiQ (50%: 50%) 30nm | LiQ 1nm |
| V4 | HATCN 5nm | SpMA1 230nm | SpMA2 20nm | SoA1:CoH3:TEG1 (44%: 44%:12%) 30nm | ST2 10nm | ST2:LiQ (50%: 50%) 30nm | LiQ 1nm |
| V5 | HATCN 5nm | SpMA1 230nm | SpMA2 20nm | SoA2:CoH3:TEG1 (44%: 44%:12%) 30nm | ST2 10nm | ST2:LiQ (50%: 50%) 30nm | LiQ 1nm |
| V6 | HATCN 5nm | SpMA1 230nm | SpMA2 20nm | SoA3:CoH3:TEG1 (44%: 44%:12%) 30nm | ST2 10nm | ST2:LiQ (50%: 50%) 30nm | LiQ 1nm |
| Ex7 | HATCN 5nm | SpMA1 230nm | SpMA2 20nm | Eg1:CoH3:TEG1 (44%: 44%:12%) 30nm | ST2 10nm | ST2:LiQ (50%: 50%) 30nm | LiQ 1nm |
| Ex8 | HATCN 5nm | SpMA1 230nm | SpMA2 20nm | Eg2:CoH3:TEG1 (44%: 44%:12%) 30nm | ST2 10nm | ST2:LiQ (50%: 50%) 30nm | LiQ 1nm |
| Ex9 | HATCN 5nm | SpMA1 230nm | SpMA2 20nm | Eg3:CoH3:TEG1 (44%: 44%:12%) 30nm | ST2 10nm | ST2:LiQ (50%: 50%) 30nm | LiQ 1nm |
| Ex10 | HATCN 5nm | SpMA1 230nm | SpMA2 20nm | Eg4:CoH3:TEG1 (44%: 44%:12%) 30nm | ST2 10nm | ST2:LiQ (50%: 50%) 30nm | LiQ 1nm |
| Ex11 | HATCN 5nm | SpMA1 230nm | SpMA2 20nm | Eg5:CoH3:TEG1 (44%: 44%:12%) 30nm | ST2 10nm | ST2:LiQ (50%: 50%) 30nm | LiQ 1nm |
| Ex12 | HATCN 5nm | SpMA1 230nm | SpMA2 20nm | Eg6:CoH3:TEG1 (44%: 44%:12%) 30nm | ST2 10nm | ST2:LiQ (50%: 50%) 30nm | LiQ 1nm |

(fortgesetzt)

| Bsp | HIL Dicke | HTL Dicke | EBL Dicke | EML Dicke | HBL Dicke | ETL Dicke | EIL Dicke |
|---|---|---|---|---|---|---|---|
| Ex13 | HATCN 5nm | SpMA1 230nm | SpMA2 20nm | Eg1:CoH2:TEG2 (21%: 72%:7%) 40nm | ST2 5nm | ST2:LiQ (50%: 50%) 30nm | LiQ 1nm |
| Ex14 | HATCN 5nm | SpMA1 230nm | SpMA2 20nm | Eg2:CoH2:TEG2 (21%: 72%:7%) 40nm | ST2 5nm | ST2:LiQ (50%: 50%) 30nm | LiQ 1nm |
| Ex15 | HATCN 5nm | SpMA1 230nm | SpMA2 20nm | Eg3:CoH2:TEG2 (21%: 72%:7%) 40nm | ST2 5nm | ST2:LiQ (50%: 50%) 30nm | LiQ 1nm |
| Ex16 | HATCN 5nm | SpMA1 230nm | SpMA2 20nm | Eg4:CoH2:TEG2 (21%: 72%:7%) 40nm | ST2 5nm | ST2:LiQ (50%: 50%) 30nm | LiQ 1nm |
| Ex17 | HATCN 5nm | SpMA1 230nm | SpMA2 20nm | Eg5:CoH2:TEG2 (21%: 72%:7%) 40nm | ST2 5nm | ST2:LiQ (50%: 50%) 30nm | LiQ 1nm |
| Ex18 | HATCN 5nm | SpMA1 230nm | SpMA2 20nm | Eg6:CoH2:TEG2 (21%: 72%:7%) 40nm | ST2 5nm | ST2:LiQ (50%: 50%) 30nm | LiQ 1nm |
| Ex19 | HATCN 5nm | SpMA1 230nm | SpMA2 20nm | Eg1:CoH1:TEG3 (21%: 72%:7%) 40nm | ST2 5nm | ST2:LiQ (50%: 50%) 30nm | LiQ 1nm |
| Ex20 | HATCN 5nm | SpMA1 230nm | SpMA2 20nm | Eg2:CoH1:TEG3 (21%: 72%:7%) 40nm | ST2 5nm | ST2:LiQ (50%: 50%) 30nm | LiQ 1nm |
| Ex21 | HATCN 5nm | SpMA1 230nm | SpMA2 20nm | Eg3:CoH1:TEG3 (21%: 72%:7%) 40nm | ST2 5nm | ST2:LiQ (50%: 50%) 30nm | LiQ 1nm |
| Ex22 | HATCN 5nm | SpMA1 230nm | SpMA2 20nm | Eg4:CoH1:TEG3 (21%: 72%:7%) 40nm | ST2 5nm | ST2:LiQ (50%: 50%) 30nm | LiQ 1nm |
| Ex23 | HATCN 5nm | SpMA1 230nm | SpMA2 20nm | Eg5:CoH1:TEG3 (21%: 72%:7%) 40nm | ST2 5nm | ST2:LiQ (50%: 50%) 30nm | LiQ 1nm |
| Ex24 | HATCN 5nm | SpMA1 230nm | SpMA2 20nm | Eg6:CoH1:TEG3 (21%: 72%:7%) 40nm | ST2 5nm | ST2:LiQ (50%: 50%) 30nm | LiQ 1nm |

Tabelle 7: Daten der OLEDs

| Bsp. | $j_0$ (mA/cm$^2$) | L1 (%) | LD (h) |
|---|---|---|---|
| V1 | 40 | 80 | 210 |
| V2 | 40 | 80 | 590 |
| V3 | 40 | 80 | 320 |
| Ex1 | 40 | 80 | 705 |
| Ex2 | 40 | 80 | 660 |
| Ex3 | 40 | 80 | 715 |
| Ex4 | 40 | 80 | 670 |
| Ex5 | 40 | 80 | 650 |
| Ex6 | 40 | 80 | 690 |
| V4 | 40 | 80 | 220 |
| V5 | 40 | 80 | 605 |
| V6 | 40 | 80 | 325 |
| Ex7 | 40 | 80 | 720 |

(fortgesetzt)

| Bsp. | $j_0$ (mA/cm$^2$) | L1 (%) | LD (h) |
|---|---|---|---|
| Ex8 | 40 | 80 | 675 |
| Ex9 | 40 | 80 | 725 |
| Ex10 | 40 | 80 | 680 |
| Ex11 | 40 | 80 | 670 |
| Ex12 | 40 | 80 | 705 |
| Ex13 | 40 | 80 | 1410 |
| Ex14 | 40 | 80 | 1330 |
| Ex15 | 40 | 80 | 1450 |
| Ex16 | 40 | 80 | 1390 |
| Ex17 | 40 | 80 | 1430 |
| Ex18 | 40 | 80 | 1420 |
| Ex19 | 40 | 80 | 1050 |
| Ex20 | 40 | 80 | 920 |
| Ex21 | 40 | 80 | 1090 |
| Ex22 | 40 | 80 | 940 |
| Ex23 | 40 | 80 | 960 |
| Ex24 | 40 | 80 | 1030 |

Tabelle 8: Strukturformeln der verwendeten Materialien der OLEDs

| | |
|---|---|
| 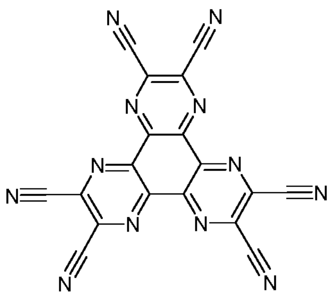 | 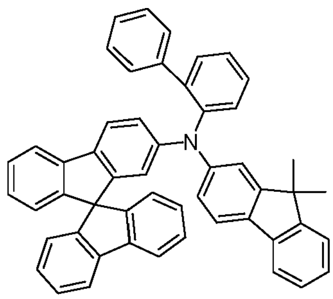 |
| HATCN | SpMA1 |
| 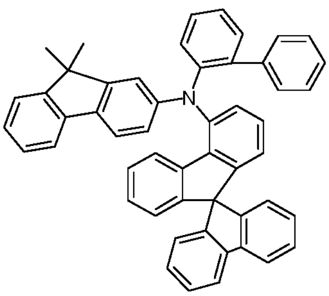 | 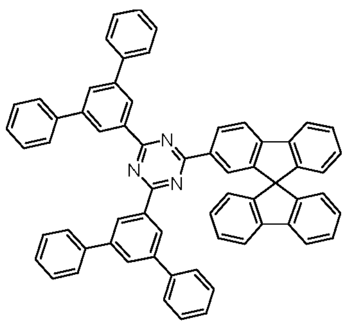 |
| SpMA2 | ST2 |

(fortgesetzt)

| | |
|---|---|
| 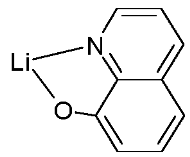 | 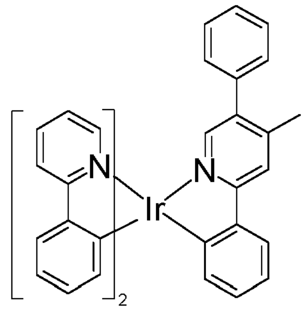 |
| LiQ | TEG1 |
| 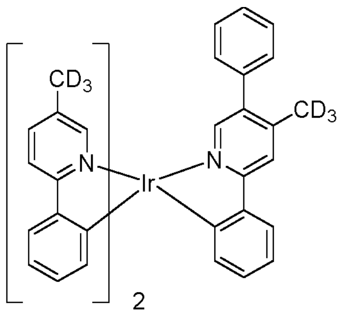 | 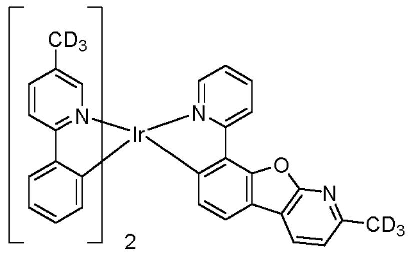 |
| TEG2 | TEG3 |
| 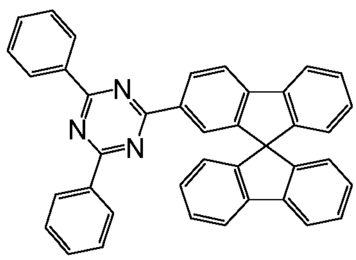 | 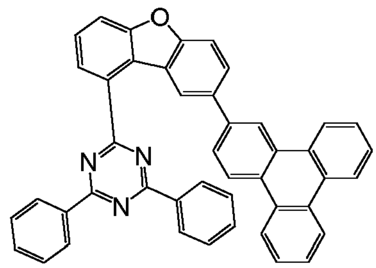 |
| SoA1 | SoA2 |
| 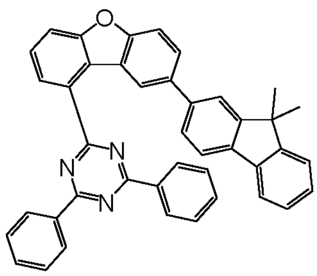 | 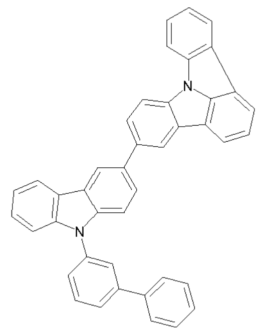 |
| SoA3 | CoH1 = 13 |

(fortgesetzt)

| | |
|---|---|
| 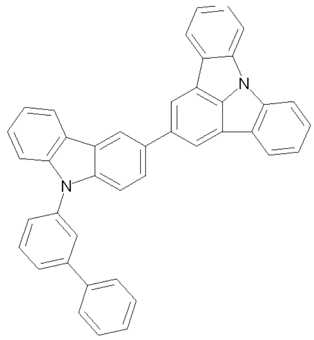 | 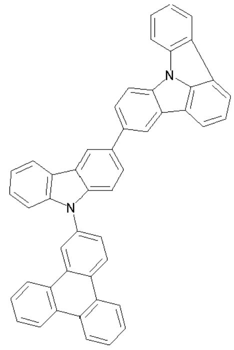 |
| CoH2 = 16 | CoH3 = 17 |
| 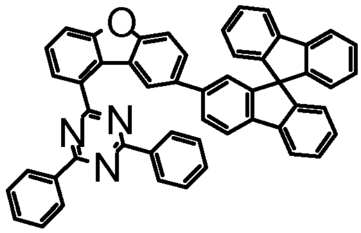 | 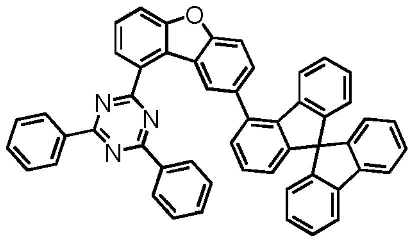 |
| Eg1 = 1 | Eg2 = 2 |
| 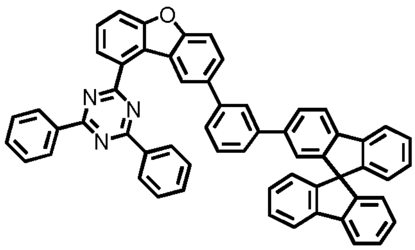 | 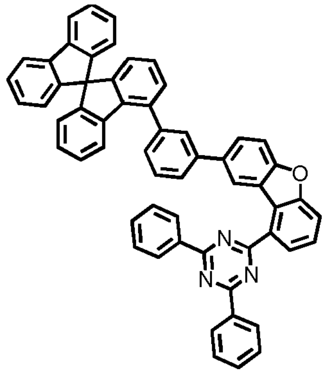 |
| Eg3 = 6 | Eg4 = 7 |
| 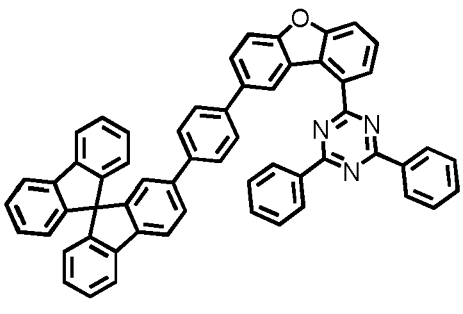 | 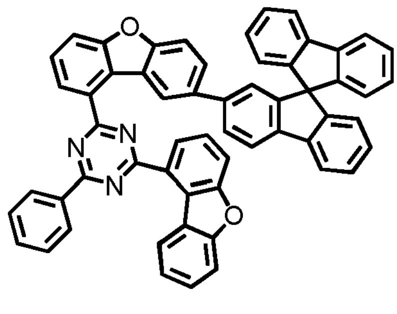 |
| Eg5 = 9 | Eg6 = 10. |

**Patentansprüche**

1. Organische elektrolumineszierende Vorrichtung umfassend eine Anode, eine Kathode und mindestens eine organische Schicht, enthaltend mindestens eine lichtemittierende Schicht, wobei die mindestens eine lichtemittierende Schicht mindestens eine Verbindung der Formel (1) als Hostmaterial 1 und mindestens eine Verbindung der Formel

(2) als Hostmaterial 2 enthält,

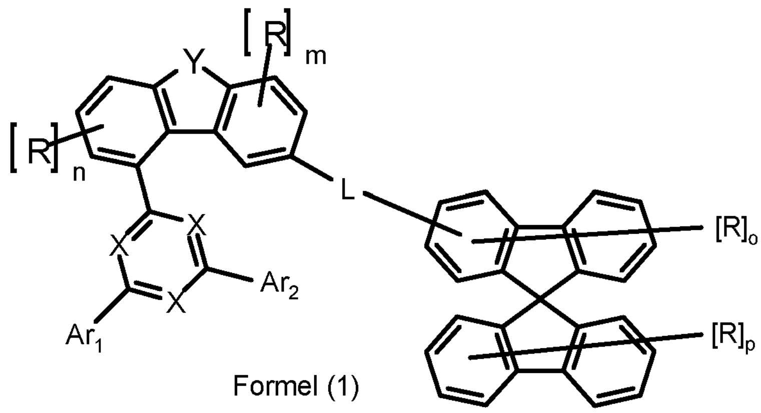

Formel (1)

,

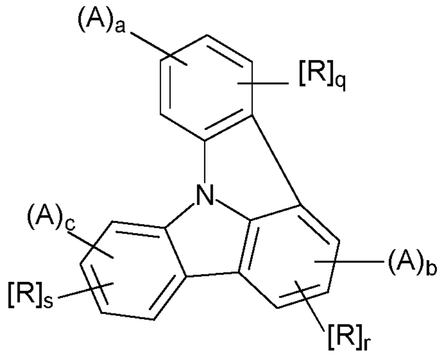

Formel (2)

,

wobei für die verwendeten Symbole und Indizes gilt:

X ist bei jedem Auftreten gleich oder verschieden $CR^0$ oder N, mit der Maßgabe, dass mindestens zwei Gruppen X für N stehen;
Y ist ausgewählt aus O oder S;
L ist bei jedem Auftreten gleich oder verschieden eine Einfachbindung oder ein Linker L-1 bis L-13,

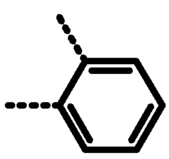

L-1

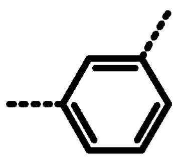

L-2

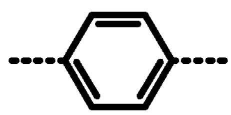

L-3

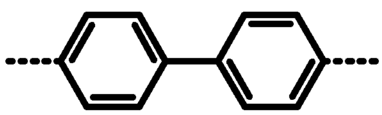

L-4

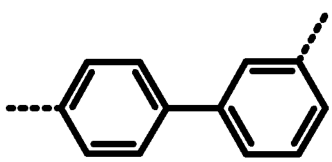

L-5

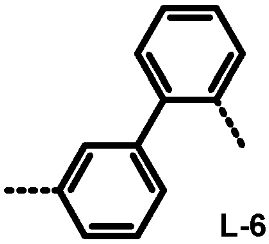 L-6 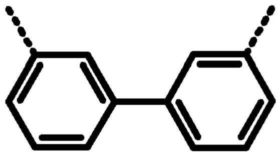 L-7 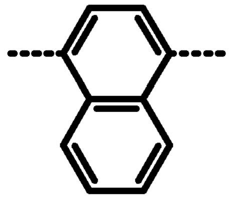 L-8

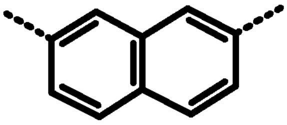 L-9 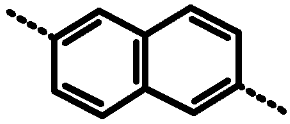 L-10 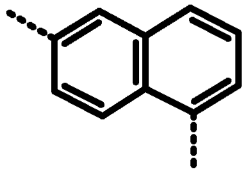 L-11

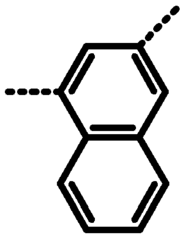 L-12 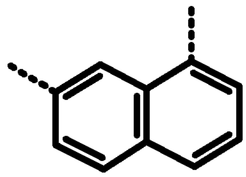 L-13 ,

wobei die Linker L-1 bis L-13 noch mit einem oder mehreren Substituenten R substituiert sein können und die gestrichelte Linie die jeweilige Bindung an den Rest der Formel (1) kennzeichnet;

R ist bei jedem Auftreten gleich oder verschieden ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen;

$Ar_1$, $Ar_2$ sind jeweils unabhängig voneinander bei jedem Auftreten eine Aryl- oder Heteroarylgruppe mit 5 bis 40 aromatischen Ringatomen, die mit einem oder mehreren Resten R substituiert sein können;

A ist bei jedem Auftreten unabhängig voneinander eine Gruppe der Formel (3) oder (4),

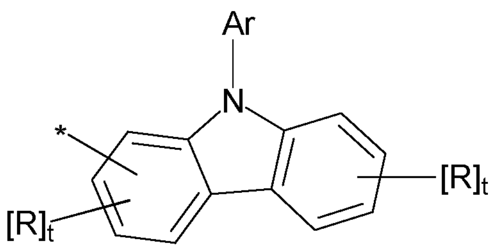

Formel (3) ,

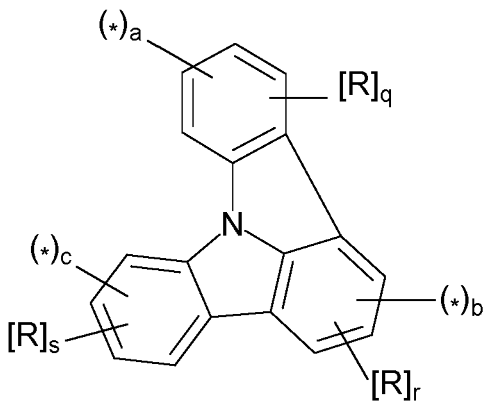

Formel (4) ;

Ar ist jeweils unabhängig voneinander bei jedem Auftreten eine Arylgruppe mit 6 bis 40 aromatischen Ringatomen, die mit einem oder mehreren Resten R substituiert sein kann, oder eine Heterarylgruppe mit 5 bis 40 aromatischen Ringatomen, enthaltend O als Heteroatom, die mit einem oder mehreren Resten R substituiert sein kann;
* kennzeichnet die Bindungsstelle an die Formel (2);
a, b, c bedeuten jeweils unabhängig voneinander bei jedem Auftreten 0 oder 1, wobei die Summe der Indizes bei jedem Auftreten a+b+c 1 bedeutet;
n und m bedeuten unabhängig voneinander bei jedem Auftreten 0, 1, 2 oder 3;
o bedeutet unabhängig bei jedem Auftreten 0, 1, 2, 3, 4, 5, 6 oder 7;
p bedeutet unabhängig bei jedem Auftreten 0, 1, 2, 3, 4, 5, 6, 7 oder 8;
q, r, s, t bedeuten jeweils unabhängig bei jedem Auftreten 0 oder 1;
$R^0$ ist bei jedem Auftreten unabhängig voneinander H oder ein unsubstituiertes oder teilweise oder vollständig deuteriertes aromatisches Ringsystem mit 6 bis 18 C-Atomen.

2. Organische elektrolumineszierende Vorrichtung nach Anspruch 1, **dadurch gekennzeichnet, dass** im Hostmaterial 1 Y O bedeutet.

3. Organische elektrolumineszierende Vorrichtung nach Anspruch 1 oder 2, **dadurch gekennzeichnet, dass** das Hostmaterial 2 einer der Formeln (2a), (2b) oder (2c) entspricht,

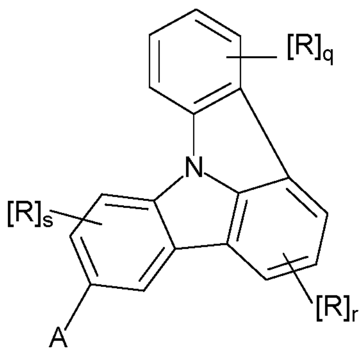

Formel (2a) ,

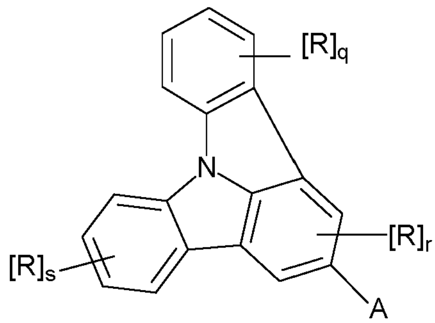

Formel (2b) ,

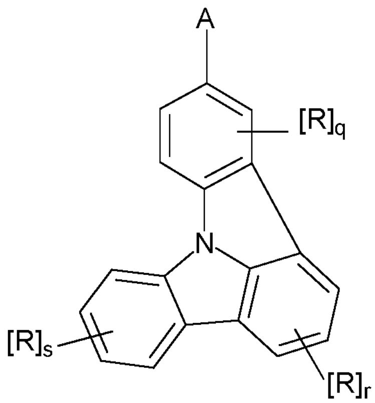

Formel (2c) ,

wobei die verwendeten Symbole und Indizes A, R, q, r und s eine Bedeutung wie in Anspruch 1 haben.

4. Organische elektrolumineszierende Vorrichtung nach einem oder mehreren der Ansprüche 1 bis 3, **dadurch gekennzeichnet, dass** im Hostmaterial 1, L eine einfache Bindung oder die Linker L-1, L-2 oder L-3 bedeutet.

5. Organische elektrolumineszierende Vorrichtung nach einem oder mehreren der Ansprüche 1 bis 4, **dadurch gekennzeichnet, dass** es sich um eine Elektrolumineszenzvorrichtung handelt, die ausgewählt ist aus den organischen

lichtemittierenden Transistoren (OLETs), organischen Feld-Quench-Devices (OFQDs), organischen lichtemittierenden elektrochemischen Zellen (OLECs, LECs, LEECs), organischen Laserdioden (O-Laser) und den organischen lichtemittierenden Dioden (OLEDs).

**6.** Organische elektrolumineszierende Vorrichtung nach einem oder mehreren der Ansprüche 1 bis 5, **dadurch gekennzeichnet, dass** diese neben der Licht-emittierenden Schicht (EML), eine Lochinjektionsschicht (HIL), eine Lochtransportschicht (HTL), eine Elektronentransportschicht (ETL), eine Elektroneninjektionsschicht (EIL) und/oder eine Lochblockierschicht (HBL) enthält.

**7.** Organische elektroluminszierende Vorrichtung nach einem oder mehreren der Ansprüche 1 bis 6, **dadurch gekennzeichnet, dass** die lichtemittierende Schicht neben dem mindestens einen Hostmaterial 1 und dem mindestens einen Hostmaterial 2 mindestens einen phosphoreszierenden Emitter enthält.

**8.** Organische elektrolumineszierende Vorrichtung nach Anspruch 7, **dadurch gekennzeichnet, dass** der phosphoreszierende Emitter der Formel (5) entspricht,

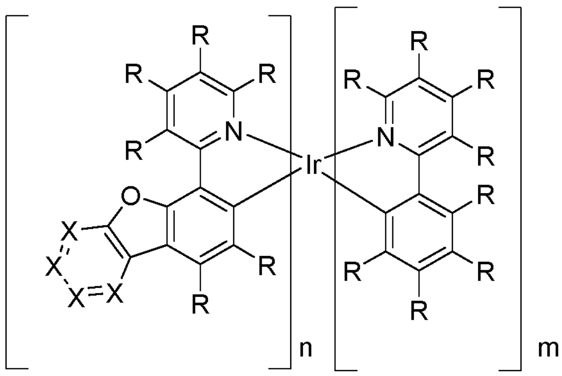

Formel (5)

,

wobei die Symbole und Indizes für diese Formel (5) die Bedeutung haben:

n+m ist 3, n ist 1 oder 2, m ist 2 oder 1,
X ist N oder CR,
R ist H, D oder eine verzweigte oder lineare Alkylgruppe mit 1 bis 10 C-Atomen oder eine teilweise oder vollständig deuterierte verzweigte oder lineare Alkylgruppe mit 1 bis 10 C-Atomen oder eine Cycloalkylgruppe mit 4 bis 7 C-Atomen, die teilweise oder vollständig mit Deuterium substituiert sein kann.

**9.** Verfahren zur Herstellung einer Vorrichtung nach einem oder mehreren der Ansprüche 1 bis 8, **dadurch gekennzeichnet, dass** die lichtemittierende Schicht durch Gasphasenabscheidung oder aus Lösung aufgebracht wird.

**10.** Verfahren nach Anspruch 9, **dadurch gekennzeichnet, dass** die mindestens eine Verbindung der Formel (1) und die mindestens eine Verbindung der Formel (2), nacheinander oder gleichzeitig aus mindestens zwei Materialquellen, gegebenenfalls mit dem mindestens einen phosphoreszierenden Emitter, aus der Gasphase abgeschieden werden und die lichtemittierende Schicht bilden.

**11.** Verfahren nach Anspruch 9, **dadurch gekennzeichnet, dass** die mindestens eine Verbindung der Formel (1) und die mindestens eine Verbindung der Formel (2) als Mischung, nacheinander oder gleichzeitig mit dem mindestens einen phosphoreszierenden Emitter, aus der Gasphase abgeschieden werden und die lichtemittierende Schicht bilden.

**12.** Verfahren nach Anspruch 9, **dadurch gekennzeichnet, dass** die mindestens eine Verbindung der Formel (1) und die mindestens eine Verbindung der Formel (2) zusammen mit dem mindestens einen phosphoreszierenden Emitter, aus einer Lösung aufgebracht werden, um die lichtemittierende Schicht zu bilden.

**13.** Mischung enthaltend mindestens eine Verbindung der Formel (1) und mindestens eine Verbindung der Formel (2),

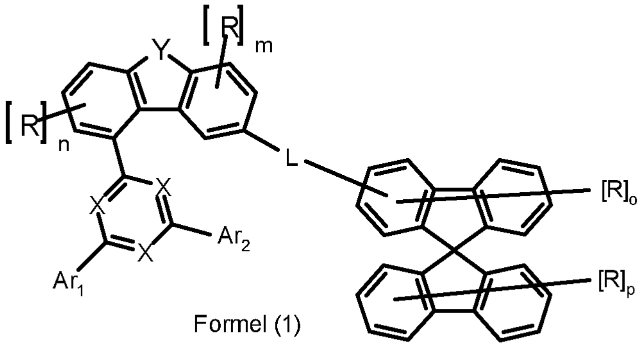

Formel (1)

,

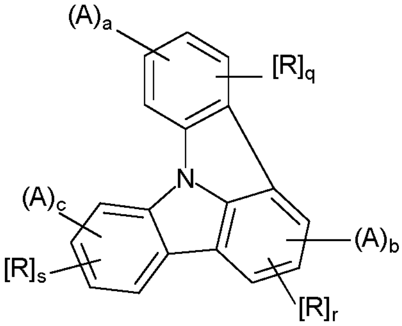

Formel (2)

,

wobei für die verwendeten Symbole und Indizes gilt:

X ist bei jedem Auftreten gleich oder verschieden $CR^0$ oder N, mit der Maßgabe, dass mindestens zwei Gruppen X für N stehen;

Y ist ausgewählt aus O oder S;

L ist bei jedem Auftreten gleich oder verschieden eine Einfachbindung oder ein Linker L-1 bis L-13,

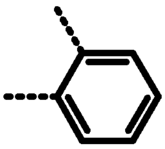

L-1

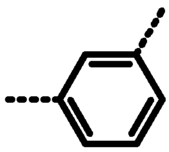

L-2

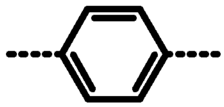

L-3

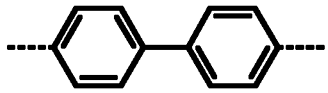

L-4

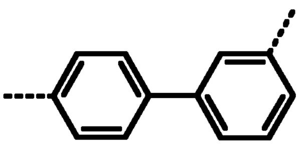

L-5

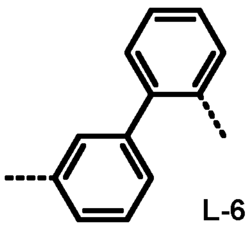

L-6

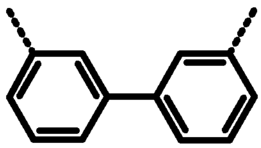

L-7

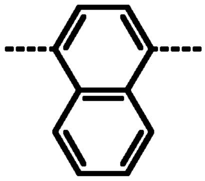 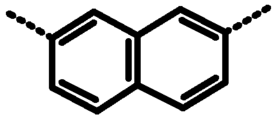 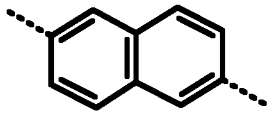

L-8 L-9 L-10

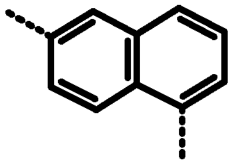 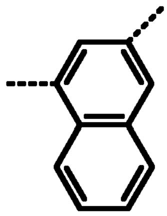 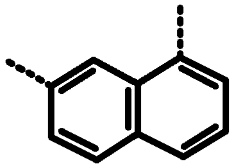

L-11 L-12 L-13 ,

wobei die Linker L-1 bis L-13 noch mit einem oder mehreren Substituenten R substituiert sein können und die gestrichelte Linie die jeweilige Bindung an den Rest der Formel (1) kennzeichnet;

R ist bei jedem Auftreten gleich oder verschieden ausgewählt aus der Gruppe bestehend aus CN, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen, einem aromatischen oder heteroaromatischen Ringsystem mit 5 bis 40 aromatischen Ringatomen, einer Aryloxy- oder Heteroaryloxygruppe mit 5 bis 40 aromatischen Ringatomen, oder einer Aralkyl- oder Heteroaralkylgruppe mit 5 bis 40 aromatischen Ringatomen;

$Ar_1$, $Ar_2$ sind jeweils unabhängig voneinander bei jedem Auftreten eine Aryl- oder Heteroarylgruppe mit 5 bis 40 aromatischen Ringatomen, die mit einem oder mehreren Resten R substituiert sein können;

A ist bei jedem Auftreten unabhängig voneinander eine Gruppe der Formel (3) oder (4),

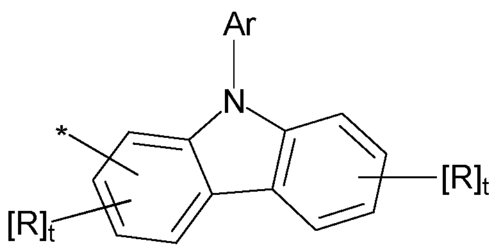

Formel (3) ,

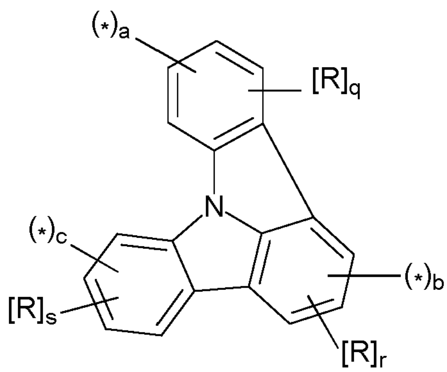

Formel (4) ;

Ar ist jeweils unabhängig voneinander bei jedem Auftreten eine Arylgruppe mit 6 bis 40 aromatischen Ringatomen, die mit einem oder mehreren Resten R substituiert sein kann, oder eine Heterarylgruppe mit 5 bis 40 aromatischen Ringatomen, enthaltend O als Heteroatom, die mit einem oder mehreren Resten R substituiert sein kann;

* kennzeichnet die Bindungsstelle an die Formel (2);

a, b, c bedeuten jeweils unabhängig voneinander bei jedem Auftreten 0 oder 1, wobei die Summe der Indizes bei jedem Auftreten a+b+c 1 bedeutet;

n und m bedeuten unabhängig voneinander bei jedem Auftreten 0, 1, 2 oder 3;

o bedeutet unabhängig bei jedem Auftreten 0, 1, 2, 3, 4, 5, 6 oder 7;

p bedeutet unabhängig bei jedem Auftreten 0, 1, 2, 3, 4, 5, 6, 7 oder 8;

q, r, s, t bedeuten jeweils unabhängig bei jedem Auftreten 0 oder 1;

X und $X^1$ sind jeweils unabhängig voneinander bei jedem Auftreten eine Bindung oder $C(R\#)_2$;
$R^0$ ist bei jedem Auftreten unabhängig voneinander H oder ein unsubstituiertes oder teilweise oder vollständig deuteriertes aromatisches Ringsystem mit 6 bis 18 C-Atomen.

**14.** Mischung nach Anspruch 13, **dadurch gekennzeichnet, dass** die Mischung aus mindestens einer Verbindung der Formel (1), mindestens einer Verbindung der Formel (2) und einem phosphoreszierenden Emitter besteht.

**15.** Formulierung enthaltend eine Mischung nach Anspruch 13 oder 14 sowie mindestens ein Lösemittel.

## Claims

**1.** Organic electroluminescent device comprising an anode, a cathode and at least one organic layer, comprising at least one light-emitting layer, where the at least one light-emitting layer comprises at least one compound of the formula (1) as host material 1 and at least one compound of the formula (2) as host material 2,

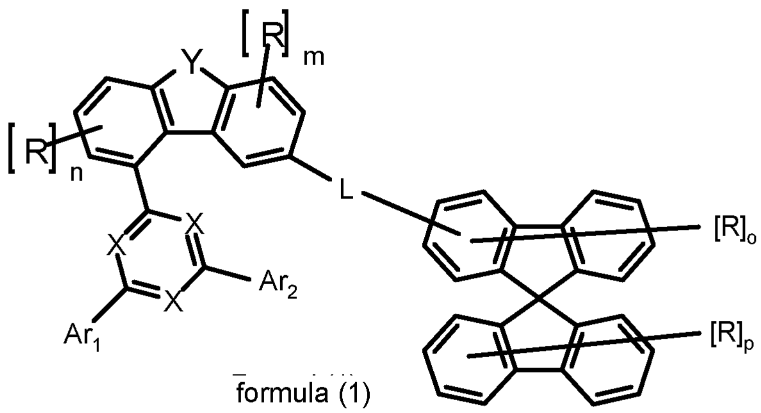

formula (1)

,

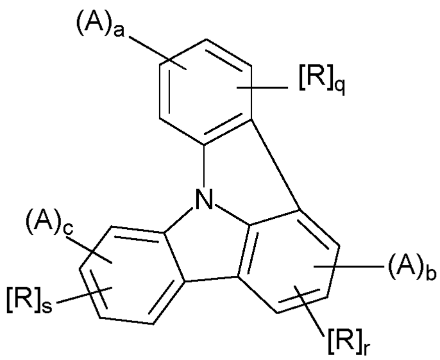

formula (2)

,

where the following applies to the symbols and indices used:

X is on each occurrence, identically or differently, $CR^0$ or N, with the proviso that at least two groups X stand for N;
Y is selected from O or S;
L is on each occurrence, identically or differently, a single bond or a linker L-1 to L-13,

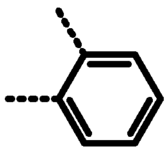 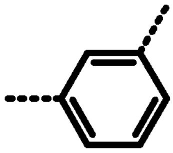 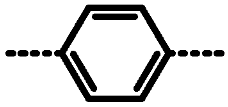

L-1 L-2 L-3

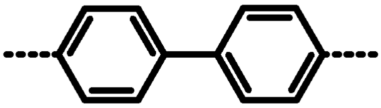 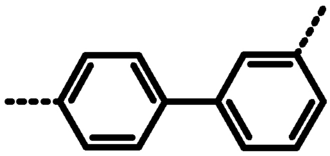

L-4 L-5

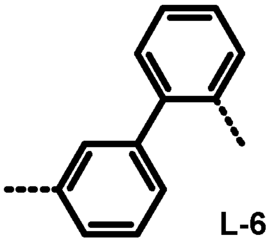 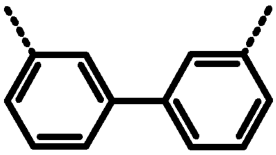 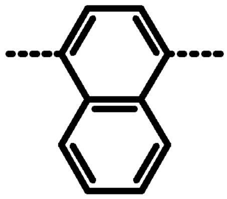

L-6 L-7 L-8

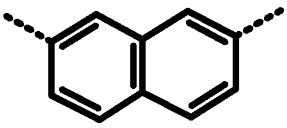 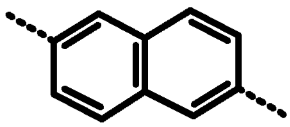 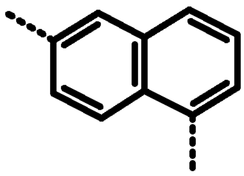

L-9 L-10 L-11

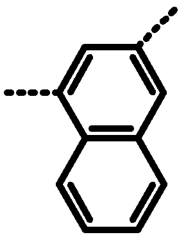 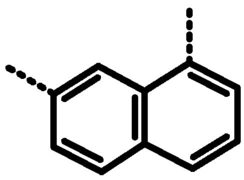

L-12 L-13 ,

where the linkers L-1 to L-13 may also be substituted by one or more substituents R and the dashed line denotes the respective bond to the remainder of the formula (1);

R is selected on each occurrence, identically or differently, from the group consisting of CN, a straight-chain alkyl, alkoxy or thioalkyl group having 1 to 20 C atoms or a branched or cyclic alkyl, alkoxy or thioalkyl group having 3 to 20 C atoms, an aromatic or hetero-aromatic ring system having 5 to 40 aromatic ring atoms, an aryloxy or heteroaryloxy group having 5 to 40 aromatic ring atoms, or an aralkyl or heteroaralkyl group having 5 to 40 aromatic ring atoms;

$Ar_1$, $Ar_2$ are on each occurrence, in each case independently of one another, an aryl or heteroaryl group having 5 to 40 aromatic ring atoms, which may be substituted by one or more radicals R;

A is on each occurrence, independently of one another, a group of the formula (3) or (4),

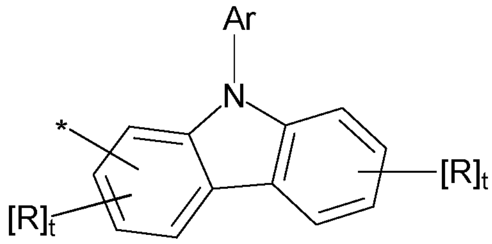

formula (3) ,

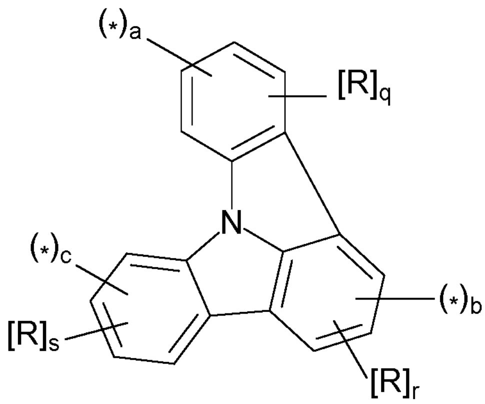

formula (4) ;

Ar is on each occurrence, in each case independently of one another, an aryl group having 6 to 40 aromatic ring atoms, which may be substituted by one or more radicals R, or a heteroaryl group having 5 to 40 aromatic ring atoms, containing O as heteroatom, which may be substituted by one or more radicals R;
* denotes the bonding site to the formula (2);
a, b, c on each occurrence, in each case independently of one another, denote 0 or 1, where the sum of the indices on each occurrence a+b+c denotes 1;
n and m on each occurrence, independently of one another, denote 0, 1, 2 or 3;
o on each occurrence, independently, denotes 0, 1, 2, 3, 4, 5, 6 or 7;
p on each occurrence, independently, denotes 0, 1, 2, 3, 4, 5, 6, 7 or 8;
q, r, s, t on each occurrence, in each case independently, denote 0 or 1;
R° is on each occurrence, independently of one another, H or an unsubstituted or partially or fully deuterated aromatic ring system having 6 to 18 C atoms.

**2.** Organic electroluminescent device according to Claim 1, **characterised in that** in host material 1 Y denotes O.

**3.** Organic electroluminescent device according to Claim 1 or 2, **characterised in that** host material 2 corresponds to one of the formulae (2a), (2b) or (2c),

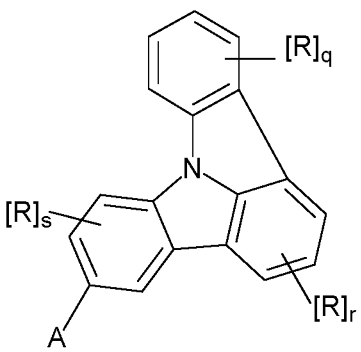

formula (2a) ,

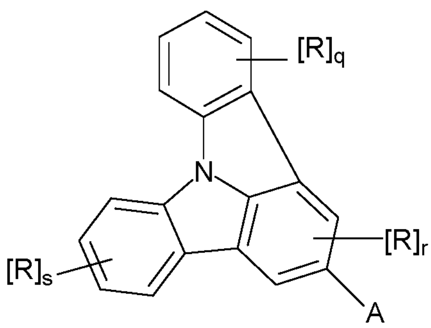

formula (2b) ,

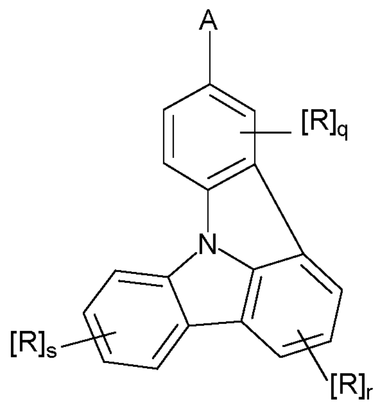

formula (2c) ,

where the symbols and indices A, R, q, r and s used have a meaning as in Claim 1.

**4.** Organic electroluminescent device according to one or more of Claims 1 to 3, **characterised in that** in host material 1 L denotes a single bond or the linkers L-1, L-2 or L-3.

**5.** Organic electroluminescent device according to one or more of Claims 1 to 4, **characterised in that** it is an electroluminescent device selected from organic light-emitting transistors (OLETs), organic field-quench devices (OFQDs), organic light-emitting electrochemical cells (OLECs, LECs, LEECs), organic laser diodes (O-lasers) and organic light-emitting diodes (OLEDs).

**6.** Organic electroluminescent device according to one or more of Claims 1 to 5, **characterised in that**, besides the light-emitting layer (EML), it comprises a hole-injection layer (HIL), a hole-transport layer (HTL), an electron-transport layer (ETL), an electron-injection layer (EIL) and/or a hole-blocking layer (HBL).

**7.** Organic electroluminescent device according to one or more of Claims 1 to 6, **characterised in that**, besides the at least one host material 1 and the at least one host material 2, the light-emitting layer comprises at least one phosphorescent emitter.

**8.** Organic electroluminescent device according to Claim 7, **characterised in that** the phosphorescent emitter corresponds to the formula (5),

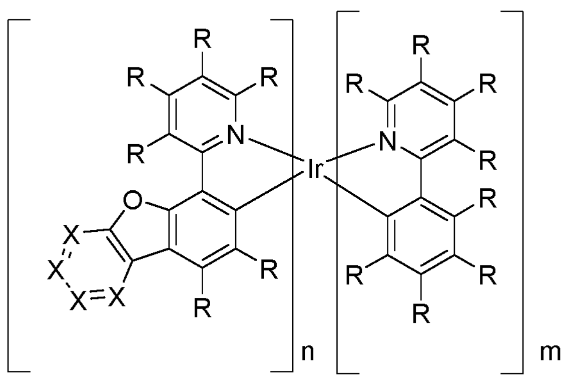

formula (5) ,

where the symbols and indices for this formula (5) have the meaning:

n+m is 3, n is 1 or 2, m is 2 or 1,
X is N or CR,
R is H, D or a branched or linear alkyl group having 1 to 10 C atoms or a partially or fully deuterated branched

or linear alkyl group having 1 to 10 C atoms or a cycloalkyl group having 4 to 7 C atoms, which may be partially or fully substituted by deuterium.

**9.** Process for the production of a device according to one or more of Claims 1 to 8, **characterised in that** the light-emitting layer is applied by gas-phase deposition or from solution.

**10.** Process according to Claim 9, **characterised in that** the at least one compound of the formula (1) and the at least one compound of the formula (2) are deposited successively or simultaneously from the gas phase from at least two material sources, optionally with the at least one phosphorescent emitter, and form the light-emitting layer.

**11.** Process according to Claim 9, **characterised in that** the at least one compound of the formula (1) and the at least one compound of the formula (2) are deposited from the gas phase as a mixture, successively or simultaneously with the at least one phosphorescent emitter, and form the light-emitting layer.

**12.** Process according to Claim 9, **characterised in that** the at least one compound of the formula (1) and the at least one compound of the formula (2) are applied from a solution together with the at least one phosphorescent emitter in order to form the light-emitting layer.

**13.** Mixture comprising at least one compound of the formula (1) and at least one compound of the formula (2),

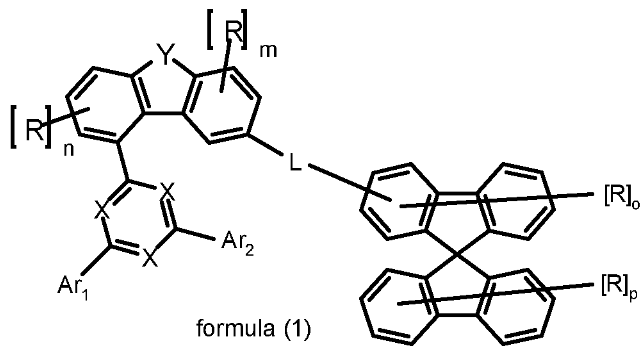

formula (1)

,

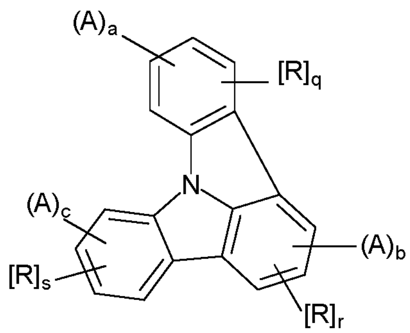

formula (2)

,

where the following applies to the symbols and indices used:

X is on each occurrence, identically or differently, $CR^0$ or N, with the proviso that at least two groups X stand for N;
Y is selected from O or S;
L is on each occurrence, identically or differently, a single bond or a linker L-1 to L-13,

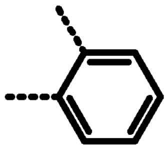 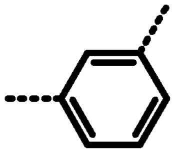 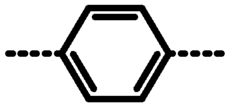

**L-1** **L-2** **L-3**

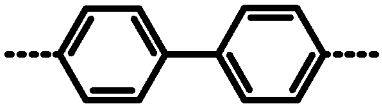 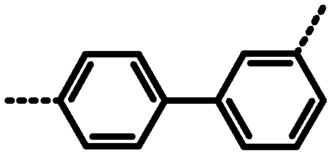

**L-4** **L-5**

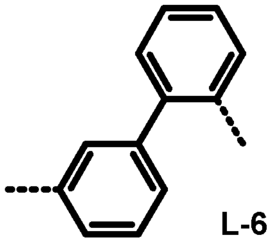 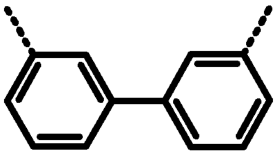 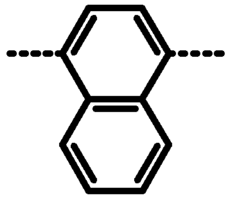

**L-6** **L-7** **L-8**

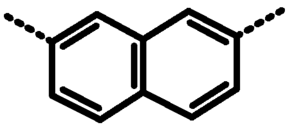 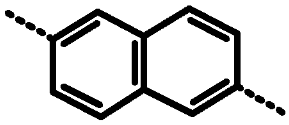 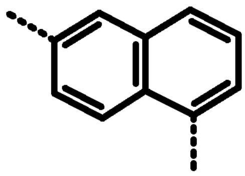

**L-9** **L-10** **L-11**

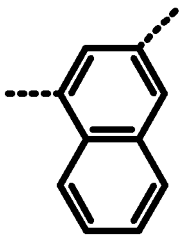 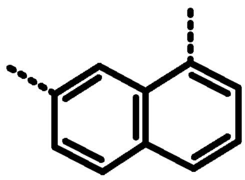

**L-12** **L-13** ,

where the linkers L-1 to L-13 may also be substituted by one or more substituents R and the dashed line denotes the respective bond to the remainder of the formula (1);

R is selected on each occurrence, identically or differently, from the group consisting of CN, a straight-chain alkyl, alkoxy or thioalkyl group having 1 to 20 C atoms or a branched or cyclic alkyl, alkoxy or thioalkyl group having 3 to 20 C atoms, an aromatic or heteroaromatic ring system having 5 to 40 aromatic ring atoms, an aryloxy or heteroaryloxy group having 5 to 40 aromatic ring atoms, or an aralkyl or heteroaralkyl group having 5 to 40 aromatic ring atoms;

$Ar_1$, $Ar_2$ are on each occurrence, in each case independently of one another, an aryl or heteroaryl group having 5 to 40 aromatic ring atoms, which may be substituted by one or more radicals R;

A is on each occurrence, independently of one another, a group of the formula (3) or (4),

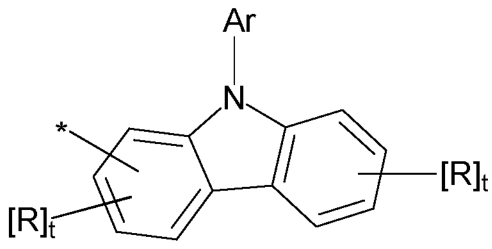

formula (3)

,

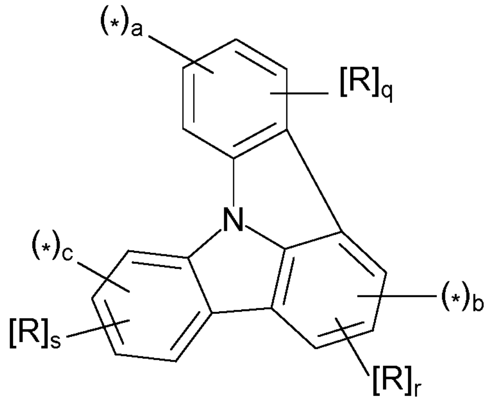

formula (4)

;

Ar is on each occurrence, in each case independently of one another, an aryl group having 6 to 40 aromatic ring atoms, which may be substituted by one or more radicals R, or a heteroaryl group having 5 to 40 aromatic ring atoms, containing O as heteroatom, which may be substituted by one or more radicals R;
* denotes the bonding site to the formula (2);
a, b, c on each occurrence, in each case independently of one another, denote 0 or 1, where the sum of the indices on each occurrence a+b+c denotes 1;
n and m on each occurrence, independently of one another, denote 0, 1, 2 or 3;
o on each occurrence, independently, denotes 0, 1, 2, 3, 4, 5, 6 or 7;
p on each occurrence, independently, denotes 0, 1, 2, 3, 4, 5, 6, 7 or 8;
q, r, s, t on each occurrence, in each case independently, denote 0 or 1;
X and $X^1$ are on each occurrence, in each case independently of one another, a bond or $C(R\#)_2$;
R° is on each occurrence, independently of one another, H or an unsubstituted or partially or fully deuterated aromatic ring system having 6 to 18 C atoms.

**14.** Mixture according to Claim 13, **characterised in that** the mixture consists of at least one compound of the formula (1), at least one compound of the formula (2) and a phosphorescent emitter.

**15.** Formulation comprising a mixture according to Claim 13 or 14 and at least one solvent.

**Revendications**

**1.** Dispositif électroluminescent organique comprenant une anode, une cathode et au moins une couche organique, comprenant au moins une couche émettrice de lumière, où la au moins une couche émettrice de lumière comprend au moins un composé de formule (1) comme matériau hôte 1 et au moins un composé de formule (2) comme matériau hôte 2,

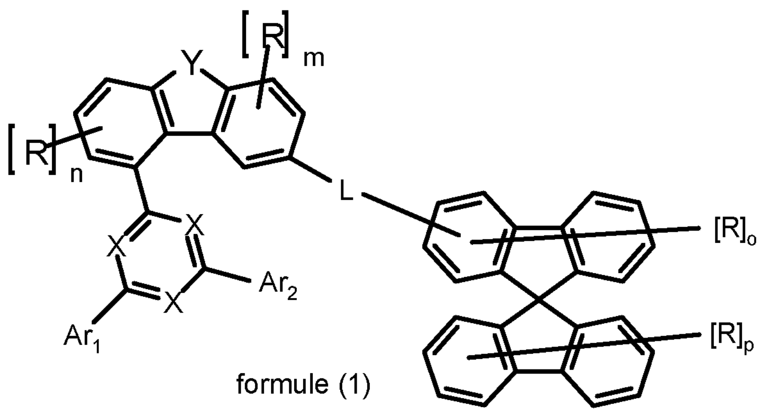

formule (1)

,

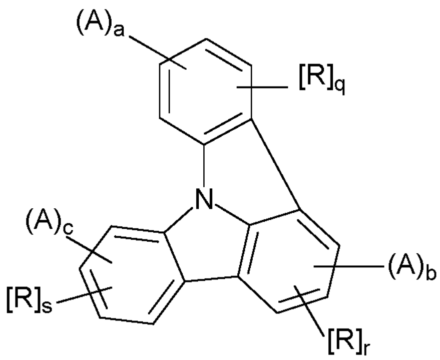

formule (2)

,

dans lesquelles ce qui suit s'applique aux symboles et indices utilisés :

X est à chaque occurrence, de manière identique ou différente, $CR^0$ ou N, à condition qu'au moins deux groupements X représentent N ;
Y est choisi parmi O ou S ;
L est à chaque occurrence, de manière identique ou différente, une liaison simple ou un bras de liaison L-1 à L-13,

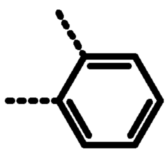

L-1

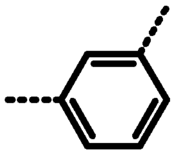

L-2

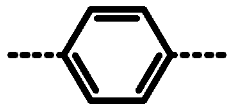

L-3

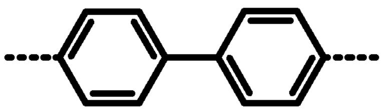

L-4

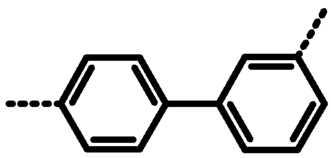

L-5

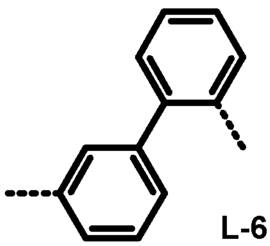

L-6

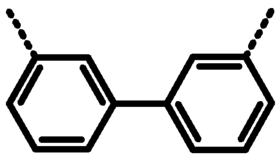

L-7

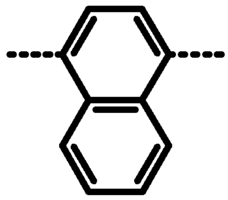

L-8

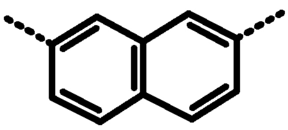

L-9

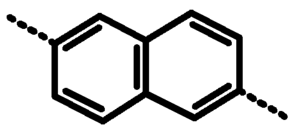

L-10

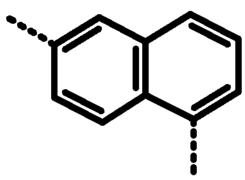

L-11

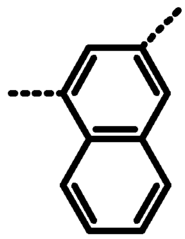 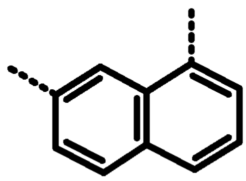

L-12 L-13 ,

où les bras de liaison L-1 à L-13 peuvent également être substitués par un ou plusieurs substituants R et la ligne en pointillés désigne la liaison respective au reste de la formule (1) ;
R est choisi à chaque occurrence, de manière identique ou différente, dans le groupe constitué par CN, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C, un noyau aromatique ou hétéroaromatique ayant de 5 à 40 atomes de cycle aromatique, un groupement aryloxy ou hétéroaryloxy ayant de 5 à 40 atomes de cycle aromatique, ou un groupement aralkyle ou hétéroaralkyle ayant de 5 à 40 atomes de cycle aromatique ;
$Ar_1$, $Ar_2$ sont à chaque occurrence, dans chaque cas indépendamment l'un de l'autre, un groupement aryle ou hétéroaryle ayant de 5 à 40 atomes de cycle aromatique, pouvant être substitué par un ou plusieurs radicaux R ;
A est à chaque occurrence, indépendamment les uns des autres, un groupement de formule (3) ou (4),

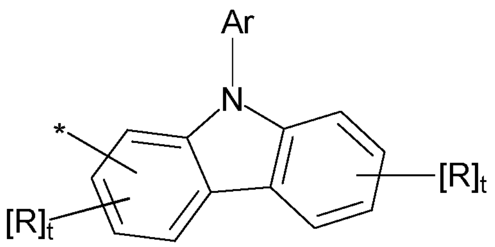

formule (3) ,

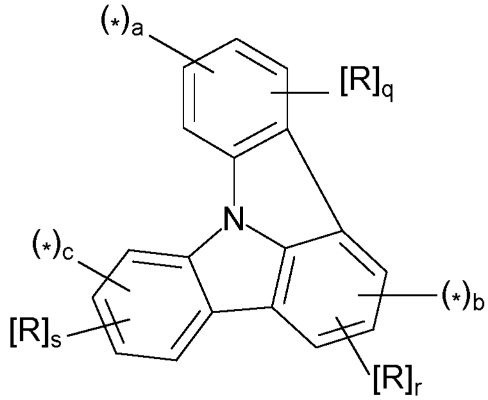

formule (4) ;

Ar est à chaque occurrence, dans chaque cas indépendamment les uns des autres, un groupement aryle ayant de 6 à 40 atomes de cycle aromatique, pouvant être substitué par un ou plusieurs radicaux R, ou un groupement hétéroaryle ayant de 5 à 40 atomes de cycle aromatique, contenant O comme hétéroatome, pouvant être substitué par un ou plusieurs radicaux R ;
* désigne le site de liaison à la formule (2) ;
a, b, c à chaque occurrence, dans chaque cas indépendamment les uns des autres, désignent 0 ou 1, où la somme des indices à chaque occurrence a+b+c désigne 1 ;
n et m à chaque occurrence, indépendamment l'un de l'autre, désignent 0, 1, 2 ou 3 ;
o à chaque occurrence, indépendamment, désigne 0, 1, 2, 3, 4, 5, 6 ou 7 ;
p à chaque occurrence, indépendamment, désigne 0, 1, 2, 3, 4, 5, 6, 7 ou 8 ;
q, r, s, t à chaque occurrence, dans chaque cas indépendamment, désignent 0 ou 1 ;
$R^0$ est à chaque occurrence, indépendamment les uns des autres, H ou un noyau aromatique non substitué ou partiellement ou totalement deutérié ayant de 6 à 18 atomes de C.

2. Dispositif électroluminescent organique selon la revendication 1, **caractérisé en ce que**, dans le matériau hôte 1, Y désigne O.

3. Dispositif électroluminescent organique selon la revendication 1 ou 2, **caractérisé en ce que** le matériau hôte 2 correspond à l'une des formules (2a), (2b) ou (2c),

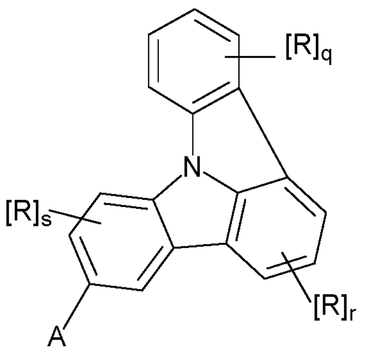

formule (2a) ,

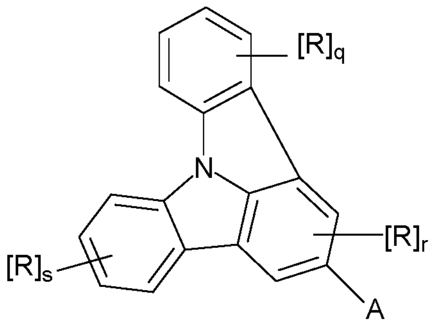

formule (2b) ,

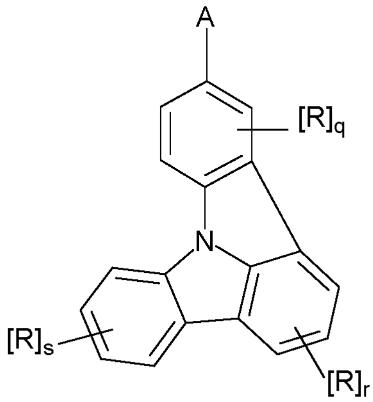

formule (2c) ,

dans lesquelles les symboles et indices A, R, q, r et s utilisés revêtent une signification selon la revendication 1.

4. Dispositif électroluminescent organique selon l'une ou plusieurs parmi les revendications 1 à 3, **caractérisé en ce que**, dans le matériau hôte 1, L désigne une liaison simple ou les bras de liaison L-1, L-2 ou L-3.

5. Dispositif électroluminescent organique selon l'une ou plusieurs parmi les revendications 1 à 4, **caractérisé en ce qu'**il s'agit d'un dispositif électroluminescent choisi parmi les transistors organiques émetteurs de lumière (OLET), les dispositifs organiques à extinction de champ (OFQD), les cellules électrochimiques organiques émettrices de lumière (OLEC, LEC, LEEC), les diodes laser organiques (O-lasers) et les diodes électroluminescentes organiques (OLED).

6. Dispositif électroluminescent organique selon l'une ou plusieurs parmi les revendications 1 à 5, **caractérisé en ce que**, outre la couche émettrice de lumière (EML), il comprend une couche d'injection de trous (HIL), une couche de transport de trous (HTL), une couche de transport d'électrons (ETL), une couche d'injection d'électrons (EIL) et/ou une couche de blocage de trous (HBL).

7. Dispositif électroluminescent organique selon l'une ou plusieurs parmi les revendications 1 à 6, **caractérisé en ce que**, outre le au moins un matériau hôte 1 et le au moins un matériau hôte 2, la couche émettrice de lumière comprend au moins un émetteur phosphorescent.

8. Dispositif électroluminescent organique selon la revendication 7, **caractérisé en ce que** l'émetteur phosphorescent correspond à la formule (5),

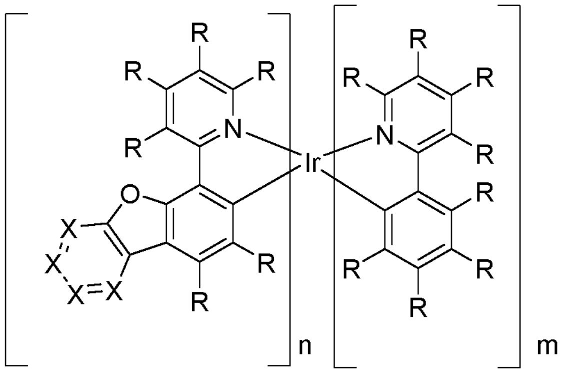

formule (5)

,

dans laquelle les symboles et indices pour cette formule (5) revêtent la signification :

n+m vaut 3, n vaut 1 ou 2, m vaut 2 ou 1,
X est N ou CR,
R est H, D ou un groupement alkyle linéaire ou ramifié ayant de 1 à 10 atomes de C ou un groupement alkyle linéaire ou ramifié partiellement ou totalement deutérié ayant de 1 à 10 atomes de C ou un groupement cycloalkyle ayant de 4 à 7 atomes de C, pouvant être partiellement ou totalement substitué par du deutérium.

9. Procédé de production d'un dispositif selon l'une ou plusieurs parmi les revendications 1 à 8, **caractérisé en ce que** la couche émettrice de lumière est appliquée par dépôt en phase gazeuse ou à partir d'une solution.

10. Procédé selon la revendication 9, **caractérisé en ce que** le au moins un composé de formule (1) et le au moins un composé de formule (2) sont déposés successivement ou simultanément à partir de la phase gazeuse à partir d'au moins deux sources de matériau, éventuellement avec le au moins un émetteur phosphorescent, et forment la couche émettrice de lumière.

11. Procédé selon la revendication 9, **caractérisé en ce que** le au moins un composé de formule (1) et le au moins un composé de formule (2) sont déposés à partir de la phase gazeuse sous forme de mélange, successivement ou simultanément avec le au moins un émetteur phosphorescent, et forment la couche émettrice de lumière.

12. Procédé selon la revendication 9, **caractérisé en ce que** le au moins un composé de formule (1) et le au moins un composé de formule (2) sont appliqués à partir d'une solution conjointement avec le au moins un émetteur phosphorescent afin de former la couche émettrice de lumière.

13. Mélange comprenant au moins un composé de formule (1) et au moins un composé de formule (2),

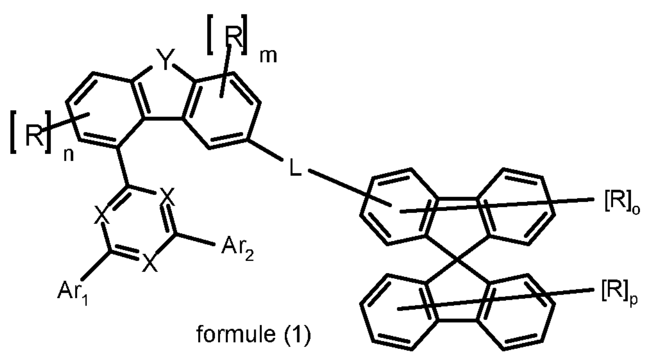

formule (1)

,

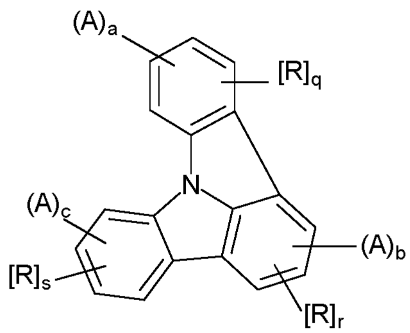

formule (2) ,

dans lesquelles ce qui suit s'applique aux symboles et indices utilisés :

X est à chaque occurrence, de manière identique ou différente, $CR^0$ ou N, à condition qu'au moins deux groupements X représentent N ;
Y est choisi parmi O ou S ;
L est à chaque occurrence, de manière identique ou différente, une liaison simple ou un bras de liaison L-1 à L-13,

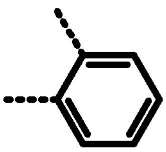

L-1

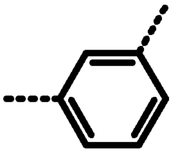

L-2

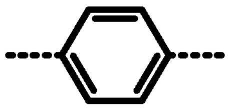

L-3

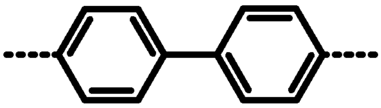

L-4

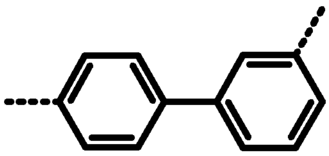

L-5

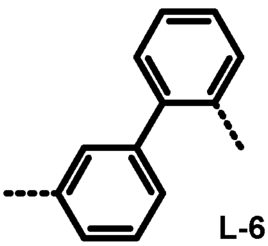

L-6

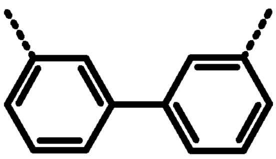

L-7

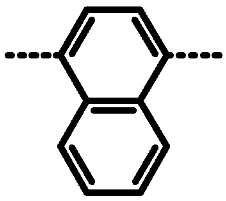

L-8

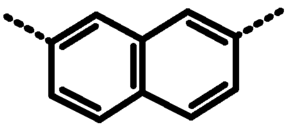

L-9

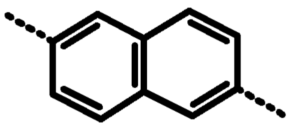

L-10

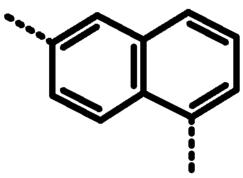

L-11

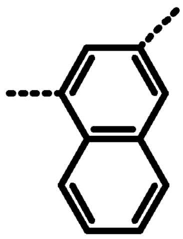
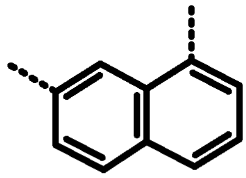

**L-12** **L-13** ,

où les bras de liaison L-1 à L-13 peuvent également être substitués par un ou plusieurs substituants R et la ligne en pointillés désigne la liaison respective au reste de la formule (1) ;
R est choisi à chaque occurrence, de manière identique ou différente, dans le groupe constitué par CN, un groupement alkyle, alcoxy ou thioalkyle à chaîne linéaire ayant de 1 à 20 atomes de C ou un groupement alkyle, alcoxy ou thioalkyle ramifié ou cyclique ayant de 3 à 20 atomes de C, un noyau aromatique ou hétéroaromatique ayant de 5 à 40 atomes de cycle aromatique, un groupement aryloxy ou hétéroaryloxy ayant de 5 à 40 atomes de cycle aromatique, ou un groupement aralkyle ou hétéroaralkyle ayant de 5 à 40 atomes de cycle aromatique ;
$Ar_1$, $Ar_2$ sont à chaque occurrence, dans chaque cas indépendamment l'un de l'autre, un groupement aryle ou hétéroaryle ayant de 5 à 40 atomes de cycle aromatique, pouvant être substitué par un ou plusieurs radicaux R ;
A est à chaque occurrence, indépendamment les uns des autres, un groupement de formule (3) ou (4),

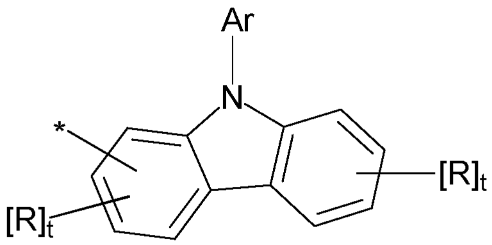

formule (3) ,

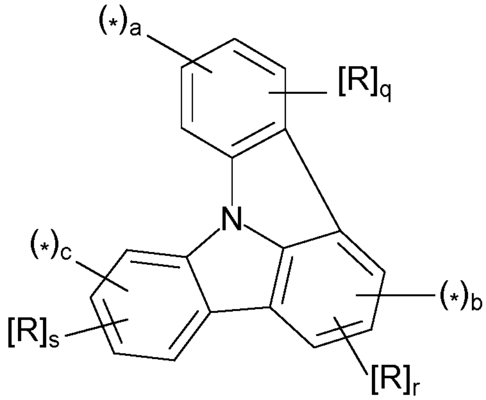

formule (4) ;

Ar est à chaque occurrence, dans chaque cas indépendamment les uns des autres, un groupement aryle ayant de 6 à 40 atomes de cycle aromatique, pouvant être substitué par un ou plusieurs radicaux R, ou un groupement hétéroaryle ayant de 5 à 40 atomes de cycle aromatique, contenant O comme hétéroatome, pouvant être substitué par un ou plusieurs radicaux R ;
* désigne le site de liaison à la formule (2) ;
a, b, c à chaque occurrence, dans chaque cas indépendamment les uns des autres, désignent 0 ou 1, où la somme des indices à chaque occurrence a+b+c désigne 1 ;
n et m à chaque occurrence, indépendamment l'un de l'autre, désignent 0, 1, 2 ou 3 ;
o à chaque occurrence, indépendamment, désigne 0, 1, 2, 3, 4, 5, 6 ou 7 ;
p à chaque occurrence, indépendamment, désigne 0, 1, 2, 3, 4, 5, 6, 7 ou 8 ;
q, r, s, t à chaque occurrence, dans chaque cas indépendamment, désignent 0 ou 1 ;
X et $X^1$ sont à chaque occurrence, dans chaque cas indépendamment l'un de l'autre, une liaison ou $C(R\#)_2$ ;
R° est à chaque occurrence, indépendamment les uns des autres, H ou un noyau aromatique non substitué ou partiellement ou totalement deutérié ayant de 6 à 18 atomes de C.

**14.** Mélange selon la revendication 13, **caractérisé en ce que** le mélange est constitué d'au moins un composé de formule (1), d'au moins un composé de formule (2) et d'un émetteur phosphorescent.

**15.** Formulation comprenant un mélange selon la revendication 13 ou 14 et au moins un solvant.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6392250 B1
- US 6803720 B1
- WO 2011088877 A
- WO 2015169412 A
- KR 20170113318
- US 20180337348 A
- US 2019013490 A
- US 2019047991 A
- WO 19031679 A
- US 2015333273 A
- WO 2016015815 A
- WO 0070655 A
- WO 200141512 A
- WO 200202714 A
- WO 200215645 A
- EP 1191613 A
- EP 1191612 A
- EP 1191614 A
- WO 05033244 A
- WO 05019373 A
- US 20050258742 A
- WO 2009146770 A
- WO 2010015307 A
- WO 2010031485 A
- WO 2010054731 A
- WO 2010054728 A
- WO 2010086089 A
- WO 2010099852 A
- WO 2010102709 A
- WO 2011032626 A
- WO 2011066898 A
- WO 2011157339 A
- WO 2012007086 A
- WO 2014008982 A
- WO 2014023377 A
- WO 2014094961 A
- WO 2014094960 A
- WO 2015036074 A
- WO 2015104045 A
- WO 2015117718 A
- WO 2016124304 A
- WO 2017032439 A
- WO 2006108497 A
- WO 2006122630 A
- WO 2008006449 A
- WO 2007140847 A
- WO 2010012328 A
- US 7294849 B
- WO 2005011013 A
- JP 2000053957 A
- WO 2003060956 A
- WO 2004028217 A
- WO 2004080975 A
- WO 2010072300 A
- WO 06122630 A
- WO 06100896 A
- EP 1661888 A
- WO 01049806 A
- US 5061569 A
- WO 9509147 A
- WO 08006449 A
- WO 07140847 A
- WO 2012034627 A
- EP 12000929
- WO 2014015937 A
- WO 2014015938 A
- WO 2014015935 A
- WO 2013083216 A
- WO 2012150001 A


### In der Beschreibung aufgeführte Nicht-Patentliteratur


- *CHEMICAL ABSTRACTS,* 1269508-30-6
- *CHEMICAL ABSTRACTS,* 1989601-68-4
- *CHEMICAL ABSTRACTS,* 1989602-19-8
- *CHEMICAL ABSTRACTS,* 1989602-70-1
- *CHEMICAL ABSTRACTS,* 1215692-34-4
- *CHEMICAL ABSTRACTS,* 1989601-69-5
- *CHEMICAL ABSTRACTS,* 1989602-20-1
- *CHEMICAL ABSTRACTS,* 1989602-71-2
- *CHEMICAL ABSTRACTS,* 1370364-40-1
- *CHEMICAL ABSTRACTS,* 1989601-70-8
- *CHEMICAL ABSTRACTS,* 1989602-21-2
- *CHEMICAL ABSTRACTS,* 1989602-72-3
- *CHEMICAL ABSTRACTS,* 1370364-42-3
- *CHEMICAL ABSTRACTS,* 1989601-71-9
- *CHEMICAL ABSTRACTS,* 1989602-22-3
- *CHEMICAL ABSTRACTS,* 1989602-73-4
- *CHEMICAL ABSTRACTS,* 1989600-74-9
- *CHEMICAL ABSTRACTS,* 1989601-72-0
- *CHEMICAL ABSTRACTS,* 1989602-23-4
- *CHEMICAL ABSTRACTS,* 1989602-74-5

- *CHEMICAL ABSTRACTS,* 1989600-75-0
- *CHEMICAL ABSTRACTS,* 1989601-73-1
- *CHEMICAL ABSTRACTS,* 1989602-24-5
- *CHEMICAL ABSTRACTS,* 1989602-75-6
- *CHEMICAL ABSTRACTS,* 1989600-77-2
- *CHEMICAL ABSTRACTS,* 1989601-74-2
- *CHEMICAL ABSTRACTS,* 1989602-25-6
- *CHEMICAL ABSTRACTS,* 1989602-76-7
- *CHEMICAL ABSTRACTS,* 1989600-78-3
- *CHEMICAL ABSTRACTS,* 1989601-75-3
- *CHEMICAL ABSTRACTS,* 1989602-26-7
- *CHEMICAL ABSTRACTS,* 1989602-77-8
- *CHEMICAL ABSTRACTS,* 1989600-79-4
- *CHEMICAL ABSTRACTS,* 1989601-76-4
- *CHEMICAL ABSTRACTS,* 1989602-27-8
- *CHEMICAL ABSTRACTS,* 1989602-78-9
- *CHEMICAL ABSTRACTS,* 1989600-82-9
- *CHEMICAL ABSTRACTS,* 1989601-77-5
- *CHEMICAL ABSTRACTS,* 1989602-28-9
- *CHEMICAL ABSTRACTS,* 1989602-79-0
- *CHEMICAL ABSTRACTS,* 1989600-83-0
- *CHEMICAL ABSTRACTS,* 1989601-78-6
- *CHEMICAL ABSTRACTS,* 1989602-29-0
- *CHEMICAL ABSTRACTS,* 1989602-80-3
- *CHEMICAL ABSTRACTS,* 1989600-84-1
- *CHEMICAL ABSTRACTS,* 1989601-79-7
- *CHEMICAL ABSTRACTS,* 1989602-30-3
- *CHEMICAL ABSTRACTS,* 1989602-82-5
- *CHEMICAL ABSTRACTS,* 1989600-85-2
- *CHEMICAL ABSTRACTS,* 1989601-80-0
- *CHEMICAL ABSTRACTS,* 1989602-31-4
- *CHEMICAL ABSTRACTS,* 1989602-84-7
- *CHEMICAL ABSTRACTS,* 1989600-86-3
- *CHEMICAL ABSTRACTS,* 1989601-81-1
- *CHEMICAL ABSTRACTS,* 1989602-32-5
- *CHEMICAL ABSTRACTS,* 1989602-85-8
- *CHEMICAL ABSTRACTS,* 1989600-87-4
- *CHEMICAL ABSTRACTS,* 1989601-82-2
- *CHEMICAL ABSTRACTS,* 1989602-33-6
- *CHEMICAL ABSTRACTS,* 1989602-86-9
- *CHEMICAL ABSTRACTS,* 1989600-88-5
- *CHEMICAL ABSTRACTS,* 1989601-83-3
- *CHEMICAL ABSTRACTS,* 1989602-34-7
- *CHEMICAL ABSTRACTS,* 1989602-87-0
- *CHEMICAL ABSTRACTS,* 1989600-89-6
- *CHEMICAL ABSTRACTS,* 1989601-84-4
- *CHEMICAL ABSTRACTS,* 1989602-35-8
- *CHEMICAL ABSTRACTS,* 1989602-88-1
- *CHEMICAL ABSTRACTS,* 1989601-11-7
- *CHEMICAL ABSTRACTS,* 1989601-85-5
- *CHEMICAL ABSTRACTS,* 1989602-36-9
- *CHEMICAL ABSTRACTS,* 1989604-00-3
- *CHEMICAL ABSTRACTS,* 1989601-23-1
- *CHEMICAL ABSTRACTS,* 1989601-86-6
- *CHEMICAL ABSTRACTS,* 1989602-37-0
- *CHEMICAL ABSTRACTS,* 1989604-01-4
- *CHEMICAL ABSTRACTS,* 1989601-26-4
- *CHEMICAL ABSTRACTS,* 1989601-87-7
- *CHEMICAL ABSTRACTS,* 1989602-38-1
- *CHEMICAL ABSTRACTS,* 1989604-02-5
- *CHEMICAL ABSTRACTS,* 1989601-28-6
- *CHEMICAL ABSTRACTS,* 1989601-88-8
- *CHEMICAL ABSTRACTS,* 1989602-39-2
- *CHEMICAL ABSTRACTS,* 1989604-03-6
- *CHEMICAL ABSTRACTS,* 1989601-29-7
- *CHEMICAL ABSTRACTS,* 1989601-89-9
- *CHEMICAL ABSTRACTS,* 1989602-40-5
- *CHEMICAL ABSTRACTS,* 1989604-04-7
- *CHEMICAL ABSTRACTS,* 1989601-33-3
- *CHEMICAL ABSTRACTS,* 1989601-90-2
- *CHEMICAL ABSTRACTS,* 1989602-41-6
- *CHEMICAL ABSTRACTS,* 1989604-05-8
- *CHEMICAL ABSTRACTS,* 1989601-40-2
- *CHEMICAL ABSTRACTS,* 1989601-91-3
- *CHEMICAL ABSTRACTS,* 1989602-42-7
- *CHEMICAL ABSTRACTS,* 1989604-06-9
- *CHEMICAL ABSTRACTS,* 1989601-41-3
- *CHEMICAL ABSTRACTS,* 1989601-92-4
- *CHEMICAL ABSTRACTS,* 1989602-43-8
- *CHEMICAL ABSTRACTS,* 1989604-07-0
- *CHEMICAL ABSTRACTS,* 1989601-42-4
- *CHEMICAL ABSTRACTS,* 1989601-93-5
- *CHEMICAL ABSTRACTS,* 1989602-44-9
- *CHEMICAL ABSTRACTS,* 1989604-08-1
- *CHEMICAL ABSTRACTS,* 1989601-43-5
- *CHEMICAL ABSTRACTS,* 1989601-94-6
- *CHEMICAL ABSTRACTS,* 1989602-45-0
- *CHEMICAL ABSTRACTS,* 1989604-09-2
- *CHEMICAL ABSTRACTS,* 1989601-44-6
- *CHEMICAL ABSTRACTS,* 1989601-95-7
- *CHEMICAL ABSTRACTS,* 1989602-46-1
- *CHEMICAL ABSTRACTS,* 1989604-10-5
- *CHEMICAL ABSTRACTS,* 1989601-45-7
- *CHEMICAL ABSTRACTS,* 1989601-96-8
- *CHEMICAL ABSTRACTS,* 1989602-47-2
- *CHEMICAL ABSTRACTS,* 1989604-11-6
- *CHEMICAL ABSTRACTS,* 1989601-46-8
- *CHEMICAL ABSTRACTS,* 1989601-97-9
- *CHEMICAL ABSTRACTS,* 1989602-48-3
- *CHEMICAL ABSTRACTS,* 1989604-13-8
- *CHEMICAL ABSTRACTS,* 1989601-47-9
- *CHEMICAL ABSTRACTS,* 1989601-98-0
- *CHEMICAL ABSTRACTS,* 1989602-49-4
- *CHEMICAL ABSTRACTS,* 1989604-14-9
- *CHEMICAL ABSTRACTS,* 1989601-48-0
- *CHEMICAL ABSTRACTS,* 1989601-99-1
- *CHEMICAL ABSTRACTS,* 1989602-50-7
- *CHEMICAL ABSTRACTS,* 1989604-15-0
- *CHEMICAL ABSTRACTS,* 1989601-49-1
- *CHEMICAL ABSTRACTS,* 1989602-00-7
- *CHEMICAL ABSTRACTS,* 1989602-51-8
- *CHEMICAL ABSTRACTS,* 1989604-16-1
- *CHEMICAL ABSTRACTS,* 1989601-50-4
- *CHEMICAL ABSTRACTS,* 1989602-01-8
- *CHEMICAL ABSTRACTS,* 1989602-52-9
- *CHEMICAL ABSTRACTS,* 1989604-17-2

- *CHEMICAL ABSTRACTS,* 1989601-51-5
- *CHEMICAL ABSTRACTS,* 1989602-02-9
- *CHEMICAL ABSTRACTS,* 1989602-53-0
- *CHEMICAL ABSTRACTS,* 1989604-18-3
- *CHEMICAL ABSTRACTS,* 1989601-52-6
- *CHEMICAL ABSTRACTS,* 1989602-03-0
- *CHEMICAL ABSTRACTS,* 1989602-54-1
- *CHEMICAL ABSTRACTS,* 1989604-19-4
- *CHEMICAL ABSTRACTS,* 1989601-53-7
- *CHEMICAL ABSTRACTS,* 1989602-04-1
- *CHEMICAL ABSTRACTS,* 1989602-55-2
- *CHEMICAL ABSTRACTS,* 1989604-20-7
- *CHEMICAL ABSTRACTS,* 1989601-54-8
- *CHEMICAL ABSTRACTS,* 1989602-05-2
- *CHEMICAL ABSTRACTS,* 1989602-56-3
- *CHEMICAL ABSTRACTS,* 1989604-21-8
- *CHEMICAL ABSTRACTS,* 1989601-55-9
- *CHEMICAL ABSTRACTS,* 1989602-06-3
- *CHEMICAL ABSTRACTS,* 1989602-57-4
- *CHEMICAL ABSTRACTS,* 1989604-22-9
- *CHEMICAL ABSTRACTS,* 1989601-56-0
- *CHEMICAL ABSTRACTS,* 1989602-07-4
- *CHEMICAL ABSTRACTS,* 1989602-58-5
- *CHEMICAL ABSTRACTS,* 1989604-23-0
- *CHEMICAL ABSTRACTS,* 1989601-57-1
- *CHEMICAL ABSTRACTS,* 1989602-08-5
- *CHEMICAL ABSTRACTS,* 1989602-59-6
- *CHEMICAL ABSTRACTS,* 1989604-24-1
- *CHEMICAL ABSTRACTS,* 1989601-58-2
- *CHEMICAL ABSTRACTS,* 1989602-09-6
- *CHEMICAL ABSTRACTS,* 1989602-60-9
- *CHEMICAL ABSTRACTS,* 1989604-25-2
- *CHEMICAL ABSTRACTS,* 1989601-59-3
- *CHEMICAL ABSTRACTS,* 1989602-10-9
- *CHEMICAL ABSTRACTS,* 1989602-61-0
- *CHEMICAL ABSTRACTS,* 1989604-26-3
- *CHEMICAL ABSTRACTS,* 1989601-60-6
- *CHEMICAL ABSTRACTS,* 1989602-11-0
- *CHEMICAL ABSTRACTS,* 1989602-62-1
- *CHEMICAL ABSTRACTS,* 1989604-27-4
- *CHEMICAL ABSTRACTS,* 1989601-61-7
- *CHEMICAL ABSTRACTS,* 1989602-12-1
- *CHEMICAL ABSTRACTS,* 1989602-63-2
- *CHEMICAL ABSTRACTS,* 1989604-28-5
- *CHEMICAL ABSTRACTS,* 1989601-62-8
- *CHEMICAL ABSTRACTS,* 1989602-13-2
- *CHEMICAL ABSTRACTS,* 1989602-64-3
- *CHEMICAL ABSTRACTS,* 1989604-29-6
- *CHEMICAL ABSTRACTS,* 1989601-63-9
- *CHEMICAL ABSTRACTS,* 1989602-14-3
- *CHEMICAL ABSTRACTS,* 1989602-65-4
- *CHEMICAL ABSTRACTS,* 1989604-30-9
- *CHEMICAL ABSTRACTS,* 1989601-64-0
- *CHEMICAL ABSTRACTS,* 1989602-15-4
- *CHEMICAL ABSTRACTS,* 1989602-66-5
- *CHEMICAL ABSTRACTS,* 1989604-31-0
- *CHEMICAL ABSTRACTS,* 1989601-65-1
- *CHEMICAL ABSTRACTS,* 1989602-16-5
- *CHEMICAL ABSTRACTS,* 1989602-67-6
- *CHEMICAL ABSTRACTS,* 1989604-32-1
- *CHEMICAL ABSTRACTS,* 1989601-66-2
- *CHEMICAL ABSTRACTS,* 1989602-17-6
- *CHEMICAL ABSTRACTS,* 1989602-68-7
- *CHEMICAL ABSTRACTS,* 1989604-33-2
- *CHEMICAL ABSTRACTS,* 1989601-67-3
- *CHEMICAL ABSTRACTS,* 1989602-18-7
- *CHEMICAL ABSTRACTS,* 1989602-69-8
- *CHEMICAL ABSTRACTS,* 1989604-34-3
- *CHEMICAL ABSTRACTS,* 1989604-35-4
- *CHEMICAL ABSTRACTS,* 1989604-88-7
- *CHEMICAL ABSTRACTS,* 1989605-52-8
- *CHEMICAL ABSTRACTS,* 1989606-07-6
- *CHEMICAL ABSTRACTS,* 1989604-36-5
- *CHEMICAL ABSTRACTS,* 1989604-89-8
- *CHEMICAL ABSTRACTS,* 1989605-53-9
- *CHEMICAL ABSTRACTS,* 1989606-08-7
- *CHEMICAL ABSTRACTS,* 1989604-37-6
- *CHEMICAL ABSTRACTS,* 1989604-90-1
- *CHEMICAL ABSTRACTS,* 1989605-54-0
- *CHEMICAL ABSTRACTS,* 1989606-09-8
- *CHEMICAL ABSTRACTS,* 1989604-38-7
- *CHEMICAL ABSTRACTS,* 1989604-92-3
- *CHEMICAL ABSTRACTS,* 1989605-55-1
- *CHEMICAL ABSTRACTS,* 1989606-10-1
- *CHEMICAL ABSTRACTS,* 1989604-39-8
- *CHEMICAL ABSTRACTS,* 1989604-93-4
- *CHEMICAL ABSTRACTS,* 1989605-56-2
- *CHEMICAL ABSTRACTS,* 1989606-11-2
- *CHEMICAL ABSTRACTS,* 1989604-40-1
- *CHEMICAL ABSTRACTS,* 1989604-94-5
- *CHEMICAL ABSTRACTS,* 1989605-57-3
- *CHEMICAL ABSTRACTS,* 1989606-12-3
- *CHEMICAL ABSTRACTS,* 1989604-41-2
- *CHEMICAL ABSTRACTS,* 1989604-95-6
- *CHEMICAL ABSTRACTS,* 1989605-58-4
- *CHEMICAL ABSTRACTS,* 1989606-13-4
- *CHEMICAL ABSTRACTS,* 1989604-42-3
- *CHEMICAL ABSTRACTS,* 1989604-96-7
- *CHEMICAL ABSTRACTS,* 1989605-59-5
- *CHEMICAL ABSTRACTS,* 1989606-14-5
- *CHEMICAL ABSTRACTS,* 1989604-43-4
- *CHEMICAL ABSTRACTS,* 1989604-97-8
- *CHEMICAL ABSTRACTS,* 1989605-61-9
- *CHEMICAL ABSTRACTS,* 1989606-15-6
- *CHEMICAL ABSTRACTS,* 1989604-45-6
- *CHEMICAL ABSTRACTS,* 1989605-09-5
- *CHEMICAL ABSTRACTS,* 1989605-62-0
- *CHEMICAL ABSTRACTS,* 1989606-16-7
- *CHEMICAL ABSTRACTS,* 1989604-46-7
- *CHEMICAL ABSTRACTS,* 1989605-10-8
- *CHEMICAL ABSTRACTS,* 1989605-63-1
- *CHEMICAL ABSTRACTS,* 1989606-17-8
- *CHEMICAL ABSTRACTS,* 1989604-47-8
- *CHEMICAL ABSTRACTS,* 1989605-11-9
- *CHEMICAL ABSTRACTS,* 1989605-64-2
- *CHEMICAL ABSTRACTS,* 1989606-18-9

- *CHEMICAL ABSTRACTS,* 1989604-48-9
- *CHEMICAL ABSTRACTS,* 1989605-13-1
- *CHEMICAL ABSTRACTS,* 1989605-65-3
- *CHEMICAL ABSTRACTS,* 1989606-19-0
- *CHEMICAL ABSTRACTS,* 1989604-49-0
- *CHEMICAL ABSTRACTS,* 1989605-14-2
- *CHEMICAL ABSTRACTS,* 1989605-66-4
- *CHEMICAL ABSTRACTS,* 1989606-20-3
- *CHEMICAL ABSTRACTS,* 1989604-50-3
- *CHEMICAL ABSTRACTS,* 1989605-15-3
- *CHEMICAL ABSTRACTS,* 1989605-67-5
- *CHEMICAL ABSTRACTS,* 1989606-21-4
- *CHEMICAL ABSTRACTS,* 1989604-52-5
- *CHEMICAL ABSTRACTS,* 1989605-16-4
- *CHEMICAL ABSTRACTS,* 1989605-68-6
- *CHEMICAL ABSTRACTS,* 1989606-22-5
- *CHEMICAL ABSTRACTS,* 1989604-53-6
- *CHEMICAL ABSTRACTS,* 1989605-17-5
- *CHEMICAL ABSTRACTS,* 1989605-69-7
- *CHEMICAL ABSTRACTS,* 1989606-23-6
- *CHEMICAL ABSTRACTS,* 1989604-54-7
- *CHEMICAL ABSTRACTS,* 1989605-18-6
- *CHEMICAL ABSTRACTS,* 1989605-70-0
- *CHEMICAL ABSTRACTS,* 1989606-24-7
- *CHEMICAL ABSTRACTS,* 1989604-55-8
- *CHEMICAL ABSTRACTS,* 1989605-19-7
- *CHEMICAL ABSTRACTS,* 1989605-71-1
- *CHEMICAL ABSTRACTS,* 1989606-26-9
- *CHEMICAL ABSTRACTS,* 1989604-56-9
- *CHEMICAL ABSTRACTS,* 1989605-20-0
- *CHEMICAL ABSTRACTS,* 1989605-72-2
- *CHEMICAL ABSTRACTS,* 1989606-27-0
- *CHEMICAL ABSTRACTS,* 1989604-57-0
- *CHEMICAL ABSTRACTS,* 1989605-21-1
- *CHEMICAL ABSTRACTS,* 1989605-73-3
- *CHEMICAL ABSTRACTS,* 1989606-28-1
- *CHEMICAL ABSTRACTS,* 1989604-58-1
- *CHEMICAL ABSTRACTS,* 1989605-22-2
- *CHEMICAL ABSTRACTS,* 1989605-74-4
- *CHEMICAL ABSTRACTS,* 1989606-29-2
- *CHEMICAL ABSTRACTS,* 1989604-59-2
- *CHEMICAL ABSTRACTS,* 1989605-23-3
- *CHEMICAL ABSTRACTS,* 1989605-75-5
- *CHEMICAL ABSTRACTS,* 1989606-30-5
- *CHEMICAL ABSTRACTS,* 1989604-60-5
- *CHEMICAL ABSTRACTS,* 1989605-24-4
- *CHEMICAL ABSTRACTS,* 1989605-76-6
- *CHEMICAL ABSTRACTS,* 1989606-31-6
- *CHEMICAL ABSTRACTS,* 1989604-61-6
- *CHEMICAL ABSTRACTS,* 1989605-25-5
- *CHEMICAL ABSTRACTS,* 1989605-77-7
- *CHEMICAL ABSTRACTS,* 1989606-32-7
- *CHEMICAL ABSTRACTS,* 1989604-62-7
- *CHEMICAL ABSTRACTS,* 1989605-26-6
- *CHEMICAL ABSTRACTS,* 1989605-78-8
- *CHEMICAL ABSTRACTS,* 1989606-33-8
- *CHEMICAL ABSTRACTS,* 1989604-63-8
- *CHEMICAL ABSTRACTS,* 1989605-27-7
- *CHEMICAL ABSTRACTS,* 1989605-79-9
- *CHEMICAL ABSTRACTS,* 1989606-34-9
- *CHEMICAL ABSTRACTS,* 1989604-64-9
- *CHEMICAL ABSTRACTS,* 1989605-28-8
- *CHEMICAL ABSTRACTS,* 1989605-81-3
- *CHEMICAL ABSTRACTS,* 1989606-35-0
- *CHEMICAL ABSTRACTS,* 1989604-65-0
- *CHEMICAL ABSTRACTS,* 1989605-29-9
- *CHEMICAL ABSTRACTS,* 1989605-82-4
- *CHEMICAL ABSTRACTS,* 1989606-36-1
- *CHEMICAL ABSTRACTS,* 1989604-66-1
- *CHEMICAL ABSTRACTS,* 1989605-30-2
- *CHEMICAL ABSTRACTS,* 1989605-83-5
- *CHEMICAL ABSTRACTS,* 1989606-37-2
- *CHEMICAL ABSTRACTS,* 1989604-67-2
- *CHEMICAL ABSTRACTS,* 1989605-31-3
- *CHEMICAL ABSTRACTS,* 1989605-84-6
- *CHEMICAL ABSTRACTS,* 1989606-38-3
- *CHEMICAL ABSTRACTS,* 1989604-68-3
- *CHEMICAL ABSTRACTS,* 1989605-32-4
- *CHEMICAL ABSTRACTS,* 1989605-85-7
- *CHEMICAL ABSTRACTS,* 1989606-39-4
- *CHEMICAL ABSTRACTS,* 1989604-69-4
- *CHEMICAL ABSTRACTS,* 1989605-33-5
- *CHEMICAL ABSTRACTS,* 1989605-86-8
- *CHEMICAL ABSTRACTS,* 1989606-40-7
- *CHEMICAL ABSTRACTS,* 1989604-70-7
- *CHEMICAL ABSTRACTS,* 1989605-34-6
- *CHEMICAL ABSTRACTS,* 1989605-87-9
- *CHEMICAL ABSTRACTS,* 1989606-41-8
- *CHEMICAL ABSTRACTS,* 1989604-71-8
- *CHEMICAL ABSTRACTS,* 1989605-35-7
- *CHEMICAL ABSTRACTS,* 1989605-88-0
- *CHEMICAL ABSTRACTS,* 1989606-42-9
- *CHEMICAL ABSTRACTS,* 1989604-72-9
- *CHEMICAL ABSTRACTS,* 1989605-36-8
- *CHEMICAL ABSTRACTS,* 1989605-89-1
- *CHEMICAL ABSTRACTS,* 1989606-43-0
- *CHEMICAL ABSTRACTS,* 1989604-73-0
- *CHEMICAL ABSTRACTS,* 1989605-37-9
- *CHEMICAL ABSTRACTS,* 1989605-90-4
- *CHEMICAL ABSTRACTS,* 1989606-44-1
- *CHEMICAL ABSTRACTS,* 1989604-74-1
- *CHEMICAL ABSTRACTS,* 1989605-38-0
- *CHEMICAL ABSTRACTS,* 1989605-91-5
- *CHEMICAL ABSTRACTS,* 1989606-45-2
- *CHEMICAL ABSTRACTS,* 1989604-75-2
- *CHEMICAL ABSTRACTS,* 1989605-39-1
- *CHEMICAL ABSTRACTS,* 1989605-92-6
- *CHEMICAL ABSTRACTS,* 1989606-46-3
- *CHEMICAL ABSTRACTS,* 1989604-76-3
- *CHEMICAL ABSTRACTS,* 1989605-40-4
- *CHEMICAL ABSTRACTS,* 1989605-93-7
- *CHEMICAL ABSTRACTS,* 1989606-48-5
- *CHEMICAL ABSTRACTS,* 1989604-77-4
- *CHEMICAL ABSTRACTS,* 1989605-41-5
- *CHEMICAL ABSTRACTS,* 1989605-94-8
- *CHEMICAL ABSTRACTS,* 1989606-49-6

- *CHEMICAL ABSTRACTS,* 1989604-78-5
- *CHEMICAL ABSTRACTS,* 1989605-42-6
- *CHEMICAL ABSTRACTS,* 1989605-95-9
- *CHEMICAL ABSTRACTS,* 1989606-53-2
- *CHEMICAL ABSTRACTS,* 1989604-79-6
- *CHEMICAL ABSTRACTS,* 1989605-43-7
- *CHEMICAL ABSTRACTS,* 1989605-96-0
- *CHEMICAL ABSTRACTS,* 1989606-55-4
- *CHEMICAL ABSTRACTS,* 1989604-80-9
- *CHEMICAL ABSTRACTS,* 1989605-44-8
- *CHEMICAL ABSTRACTS,* 1989605-97-1
- *CHEMICAL ABSTRACTS,* 1989606-56-5
- *CHEMICAL ABSTRACTS,* 1989604-81-0
- *CHEMICAL ABSTRACTS,* 1989605-45-9
- *CHEMICAL ABSTRACTS,* 1989605-98-2
- *CHEMICAL ABSTRACTS,* 1989606-61-2
- *CHEMICAL ABSTRACTS,* 1989604-82-1
- *CHEMICAL ABSTRACTS,* 1989605-46-0
- *CHEMICAL ABSTRACTS,* 1989605-99-3
- *CHEMICAL ABSTRACTS,* 1989606-62-3
- *CHEMICAL ABSTRACTS,* 1989604-83-2
- *CHEMICAL ABSTRACTS,* 1989605-47-1
- *CHEMICAL ABSTRACTS,* 1989606-00-9
- *CHEMICAL ABSTRACTS,* 1989606-63-4
- *CHEMICAL ABSTRACTS,* 1989604-84-3
- *CHEMICAL ABSTRACTS,* 1989605-48-2
- *CHEMICAL ABSTRACTS,* 1989606-01-0
- *CHEMICAL ABSTRACTS,* 1989606-67-8
- *CHEMICAL ABSTRACTS,* 1989604-85-4
- *CHEMICAL ABSTRACTS,* 1989605-49-3
- *CHEMICAL ABSTRACTS,* 1989606-04-3
- *CHEMICAL ABSTRACTS,* 1989606-69-0
- *CHEMICAL ABSTRACTS,* 1989604-86-5
- *CHEMICAL ABSTRACTS,* 1989605-50-6
- *CHEMICAL ABSTRACTS,* 1989606-05-4
- *CHEMICAL ABSTRACTS,* 1989606-70-3
- *CHEMICAL ABSTRACTS,* 1989604-87-6
- *CHEMICAL ABSTRACTS,* 1989605-51-7
- *CHEMICAL ABSTRACTS,* 1989606-06-5
- *CHEMICAL ABSTRACTS,* 1989606-74-7
- *CHEMICAL ABSTRACTS,* 1989658-39-0
- *CHEMICAL ABSTRACTS,* 2088184-56-7
- *CHEMICAL ABSTRACTS,* 2088185-07-1
- *CHEMICAL ABSTRACTS,* 2088185-66-2
- *CHEMICAL ABSTRACTS,* 1989658-41-4
- *CHEMICAL ABSTRACTS,* 2088184-57-8
- *CHEMICAL ABSTRACTS,* 2088185-08-2
- *CHEMICAL ABSTRACTS,* 2088185-67-3
- *CHEMICAL ABSTRACTS,* 1989658-43-6
- *CHEMICAL ABSTRACTS,* 2088184-58-9
- *CHEMICAL ABSTRACTS,* 2088185-09-3
- *CHEMICAL ABSTRACTS,* 2088185-68-4
- *CHEMICAL ABSTRACTS,* 1989658-47-0
- *CHEMICAL ABSTRACTS,* 2088184-59-0
- *CHEMICAL ABSTRACTS,* 2088185-10-6
- *CHEMICAL ABSTRACTS,* 2088185-69-5
- *CHEMICAL ABSTRACTS,* 1989658-49-2
- *CHEMICAL ABSTRACTS,* 2088184-60-3
- *CHEMICAL ABSTRACTS,* 2088185-11-7
- *CHEMICAL ABSTRACTS,* 2088185-70-8
- *CHEMICAL ABSTRACTS,* 2088184-07-8
- *CHEMICAL ABSTRACTS,* 2088184-61-4
- *CHEMICAL ABSTRACTS,* 2088185-12-8
- *CHEMICAL ABSTRACTS,* 2088185-71-9
- *CHEMICAL ABSTRACTS,* 2088184-08-9
- *CHEMICAL ABSTRACTS,* 2088184-62-5
- *CHEMICAL ABSTRACTS,* 2088185-13-9
- *CHEMICAL ABSTRACTS,* 2088185-72-0
- *CHEMICAL ABSTRACTS,* 2088184-09-0
- *CHEMICAL ABSTRACTS,* 2088184-63-6
- *CHEMICAL ABSTRACTS,* 2088185-14-0
- *CHEMICAL ABSTRACTS,* 2088185-73-1
- *CHEMICAL ABSTRACTS,* 2088184-10-3
- *CHEMICAL ABSTRACTS,* 2088184-64-7
- *CHEMICAL ABSTRACTS,* 2088185-15-1
- *CHEMICAL ABSTRACTS,* 2088185-74-2
- *CHEMICAL ABSTRACTS,* 2088184-11-4
- *CHEMICAL ABSTRACTS,* 2088184-65-8
- *CHEMICAL ABSTRACTS,* 2088185-16-2
- *CHEMICAL ABSTRACTS,* 2088185-75-3
- *CHEMICAL ABSTRACTS,* 2088184-13-6
- *CHEMICAL ABSTRACTS,* 2088184-66-9
- *CHEMICAL ABSTRACTS,* 2088185-17-3
- *CHEMICAL ABSTRACTS,* 2088185-76-4
- *CHEMICAL ABSTRACTS,* 2088184-14-7
- *CHEMICAL ABSTRACTS,* 2088184-67-0
- *CHEMICAL ABSTRACTS,* 2088185-18-4
- *CHEMICAL ABSTRACTS,* 2088185-77-5
- *CHEMICAL ABSTRACTS,* 2088184-15-8
- *CHEMICAL ABSTRACTS,* 2088184-68-1
- *CHEMICAL ABSTRACTS,* 2088185-19-5
- *CHEMICAL ABSTRACTS,* 2088185-78-6
- *CHEMICAL ABSTRACTS,* 2088184-16-9
- *CHEMICAL ABSTRACTS,* 2088184-69-2
- *CHEMICAL ABSTRACTS,* 2088185-20-8
- *CHEMICAL ABSTRACTS,* 2088185-79-7
- *CHEMICAL ABSTRACTS,* 2088184-17-0
- *CHEMICAL ABSTRACTS,* 2088184-70-5
- *CHEMICAL ABSTRACTS,* 2088185-21-9
- *CHEMICAL ABSTRACTS,* 2088185-80-0
- *CHEMICAL ABSTRACTS,* 2088184-18-1
- *CHEMICAL ABSTRACTS,* 2088184-71-6
- *CHEMICAL ABSTRACTS,* 2088185-22-0
- *CHEMICAL ABSTRACTS,* 2088185-81-1
- *CHEMICAL ABSTRACTS,* 2088184-19-2
- *CHEMICAL ABSTRACTS,* 2088184-72-7
- *CHEMICAL ABSTRACTS,* 2088185-23-1
- *CHEMICAL ABSTRACTS,* 2088185-82-2
- *CHEMICAL ABSTRACTS,* 2088184-20-5
- *CHEMICAL ABSTRACTS,* 2088184-73-8
- *CHEMICAL ABSTRACTS,* 2088185-32-2
- *CHEMICAL ABSTRACTS,* 2088185-83-3
- *CHEMICAL ABSTRACTS,* 2088184-21-6
- *CHEMICAL ABSTRACTS,* 2088184-74-9
- *CHEMICAL ABSTRACTS,* 2088185-33-3
- *CHEMICAL ABSTRACTS,* 2088185-84-4

- *CHEMICAL ABSTRACTS,* 2088184-22-7
- *CHEMICAL ABSTRACTS,* 2088184-75-0
- *CHEMICAL ABSTRACTS,* 2088185-34-4
- *CHEMICAL ABSTRACTS,* 2088185-85-5
- *CHEMICAL ABSTRACTS,* 2088184-23-8
- *CHEMICAL ABSTRACTS,* 2088184-76-1
- *CHEMICAL ABSTRACTS,* 2088185-35-5
- *CHEMICAL ABSTRACTS,* 2088185-86-6
- *CHEMICAL ABSTRACTS,* 2088184-24-9
- *CHEMICAL ABSTRACTS,* 2088184-77-2
- *CHEMICAL ABSTRACTS,* 2088185-36-6
- *CHEMICAL ABSTRACTS,* 2088185-87-7
- *CHEMICAL ABSTRACTS,* 2088184-25-0
- *CHEMICAL ABSTRACTS,* 2088184-78-3
- *CHEMICAL ABSTRACTS,* 2088185-37-7
- *CHEMICAL ABSTRACTS,* 2088185-88-8
- *CHEMICAL ABSTRACTS,* 2088184-26-1
- *CHEMICAL ABSTRACTS,* 2088184-79-4
- *CHEMICAL ABSTRACTS,* 2088185-38-8
- *CHEMICAL ABSTRACTS,* 2088185-89-9
- *CHEMICAL ABSTRACTS,* 2088184-27-2
- *CHEMICAL ABSTRACTS,* 2088184-80-7
- *CHEMICAL ABSTRACTS,* 2088185-39-9
- *CHEMICAL ABSTRACTS,* 2088185-90-2
- *CHEMICAL ABSTRACTS,* 2088184-28-3
- *CHEMICAL ABSTRACTS,* 2088184-81-8
- *CHEMICAL ABSTRACTS,* 2088185-40-2
- *CHEMICAL ABSTRACTS,* 2088185-91-3
- *CHEMICAL ABSTRACTS,* 2088184-29-4
- *CHEMICAL ABSTRACTS,* 2088184-82-9
- *CHEMICAL ABSTRACTS,* 2088185-41-3
- *CHEMICAL ABSTRACTS,* 2088185-92-4
- *CHEMICAL ABSTRACTS,* 2088184-30-7
- *CHEMICAL ABSTRACTS,* 2088184-83-0
- *CHEMICAL ABSTRACTS,* 2088185-42-4
- *CHEMICAL ABSTRACTS,* 2088185-93-5
- *CHEMICAL ABSTRACTS,* 2088184-32-9
- *CHEMICAL ABSTRACTS,* 2088184-84-1
- *CHEMICAL ABSTRACTS,* 2088185-43-5
- *CHEMICAL ABSTRACTS,* 2088185-94-6
- *CHEMICAL ABSTRACTS,* 2088184-34-1
- *CHEMICAL ABSTRACTS,* 2088184-85-2
- *CHEMICAL ABSTRACTS,* 2088185-44-6
- *CHEMICAL ABSTRACTS,* 2088185-95-7
- *CHEMICAL ABSTRACTS,* 2088184-35-2
- *CHEMICAL ABSTRACTS,* 2088184-86-3
- *CHEMICAL ABSTRACTS,* 2088185-45-7
- *CHEMICAL ABSTRACTS,* 2088185-96-8
- *CHEMICAL ABSTRACTS,* 2088184-36-3
- *CHEMICAL ABSTRACTS,* 2088184-87-4
- *CHEMICAL ABSTRACTS,* 2088185-46-8
- *CHEMICAL ABSTRACTS,* 2088185-97-9
- *CHEMICAL ABSTRACTS,* 2088184-37-4
- *CHEMICAL ABSTRACTS,* 2088184-88-5
- *CHEMICAL ABSTRACTS,* 2088185-47-9
- *CHEMICAL ABSTRACTS,* 2088185-98-0
- *CHEMICAL ABSTRACTS,* 2088184-38-5
- *CHEMICAL ABSTRACTS,* 2088184-89-6
- *CHEMICAL ABSTRACTS,* 2088185-48-0
- *CHEMICAL ABSTRACTS,* 2088185-99-1
- *CHEMICAL ABSTRACTS,* 2088184-39-6
- *CHEMICAL ABSTRACTS,* 2088184-90-9
- *CHEMICAL ABSTRACTS,* 2088185-49-1
- *CHEMICAL ABSTRACTS,* 2088186-00-7
- *CHEMICAL ABSTRACTS,* 2088184-40-9
- *CHEMICAL ABSTRACTS,* 2088184-91-0
- *CHEMICAL ABSTRACTS,* 2088185-50-4
- *CHEMICAL ABSTRACTS,* 2088186-01-8
- *CHEMICAL ABSTRACTS,* 2088184-41-0
- *CHEMICAL ABSTRACTS,* 2088184-92-1
- *CHEMICAL ABSTRACTS,* 2088185-51-5
- *CHEMICAL ABSTRACTS,* 2088186-02-9
- *CHEMICAL ABSTRACTS,* 2088184-42-1
- *CHEMICAL ABSTRACTS,* 2088184-93-2
- *CHEMICAL ABSTRACTS,* 2088185-52-6
- *CHEMICAL ABSTRACTS,* 2088195-88-2
- *CHEMICAL ABSTRACTS,* 2088184-43-2
- *CHEMICAL ABSTRACTS,* 2088184-94-3
- *CHEMICAL ABSTRACTS,* 2088185-53-7
- *CHEMICAL ABSTRACTS,* 2088195-89-3
- *CHEMICAL ABSTRACTS,* 2088184-44-3
- *CHEMICAL ABSTRACTS,* 2088184-95-4
- *CHEMICAL ABSTRACTS,* 2088185-54-8
- *CHEMICAL ABSTRACTS,* 2088195-90-6
- *CHEMICAL ABSTRACTS,* 2088184-45-4
- *CHEMICAL ABSTRACTS,* 2088184-96-5
- *CHEMICAL ABSTRACTS,* 2088185-55-9
- *CHEMICAL ABSTRACTS,* 2088195-91-7
- *CHEMICAL ABSTRACTS,* 2088184-46-5
- *CHEMICAL ABSTRACTS,* 2088184-97-6
- *CHEMICAL ABSTRACTS,* 2088185-56-0
- *CHEMICAL ABSTRACTS,* 861806-70-4
- *CHEMICAL ABSTRACTS,* 2088184-47-6
- *CHEMICAL ABSTRACTS,* 2088184-98-7
- *CHEMICAL ABSTRACTS,* 2088185-57-1
- *CHEMICAL ABSTRACTS,* 2088184-48-7
- *CHEMICAL ABSTRACTS,* 2088184-99-8
- *CHEMICAL ABSTRACTS,* 2088185-58-2
- *CHEMICAL ABSTRACTS,* 2088184-49-8
- *CHEMICAL ABSTRACTS,* 2088185-00-4
- *CHEMICAL ABSTRACTS,* 2088185-59-3
- *CHEMICAL ABSTRACTS,* 2088184-50-1
- *CHEMICAL ABSTRACTS,* 2088185-01-5
- *CHEMICAL ABSTRACTS,* 2088185-60-6
- *CHEMICAL ABSTRACTS,* 2088184-51-2
- *CHEMICAL ABSTRACTS,* 2088185-02-6
- *CHEMICAL ABSTRACTS,* 2088185-61-7
- *CHEMICAL ABSTRACTS,* 2088184-52-3
- *CHEMICAL ABSTRACTS,* 2088185-03-7
- *CHEMICAL ABSTRACTS,* 2088185-62-8
- *CHEMICAL ABSTRACTS,* 2088184-53-4
- *CHEMICAL ABSTRACTS,* 2088185-04-8
- *CHEMICAL ABSTRACTS,* 2088185-63-9
- *CHEMICAL ABSTRACTS,* 2088184-54-5
- *CHEMICAL ABSTRACTS,* 2088185-05-9
- *CHEMICAL ABSTRACTS,* 2088185-64-0

- *CHEMICAL ABSTRACTS,* 2088184-55-6
- *CHEMICAL ABSTRACTS,* 2088185-06-0
- *CHEMICAL ABSTRACTS,* 2088185-65-1
- **Y. SHIROTA et al.** *Chem. Rev.,* 2007, vol. 107 (4), 953-1010
- **B. M. S. ARNOLD et al.** *Appl. Phys. Lett.,* 2008, vol. 92, 053301